(19) Europäisches Patentamt
European Patent Office
Office européen des brevets

(11) **EP 2 619 540 B1**

(12) **EUROPEAN PATENT SPECIFICATION**

(45) Date of publication and mention
of the grant of the patent:
**21.03.2018 Bulletin 2018/12**

(51) Int Cl.:
***G01N 1/28*** *(2006.01)*    ***G01N 33/50*** *(2006.01)*
***G01N 33/68*** *(2006.01)*

(21) Application number: **11826220.3**

(22) Date of filing: **20.09.2011**

(86) International application number:
**PCT/AU2011/001221**

(87) International publication number:
**WO 2012/037609 (29.03.2012 Gazette 2012/13)**

(54) **SERUM PREPARATION**

SERUMSPRÄPARAT

PRÉPARATION DE SÉRUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2010 AU 2010904233**

(43) Date of publication of application:
**31.07.2013 Bulletin 2013/31**

(73) Proprietor: **Q-Sera Pty Ltd**
**Melbourne, Victoria 3000 (AU)**

(72) Inventors:
• **MASCI, Paul**
**St Lucia, Queensland 4072 (AU)**
• **DE JERSEY, John**
**St Lucia, Queensland 4072 (AU)**
• **LAVIN, Martin**
**St Lucia, Queensland 4072 (AU)**
• **PHILLIPS, Julie**
**Denistone**
**New South Wales 2114 (AU)**
• **DIMESKI, Goce**
**Hamilton, Queensland 4007 (AU)**

(74) Representative: **Maschio, Antonio**
**Maschio & Soames IP Limited**
**30 Carlton Crescent**
**Southampton SO15 2EW (GB)**

(56) References cited:
WO-A1-03/082914    WO-A1-2009/079690
JP-A- H05 157 747    JP-A- S57 142 561
US-A- 5 089 415    US-B1- 6 562 837
US-B2- 7 745 192

• HOFMANN, H. ET AL.: 'Blood Coagulation Induced by the Venom of Bothrops atrox. 1. Identification, Purification, and Properties of a Prothrombin Activator' BIOCHEMISTRY vol. 26, 1987, pages 772 - 780, XP001063236
• SCHONI, R.: 'The Use of Snake Venom-Derived Compounds for New Functional Diagnostic Test Kits in the Field of Haemostasis' PATHOPHYSIOLOGY OFHAEMOSTASIS AND THROMBOSIS vol. 34, 2005, pages 234 - 240, XP055105391
• KINI, R.M.: 'The Intriguing World of Prothrombin Activators from Snake Venom' TOXICON vol. 45, 2005, pages 1133 - 1145, XP027611236

**Description**

This application claims priority to Australian provisional application 2010904233 entitled "Serum Preparation" filed 20 September 2010.

**FIELD OF THE INVENTION**

This invention relates generally to using procoagulants to produce high quality blood serum samples for pathology and other biological assays. The present invention is defined by the appended claims. Any subject-matter identified in the application as "invention", "embodiment", "aspect", etc., which exceeds the scope of the invention as represented by the claims does not form part the claimed invention but only serves as background information to better understand the invention.

**BACKGROUND OF THE INVENTION**

Blood collection devices, including tubes, are, used to collect blood to produce serum or plasma which is in turn used for biochemical or other pathology assays.

Serum is produced by allowing the blood sample to clot and then centrifuging the sample to separate the blood clot including cells from the serum. Plastic tubes (in place of glass) are now typically used and require procoagulants (often micronised silica particles) to enhance the clotting process. Serum is usually preferred over plasma for biological testing unless urgent results are required, in which case the clotting time for a serum tube is considered too long. Even with existing procoagulants, in most commercial tubes the minimum required clotting time recommended by manufacturers is 30 minutes for blood samples from normal patients, and much longer (typically 60 minutes or longer) for samples from patients taking anti-clotting therapeutic agents such as warfarin or heparin. For patient samples from emergency situations (emergency departments, intensive care, operating theatres *etc.*) the time is too long and therefore plasma, which can be produced much faster, is often preferred over serum. An alternative purported to address this issue is a blood collection tube for serum production recently developed by Becton-Dickinson (designated BD Rapid Serum Tube, BDT or BD RST) which contains thrombin designed to increase the rate and extent of blood clotting in blood samples.

Plasma is formed by collecting blood in tubes containing anticoagulants followed by centrifugation which can be performed immediately after collection to separate the cells and thus obtain plasma for analysis. Lithium heparin is the most commonly used anticoagulant in these tubes. Citrate, sodium fluoride/potassium oxalate and EDTA are other anticoagulants that are used in some tubes to produce plasma for estimation of a small number of other analytes.

Incomplete clotting

The coagulation process in preparing a serum sample consumes fibrinogen and entraps platelets and other cells within a network of fibrin. Upon centrifugation the serum is separated from the clot, either by serum separator in the collection device or by aliquoting the serum into a secondary container, to prevent contact with cells. This separation permits the sample to remain stable for extended periods of time. This stability is particularly important if samples are not analysed immediately, or if re-analysis or additional analyses are required.

For some serum samples, coagulation is incomplete after the recommended waiting times. This problem of incomplete clotting is especially prevalent in patients on anti-clotting therapy or specimens collected from anticoagulated taps or cannulae. Contamination of the specimen with anticoagulant agents during collection may also occur. Such blood can take much longer than the manufacturer's recommended waiting time to clot, or in fact may never fully clot in a standard serum tube (e.g. blood from cardiac surgery patients who are fully heparinised). If a serum sample is centrifuged before clotting is complete, clotting can continue in the serum, leading to clots, microclots or formation of fibrin strings capable of causing analyser or analyte specific problems. The formation of microclots and fibrinogen strings during sample preparation may also occur in plasma tubes, especially post-storage at low temperatures. Lack of timely inversion of lithium heparin tubes after blood collection can lead to small clot formation around the rubber stopper. Droplets of blood not heparinised in a timely manner will clot, and clots do not disintegrate upon heparinisation.

Even the smallest clots are capable of producing clinically significant errors. Thus for accuracy, samples must be manually checked by eye or using automated detection systems if available to ensure they are free of fibrin strands or clots. If strands or clots of insoluble material are present, the sample requires sub-sampling into a new container and re-centrifugation prior to test analysis. Samples that exhibit repeated latent clotting may need to be transferred to a lithium heparin tube to stop ongoing clotting. These actions take additional time. Further, fibrin strands or clots are not always detected (e.g. they may even occur post analyser sampling), and consequential sampling errors may lead to patient care decisions being made on inaccurate results.

Cell contamination in plasma tubes

Specimens obtained in plasma tubes, lithium heparin plasma specifically, may be contaminated with cells. Lithium heparin gel tubes when centrifuged will always present a small "buffy coat like layer" on top of the gel at the bottom of the plasma. This layer contains fibrin, cells and cell stroma. The rapid gel movement during centrifugation leaves some cells in the plasma. If the plasma specimen is mixed (e.g. during sub-sampling or handling), it will become turbid due to suspension of cell-containing material and fibrin, which decreases the specimen integrity. In addition, platelet aggregates can form which may also contain fibrin and/or white blood cells. These aggregates can be large enough to be visible to the unaided eye and have been termed "white particulate matter" due to their typical white colour, and present similar problems to incomplete clotting discussed above.

The presence of cells in the sample can affect analyte concentrations. Certain analytes (e.g. glucose) may be decreased by cell activity and others may be increased by leakage or cell lysis (e.g. lactate dehydrogenase, potassium, phosphate).

Analyte interference

Although generally there is no difference in concentration of analytes measured in serum or plasma tubes, there are some exceptions.

Plasma tubes that use heparin are not suitable for heparin analysis or cell-based assays. Lithium heparin plasma tubes are not suitable for lithium analysis. Plasma may be unreliable for additional testing or re-testing, due to presence of cells and insoluble fibrin formation upon prolonged storage at 2-8°C.

Further, there have been reports of some serum or plasma tubes producing inaccurate results of analyte levels, due to interaction with the procoagulant or anticoagulant agents within the tubes, or otherwise (Ciuti *et al.,* 1989; Cowley *et al.,* 1985; Davidson *et al.,* 2006; Dimeski *et al.,* 2004; Dimeski *et al.,* 2005; Dimeski *et al.,* 2010; Hartland *et al.,* 1999; Miles *et al.,* 2004; O'Keane *et al.,* 2006; Wannaslip *et al.,* 2006).

Sample size

It is desirable to reduce the sample size needed for testing, especially in critically ill patients, patients receiving blood transfusions, and infants, in order to reduce the volume of blood taken from a patient. It is therefore optimal to be able to run all necessary tests using a sample taken in a single blood collection tube. To achieve this, testing methods have been developed using very small sample volumes (*e.g.* 2 $\mu$L) so that typically one serum or plasma tube is used for at least 21 tests, but can be used for between 50-60 or even 70-80 tests, depending on the volume of sample needed for each test. However, where there is doubt over the accuracy of measuring a particular analyte in a serum or plasma tube, it may be necessary to take both a serum tube and a plasma tube from the patient and doing so defeats the goal of reducing the volume of blood taken from the patient.

Problems arising from the use of current methodologies for serum and plasma preparation from blood show that improvements are required to achieve timely, reliable analytical results from a wider variety of blood samples generally.

Snake venom prothrombin activators

Many snake venoms contain prothrombin activators for the purpose of rapid clotting of the blood of their prey. These prothrombin activators are proteolytic enzymes which convert prothrombin present in blood to thrombin which in turn causes clotting.

While snake venom prothrombin activators are known procoagulants, they are also known to possess proteolytic trypsin-like activity (Schieck *et al.,* 1972; Parker, H. W. and Grandison A. G. C., 1977; Masci, P. P., 1986; Nicholson *et al.,* 2006; Lavin and Masci, 2009). It has been postulated that there may be an evolutionary reason that prothrombin activators possess both procoagulant and proteolytic properties in that they act to both kill and digest the prey (Masci, P. P., 1986, page 143). For example, ecarin (prothrombin activator purified from *Echis carinatus* venom) has been shown to have procoagulant activity and as well several other proteolytic activities such as fibrinogenolysis, gelatinolysis, caesionlysis and haemorrhage (Schieck *et al.,* 1972), and a prothrombin activator purified from the venom of *Pseudonaja textilis* (PtPA) is active against a range of chromogenic peptide substrates designed for different proteolytic enzymes (Masci, P.P., 1986).

Many analyte tests that may be performed on blood, serum, or plasma samples involve proteins, including tests measuring proteins as analytes (e.g. total protein, albumin); tests measuring enzyme activity of blood proteins (*e.g.* gamma-glutamyl transpeptidase used in test for gamma-glutamyl transferase, aspartate aminotransferase, lactate dehydrogenase, creatine kinase, lipase); tests using proteins as reagents (*e.g.* immunoassays); tests using enzymes in the analytical method (*e.g.* glucose oxidase). Other commonly used tests involving protein include assays for glucose, urea, urate, alanine aminotransferase, creatine kinase, high-density lipoprotein cholesterol, cholesterol, triglycerides, transferrin, C reactive protein, troponin, cortisol, free thyroxine, free triiodothyronine, thyroid stimulating hormone, and ferritin.

Therefore, despite their procoagulant properties, these snake venom prothrombin activators have never been considered suitable for use in serum tubes for analyte tests, on the basis that their proteolytic activity would degrade analytes being measured (e.g. where the analyte is a protein), or would degrade proteins being used in the reaction to measure analyte levels (*e.g.* where the analyte test involves use of a protein such as glucose oxidase).

Thrombin tubes

While thrombin-containing tubes have recently become available as 'faster' clotting tubes, and thrombin possesses both procoagulant and proteolytic activity, thrombin is known to have high specificity for cutting bonds in fibrinogen, activated protein C. (APC) and Factor Va. Therefore, unlike the reported trypsin-like activity of the snake venom prothrombin activators, thrombin would not be expected to interfere with analyte tests.

In work leading up to the present invention, it was found that thrombin-containing tubes cannot be used with all blood samples. Thrombin is known to be rapidly and completely inhibited by the heparin-antithrombin III complex present in heparinised blood samples. In investigating the BD RST tubes, it was found that these tubes are ineffective in clotting patient samples containing high doses of heparin (Dimeski *et al.,* 2010).

Development of the invention

Surprisingly, the present inventors discovered that when used in blood collection devices, including tubes, prothrombin activators are generally capable of producing high quality serum in an acceptable time from a wide variety of blood samples (including those taken from patients on high concentration of anti-clotting therapy, including heparin), decreasing both the serum sample preparation time and the risk of analysis problems due to incomplete clotting and contamination by cells and cell components.

Moreover, the inventors also surprisingly discovered that serum samples obtained from blood samples by addition of prothrombin activators give the same results in a wide range of standard biochemistry analytical tests as serum samples produced in existing blood collection tubes.

These discoveries suggested that prothrombin activators would be suitable for producing serum for the purpose of measuring a wide range of analytes, and have been reduced to practice in blood collection containers for preparing serum samples useful in detecting analytes, related uses and methods, as described hereafter.

## SUMMARY OF THE INVENTION

Accordingly, in one aspect the present invention provides the use of a clotting composition comprising, consisting essentially of, or consisting of a prothrombin activator in the preparation of a serum sample that is suitable for detecting an analyte.

The prothrombin activator (sometimes known as prothrombinase) suitably exhibits trypsin-like activity and activates prothrombin (*i.e.* converts prothrombin to thrombin).

The present invention also provides a container for preparing a serum sample that is suitable for detecting an analyte of interest that is present in the sample, wherein the container contains a clotting composition comprising, consisting essentially of, or consisting of a prothrombin activator as defined herein.

In another aspect, the present invention provides the use of a clotting composition comprising, consisting essentially of, or consisting of a prothrombin activator as defined herein in the preparation or manufacture of a container for preparing a serum sample suitable for detecting an analyte. In another aspect, the present invention provides a container comprising a clotting composition comprising, consisting essentially of, or consisting of a prothrombin activator as defined herein and a blood sample, for preparing a serum sample suitable for detecting an analyte.

In another aspect the present invention provides a method of preparing a serum sample for detecting an analyte of interest, the method comprising contacting a blood sample with a clotting composition comprising, consisting essentially of, or consisting of a prothrombin activator as defined herein for a time and under conditions sufficient to prepare a serum sample. Suitably, the method is carried out in a container as broadly defined above. Suitably, the blood is contacted with the clotting composition for a time and under conditions sufficient to prepare a serum sample and clotted cells. Suitably, the method further comprises separating the serum sample from the clotted cells. In some embodiments the method comprises mixing the clotting composition and blood sample by providing a container containing the blood sample and adding the clotting composition to the container, or providing a container containing the clotting composition and adding or collecting the blood sample into the container.

The present invention also provides a serum sample produced by contacting a blood sample with a clotting composition as broadly described above for a time and under conditions sufficient to produce the serum sample.

The present invention further provides methods of detecting an analyte of interest. These methods generally comprise analysing a serum sample prepared by the method of the present invention for the presence or amount of the analyte of interest.

The present invention also provides methods of diagnosing the presence, absence or severity of a disease or condition in a subject, wherein the presence, absence or severity of the disease or condition is associated with the presence, absence or an aberrant amount of an analyte of interest in the subject. These methods generally comprise providing a serum sample prepared according to the methods broadly described above; and detecting the presence, absence or aberrant amount of the analyte in the serum sample to thereby determine the presence, absence or severity of the disease or condition in the subject.

## BRIEF DESCRIPTION OF THE SEQUENCES

A brief description of the sequences in the sequence listing is provided below.

| SEQUENCE ID NUMBER | SEQUENCE |
| --- | --- |
| SEQ ID NO: 1 | Polypeptide sequence for ecarin from *Echis carinatus* |
| SEQ ID NO: 2 | Partial polypeptide sequence for basparin from *Bothrops asper* venom |
| SEQ ID NO: 3 | Partial polypeptide sequence for carinactivase-1 from *Echis carinatus* venom (prepared as described in Yamada, *D.*, *et al.,* (1996)) - 62 kDa subunit |
| SEQ ID NO: 4 | Partial polypeptide sequence for multactivase from *Echis multisquamatus* venom (prepared as described in Yamada, *D.*, *et al.,* (1997)) |
| SEQ ID NO: 5 | Nucleotide sequence encoding Factor V-like component of PtPA (or pseutarin C) from *Pseudonaja textilis* |
| SEQ ID NO: 6 | Nucleotide sequence encoding Factor V-like component of PtPA (or pseutarin C) from *Pseudonaja textilis* |
| SEQ ID NO: 7 | Polypeptide sequence for Factor V-like component of PtPA (or pseutarin C) from *Pseudonaja textilis* |
| SEQ ID NO: 8 | Polypeptide sequence for Factor V-like component of PtPA (or pseutarin C) from *Pseudonaja textilis* |
| SEQ ID NO: 9 | Nucleotide sequence encoding Factor V-like component of OsPA (or oscutarin C) from *Oxyuranus scutellatus* |
| SEQ ID NO: 10 | Nucleotide sequence encoding Factor V-like component of OsPA (or oscutarin C) from *Oxyuranus scutellatus scutellatus* |
| SEQ ID NO: 11 | Polypeptide sequence for Factor V-like component of OsPA (or oscutarin C) from *Oxyuranus cutellatus scutellatus* |
| SEQ ID NO: 12 | Polypeptide sequence for Factor V-like component of OsPA (or oscutarin C) from *Oxyuranus scutellatus* |
| SEQ ID NO: 13 | Polypeptide sequence for Factor V-like component of OsPA (or oscutarin C) from *Oxyuranus scutellatus* |
| SEQ ID NO: 14 | Nucleotide sequence encoding Factor V-like component of omicarin C from *Oxyuranus microlepidotus* |
| SEQ ID NO: 15 | Nucleotide sequence encoding factor V from *Homo sapiens* |
| SEQ ID NO: 16 | Polypeptide sequence for factor V from *Homo sapiens* |
| SEQ ID NO: 17 | Nucleotide sequence encoding factor V from *Bos taurus* |
| SEQ ID NO: 18 | Polypeptide sequence for factor V from *Bos taurus* |
| SEQ ID NO: 19 | Nucleotide sequence encoding Factor X-like component of PtPA (or pseutarin C) from *Pseudonaja textilis* |

(continued)

| SEQUENCE ID NUMBER | SEQUENCE |
|---|---|
| SEQ ID NO: 20 | Nucleotide sequence encoding Factor X-like component of PtPA (or pseutarin C) from *Pseudonaja textilis* |
| SEQ ID NO: 21 | Nucleotide sequence encoding Factor X-like component of PtPA (or pseutarin C) from *Pseudonaja textilis* |
| SEQ ID NO: 22 | Nucleotide sequence encoding Factor X-like component of PtPA (or pseutarin C) from *Pseudonaja textilis textilis* |
| SEQ ID NO: 23 | Nucleotide sequence encoding Factor X-like component of PtPA (or pseutarin C) from *Pseudonaja textilis textilis* |
| SEQ ID NO: 24 | Nucleotide sequence encoding Factor X-like component of PtPA (or pseutarin C) from *Pseudonaja textilis* |
| SEQ ID NO: 25 | Nucleotide sequence encoding Factor X-like component of PtPA (or pseutarin C) from *Pseudonaja textilis* |
| SEQ ID NO: 26 | Polypeptide sequence for Factor X-like component of PtPA (or pseutarin C) from *Pseudonaja textilis* |
| SEQ ID NO: 27 | Polypeptide sequence for Factor X-like component of PtPA (or pseutarin C) from *Pseudonaja textilis* |
| SEQ ID NO: 28 | Polypeptide sequence for Factor X-like component of PtPA (or pseutarin C) from *Pseudonaja textilis textilis* |
| SEQ ID NO: 29 | Polypeptide sequence for Factor X-like component of PtPA (or pseutarin C) from *Pseudonaja textilis textilis* |
| SEQ ID NO: 30 | Polypeptide sequence for Factor X-like component of PtPA (or pseutarin C) from *Pseudonaja textilis* |
| SEQ ID NO: 31 | Nucleotide sequence encoding Factor X-like component of OsPA (or oscutarin C) from *Oxyuranus scutellatus* |
| SEQ ID NO: 32 | Polypeptide sequence for Factor X-like component of OsPA (or oscutarin C) from *Oxyuranus scutellatus* |
| SEQ ID NO: 33 | Nucleotide sequence encoding Factor X-like component of omicarin C from *Oxyuranus microlepidotus* |
| SEQ ID NO: 34 | Polypeptide sequence for Factor X-like component of omicarin C from *Oxyuranus microlepidolus* |
| SEQ ID NO: 35 | Nucleotide sequence encoding Factor X-like components of porpharin D from *Pseudechis porphyriacus* |
| SEQ ID NO: 36 | Polypeptide sequence for Factor X-like component of porpharin D from *Pseudechis porphyriacus* |
| SEQ ID NO: 37 | Nucleotide sequence encoding Factor X-like component of hopsarin D from *Hoplocephalus stephensii* |
| SEQ ID NO: 38 | Polypeptide sequence for Factor X-like component of hopsarin D from *Hoplocephalus stephensii* |
| SEQ ID NO: 39 | Nucleotide sequence encoding Factor X-like component of notecarin D from *Notechis scutatus* |
| SEQ ID NO: 40 | Polypeptide sequence for Factor X-like component of notecarin D from *Notechis scutatus* |
| SEQ ID NO: 41 | Nucleotide sequence encoding Factor X-like component of trocarin D from *Tropidechis carinatus* |

(continued)

| SEQUENCE ID NUMBER | SEQUENCE |
|---|---|
| SEQ ID NO: 42 | Polypeptide sequence for Factor X-like component of trocarin D from *Tropidechis carinatus* |
| SEQ ID NO: 43 | Nucleotide sequence encoding Factor X-like component of prothrombin activator from *Demansia vestigiata* |
| SEQ ID NO: 44 | Polypeptide sequence for Factor X-like component of prothrombin activator from *Demansia vestigiata* |
| SEQ ID NO: 45 | Nucleotide sequence encoding Factor X-like component of prothrombin activator from *Demansia vestigiata* |
| SEQ ID NO: 46 | Polypeptide sequence for Factor X-like component of prothrombin activator from *Demansia vestigiata* |
| SEQ ID NO: 47 | Nucleotide sequence encoding factor X from *Homo sapiens* |
| SEQ ID NO: 48 | Polypeptide sequence for factor X from *Homo sapiens* |
| SEQ ID NO: 49 | Nucleotide sequence encoding factor X from *Bos taurus* |
| SEQ ID NO: 50 | Polypeptide sequence for factor X from *Bos taurus* |
| SEQ ID NO: 51 | Partial polypeptide sequence for carinactivase-1 from *Echis carinatus* venom (prepared as described in Yamada, D., *et al.,* (1996)) - 17 kDa subunit |
| SEQ ID NO: 52 | Partial polypeptide sequence for carinactivase-1 from *Echis carinatus* venom (prepared as described in Yamada, D., *et al.,* (1996)) - 14 kDa subunit |

**BRIEF DESCRIPTION OF THE DRAWINGS**

Figure 1 shows the elution profile in the isolation of ecarin, carinactivase-1, and carinactivase-2 from *E. carinatus* venom using gel filtration on a Superdex 200 column as described in Example 1a. The *E. carinatus* venom (157 mg, 122 $A_{280}$ units) was subjected to gel filtration on Superdex 200 (2.5 x 95 cm) using of 0.05 M Tris-HCl buffer at pH 8.0. The pooled fractions (28.3 $A_{280}$ units) of the three prothrombin activators (ecarin, carinactivase-1, and carinactivase-2) are indicated by the bar.

Figure 2 shows the elution profile of the Blue Sepharose chromatography of the active (procoagulant) fractions from the Superdex 200 gel filtration chromatography described in Example 1a (also shown in Figure 1). The active fractions were subjected to a column of Blue Sepharose and eluted with a linear gradient of NaCl. Three mL fractions were collected. The fractions indicated by the bars were pooled as carinactivase-1, carinactivase-2, and ecarin, respectively.

Figure 3 shows the elution profile for chromatography of reconstituted *P. textilis* venom (50 mg in 5 mL) on a column (2.5 x 95 cm) of Sephacryl S-300 in 0.05 Tris-HCl buffer, pH 7.4; 4°C; flow rate, 17 mL/h; $A_{280}$ (•); S-2222 specific activity (°); 'A' and 'B' represent the void volume (167 mL) and the elution volume of the peak of S-2222 activity (250 mL) respectively, as described in Example 1b.

Figure 4 shows the elution profile of *P. textilis* venom (1 g : 30 mL) using the Con A-Sepharose affinity chromatography method described in Example 1b. The arrow denotes the position of the application of 0.2 M methyl $\alpha$-D-mannopyranoside to elute PtPA (labelled "a").

Figure 5 shows the results of native PAGE at pH 8.6 of pooled fractions labelled "a" in Figure 4 where lane A is 25 $\mu$g and lane B is 50 $\mu$g.

Figure 6 shows the results of native PAGE at pH 8.6 of purified PtPA where the gel was stained with Coomassie blue and a duplicate gel was cut into 5 mm slices which were each equilibrated with 1 mL of S-2222 assay mixture to locate the activity, as described in Example 1b. The graph shows a plot of S-2222 activity (Y axis reads: "RATE OF HYDROLYSIS OF S-222 ($\Delta A_{410}$ / min)) against gel slice number (X axis reads: "GEL SLICE NUMBER").

Figure 7 shows the SDS-PAGE results of the affinity purified preparations of PtPA and OsPA under reducing and non-reducing conditions as described in Example 1b where the lanes are (from left to right): marker; OsPA red. (mg/mL); PtPA red. (mg/mL); OsPA red. (2 mg/mL); PtPA red. (2 mg/mL); OsPA unred. (mg/mL); PtPA unred. (1 mg/mL); OsPA unred. (2 mg/mL); PtPA unred. (2 mg/mL); marker; where "red." designates the components in the presence of β-mercaptoethanol (*i.e.* reduced) and "unred." designates the components in the absence of β-mercaptoethanol (*i.e.* unreduced).

Figure 8 shows the elution profile in the isolation of notecarin from *N. scutatus* venom using Sephacryl S-300 chromatography as described in Example 1c. The pooled fractions of notecarin are indicated by the bar labelled "PA".

Figure 9 shows the results of native PAGE at pH 8.9 of the prothrombin activators: carinactivase-1, carinactivase-2, ecarin, PtPA, OsPA, and notecarin, prepared in Examples 1a, 1b, and 1c. In this Figure, the labels represent the following: (I) prothrombin; (II) alpha-thrombin; (III) ecarin; (IV) carinactivase-1; (V) carinactivase-2; (VI) PtPA; (VII) OsPA; (VIII) notecarin (20 μg of each prothrombin activator was loaded).

Figure 10 shows SDS-PAGE characterisation of the prothrombin activators prepared in Examples 1a, 1b, and 1c, in the presence of β-mercaptoethanol, where the lanes are as follows: (1) carinactivase-1; (2) carinactivase-2; (3) ecarin; (4) PtPA; (5) OsPA; (6) notecarin; (7) thrombin; and (M) molecular weight marker.

Figure 11 shows SDS-PAGE characterisation of the prothrombin activators prepared in Examples 1a, 1b, and 1c, in the absence of β-mercaptoethanol, where the lanes are as follows: (1) carinactivase-1; (2) carinactivase-2; (3) ecarin; (4) PtPA; (5) OsPA; (6) notecarin; (7) thrombin; and (M) molecular weight marker.

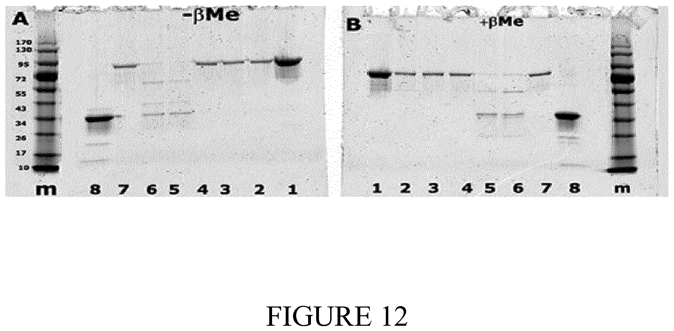

Figure 12 shows the SDS-PAGE without β-mercaptoethanol (A) and with β-mercaptoethanol (B) of samples incubated for 5 minutes at room temperature in the presence of 5mM Ca$^{2+}$, and with the following prothrombin activators: carinactivase-1 (lane 2); carinactivase-2 (lane 3); ecarin (lane 4); PtPA (lane 5); OsPA (lane 6); notecarin (lane 7). Lane 1 contained the sample of human prothrombin alone in buffer (no prothrombin activator), and lane 8 contained the sample of highly purified α-thrombin (no prothrombin activator), and "m" represents the molecular weight marker. This experiment is described in more detail in Example 2a.

Figure 13 shows the SDS-PAGE of the time course of the prothrombin (14 μM) to thrombin activation by PtPA (6 nM) and by notecarin (6 nM), as described in Example 2b.

Figure 14 is an annotated image of part of the SDS-PAGE results in Figure 13, where selected bands were eluted and subjected to N-terminal sequencing using mass-spectrometry analysis in order to assign to specific molecular domains.

Figure 15 is a graph of the absorbance of the pNA generated from S-2238 by different thrombin concentrations over 155 seconds as described in Example 2d. The calculations on the right hand side of the graph align with each line of the graph, for example the top calculation is the highest line, and so on.

Figure 16 is a graph of the standard curve derived from the results in Figure 15, specifically the slopes of the equations for each reaction up to 180 seconds in Figure 15 were plotted against the thrombin concentration to provide the linear regression equation shown in Figure 16.

Figure 17 is a graph showing the rate of thrombin generation by 0.6 nM concentrations of the prothrombin activators: PtPA, OsPA, ecarin (Ecr), notecarin (Ntcr), carinactivase-1 (CA-1), and carinactivase-2 (CA-2) over 180 seconds as described in Example 2d.

Figure 18 shows the curve fitting analysis of the thrombin catalysed hydrolysis of S-2238 as described in Example 2e. Thrombin concentrations are indicated on progress curves.

Figure 19 plots the PtPA activation of prothrombin at the concentrations of PtPA as described in Example 2e.

Figure 20 plots the OsPA activation of prothrombin at the concentrations of OsPA at the concentrations of OsPA as described in Example 2e.

Figure 21 plots the ecarin activation of prothrombin at the concentrations of ecarin as described in Example 2e.

Figure 22 plots the carinactivase-1 activation of prothrombin at the concentrations of carinactivase-1 as described in Example 2e.

Figure 23 plots the carinactivase-2 activation of prothrombin at the concentrations of carinactivase-2 as described in Example 2e.

Figure 24 plots the notecarin activation of prothrombin at the concentrations of notecarin as described in Example 2e.

Figure 25 shows an example of a TEG plot (clot formation part) with the clotting parameters labelled, as discussed in the Examples section.

Figure 26 plots the progress curves for hydrolysis of S-2238 by different thrombin concentrations in duplicate from 45-225 nM over a 5 minute incubation period in normal citrated plasma as described in Example 3g.

Figure 27 shows the thrombin standard curve derived from the slope of the reactions in Figure 26 at the 5 minute incubation mark in logarithmic form, where the *x*-axis shows the slope In (absorbance units / minute); and the *y*-axis shows In (molar concentration of thrombin).

Figure 28 shows assays of thrombin remaining after clot removal in sera generated by three different PtPA concentrations (in duplicate), as described in Example 3g.

Figure 29 shows assays of thrombin remaining after clot removal in sera generated by three different OsPA concentrations (in duplicate), as described in Example 3g.

Figure 30 plots the slopes from Figure 28 (proportional to thrombin concentrations) against PtPA concentration, as described in Example 3g.

Figure 31 plots the slopes from Figure 29 (proportional to thrombin concentrations) against OsPA concentration, as described in Example 3g.

Figure 32 shows progress curves for the hydrolysis of S-2238 by thrombin remaining in serum after clot removal in the presence of 4300 nM (10 IU) heparin, as described in Example 3g. Thrombin was generated by PtPA and OsPA, or was added.

Figure 33 shows the reaction curves with samples containing different heparin concentrations using 1.5 nM PtPA as described in Example 3g.

Figure 34 shows the reaction curves with samples containing different heparin concentrations using 1.5 nM OsPA as described in Example 3g.

Figure 35 shows the changes of absorbances over the 5 minute period in pooled "normal" citrated plasma, with each line representing a BD RST tube filled with a different volume of normal pooled citrate plasma, as described in Example 3h.

Figure 36 shows the relationship between the measured slopes from Figure 35 and the corresponding thrombin concentrations read from the standard curve in Figure 16, as described in Example 3h.

Figure 37 shows the changes of absorbances over the 5 minute period in pooled "normal" citrated plasma, with each line representing a BD RST tube filled with 1 mL or 4 mL normal pooled citrate plasma and a different concentration of heparin, as described in Example 3h.

Figure 38 shows TEG traces for comparison of plain tube, commercially available serum tubes, and PtPA-containing tube as described in Example 4c.

Figure 39 shows TEG plots of the results in Example 5a.

Figure 40 shows TEG plots of the results for volunteer "W1" in Example 5c.

Figure 41 shows TEG plots of the results for volunteer "W2" in Example 5c.

Figure 42 shows TEG traces for clotting of recalcified citrated blood from a heparinised participant with PtPA or OsPA as described in Example 5d.

Figure 43 shows the TEG traces of the Example 5e results.

Figure 44 shows the TEG plots of the Example 6a results.

Figure 45 shows the TEG plots of the Example 6b results.

Figure 46 plots the clotting times of prothrombin activator-containing snake venoms from the species: *Pseudonaja textilis* (Pt), *Oxyuranus scutellatus* (Os), *Oxyuranus microlepidotus* (Om), *Notechis scutatus* (Ns), and *Echis carinatus* (Ec) at different concentrations as described in Example 7.

Figure 47 shows a Greiner plasma tube with gelatinous precipitate as described in Example 8.

Figure 48 shows post-centrifugation (latent) clotting in a number of tubes as described in Example 8.

Figure 49 shows a comparison of serum samples in different tubes as described in Example 8.

Figure 50 shows the range of fibrinogen/fdp/FDP concentrations measured in Greiner serum tubes from 48 randomly selected patients requiring analyte determination as described in Example 9a. The bar shows a mean of 17.5 $\mu$g/mL and range is 4.4-32 $\mu$g/mL.

Figure 51 shows the comparison of the fibrinogen/fdp/FDP concentration measured by ELISA in 36 normal serum samples prepared using Greiner serum (GS) tubes with or without the addition of PtPA as described in Example 9b.

Figure 52 shows the fibrinogen/fdp/FDP concentrations measured by ELISA in the sera from 9 normal blood samples collected in four different serum tubes: Greiner serum (GRS), BD serum (BDS), BD RST and PtPA (300 ng/mL) as described in Example 9b.

Figure 53 shows the fibrinogen/fdp/FDP concentrations measured by ELISA in the sera from 5 normal blood samples collected in Greiner serum tubes (GRS), Greiner No Additive tubes with 300 ng/mL of PtPA (PTPA), Greiner No Additive tubes containing 125 ng/mL of OsPA (OSPA) and Greiner No Additive tubes containing 0.16 U/mL of purified ecarin (ECARIN) as described in Example 9b. The bars represent mean $\pm$ standard deviation.

Figure 54 shows the fibrinogen/fdp/FDP concentrations measured by ELISA of sera from 3 renal dialysis patients collected in Greiner serum tubes (GRS), BD SST II tubes (BDS), BD RST tubes (BDRST) and Greiner No Additive tubes with added 1.2 $\mu$g/4 mL tube PtPA (PTPA) as described in Example 9c.

Figure 55 shows the fibrinogen/fdp/FDP concentrations measured by ELISA of plasma and sera from 2 cardiac patients collected in Greiner plasma tubes (GRLH), Greiner serum tubes (GRS), BD RST tubes (BDRST), Greiner Vacuette™ No Additive tube with added 300 ng/mL PtPA (PTPA), Greiner Vacuette™ No Additive tube with added 125 ng/mL OsPA (OSPA), Greiner Vacuette™ No Additive tube with added 0.31 U/mL of ecarin (EC1) and Greiner Vacuette™ No Additive tube with added 0.63 U/mL of ecarin (EC2) as described in Example 9c.

Figure 56 shows Giemsa-stained Cytospin slides showing cell content above the gel barrier as described in Example 9b where the slides from left to right are: (P) - PtPA serum; (S) - Greiner serum; (LH) - diluted Greiner lithium heparin plasma; and (LH) - undiluted Greiner lithium heparin plasma.

Figure 57 shows total protein determination carried out under standard Pathology Queensland procedures on normal serum and plasma samples, (n = 26), all serum and plasma samples (n = 61) and cardiac patient samples (n = 11) as described in Example 12a.

Figure 58 shows the time taken for two different PtPA concentrations to clot plasma at selected time points over a period of two weeks (336 hours) when stored at different temperatures, as described in Example 15a.

Figure 59 shows the activity of OsPA against the chromogenic substrate S-2765 after irradiation in Greiner plain tubes (P) and in Greiner serum tubes containing silica and surfactant (siP), as described in Example 15b.

Figure 60 shows the citrated plasma clotting activity of OsPA in Greiner plain tubes (P) and in Greiner serum tubes containing silica and surfactant, as described in Example 15b.

Figure 61 shows the activity ($A_{405}$/mm) of OsPA against the chromogenic substrate S-2765 over a period of up to 14 days at 23 °C compared to a fresh dilution of stock, as described in Example 15c.

Figure 62 shows an example of a device (point-of-care device in the form of a syringe) designed to produce high quality serum for analysis, as described in Example 17.

## DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much 15, 10, 9, 8, 7, 6, 5, 4, 3, 2 or 1% to a reference quantity, level, value, number, frequency, percentage, dimension, size, amount, weight or length.

The term "biologically active fragment", as applied to fragments of a reference or full-length polynucleotide or polypeptide sequence, refers to a fragment that has at least about 0.1, 0.5, 1, 2, 5, 10, 12, 14, 16, 18, 20, 22, 24, 26, 28, 30, 35, 40, 45, 50, 55, 60, 65, 70, 75, 80, 85, 90, 95, 96, 97, 98, 99% of the activity of a reference sequence. Included within the scope of the present invention are biologically active fragments, including those of at least about 18, 19, 20, 21, 22, 23, 24, 25, 26, 27, 28, 29, 30, 40, 50, 60, 70, 80, 90, 100, 120, 140, 160, 180, 200, 250, 300, 400, 500, 600, 700, 800, 900, 1,000, 1,500, 2,000 nucleotides or residues in length, which comprise or encode an activity of a reference polynucleotide or polypeptide. Representative biologically active fragments generally participate in an interaction, e.g. an intramolecular or an inter-molecular interaction. An inter-molecular interaction can be a specific binding interaction or an enzymatic interaction (e.g., the interaction can be transient and a covalent bond is formed or broken). Biologically active fragments of a full-length polypeptide include peptides may comprise amino acid sequences sufficiently similar to or derived from the amino acid sequences of a (putative) full-length polypeptide. Typically, biologically active fragments comprise a domain or motif with at least one activity of a full-length polypeptide. Suitably, the biologically-active fragment has no less than about 1%, 10%, 25% 50% of an activity of the full-length polypeptide from which it is derived.

By "coding sequence" is meant any nucleic acid sequence that contributes to the code for the polypeptide product of a gene. By contrast, the term "non-coding sequence" refers to any nucleic acid sequence that does not contribute to the code for the polypeptide product of a gene.

Throughout this specification, unless the context requires otherwise, the words "comprise," "comprises" and "comprising" will be understood to imply the inclusion of a stated step or element or group of steps or elements but not the exclusion of any other step or element or group of steps or elements. Thus, use of the term "comprising" and the like indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridisation between nucleic acid strands.

By "corresponds to" or "corresponding to" is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

As used herein, the term "detecting an analyte" means determining the presence, absence, amount or concentration of one or more analytes in a sample.

By "gene" is meant a unit of inheritance that occupies a specific locus on a chromosome and consists of transcriptional and/or translational regulatory sequences and/or a coding region and/or non-translated sequences (i.e., introns, 5' and 3' untranslated sequences).

"Homology" refers to the percentage number of nucleic or amino acids that are identical or constitute conservative substitutions. Homology may be determined using sequence comparison programs such as GAP (Devereux et al., 1984). In this way sequences of a similar or substantially different length to those cited herein could be compared by insertion of gaps into the alignment, such gaps being determined, for example, by the comparison algorithm used by GAP.

The term "host cell" includes an individual cell or cell culture which can be or has been a recipient of any recombinant vector(s) or isolated polynucleotide of the invention. Host cells include progeny of a single host cell, and the progeny may not necessarily be completely identical (in morphology or in total DNA complement) to the original parent cell due to natural, accidental, or deliberate mutation and/or change. A host cell includes cells transfected or infected in vivo or in vitro with a recombinant vector or a polynucleotide of the invention. A host cell which comprises a recombinant vector of the invention is a recombinant host cell.

"Hybridisation" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA U pairs with A and C pairs with G. In this regard, the terms "match" and "mismatch" as used herein refer to the hybridisation potential of paired nucleotides in complementary nucleic acid strands. Matched nucleotides hybridise efficiently, such as the classical A-T and G-C base pair mentioned above. Mismatches are other combinations of nucleotides that do not hybridise efficiently.

By "isolated" is meant material that is substantially or essentially free from components that normally accompany it in its native state. For example, an "isolated polynucleotide," as used herein, refers to a polynucleotide, which has been purified from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to in vitro isolation and/or purification of a peptide or polypeptide molecule from its natural cellular environment, and from association with other components of the cell, i.e., it is not associated with in vivo substances.

By "obtained from" is meant that the polypeptide or complex, for example, is isolated from, or derived from, a particular source.

The term "oligonucleotide" as used herein refers to a polymer composed of a multiplicity of nucleotide residues (deoxyribonucleotides or ribonucleotides, or related structural variants or synthetic analogues thereof) linked via phosphodiester bonds (or related structural variants or synthetic analogues thereof). Thus, while the term "oligonucleotide" typically refers to a nucleotide polymer in which the nucleotide residues and linkages between them are naturally occurring, it will be understood that the term also includes within its scope various analogues including, but not restricted to, peptide nucleic acids (PNAs), phosphoramidates, phosphorothioates, methyl phosphonates, 2-O-methyl ribonucleic acids, and the like. The exact size of the molecule can vary depending on the particular application. An oligonucleotide is typically rather short in length, generally from about 10 to 30 nucleotide residues, but the term can refer to molecules of any length, although the term "polynucleotide" or "nucleic acid" is typically used for large oligonucleotides.

The term "operably linked" as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e., the genes from which it is derived.

The terms "patient", "subject" and "individual" are used interchangeably and refer to patients, subjects and individuals of human or other mammals and includes any one for whom it is desired to detect analyte levels or to diagnose the presence, absence or severity of a disease or condition using the invention. However, it will be understood that "patient" does not imply that symptoms are present. Suitable mammals that fall within the scope of the invention include, but are not restricted to, primates (*e.g.* humans, chimpanzees), livestock animals (*e.g.* sheep, cows, horses, donkeys, pigs), laboratory test animals (*e.g.* rabbits, mice, rats, guinea pigs, hamsters), companion animals (*e.g.* cats, dogs) and captive wild animals (*e.g.* foxes, deer, dingoes).

The term *"polynucleotide"* or *"nucleic acid"* as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA.

The terms *"polynucleotide variant"* and *"variant"* and the like refer to polynucleotides displaying substantial sequence identity with a reference polynucleotide sequence or polynucleotides that hybridise with a reference sequence under stringent conditions that are defined hereinafter. These terms also encompass polynucleotides that are distinguished from a reference polynucleotide by the addition, deletion or substitution of at least one nucleotide. Accordingly, the terms *"polynucleotide variant"* and *"variant"* include polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide. The terms *"polynucleotide variant"* and *"variant"* also include naturally occurring allelic variants.

*"Polypeptide"*, *"peptide"* and *"protein"* are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers.

The term *"polypeptide variant"* refers to polypeptides that are distinguished from a reference polypeptide by the addition, deletion or substitution of at least one amino acid residue. In certain embodiments, a polypeptide variant is distinguished from a reference polypeptide by one or more substitutions, which may be conservative or non-conservative. In certain embodiments, the polypeptide variant comprises conservative substitutions and, in this regard, it is well understood in the art that some amino acids may be changed to others with broadly similar properties without changing the nature of the activity of the polypeptide. Polypeptide variants also encompass polypeptides in which one or more amino acids have been added or deleted, or replaced with different amino acid residues.

By *"primer"* is meant an oligonucleotide which, when paired with a strand of DNA, is capable of initiating the synthesis of a primer extension product in the presence of a suitable polymerising agent. The primer is preferably single-stranded for maximum efficiency in amplification but can alternatively be double-stranded. A primer must be sufficiently long to prime the synthesis of extension products in the presence of the polymerization agent. The length of the primer depends on many factors, including application, temperature to be employed, template reaction conditions, other reagents, and source of primers. For example, depending on the complexity of the target sequence, the oligonucleotide primer typically contains 15 to 35 or more nucleotide residues, although it can contain fewer nucleotide residues. Primers can be large polynucleotides, such as from about 200 nucleotide residues to several kilobases or more. Primers can be selected to be *"substantially complementary"* to the sequence on the template to which it is designed to hybridise and serve as a site for the initiation of synthesis. By *"substantially complementary"*, it is meant that the primer is sufficiently complementary to hybridise with a target polynucleotide. Preferably, the primer contains no mismatches with the template to which it is designed to hybridise but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridise therewith and thereby form a template for synthesis of the extension product of the primer.

*"Probe"* refers to a molecule that binds to a specific sequence or subsequence or other moiety of another molecule. Unless otherwise indicated, the term *"probe"* typically refers to a polynucleotide probe that binds to another polynucleotide, often called the *"target polynucleotide"*, through complementary base pairing. Probes can bind target polynucleotides lacking complete sequence complementarity with the probe, depending on the stringency of the hybridisation conditions. Probes can be labelled directly or indirectly.

The term *"reference result"* includes a result taken from the same subject at a different time, a result from a normal subject or a group of normal subjects, or a reference standard used in an analytical test.

By *"regulatory element"* or *"regulatory sequence"* is meant nucleic acid sequences (e.g., DNA) necessary for expression of an operably linked coding sequence in a particular host cell. The regulatory sequences that are suitable for prokaryotic cells for example, include a promoter, and optionally a *cis*-acting sequence such as an operator sequence and a ribosome binding site. Control sequences that are suitable for eukaryotic cells include promoters, polyadenylation signals, transcriptional enhancers, translational enhancers, leader or trailing sequences that modulate mRNA stability, as well as targeting sequences that target a product encoded by a transcribed polynucleotide to an intracellular compartment within a cell or to the extracellular environment.

The term *"sequences identity"* as used herein refers to the extent that sequences are identical on a nucleotide-by-nucleotide basis or an amino acid-by-amino acid basis over a window of comparison. Thus, a *"percentage of sequence*

*identity"* is calculated by comparing two optimally aligned sequences over the window of comparison, determining the number of positions at which the identical nucleic acid base (e.g., A, T, C, G, I) or the identical amino acid residue *(e.g.,* Ala, Pro, Ser, Thr, Gly, Val, Leu, Ile, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (*i.e.,* the window size), and multiplying the result by 100 to yield the percentage of sequence identity. For the purposes of the present invention, *"sequence identity"* may be understood to mean the "match percentage" calculated by the DNASIS computer program (Version 2.5 for Windows; available from Hitachi Software Engineering Co., Ltd., South San Francisco, California, USA) using standard defaults as used in the reference material accompanying the software.

The term *"sequences similarity"* refers to the percentage number of amino acids that are identical or constitute conservative substitutions as defined in Table 2 *infra.* Similarity may be determined using sequence comparison programs such as GAP (Devereux *et al.,* 1984). In this way, sequences of a similar or substantially different length to those cited herein might be compared by insertion of gaps into the alignment, such gaps being determined, for example, by the comparison algorithm used by GAP.

Terms used to describe sequence relationships between two or more polynucleotides or polypeptides include *"reference sequence", "comparison window", "sequences identity", "percentage of sequence identity"* and *"substantial identity".* A *"reference sequence"* is at least 12 but frequently 15 to 18 and often at least 25 monomer units, inclusive of nucleotides and amino acid residues, in length. Because two polynucleotides may each comprise (1) a sequence *(i.e.,* only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a *"comparison window"* to identify and compare local regions of sequence similarity. A *"comparison window"* refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions *(i.e.,* gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (*i.e.*, resulting in the highest percentage homology over the comparison window) generated by any of the various methods selected. Reference also may be made to the BLAST family of programs as for example disclosed by Altschul *et al.,* 1997. A detailed discussion of sequence analysis can be found in Unit 19.3 of Ausubel et al., "Current Protocols in Molecular Biology", John Wiley & Sons Inc, 1994-1998, Chapter 15.

*"Stringency"* as used herein, refers to the temperature and ionic strength conditions, and presence or absence of certain organic solvents, during hybridisation and washing procedures. The higher the stringency, the higher will be the degree of complementarity between immobilized target nucleotide sequences and the labelled probe polynucleotide sequences that remain hybridised to the target after washing. The term *"high stringency"* refers to temperature and ionic conditions under which only nucleotide sequences having a high frequency of complementary bases will hybridise. The stringency required is nucleotide sequence dependent and depends upon the various components present during hybridisation. Generally, stringent conditions are selected to be about 10 to 20°C lower than the thermal melting point ($T_m$) for the specific sequence at a defined ionic strength and pH. The $T_m$ is the temperature (under defined ionic strength and pH) at which 50% of a target sequence hybridises to a complementary probe.

The term *"transformation"* means alteration of the genotype of an organism, for example a bacterium, yeast, mammal, avian, reptile, fish or plant, by the introduction of a foreign or endogenous nucleic acid.

By *"vector"* is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, *i.e.,* a vector that exists as an extra-chromosomal entity, the replication of which is independent of chromosomal replication, e.g., a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In the present case, the vector is preferably a viral or viral-derived vector, which is operably functional in animal and preferably mammalian cells. Such vector may be derived from a poxvirus, an adenovirus or yeast. The vector can also include a selection marker such as an antibiotic resistance gene that can be used for selection of suitable transformants. Examples of such resistance genes are known to those of skill in the art and include the *nptII* gene that confers resistance to the antibiotics kanamycin and G418 (Geneticin®) and the hph gene which confers resistance to the antibiotic hygromycin B.

The terms *"wild-type"* and *"naturally occurring"* are used interchangeably to refer to a gene or gene product that has the characteristics of that gene or gene product when isolated from a naturally occurring source. A wild type gene or gene product (e.g., a polypeptide) is that which is most frequently observed in a population and is thus arbitrarily designed the *"normal"* or *"wild-type"* form of the gene.

### 2. Prothrombin activators

The present invention is based in part on the discovery that prothrombin activators are suitable clotting agents for preparing serum that is used to detect analytes, despite their known proteolytic activity. Prothrombin activators (sometimes known as prothrombinases) exhibit trypsin-like activity and activate prothrombin (*i.e.* convert prothrombin to thrombin which in turn converts fibrinogen to fibrin and hence cause clot formation).

In some embodiments, the prothrombin activator is an exogenous prothrombin activator. As used herein, an *"exogenous prothrombin activator"* means a prothrombin activator obtained from a source other than the blood sample from which the serum sample is to be prepared.

### 2.1 Wild-type or naturally-occurring prothrombin activators

The prothrombin activators used in the present invention may comprise wild-type or naturally-occurring prothrombin activators including those obtained from any suitable organism, including snake, human, bovine and bacterial prothrombin activator. The prothrombin activator may comprise a full-length wild-type or naturally occurring polypeptide.

In certain embodiments, the prothrombin activator is a snake prothrombin activator. Suitably, the prothrombin activator is a snake venom prothrombin activator. Snake venom prothrombin activators are generally classified in four groups (A, B, C, and D) depending on their structure, function and requirements for co-factors.

Suitably, the snake venom prothrombin activator is a group A prothrombin activator. Group A prothrombin activators are metalloproteinases consisting of three domains: a metalloproteinase, a disintegrin, and a Cys-rich domain. The metalloproteinase domain contains the consensus sequence HEXXHXXGXXH, corresponding to the zinc-chelating active site. These prothrombin activators are found at least in several viper venoms, and include ecarin from *Echis carinatus* venom and basparin from *Bothrops asper* venom.

Suitably, the snake venom prothrombin activator is a group B prothrombin activator. Group B prothrombin activators are metalloproteinases consisting of two subunits held non-covalently: a metalloproteinase and a C-type lectin-like disulfide-liked dimer. These prothrombin activators are found in several viper venoms, and include carinactivase-1 and carinactivase-2 from *Echis carinatus* venom and multactivase from *Echis multisquamatus* venom.

Suitably, the snake venom prothrombin activator is a group C prothrombin activator. Group C prothrombin activators are serine proteases and resemble the mammalian factor Xa-factor Va complex. Pseutarin C (or PtPA) and oscutarin C (or OsPA) are group C prothrombin activators from the venoms of *Pseudonaja textilis* and *Oxyuranus scutellatus* respectively. Omicarin C is the prothrombin activator from *Oxyuranus microlepidotus* venom.

Suitably, the snake venom prothrombin activator is a group D prothrombin activator. Group D prothrombin activators are serine proteases and are functionally similar to mammalian factor Xa. Porpharin D (from *Pseudechis porphyriacus),* notecarin D (from *Notechis scutatus scutatus),* trocarin D (from *Tropidechis carinatus),* hopsarin D (from *Hoplocephalus stephensi),* and notenarin D (from *Notechis ater niger)* are all group D prothrombin activators.

A review of snake prothrombin activators is provided in Kini, R. M. (2005), and of those specifically from the venom of Australian Elapids (group C and D prothrombin activators) is in St. Pierre *et al.* (2005). These two reviews use the classification of snake prothrombin activators into groups A-D as described above. This classification supersedes the previous classification system using groups I-III (group I encompasses groups A and B; group II is now group D and group III is now group C), and sometimes additional groups IV (snake venom activators that cleave peptide bonds in prothrombin but do not convert the prothrombin to an enzymatically active product - *i.e.* thrombin or meizothrombin) and V (bacterial prothrombin activators) as described in earlier review articles, including Rosing, J. *et al.* (1991) and Rosing, J. *et al.* (1992). For an explanation on the change to the classification system, see Kini, R, M., *et al.* (2001).

In specific embodiments, the snake prothrombin activator is obtained from the Family *Elapidae,* illustrative examples of which include species from the genera *Demansia, Hoplocephalus, Notechis, Oxyuranus, Pseudechis, Pseudonaja, Rhinoplocephalus,* and *Tropidechis* including but not limited to *Demansia vestigiata, Hoplocephalus stephensii, Notechis ater humphreysi, Notechis ater niger, Notechis ater serventyi, Notechis flinkders, Notechis humphreysi, Notechis niger, Notechis occidentalis, Notechis scutatus, Notechis scutatus scutatus, Notechis serventyi, Oxyuranus microlepidotus, Oxyuranus scutellatus, Pseudechis porphyriacus, Pseudonaja affinis, Pseudonaja inframaculata, Pseudonaja nuchalis, Pseudonaja textilis, Rhinoplocephalus nigrescens,* and *Tropidechis carinatus.*

In specific embodiments, the snake prothrombin activator is obtained from the Family *Viperidae,* illustrative examples of which include species from the genera *Botrhops, Echis* and *Trimeresurus,* including but not limited to *Bothrops alternatus, Bothrops asper, Bothrops atrox, Bothrops atrox asper, Bothrops brasili, Bothrops castelnaudi, Bothrops columbiensis, Bothrops erythromelas, Bothrops fonsecai, Bothrops itapetiningae, Bothrops jararaca, Bothrops neuwiedi, Bothrops venezuelensis, Echis carinatus, Echis coloratus, Echis multisquamatus,* and *Trimeresurus okinavensis.*

In specific embodiments, the snake prothrombin activator is obtained from the Family *Colubridae,* illustrative examples of which include species from the genera *Dispholidus, Rhabdophis* and *Thelotornis,* including but not limited to *Dispholidus typus, Rhabdophis tigrinus tigrinus, Thelotornis kirtlandii,* and *Thelotornis capensis.*

In some embodiments the snake prothrombin activator is from or is obtained from snake venom. The purification and characterisation of PtPA from *P. textilis* snake venom is described in Masci (1986) and Masci *et al.,* (1998), and OsPA from *O. scutellatus* venom is described in Speijer *et al.*, (1986). The purification and characterisation of ecarin from *Echis carinatus* venom is described in Morita, T *et al.* (1981) and Nishida, S *et al.* (1995), of carinactivase from *Echis carinatus* venom is described in Yamada, D *et al.* (1996), of multactivase from *Echis multisquamatus* is described in Yamada, D. *et al.*, (1997), and of notecarin from *Notechis scutatus* is described in Tans, G *et al.*, (1985).

In certain embodiments, the prothrombin activator is a mammalian prothrombin activator. Mammalian prothrombin activators include those derived from human blood and/or tissue and those derived from bovine blood and/or tissue.

In certain embodiments, the prothrombin activator is a bacterial prothrombin activator. Bacterial prothrombin activators include those from *Staphylococcus aureus, Peptococcus indolicus, Bacteroides melaninogenicus,* and *Pseudomonas aeruginosa* (Rosing, J. *et al.* (1991).

As will be appreciated by those skilled in the art, the prothrombin activator may comprise, consist essentially of, or consist of one or more polypeptides. In some embodiments, the prothrombin activator comprises, consists essentially of, or consists of a single polypeptide. In other embodiments, the prothrombin activator comprises, consists essentially of, or consists of more than one polypeptide, including but not limited to complexes of polypeptides. Where the prothrombin activator comprises, consists essentially of, or consists of more than one polypeptide, each polypeptide may be from the organisms from the same or different genera, and/or the same or different species.

In certain embodiments, the prothrombin activator comprises an amino acid sequence selected from those set forth in SEQ ID NOs: 1, 2, 3, 4, 7, 8, 11, 12, 13, 16, 18, 26, 27, 28, 29, 30, 32, 34, 36, 38, 40, 42, 44, 46, 48, 50, 51, and 52 or comprises an amino acid sequence encoded by a nucleotide sequence selected from those set forth in SEQ ID NOs: 5, 6, 9, 10, 14, 15, 17, 19, 20, 21, 22, 23, 24, 25, 31, 33, 35, 37, 39, 41, 43, 45, 47, and 49.

### 2.2 Chimeric prothrombin activators and fusion polypeptides

The present invention also contemplates the use of prothrombin activators comprising a chimeric polypeptide. As used herein, a *"chimeric polypeptide"* includes a first polypeptide component comprising a polypeptide obtained from a first organism linked to a second polypeptide component obtained from a second organism. In some embodiments, the first organism and the second organism are from different genera. In other embodiments, the first organism and the second organism are different species of the same genus. In certain embodiments, the prothrombin activator comprises a chimeric polypeptide that resembles a factor Xa-factor Va complex, wherein the first polypeptide comprises a factor Xa-like polypeptide and the second polypeptide comprises a factor Va-like polypeptide. In certain specific embodiments, the first polypeptide comprises an amino acid sequence selected from those set forth in SEQ ID NOs: 26, 27, 28, 29, 30, 32, 34, 36, 38, 40, 42, 44, 46, 48, and 50, or comprises an amino acid sequence encoded by a nucleotide sequence selected from those set forth in SEQ ID NOs: 19, 20, 21, 22, 23, 24, 25, 31, 33, 35, 37, 39, 41, 43, 45, 47, and 49, and the second polypeptide comprises an amino acid sequence selected from those set forth in SEQ ID NOs: 7, 8, 11, 12, 13, 16, and 18, or comprises an amino acid sequence encoded by a nucleotide sequence selected from those set forth in SEQ ID NOs: 5, 6, 9, 10, 14, 15, and 17.

The present invention also contemplates the use of prothrombin activators comprising a fusion polypeptide. As used herein, a *"fusion polypeptide"* includes a first polypeptide component linked to a second polypeptide component. The first polypeptide component may be obtained from a first organism and the second polypeptide component may be obtained from a second organism. In some embodiments, the first organism and the second organism are from different genera. In other embodiments, the first organism and the second organism are different species of the same genus. The first polypeptide component or the second polypeptide component of the fusion polypeptide can correspond to all or a portion (*e.g.*, a fragment as described herein) of a wild-type or naturally occurring amino acid sequence. The second polypeptide component can be fused to the N-terminus or C-terminus of the first polypeptide component.

## 2.3 Fragments of wild-type or naturally occurring polypeptides

The prothrombin activator may comprise a fragment of a full-length wild-type or naturally occurring polypeptide, wherein the prothrombin activator exhibits prothrombin activating activity.

Typically, fragments of a full-length polypeptide may participate in an interaction, for example an intramolecular or an intermolecular interaction. Such fragments include peptides comprising the amino acid sequences shown in SEQ ID NOs: 2, 3, 4, 51, and 52 and peptides comprising amino acid sequences sufficiently similar to or derived from the amino acid sequences of a (putative) full-length polypeptide, for example, the amino acid sequences shown in SEQ ID NOs: 1, 7,8, is, 12, 13, 16, 18, 26, 27, 28, 29, 30, 32, 34, 36, 38, 40, 42, 44, 46, 48, and 50, or the amino acid sequences encoded by a nucleotide sequence selected from those set forth in SEQ ID NOs: 5, 6, 9, 10, 14, 15, 17, 19, 20, 21, 22, 23, 24, 25, 31, 33, 35, 37, 39, 41, 43, 45, 47, and 49, which includes less amino acids than a full-length polypeptide, and exhibit one activity of that polypeptide.

## 2.4 Variants of naturally-occurring prothrombin activators (polypeptide)

The present invention also contemplates prothrombin activators comprising polypeptide(s) that is/are variant(s) of the wild-type or naturally-occurring polypeptide(s). Prothrombin activators comprising one or more variant polypeptides encompassed by the present invention are biologically active, that is, they continue to possess prothrombin activating activity.

Such *"variant"* prothrombin activators include polypeptides derived from the native polypeptide, wherein the polypeptides are derived from the corresponding native polypeptide(s) by deletion (so-called truncation) or addition of one or more amino acids to the N-terminal and/or C-terminal end of the native polypeptide(s); deletion or addition of one or more amino acids at one or more sites in the native polypeptide(s); or substitution of one or more amino acids at one or more sites in the native polypeptide(s). These variant prothrombin activators may result from, for example, genetic polymorphism or human manipulation.

Further non-limiting examples of variant polypeptides include precursor polypeptide or polypeptide in zymogen form processed forms of a full-length or precursor polypeptide or polypeptide in zymogen form.

Variants of a wild-type or naturally-occurring polypeptide will have at least 40%, 50%, 60%, 70%, generally at least 75%, 80%, 85%, usually about 90%, 91%, 92%, 93%, 94%, 95% or more, and typically about 96%, 97%, 98% or more (and all integer percentages in between) sequence similarity or identity with the amino acid sequence for the wild-type or naturally-occurring polypeptide, including but not limited to the sequences in SEQ ID NOs: 1, 2, 3, 4, 7, 8, 11, 12, 13, 16, 18, 26, 27, 28, 29, 30, 32, 34, 36, 38, 40, 42, 44, 46, 48, 50, 51, and 52, or the amino acid sequences encoded by the nucleotide sequences in SEQ ID NOs: 5, 6, 9, 10, 14, 15, 17, 19, 20, 21, 22, 23, 24, 25, 31, 33, 35, 37, 39, 41, 43, 45, 47, and 49, as determined by sequence alignment programs described elsewhere herein using default parameters. A variant of a wild-type or naturally-occurring polypeptide, which falls within the scope of a variant polypeptide, may differ from that polypeptide generally by as much 200, 100, 50 or 20 amino acid residues or suitably by as few as 1-15 amino acid residues, as few as 1-10, such as 6-10, as few as 5, as few as 4, 3, 2, or even 1 amino acid residue. In some embodiments, a variant polypeptide differs from the corresponding sequences in SEQ ID NOs: 1, 2, 3, 4, 7, 8, 11, 12, 13, 16, 18, 26, 27, 28, 29, 30, 32, 34, 36, 38, 40, 42, 44, 46, 48, 50, 51, or 52, or the amino acid sequences encoded by the nucleotide sequences in SEQ ID NOs: 5, 6, 9, 10, 14, 15, 17, 19, 20, 21, 22, 23, 24, 25, 31, 33, 35, 37, 39, 41, 43, 45, 47, or 49, by at least 1 but by less than 15, 10 or 5 amino acid residues. In other embodiments, it differs from the corresponding sequences in SEQ ID NOs: 1,2,3,4, 7, 8, 11, 12, 13, 16, 18, 26, 27, 28, 29, 30, 32, 34, 36, 38, 40, 42, 44, 46, 48, 50, 51, or 52, or the amino acid sequences encoded by the nucleotide sequences in SEQ ID NOs: 5, 6, 9, 10, 14, 15, 17, 19, 20, 21, 22, 23, 24, 25, 31, 33, 35, 37, 39, 41, 43, 45, 47, or 49, by at least one residue but less than 20%, 15%, 10% or 5% of the residues.

A polypeptide may be altered in various ways including amino acid substitutions, deletions, truncations, and insertions. Methods for such manipulations are generally known in the art. For example, amino acid sequence variants of a polypeptide can be prepared by mutations in the DNA. Methods for mutagenesis and nucleotide sequence alterations are well known in the art. See, for example, Kunkel (1985), Kunkel *et al.,* (1987), US patent 4,873,192, Watson et al., (1987) and the references cited therein. Guidance as to appropriate amino acid substitutions that do not affect biological activity of the protein of interest may be found in the model of Dayhoff *et al.* (1978). Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of polypeptides. Recursive ensemble mutagenesis (REM), a technique which enhances the frequency of functional mutants in the libraries, can be used in combination with the screening assays to identify polypeptide variants, see for example Arkin *et al.* (1992) and Delagrave *et al.* (1993). Conservative substitutions, such as exchanging one amino acid with another having similar properties, may be desirable as discussed in more detail below.

Variant polypeptides may contain conservative amino acid substitutions at various locations along their sequence, as compared to a parent (e.g., naturally-occurring or reference) amino acid sequence. A *"conservative amino acid substitution"* is one in which the amino acid residue is replaced with an amino acid residue having a similar side chain. Families of amino acid residues having similar side chains have been defined in the art, which can be generally sub-classified as follows:

Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.

Basic: The residue has a positive charge due to association with H ion at physiological pH or within one or two pH units thereof (*e.g.*, histidine) and the residue is attracted by aqueous solution so as to seek the surface positions in the conformation of a peptide in which it is contained when the peptide is in aqueous medium at physiological pH. Amino acids having a basic side chain include arginine, lysine and histidine.

Charged: The residues are charged at physiological pH and, therefore, include amino acids having acidic or basic side chains (*i.e.,* glutamic acid, aspartic acid, arginine, lysine and histidine).

Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.

Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterises certain amino acids as *"small"* since their side chains are not sufficiently large, even if polar groups are lacking, to confer hydrophobicity. With the exception of proline, *"small"* amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the α-amino group, as well as the α-carbon. Several amino acid similarity matrices (*e.g.* PAM120 matrix and PAM250 matrix as disclosed for example by Dayhoff *et al.* (1978) and by Gonnet *et al.* (1992)), however, include proline in the same group as glycine, serine, alanine and threonine. Accordingly, for the purposes of the present invention, proline is classified as a *"small"* amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behaviour.

Amino acid residues can be further sub-classified as cyclic or non-cyclic, and aromatic or non-aromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always non-aromatic. Dependent on their structural properties, amino acid residues may fall in two or more classes. For the naturally-occurring protein amino acids, sub-classification according to this scheme is presented in Table 1.

**TABLE 1**

| AMINO ACID SUB-CLASSIFICATION | |
|---|---|
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, Threonine |

(continued)

| AMINO ACID SUB-CLASSIFICATION | |
|---|---|
| Polar/large | Asparagine, Glutamine |
| Hydrophobic | Tyrosine, Valine, Isoleucine, Leucine, Methionine, Phenylalanine, Tryptophan |
| Aromatic | Tryptophan, Tyrosine, Phenylalanine |
| Residues that influence chain orientation | Glycine and Proline |

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional polypeptide can readily be determined by assaying its activity. Conservative substitutions are shown in Table 2 under the heading of exemplary and preferred substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants may be screened for biological activity.

TABLE 2

| EXEMPLARY AND PREFERRED AMINO ACID SUBSTITUTIONS | | |
|---|---|---|
| Ala | Val, Leu, Ile | Val |
| Arg | Lys, Gln, Asn | Lys |
| Asn | Gin, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | He |
| Lys | Arg, Gin, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleu | Leu |

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G. (1993).

Thus, a predicted non-essential amino acid residue in a polypeptide is typically replaced with another amino acid residue from the same side chain family. Alternatively, mutations can be introduced randomly along all or part of a polypeptide gene coding sequence, such as by saturation mutagenesis, and the resultant mutants can be screened for an activity of the parent polypeptide to identify mutants which retain that activity. Following mutagenesis of the coding sequences, the encoded peptide can be expressed recombinantly and the activity of the peptide can be determined. A *"non-essential"* amino acid residue is a residue that can be altered from the wild-type sequence of a polypeptide without abolishing or substantially altering one or more of its activities. Suitably, the alteration does not substantially alter one of these activities, for example, the activity is at least 20%, 40%, 60%, 70% or 80% of wild-type. An *"essential"* amino acid residue is a residue that, when altered from the wild-type sequence of a reference polypeptide, results in abolition of an activity of the parent molecule such that less than 20% of the wild-type activity is present.

Accordingly, the present invention also contemplates variants of the naturally-occurring polypeptide sequences or their biologically-active fragments, wherein the variants are distinguished from the naturally-occurring sequence by the addition, deletion, or substitution of one or more amino acid residues. In general, variants will display at least about 30, 40, 50, 55, 60, 65, 70, 75, 80, 85, 90, 91, 92, 93, 94, 95, 96, 97, 98, 99 % similarity to a parent or reference polypeptide sequence as, for example, set forth in SEQ ID NO: 1, 2, 3, 4, 7, 8, 11, 12, 13, 16, 18, 26, 27, 28, 29, 30, 32, 34, 36, 38, 40, 42, 44, 46, 48, 50, 51, or 52, or the parent or reference polypeptide sequence as, for example, encoded by the nucleotide sequence set forth in SEQ ID NO: 5, 6, 9, 10, 14, 15, 17, 19, 20, 21, 22, 23, 24, 25, 31, 33, 35, 37, 39, 41, 43, 45, 47, or 49. Desirably, variants will have at least 30, 40, 50, 55, 60, 65, 70, 75, 80, 85, 90, 91, 92, 93, 94, 95, 96, 97, 98, 99% sequence identity to a parent polypeptide sequence as, for example, set forth in SEQ ID NO: 1, 2, 3, 4, 7, 8, 11, 12, 13, 16, 18, 26, 27, 28, 29, 30, 32, 34, 36, 38, 40, 42, 44, 46, 48, 50, 51, or 52, or the parent polypeptide sequence as, for example, encoded by the nucleotide sequence set forth in SEQ ID NO: 5, 6, 9, 10, 14, 15, 17, 19, 20, 21, 22, 23, 24, 25, 31, 33, 35, 37, 39, 41, 43, 45, 47, or 49. Moreover; sequences differing from the native or parent sequences by the addition, deletion, or substitution of 1, 2, 3, 4, 5, 6, 7, 8, 9, 10, 11, 12, 13, 14, 15, 16, 17, 18, 19, 20, 30, 40, 50, 60, 70, 80, 90, 100 or more amino acids but which retain the properties of the parent polypeptide are contemplated. Polypeptides also include polypeptides that are encoded by polynucleotides that hybridise under stringency conditions as defined herein, especially high stringency conditions, to parent-coding polynucleotide sequences, or the non-coding strand thereof, as described below. Illustrative parent polynucleotide sequences are set forth in SEQ ID NO: 5,6,9, 10, 14, 5, 17, 19, 20, 21, 22, 23, 24, 25, 31, 33, 35, 37, 39, 41, 43, 45, 47, and 49.

In some embodiments, variant polypeptides differ from a reference sequence by at least one but by less than 50, 40, 30, 20, 15, 10, 8, 6, 5, 4, 3 or 2 amino acid residue(s). In other embodiments, variant polypeptides differ from the corresponding sequences of SEQ ID NO: 1,2,3,4, 7, 8, 11, 12, 13, 16, 18, 26, 27, 28, 29, 30, 32, 34, 36, 38, 40, 42, 44, 46, 48, 50,51, or 52, or the amino acid sequences encoded by the nucleotide sequences of SEQ ID NO: 5, 6, 9, 10, 14, 15, 17, 19, 20, 21, 22, 23, 24, 25, 31, 33, 35, 37, 39, 41, 43, 45, 47, or 49, by at least 1% but less than 20%, 15%, 10% or 5% of the residues. (If this comparison requires alignment, the sequences should be aligned for maximum similarity. "Looped" out sequences from deletions or insertions, or mismatches, are-considered differences). The differences are, suitably, differences or changes at a non-essential residue or a conservative substitution.

Variants of a protein can be identified by screening combinatorial libraries of mutants, *e.g.,* truncation mutants, of a protein. Libraries or fragments e.g., N terminal, C terminal, or internal fragments, of a protein coding sequence can be used to generate a variegated population of fragments for screening and subsequent selection of variants of a protein.

Methods for screening gene products of combinatorial libraries made by point mutation or truncation, and for screening cDNA libraries for gene products having a selected property are known in the art. Such methods are adaptable for rapid screening of the gene libraries generated by combinatorial mutagenesis of proteins.

Some variants of the snake prothrombin activator ecarin are described in US patent 6,413,737.

2.5 Variants of naturally-occurring prothrombin activators (nucleotide)

The present invention also contemplates prothrombin activators comprising polypeptide(s) that is/are encoded by variant(s) of the wild-type or naturally-occurring polynucleotide(s) encoding the wild-type or naturally-occurring polynucleotide(s).

Variants of a wild-type or naturally-occurring polynucleotides will have at least 40%, 50%, 60%, 70%, generally at least 75%, 80%, 85%, 86%, 87%, 88%, 89%, usually about 90%, 91%, 92%, 93%, 94%, 95% or more, and typically about 96%, 97%, 98% or more (and all integer percentages in between) sequence similarity or identity with the nucleotide sequence for the wild-type or naturally-occurring polynucleotide, including but not limited to the sequences encoded by the sequences of SEQ ID NOs: 1, 2, 3, 4, 7, 8, 11, 12, 13, 16, 18, 26, 27, 28, 29, 30, 32, 34, 36, 38, 40, 42, 44, 46, 48, 50, 51, or 52 or the sequences of SEQ ID NOs: 5, 6, 9, 10, 14, 15, 17, 19, 20, 21, 22, 23, 24, 25, 31, 33, 35, 37, 39, 41, 43, 45, 47, and 49, or a complement thereof, as determined by sequence alignment programs described elsewhere herein using default parameters.

Exemplary nucleotide sequences that encode the polypeptides encompass full-length genes as well as portions of the full-length or substantially full-length nucleotide sequences of the genes or their transcripts or DNA copies of these transcripts. Portions of a nucleotide sequence may encode polypeptide portions or segments that retain the biological activity of the native polypeptide. A portion of a nucleotide sequence that encodes a biologically active fragment of a polypeptide may encode at least about 20, 21, 22, 23, 24, 25, 30, 40, 50, 60, 70, 80, 90, 100, 120, 150, 300 or 400 contiguous amino acid residues, or almost up to the total number of amino acids present in a full-length polypeptide.

Also contemplated are variants of the nucleotide sequences. Nucleic acid variants can be naturally-occurring, such as allelic variants (same locus), homologs (different locus), and orthologs (different organism) or can be non naturally-occurring. Naturally-occurring variants such as these can be identified with the use of well-known molecular biology techniques, as, for example, with polymerase chain reaction (PCR) and hybridisation techniques as known in the art. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of a reference polypeptide. Variant nucleotide sequences also include synthetically derived nucleotide sequences, such as those generated, for example, by using site-directed mutagenesis but which still encode a polypeptide. Generally, variants of a particular nucleotide sequence will have at least about 30%, 40% 50%, 55%, 60%, 65%, 70%, generally at least about 75%, 80%, 85%, 86%, 87%, 88%, 89%, desirably about 90%, 91%, 92%, 93%, 94%, 95% or more, and more suitably about 96%, 97%, 98% or more sequence identity to that particular nucleotide sequence as determined by sequence alignment programs described elsewhere herein using default parameters.

Nucleotide sequences can be used to isolate corresponding sequences and alleles from other organisms, particularly other snakes. Methods are readily available in the art for the hybridisation of nucleic acid sequences. Coding sequences from other organisms may be isolated according to well known techniques based on their sequence identity with the coding sequences set forth herein. In these techniques all or part of the known coding sequence is used as a probe which selectively hybridises to other coding sequences present in a population of cloned genomic DNA fragments or cDNA fragments (i.e., genomic or cDNA libraries) from a chosen organism (e.g., a snake). Accordingly, the present invention also contemplates polynucleotides that hybridise to reference nucleotide sequences, or to their complements, under stringency conditions described below. As used herein, the term *"hybridises under low stringency, medium stringency, high stringency, or very high stringency conditions"* describes conditions for hybridisation and washing.

Guidance for performing hybridisation reactions can be found in Ausubel *et al., supra,* sections 6.3.1-6.3.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridisation at 42°C, and at least about 1 M to at least about 2 M salt for washing at 42°C. Low stringency conditions also may include 1% Bovine Serum Albumin (BSA), 1 mM EDTA, 0.5 M NaHPO$_4$ (pH 7.2), 7% SDS for hybridisation at 65°C, and (i) 2 x SSC, 0.1% SDS; or (ii) 0.5% BSA, 1 mM EDTA, 40 mM NaHPO$_4$ (pH 7.2), 5% SDS for washing at room temperature. One embodiment of low stringency conditions includes hybridisation in 6 x sodium chloride/sodium citrate (SSC) at about 45°C, followed by two washes in 0.2 x SSC, 0.1% SDS at least at 50°C (the temperature of the washes can be increased to 55°C for low stringency conditions). Medium stringency conditions include and encompass from at least about 16% v/v to at least about 30% v/v formamide and from at least about 0.5 M to at least about 0.9 M salt for hybridisation at 42°C, and at least about 0.1 M to at least about 0.2 M salt for washing at 55°C. Medium stringency conditions also may include 1% Bovine Serum Albumin (BSA), 1 mM EDTA, 0.5 M NaHPO$_4$ (pH 7.2), 7% SDS for hybridisation at 65°C, and (i) 2 x SSC, 0.1% SDS; or (ii) 0.5% BSA, 1 mM EDTA, 40 mM NaHPO$_4$ (pH 7.2), 5% SDS for washing at 60-65°C. One embodiment of medium stringency conditions includes hybridising in 6 x SSC at about 45°C, followed by one or more washes in 0.2 x SSC, 0.1% SDS at 60°C. High stringency conditions include and encompass from at least about 31 % v/v to at least about 50% v/v formamide and from about 0.01 M to about 0.15 M salt for hybridisation at 42°C, and about 0.01 M to about 0.02 M salt for washing at 55°C. High stringency conditions also may include 1% BSA, 1 mM EDTA, 0.5 M NaHPO$_4$ (pH 7.2), 7% SDS for hybridisation at 65°C, and (i) 0.2 x SSC, 0.1% SDS; or (ii) 0.5% BSA, 1 mM EDTA, 40 mM NaHPO$_4$ (pH 7.2), 1 % SDS for washing at a temperature in excess of 65°C. One embodiment of high stringency conditions includes hybridising in 6 x SSC at about 45°C, followed by one or more washes in 0.2 x SSC, 0.1% SDS at 65°C.

In certain embodiments, a polypeptide is encoded by a polynucleotide that hybridises to a disclosed nucleotide sequence under low, medium, high, or very high stringency conditions. One embodiment of very high stringency conditions includes hybridising 0.5 M sodium phosphate, 7% SDS at 65°C, followed by one or more washes at 0.2 x SSC, 1% SDS at 65°C.

Other stringency conditions are well known in the art and a skilled addressee will recognise that various factors can be manipulated to optimise the specificity of the hybridisation. Optimisation of the stringency of the final washes can serve to ensure a high degree of hybridisation. For detailed examples, see Ausubel *et al., supra* at pages 2.10.1 to 2.10.16 and Sambrook, J. *et al.* (2001) at sections 1.101 to 1.104.

While stringent washes are typically carried out at temperatures from about 42°C to 68°C, one skilled in the art will appreciate that other temperatures may be suitable for stringent conditions. Maximum hybridisation rate typically occurs at about 20°C to 25°C below the $T_m$ for formation of a DNA-DNA hybrid. It is well known in the art that the $T_m$ is the melting temperature, or temperature at which two complementary polynucleotide sequences dissociate. Methods for estimating $T_m$ are well known in the art (see Ausubel *et al.*, *supra* at page 2.10.8). In general, the $T_m$ of a perfectly matched duplex of DNA may be predicted as an approximation by the formula:

$$T_m = 81.5 + 16.6 \, (\log_{10} M) + 0.41 \, (\%G{+}C) - 0.63 \, (\% \text{ formamide}) - (600/\text{length})$$

wherein: M is the concentration of $Na^+$, preferably in the range of 0.01 molar to 0.4 molar; %G+C is the sum of guanine and cytosine bases as a percentage of the total number of bases, within the range between 30% and 75% G+C; % formamide is the percent formamide concentration by volume; length is the number of base pairs in the DNA duplex. The $T_m$ of a duplex DNA decreases by approximately 1°C with every increase of 1% in the number of randomly mismatched base pairs. Washing is generally carried out at $T_m$ - 15°C for high stringency, or $T_m$ - 30°C for moderate stringency.

In one example of a hybridisation procedure, a membrane (*e.g.*, a nitrocellulose membrane or a nylon membrane) containing immobilized DNA is hybridised overnight at 42°C in a hybridisation buffer (50% deionized formamide, 5 x SSC, 5 x Denhardt's solution (0.1 % ficoll, 0.1 % polyvinylpyrollidone and 0.1 % bovine serum albumin), 0.1% SDS and 200 mg/mL denatured salmon sperm DNA) containing labelled probe. The membrane is then subjected to two sequential medium stringency washes (i.e., 2 x SSC, 0.1% SDS for 15 min at 45°C, followed by 2 x SSC, 0.1% SDS for 15 min at 50°C), followed by two sequential higher stringency washes (i.e., 0.2 x SSC, 0.1% SDS for 12 min at 55°C followed by 0.2 x SSC and 0.1% SDS solution for 12 min at 65-68°C.

### 3. Preparing prothrombin activators

Prothrombin activators may be prepared by any suitable procedure known to those of skill in the art, like e.g. disclosed in WO03/082914.

For example, the prothrombin activators may be produced by any convenient method such as by purifying the polypeptide from naturally-occurring reservoirs, including but not limited to snake venom, blood and blood-derived products (*e.g.* serum). Methods of purification include affinity chromatography, including lectin (*e.g.* wheat germ agglutinin) affinity chromatography or other separation. The identity and purity of derived prothrombin activator can be determined for example by SDS-polyacrylamide electrophoresis or chromatographically such as by high performance liquid chromatography (HPLC). For example, the purification and characterisation of pseutarin C (also abbreviated to PtPA) from *P. textilis* snake venom is described in Masci (1986) and *Masci et al.* (1988), and oscutarin C (OsPA) from *O. scutellatus* venom is described in Speijer *et al.* (1986). The purification of ecarin from *E. carinatus* venom is described in Morita, T *et al.* (1981).

Alternatively, the prothrombin activators may be produced from venom gland cells in culture using methods known in the art, including for example the method described in Yamanouye, N., *et al.* (2007), which describes the primary culture of secretory cells from the venom gland of *Bothrops jararaca* for *in vitro* venom production.

Alternatively, the prothrombin activators may be synthesised by chemical synthesis, e.g. using solution synthesis or solid phase synthesis as described, for example, in Chapter 9 of Atherton and Shephard (1989) and in Roberge *et al.* (1995).

Alternatively, the prothrombin activators may be prepared by recombinant techniques. For example, the prothrombin activators used in the invention may be prepared by a procedure including the steps of: (a) preparing a construct comprising a polynucleotide sequence that encodes a polypeptide and that is operably linked to a regulatory element; (b) introducing the construct into a host cell; (c) culturing the host cell to express the polypeptide; (d) isolating the polypeptide from the host cell. If the prothrombin activator comprises a complex or two polypeptides, then the prothrombin activator may be prepared by a procedure including the steps of: (a) preparing a construct comprising a polynucleotide sequence that encodes a first polypeptide and that is operably linked to a regulatory element; (b) introducing the construct into a host cell; (c) culturing the host cell to express the first polypeptide; (d) isolating the polypeptide from the host cell; repeating steps (a) to (d) for a second polypeptide; and linking the first polypeptide and the second polypeptide. In illustrative examples, the nucleotide sequence that encodes a polypeptide encodes at least a biologically active portion of the sequences set forth in SEQ ID NO: 5, 6, 9, 10, 14, 15, 17, 19, 20, 21, 22, 23, 24, 25, 31, 33, 35, 37, 39, 41, 43, 45, 47, 49, or a variant thereof.

Recombinant prothrombin activators can be conveniently prepared using standard protocols as described for example in Sambrook, J. *et al.* (2001), in particular Chapters 16 and 17; Ausubel *et al.* (1994, *supra*), in particular Chapters 10 and 16; and Coligan et al., Current Protocols in Protein Science (John Wiley & Sons, Inc. 1995-1997), in particular Chapters 1, 5 and 6. For example, the recombinant production of snake factor V and snake factor X, which can be used to produce group C and group D prothrombin activators, is described in Filippovic, I. *et al* (2005) and Bos, M. H. A. *et al* (2009). An illustrative process for the recombinant production of ecarin and variants of ecarin is provided in Yonemura, H. *el al.* (2004) and in US patent 6,413,737.

## *4. Containers*

The present invention contemplates any suitable container for preparing a suitable serum sample. Many suitable containers are well known in the art, including those described in US patent 4,227,620; US patent 4,256,120; US patent 6,416,717; US patent 6,592,613; US patent 6,686,204; US patent 7,488,287; US patent 7,699,828; European patent 0 628 816; and commercially available containers including those used in the Examples of the present specification.

In some embodiments, the containers used in accordance with the present invention are tubes, including glass or plastic tubes. Suitable plastics include polyvinyl chloride, polypropylene, polyethylene terephthalate, and polystyrene.

The containers may be evacuated and the end sealed with an appropriate puncturable septum or cap. This allows for a double-ended needle to be used where one end is inserted into a patient's vein and the other end of the needle then punctures the septum or cap covering the end of the tube so that the vacuum in the tube draws the blood sample through the needle into the tube.

The containers may be of any suitable size. In some embodiments, the containers are designed to hold a blood sample of between 50 $\mu$L and 10 mL. Suitably, the containers are designed to hold at least 50 $\mu$L, 100 $\mu$L, 150 $\mu$L, 200 $\mu$L, 250 $\mu$L, 300 $\mu$L, 350 $\mu$L, 400 $\mu$L, 450 $\mu$L, 500 $\mu$L, 1 mL, 2 mL, 3 mL, 4 mL, 5 mL, 8 mL, or 10 mL of blood sample.

In some embodiments, the containers contain a clotting composition comprising, consisting essentially of, or consisting of 0.01 to 100 $\mu$g of prothrombin activator. Suitably, the containers contain a clotting composition comprising, consisting essentially of, or consisting of 0.1 to 10 $\mu$g of prothrombin activator. In representative embodiments, the containers contain a clotting composition comprising, consisting essentially of, or consisting of 0.1 to 10 $\mu$g of prothrombin activator and hold a 4 mL blood sample providing a final concentration of prothrombin activator in the 4 mL blood sample of 25 ng/mL to 2.5 $\mu$g/mL.

In some embodiments, the containers contain a clotting composition comprising, consisting essentially of, or consisting of 0.0005 to 15 $\mu$g/mL of prothrombin activator. Suitably, the containers contain a clotting composition comprising, consisting essentially of, or consisting of 0.005 to 10 $\mu$g of prothrombin activator.

In some embodiments, the containers contain a clotting composition comprising, consisting essentially of, or consisting of 0.0005 to 15 U/mL of prothrombin activator. Suitably, the containers contain a clotting composition comprising, consisting essentially of, or consisting of 0.0015 to 10 U/mL of prothrombin activator. In some embodiments, a unit meansurement is a prothrombin activator unit measurement that is defined as follows: 1 unit will activate prothrombin to produce one unit of amidolytic activity at pH 8.4 at 37 °C, and 1 amidolytic unit will hydrolyzs 1.0 $\mu$mole of N-p-tosyl-Gly-Pro-Arg-p-nitroanilide per min at pH 8.4 at 37 °C.

In some embodiments, the clotting composition may be contained within the container before the blood sample is added to the container. In some embodiments, the clotting composition may be added to the container after the blood sample is added to the container.

Where the clotting composition is contained within the container before the blood sample is added, it may have been added to the container by any suitable method known in the art. In some embodiments, the clotting composition is dissolved into a suitable solvent and is then added to the container and dried onto the inner surface of the container. The solvent may be a neutral buffer. The clotting composition in solution may be dried onto the inner surface of the container by spray-drying or by freeze-drying or by any other suitable method known in the art. In some other embodiments, the clotting composition is dissolved into a suitable solvent and added to the container without drying so that the container contains an aqueous solution comprising the clotting composition. The solvent may be a neutral buffer.

In some embodiments, beads are coated with the clotting composition and these beads are added to the container. The beads may be glass beads or synthetic resinous beads, including polystyrene and propylene beads. The beads may have a spherical shape. In some embodiments, the mean diameter of the beads is between 0.1 mm and 1 mm.

In some embodiments, the container provides for separation of the serum from the clotted cells after clotting has occurred. In some embodiments, the container comprises or contains a gel that provides a barrier between the clotted cells and the serum sample. In some embodiments, the container is a suitable shape and a suitable material to permit centrifugation to separate or assist in maintaining separation of the clotted cells and the serum sample. In some embodiments, the serum sample is removed from the clotted cells, or the clotted cells are removed from the serum sample.

In some embodiments, the container may comprise one or more further components. The other components may include, for example, one or more co-factors, one or more surfactants, and/or one or more clotting agents in addition to the clotting composition.

### 5. Further components

The clotting composition described herein consists of, consists essentially of, or comprises a prothrombin activator as herein defined.

As used in the above statement and in similar statements elsewhere in this specification, the term "*comprises*" (and the like) means the clotting composition includes the prothrombin activator and may also include any one or more further components. Thus, the prothrombin activator is a mandatory component, and any other components are optional and may or may not be present. Other components may include, for example, one or more co-factors, one or more surfactants, and/or one or more additional clotting agents.

As used in the above statement and in similar statements elsewhere in this specification, the term "*consists essentially of*" (and the like) means that the clotting composition includes the prothrombin activator, and may also include one or more other components, provided those components do not interfere with or contribute to the activity or action of the prothrombin activator. Thus, the prothrombin activator is a mandatory component, and other components are optional and may or may not be present, depending upon whether or not they affect the activity or action of the prothrombin activator.

As used in the above statement and in similar statements elsewhere in this specification, the term "*consists of*" (and the like) means that the clotting composition includes, and is limited to, the prothrombin activator. Thus, the phrase "*consists of*" indicates that the prothrombin activator is a mandatory component, and that no other components (such as co-factors or clotting agents) may be present.

Accordingly, in some embodiments, the clotting composition may comprise snake venom, including but not limited to crude snake venom. In some other embodiments, the clotting composition may comprise a preparation of prothrombin activator prepared by partial or full purification of snake venom. Such preparations may be prepared by any suitable method known in the art, including chromatographic and gel filtration methods, including those described herein, and elsewhere. In some other embodiments, the clotting composition may comprise a purified prothrombin activator or isolated prothrombin activator. Purified and isolated prothrombin activators may be prepared by any suitable method known in the art, including those described herein, and elsewhere.

#### 5.1 Co-factors

The ability of prothrombin activators as herein defined to activate prothrombin to thrombin may be improved with the addition of co-factors, including but not limited to calcium, phospholipid(s) and polypeptides comprising FVa activity.

#### 5.2 Surfactants

Suitable surfactants include any suitable surfactant, including but not limited to sodium dodecyl sulphate (SDS), ammonium lauryl sulphate, sodium laureth sulphate, and sodium myreth sulphate.

#### 5.3 Clotting agents

Clotting agents or coagulants are classified as either intrinsic clotting agents or extrinsic clotting agents according to the blood cascade stimulated (see for example US patent 6,686,204).

Suitable clotting agents include, but not limited to, diatomaceous earth, microparticles or particles of inorganic silicates, microsilica, glass microparticles, ellagic acid, thrombin, heparinase, thromboplastin, batroxobin, hydroyapitite, kaolin, kaolin particles, prothrombin (including microparticulated prothrombin), fibrinogen, and depolymerised collagen.

### 6. Serum samples

As discussed above, the present invention is predicated in part on the discovery that prothrombin activators as defined herein are suitable clotting agents for preparing serum samples that are suitable for detecting analytes. A serum sample that is suitable for detecting analytes is one of suitable quality as discussed herein, and/or one that is prepared within a suitable time as discussed herein.

#### 6.1 Serum quality

An important factor in the preparation of a serum sample suitable for detecting analytes is the extent to which the clotting process removes fibrinogen from the serum. Serum containing residual fibrinogen or partially degraded fibrinogen, or fibrin as a result of incomplete clotting can lead to analytical accuracy problems because of the formation of precipitates (microclots or strings), latent clotting post-centrifugation and on storage of the serum. Hence complete or substantially complete clotting is pivotal in ensuring highest quality serum and subsequent result accuracy is obtained.

Accordingly, some embodiments of the present invention provide the use of a clotting composition comprising, consisting essentially of, or consisting of a prothrombin activator in the preparation of a serum for detecting an analyte, where the serum comprises ≤ 30 μg/mL of fibrinogen or fibrinogen/fibrin related products. In more specific embodiments, the serum comprises ≤ 25 μg/mL, ≤ 20 μg/mL, ≤ 15 μg/mL, ≤ 10 μg/mL, ≤ 8 μg/mL, or ≤ 6 μg/mL of fibrinogen or fibrinogen/fibrin related products.

In some embodiments, the serum comprises ≤ 30%, ≤ 20%, ≤ 10%, ≤ 9%, ≤ 8%, ≤ 7%, ≤ 6%, ≤ 5%, ≤ 4%, ≤ 3%, ≤ 2%, ≤ 1%, ≤ 0.5%, ≤ 0.2%, ≤ 0.1% of fibrinogen or fibrinogen/fibrin related products present in the original sample from which the serum was produced.

Levels of fibrinogen and/or fibrinogen/fibrin related products can be detected by any suitable method known in the art, including a sandwich immunoassay using antibodies from MP Biomedicals and standard fibrinogen preparations purchased from NIBSC, Potters Bar, Hertsfordshire, London, UK.

Another important factor in the preparation of a serum sample suitable for detecting analytes is the activity or number of cells or cellular debris that remain in the serum after clotting. The presence of cells can have two effects during storage and analysis of serum or plasma. Firstly, cells may lyse, releasing cellular contents (*e.g.* potassium, lactate dehydrogenase) into the serum or plasma. This can lead to significant differences between measurements made immediately after centrifugation and measurements after a period of storage. Secondly, cells continue to be metabolically active and may use up significant amounts of nutrients (*e.g.* glucose) and release metabolic products (*e.g.* lactate) on storage. Changes can even be observed in the samples of many tubes when the samples are stored for the usual recommended 30 minute clotting time when the samples are from healthy participants. The degree of cellular contamination is therefore an important quality criterion for serum samples and an important advantage of using serum over plasma.

Accordingly, in some embodiments, the serum sample comprises less than 50%, 40%, 30%, 20%, 10%, 5%, or 1% of cells in the blood sample from which it has been prepared.

In some embodiments, the serum sample comprises a change of lactate dehydrogenase activity or phosphate concentration (typically measured in U/L and mmol/L respectively) of < 25%, < 20%, < 15% or < 10% over a period of 24 hours, 12 hours, 8 hours, 6 hours, 5 hours, 4 hours, 3 hours, 2 hours, 1 hour or 30 minutes. In some embodiments, the serum sample comprises a change of glucose concentration or potassium concentration (both typically measured in mmol/L) of < 5%, < 4%, < 3%, < 2%, < 1%, < 0.5%, or < 0.1% over a period of 24 hours, 12 hours, 8 hours, 6 hours, 5 hours, 4 hours, 3 hours, 2 hours, 1 hour or 30 minutes (for example, from the time of preparing the serum sample). Methods for measuring lactate dehydrogenase activity are well known in the art, see, for example, Dimeski, G., *et al.* (2004).

The haemoglobin concentration of a serum sample can also be used to determine whether the serum sample is suitable for detecting analytes. Accordingly, in some embodiments, the serum sample comprises a haemoglobin concentration of < 150 mg/L, < 100 mg/L, < 90 mg/L, < 80 mg/L, < 70 mg/L, < 60 mg/L, < 50 mg/L, < 40 mg/L, < 30 mg/L, < 20 mg/L, or < 10 mg/L.

6.2 Clotting time

As a sample for testing, serum is usually preferred over plasma unless urgent results are required and thus the clotting time for a serum tube is considered too long. Another downside to prolonged clotting time is that it can lead to clinically significant analyte concentration changes due to cellular activity in the blood sample, this problem being most pronounced in leukocytosis.

Thus in some embodiments, the present invention provides a method of producing a serum sample for detecting an analyte of interest, the method comprising contacting a blood sample with a clotting composition comprising, consisting essentially of, or consisting of a prothrombin activator as defined herein, where the serum sample is prepared within 25, 20, 15, 10, 8, 6, 5, 4, 3, 2, 1, or 0.5 minutes from contact with the clotting composition.

*7. Blood samples*

As discussed herein, there is a desire to provide a clotting composition that is suitable for producing a serum sample from all blood samples, or a container comprising a clotting composition that will clot all blood samples, in a suitable time.

Examples of different types of blood sample for which testing may be desired include blood from healthy individuals, citrated blood, blood with EDTA added, blood from patients on anti-clotting therapy such as heparin, warfarin, citrate, or rivaroxaban, patients taking anti-thrombotic agents including aspirin, thrombocytopenic patients (patients with low platelet counts), and patients with prolonged aPTT.

In some embodiments, the blood sample is a whole blood sample. In some other embodiments, the blood sample is a serum sample derived from a whole blood sample. Exemplary serum samples in this instance include serum samples where a better quality serum sample is desired, including those where the amount of fibrinogen or fibrinogen/fibrin related products and/or the amount of cells or cellular material in the serum sample and/or the amount of haemoglobin is considered too high for the serum sample to be a sample suitable to detect analytes. For example, the serum sample may exhibit microclots or latent clotting. In some other embodiments, the blood sample is a plasma sample derived from a whole blood sample. For example, the plasma sample may exhibit microclots or latent clotting.

### 8. Detecting analytes

In some embodiments the present invention further provides methods of detecting an analyte, the method comprising analysing a serum sample prepared by the method of the present invention for the presence or amount of the analyte of interest.

In specific embodiments, the serum sample prepared by the method of the present invention is suitable for more than one analyte test, so that the serum sample can be used to detect more than one analyte. As discussed herein, often a clinician will desire more than one analyte test to be performed on a blood sample from a patient, and it is not uncommon for one serum sample to be used for at least 20 tests, or even more, sometimes between 50-60 or even 70-80 tests. It will be appreciated by those skilled in the art that in specific embodiments the present invention provides for the production of a serum sample where the serum sample is of sufficient volume and quality to enable all desired analyte tests to be performed on the one serum sample. The advantage of this is that both the volume of blood to be taken from the subject and the time taken to perform the analyte tests are reduced.

Illustrative analyte tests are described below. Methods for performing these analyte tests may be performed in a number of ways and are well known in the art.

8.1 Sodium (Na$^+$)

This test measures the amount of sodium in a serum or plasma sample. Sodium plays an important role in salt and water balance in the body. Low sodium levels may indicate too much water intake, heart failure, kidney failure, or loss of sodium from the body due to diarrhea or vomitting. High sodium levels may indicate too much salt intake or insufficient water intake.

8.2 Potassium (K$^+$)

This test measures the amount of potassium in a serum or plasma sample. Levels of potassium that are too high (hyperkalaemia) may be the result of kidney disease, diabetes, ketoacidosis or drugs that decrease the amount of potassium excreted from the body. Levels of potassium that are too low (hypokalaemia) may be caused by dehydration, for example from diarrhoea or vomiting, or excessive sweating. Levels of potassium may also be low as a result of taking drugs that cause the kidneys to lose potassium, for example diuretics.

Potassium levels are often monitored in those patients that take diuretics or heart medications, those with high blood pressure or kidney disease, critical acidosis and alkalosis conditions, and those receiving kidney dialysis or intravenous therapy on a drip.

8.3 Chloride (Cl$^-$)

This test measures the amount of chloride in serum or plasma. Chloride is typically measured to assess whether there is an electrolyte imbalance in the patient. Low chloride and normal sodium can be indicative of vomiting or loss of gastric fluid.

8.4 Bicarbonate (HCO$_3^-$)

This test measures the amount of three forms of carbon dioxide (bicarbonate, carbonic acid, and dissolved carbon dioxide) in serum or plasma. This test is often performed if the patient is having breathing problems. A high level of carbon dioxide may be caused by some diseases including chronic obstructive pulmonary disease, emphysema, pneumonia, Cushing's syndrome or alcoholism, or vomiting. A low level may be caused by some diseases including pneumonia, cirrhosis, hyperventilation, diabetes, dehydration, kidney or heart failure.

### 8.5 Glucose Gluc)

This test measures the amount of glucose in serum or plasma. Glucose levels are often tested in those patients exhibiting symptoms of high blood glucose (hyperglycaemia) or hypoglycemia, those who are pregnant, those who have diabetes.

### 8.6 Urea (Urea)

This test measures the amount of urea in serum or plasma. This test can help evaluate kidney function and monitor the effectiveness of dialysis.

### 8.7 Creatinine (Creat)

This test measures the amount of creatinine in serum or plasma. This test is pivotal in helping to evaluate kidney function and monitor treatment of kidney disease.

### 8.8 Urate (Urate)

This test measures the amount of urate (or uric acid) in serum or plasma. High levels of uric acid may be a sign of gout. Uric acid levels are also monitored in patients that are undergoing chemotherapy or radiotherapy to detect tumour lysis syndromes.

### 8.9 Total protein (TP or T Prot)

This test measures the total amount of protein in serum or plasma. Although the results of a total protein test will not indicate a specific disease, a high or low protein level often indicates that additional tests are required to determine if there is a problem. Total protein tests are often used to screen for certain liver disorders, kidney disorders, multiple myeloma and hydration status.

### 8.10 Albumin (Alb)

This test measures the amount of albumin in serum or plasma. Albumin levels are often measured to screen for liver or kidney disease, or to evaluate nutritional status, especially in hospitalised patients.

### 8.11 Total Bilirubin (T Bili)

This test measures the amount of bilirubin in serum or plasma. Bilirubin levels are measured to screen for and monitor liver disorders, such as jaundice, or liver diseases, such as cirrhosis. Bilirubin levels are also measured in babies to help detect certain rare genetic disorders and to avoid brain damage in those babies with jaundice.

### 8.12 Alkaline Phosphatase (ALP)

This test measures the amount of alkaline phosphatase in serum or plasma. This test is typically performed to screen for or monitor treatment of a liver or bone disorders.

### 8.13 Gamma-Glutamyl Transferase (GGT)

This test measures the amount of gamma-glutamyl transferase in serum or plasma. This test is used to screen for liver disease and alcohol abuse. It can also be used to determine if a raised level of ALP is due to liver or bone disease.

### 8.14 Alanine Aminotransferase (ALT)

This test measures the amount of alanine aminotransferase in serum or plasma. This test is used to screen for liver disease.

### 8.15 Aspartate Aminotransferase (AST)

This test measures the amount of aspartate aminotransferase in serum or plasma. This test is used to detect liver damage, muscular damage, and other conditions as the enzyme is present in many organs and tissue cells.

### 8.16 Lactate Dehydrogenase (LD)

This test measures the amount of lactate dehydrogenase in serum or plasma. This test is typically used to identify the cause and location of tissue damage in the body, tissue ischemia, and to monitor its progress.

### 8.17 Creatine Kinase (CK)

This test measures the amount of creatine kinase in serum or plasma. Creatine kinase is measured in patient's with chest pain or muscle pain or weakness to determine if they have had a heart attack and if other muscles in the body have been damaged.

### 8.18 Total Calcium (TCa)

This test measures the amount of calcium in serum or plasma. Calcium levels are often measured in patients with kidney, bone or nerve disease, or when symptoms of significantly increased or decreased calcium are present.

### 8.19 Phosphate (Pi or Phos)

This test measures the amount of phosphate in serum or plasma. Phosphate levels may be measured as a follow-up to a test result of abnormal calcium levels. Phosphate levels may also be measured in patients with kidney disorders, uncontrolled diabetes, or where the patient is taking calcium or phosphate supplements.

### 8.20 Magnesium ($Mg^{2+}$)

This test measures the amount of magnesium in serum or plasma. This test may be performed if the patient has symptoms of too much or too little magnesium, including weakness, irritability, cardiac arrhythmia, nausea or diarrhoea. Magnesium levels may also be measured if abnormal calcium or potassium levels have been detected.

### 8.21 Lipase (Lipase)

This test measures the amount of lipase in serum or plasma. This test is typically used to diagnose pancreatitis or other pancreatic diseases.

### 8.22 Cholesterol (Chol)

This test measures the amount of cholesterol in serum or plasma. Cholesterol levels are measured to screen for risk of developing heart disease.

### 8.23 Triglycerides (Trig)

This test measures the amount of triglycerides in serum or plasma. As for cholesterol levels, this test is typically used to screen for risk of developing heart disease.

### 8.24 High-Density Lipoprotein Cholesterol (HDL-C or HDL)

This test measures the amount of HDL cholesterol in serum or plasma. This test is typically used to determine the risk of developing heart disease.

### 8.25 Iron ($Fe^{2+}$)

This test measures the amount of iron in serum or plasma. Iron is measured to check if a patient has low or high iron levels. Low iron levels can cause anaemia, and is usually due to long-term or heavy bleeding, pregnancy or rapid growth (in children). High iron levels can be due to a genetic condition or extensive blood transfusions.

### 8.26 Transferrin (Trf)

This test measures the amount of transferrin in serum or plasma. Transferrin is a plasma protein that transports iron through the blood to the liver, spleen and bone marrow. Thus the blood transferrin level is tested to determine the cause of anaemia, to examine iron metabolism (for example, in iron deficiency anaemia) and to determine the iron-carrying capacity of the blood.

### 8.27 C Reactive Protein (CRP)

This test measures the amount of C reactive protein in serum or plasma. This test is used to identify the presence of inflammation, to determine its severity, and to monitor response to treatment.

### 8.28 Cortisol (Cortisol)

This test measures the amount of cortisol in serum or plasma. Cortisol levels are measured to help diagnose Cushing's syndrome or Addison's disease.

### 8.29 Free Thyroxine (fT4)

This test measures the amount of free thyroxine in serum or plasma. The test is typically used to diagnose hypothyroidism or hyperthyroidism.

### 8.30 Thyroid Stimulating Hormone (TSH)

This test measures the amount of thyroid stimulating hormone in serum or plasma. The test is typically used to screen for, diagnose and monitor thyroid disorders.

### 8.31 Ferritin

This test is used to measure ferritin in serum or plasma. Low ferritin levels are indicative of iron deficiency. Elevated levels are indicative of iron overload such as in haematochromatosis.

### 8.32 Troponin (TnI)

This test measures the amount of troponin in serum or plasma. This test is typically used in a patient with chest pains to determine if the patient has had myocardial damage.

### 8.33 Haemolytic Index

The haemolytic index test measures the degree of red cell lysis. Haemolysis is the most common interference encountered in a biochemistry laboratory. The test is predominantly used to detect *in vitro* haemolysis and sample suitability for reporting of certain or all analytes, and in detection of haemolytic anaemias (hereditary spherocytosis, spontaneous haemolysis, RBC enzyme deficiency). Haemolysis or haemolytic index (concentration of free haemoglobin in serum or plasma) is currently estimated by all general chemistry analysers. The value is then used as a guide in determining which analytes and at what haemolysis level may be affected or not reported.

### 8.34 Icteric Index

The icteric index test returns a value indicating the relative level of bilirubin in a test sample by a purely spectrophotometric method. It is used in determining sample suitability for reporting of certain analytes and cross checking accuracy of bilirubin results in rare cases of interference with the total bilirubin photometric estimation methods. The icteric index has been shown to be of value in detecting cancer paraproteins interference (precipitation and false high total bilirubin) with Roche Total Bilirubin method (Sheppard *et al*., 2005), where the icteric index has stayed unaffected. Bilirubin can interfere with some creatinine assays at high concentration (*e.g.* >200 $\mu$M/L) as discussed in Dimeski *et al*., 2008.

8.35 Lipemia Index

The lipemia index has been employed to predict possible interference with assays due to lipaemia.

## 9. Methods or diagnosis

The present invention also provides methods of diagnosing the presence, absence or severity of a disease or condition in a subject, wherein the presence, absence or severity of the disease or condition is associated with the presence, absence or an aberrant amount of an analyte of interest in the subject. These methods generally comprise providing a serum sample prepared according to the methods broadly described above; and detecting the presence, absence or aberrant amount of the analyte in the serum sample to thereby determine the presence, absence or severity of the disease or condition in the subject.

In some embodiments, the methods of the present invention involve comparing the result of the analyte test to a reference result in order to obtain the diagnosis.

The disease or condition may be any suitable disease or condition that can be diagnosed using a serum sample, including but not limited to, the diseases or conditions outlined above with reference to different analyte tests.

In some embodiments, the methods may comprise diagnosing the presence or absence of a disease or condition not previously presented by the subject. In other embodiments, the methods may comprise diagnosing the presence, absence or severity of a disease or condition that the subject has previously presented. The methods may comprise reference to a result obtained from the subject at an earlier time. Alternatively, the reference result may be a standard analytical reference.

In some embodiments, the methods are performed in a testing facility such as a pathology laboratory. In some other embodiments, the methods are "point-of-care" methods. As used herein, a "*point-of-care method*" means that the method is performed at or near the site of patient care. Point-of-care methods are increasingly popular in hospital and other environments where there is a desire to obtain fast results, and is often accomplished through the use of transportable, portable, and hand-held instruments and test kits.

The advantages of point of care testing include the ability to obtain rapid analytical results at the bedside in hospitals, especially in emergency situations and the ability to obtain analytical results at home or in doctors' surgeries (*e.g.*, using droplets of capillary blood obtained by skin puncture).

Devices for point-of-care devices currently available on the market include the immunoassay analyser sold by Siemens called Siemens' DCA Vantage Analyzer, the Retro-STATUS HIV/CD4 350 rapid test device sold by Millenium Biotechnology, Inc. which is used to simultaneously determine a patient's HIV infection status as well as their current immune status, and the Triage PLGF test sold by Alere International which is used for detecting early onset pre-eclampsia.

## 10. Research tools

The present invention also contemplates the use of research tools that employ serum samples produced in accordance with the present invention. These methods generally comprise providing a serum sample prepared according to the methods broadly described above; and employing the serum sample in a research tool study, including but not limited to a genomics, proteomics, metabolomics, systems biology, molecular imaging or assay study.

Suitable research tools are well known in the art and include those described in Scaros, O. *et al*., 2005. Genomics includes pharmacogenomics which studies the correlation between genetics and gene expression patterns with response to therapeutics. Proteomics permits the analysis of the abundance and distribution of proteins in a system. Metabolomics, or biochemical profiling, is the study of metabolites in a system. Systems biology looks at the entire biological system as a functional unit, producing models of behaviour that can potentially predict hwo that system will respond to stimulus. Molecular imaging technologys have the ability to demonstrate both the level of a specific molecular target and the functional state of that target *in vivo,* and can be used for diagnostic methods.

In order that the invention may be readily understood and put into practical effect, particular preferred embodiments will now be described by way of the following nonlimiting examples.

**EXAMPLES**

EXAMPLE 1: PURIFICATION AND CHARACTERISATION OF PROTHROMBIN ACTIVATORS FROM SNAKE VENOM

Example 1a: Purification and characterisation of ecarin, carinactivase-1 and carinactivase-2 from *Echis carinatus* venom

This example describes the purification and characterisation of a group A prothrombin activator termed ecarin and two group B prothrombin activators termed carinactivase-1 (CA-1) and carinactivase-2 (CA-2) from the venom of *Echis carinatus.*

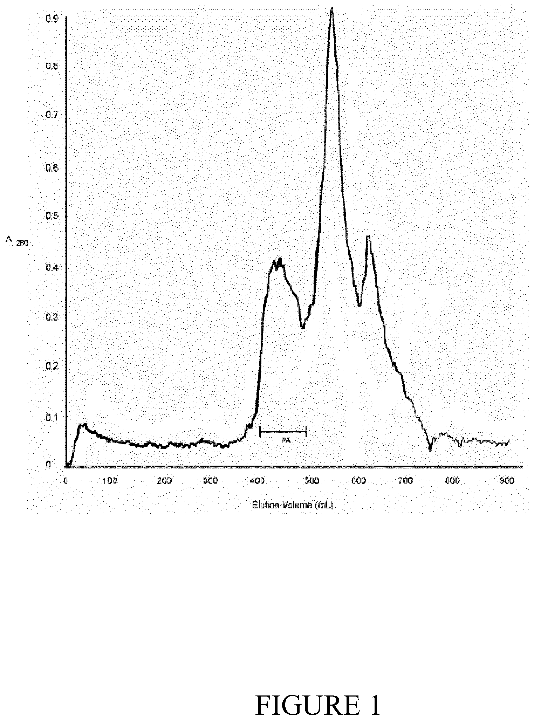

Freeze-dried *Echis carinatus* venom (157 mg; Sigma Chemical Co, USA; cat no. V8250) was dissolved in 8 mL of 0.05 M Tris-HCl pH 8.0 buffer and left standing to allow to dissolve for 30 minutes and then centrifuged (1000 g and 10 minutes) to remove insoluble materials. A volume of 7.7 mL of the reconstituted clarified solution was loaded onto a Superdex 200 gel filtration chromatography column (cat no 17-1043-02) (2.5 x 95 cm) GE Healthcare Bio-Sciences, Sweden) using the same Tris-HCl buffer for the elution. The column flow rate was 20 mL/hr and 4 mL fractions were collected (15 minute fraction). The active fractions were determined by their clotting activity with normal citrated plasma as described in more detail below. The pooled fractions exhibiting prothrombin activator activity contained a total of 100 mL with a total $A_{280}$ of 28.3 units. Figure 1 shows the elution profile in the isolation of ecarin, carinactivase-1, and carinactivase-2 from *E. carinatus* venom using gel filtration on a Superdex 200 column as described above. The pooled fractions of the three prothrombin activators (ecarin, carinactivase-1, and carinactivase-2) are indicated by the bar in Figure 1.

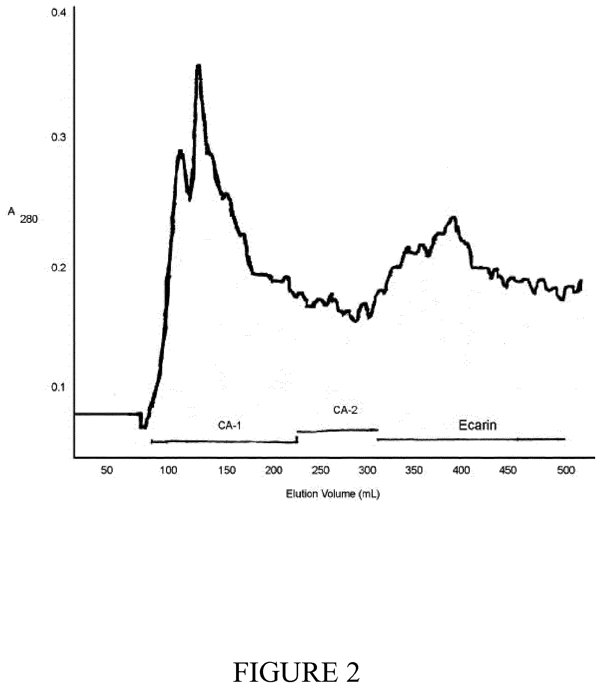

These pooled fractions were immediately applied to a column of Blue Sepharose (2.0 x 12 cm) (Cibacron Blue 3G attached to Sepharose 6 Fast Flow; cat no. 17-0948-01) (GE Healthcare Bio-Sciences, Sweden) pre-equilibrated with the same 0.05 M Tris-HCl buffer pH 8.0. After washing with initial buffer of approximately 2 column volumes, the bound material was eluted with a linear gradient of NaCl in the same buffer (0-1.0 M; 200 mL of each beaker). Carinactivase-1 was recovered in the unbound fractions and carinactivase-2 and ecarin were eluted at 0.2 and 0.5 M NaCl respectively. Prothrombin activator activities were identified by normal citrated plasma clotting assay in the presence of 30 mM calcium and in the absence of added calcium as described in more detail below. Fractions (three mL each) with specific prothrombin activator activities were pooled by their respective specific clotting activity of the clotting times followed by concentrating to 1 mg/mL using a YM 10 membrane in an Amicon stirred cell concentrator Model no. 42. All chromatography was performed at 4 °C using an Gilson peristaltic pump using black to purple chamber set at the required flow rates, an Altex dual wave UV detector set at two attenuation of 280 nm and an LKB 7000 ULTRORAC fraction collector using a time-base fraction collection. Figure 2 shows the elution profile of this Blue Sepharose chromatography, and the bars indicate the pooled fractions of carinactivase-1, carinactivase-2, and ecarin. The fractions obtained were stored at either 4°C, -20°C in buffer or -20 °C in 50% glycerol-buffer. These fractions (preparations) containing prothrombin activators were used in all subsequent experiments, except where otherwise indicated.

The prothrombin activator activity was assayed using the method described by Masci *et al.*, 1988. Essentially, this assay method involved coagulation assays performed using a Hyland-Clotek instrument as described by Austen, D. E., *et al.* 1975. Fresh-pooled citrated plasma from normal volunteers was used for each group of experiments. The clotting time assays for the preparations were performed based on the criteria defined for group A and group B prothrombin activators in Kini, R. M., 2005. The assays were carried in the presence of calcium (30 mM $Ca^{2+}$) to identify the group B prothrombin activators (carinactivase-1 and carinactivase-2) and in the absence of added calcium to identify the group A prothrombin activator (ecarin). The hydrolytic activity against the peptide p-nitroanilide S-2238, measuring the formation of thrombin generation by prothrombin activators, was determined by equilibration of 0.90 mL of 0.02 M Tris-HCl buffer, pH 7.4, with or without 10 mM $CaCl_2$, and 100 $\mu$L of S-2238 (3 mM in water) in the cell compartment of a spectrophotometer (Hitachi U2800) at 25°C. The reaction was initiated by addition of a 25 $\mu$L aliquot of prothrombin activator preparation to prothrombin (250 nM), and the release of p-nitroaniline was measured at 405 nm. One unit of activity is equivalent to the hydrolysis of 1 pmol of substrate/minute. Assays using other peptide nitroanilides as substrate were performed in the same way, with a final concentration of 100 $\mu$M.

Example 1b: Purification and characterisation of PtPA from *Pseudonaja textilis* venom and OsPA from *Oxyuranus scutellatus* venom

This example describes the purification and characterisation of group C prothrombin activators from the venom of *Pseudonaja textilis* and *Oxyuranus scutellatus* snakes. These prothrombin activators are abbreviated as PtPA and OsPA respectively.

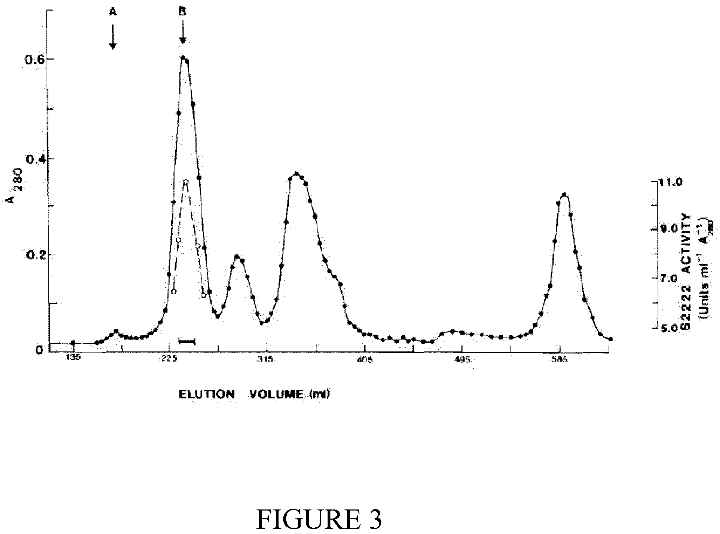

Dried lyophilised *P. textilis* venom was reconstituted in 0.05 M Tris-HCl with a pH of 7.4. Figure 3 provides an elution profile of the reconstituted *P. textilis* venom on a Sephracryl S-300 column in 0.05 M Tris-HCl buffer at pH 7.4. 'A' refers to void volume and 'B' denotes elution position (Ve) for PtPA which is 250 mL on this column and equates to an approximate molecular mass of 250 kDa. The absorbance at 280 nm ($A_{280}$) under 'B' represents 40% of the total $A_{280}$ units applied to the column. A similar gel filtration experiment with *O. scutellatus* venom showed that approximately 10% of the total $A_{280}$ loaded eluted in the 250 kDa peak (corresponding to the prothrombin activator).

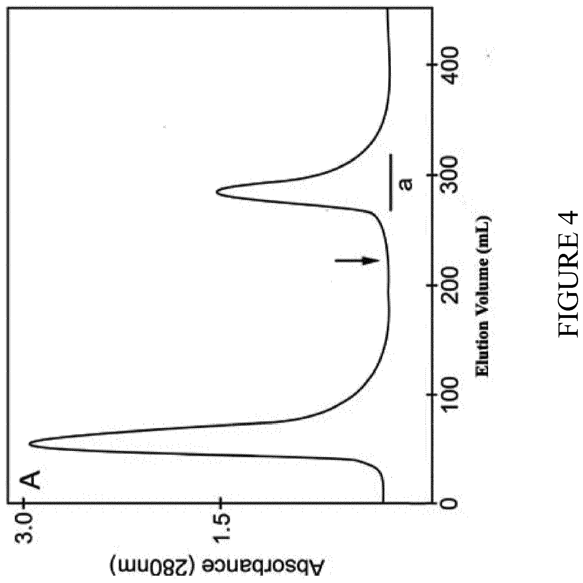

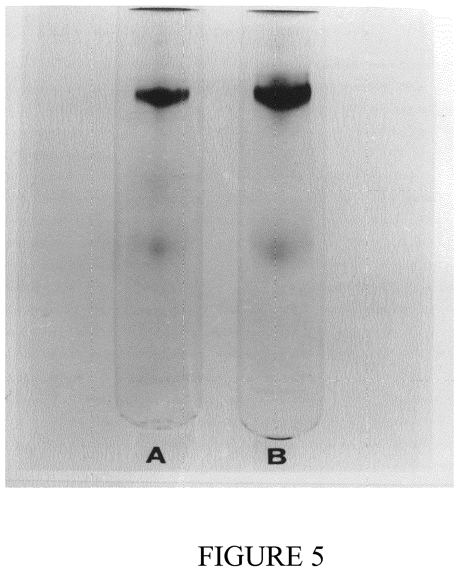

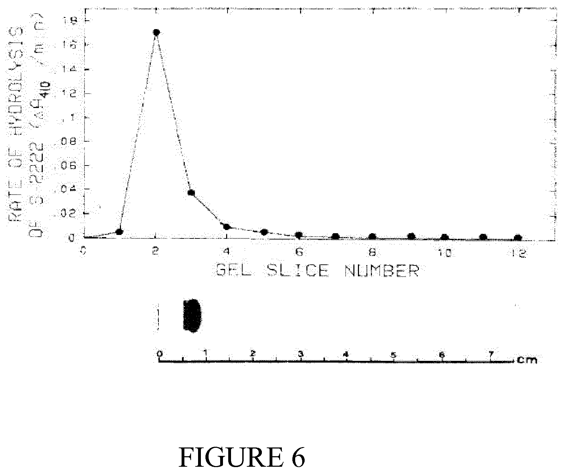

Affinity chromatography using Con A-Sepharose 4B in 0.05 M Tris-HCl, pH 7.4, was used to purify the prothrombin activator from the crude *P. textilis and O. scutellatus* venom. The eluting buffer was 0.2 M methyl $\alpha$-D-mannopyranoside with 0.01% sodium azide. The elution profile on Con A-Sepharose for PtPA is shown in Figure 4. The arrow indicates the application of eluting buffer and "a" indicates the fractions pooled to provide prothrombin activator. Figure 5 shows the results of native PAGE at pH 8.6. Peak "a" from Figure 4 showed a major protein band and a trace minor band at loadings of 25 μg (A) and 50 μg (B) per lane. The 250 kDa band was excised from the gel in a parallel experiment and shown to have Factor Xa-like activity against the chromogenic substrate S-2222, confirming its identification as PtPA (Figure 6). A similar pattern of elution was obtained for OsPA.

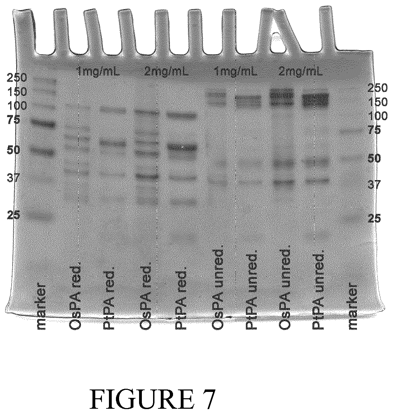

PtPA and OsPA were characterised further by SDS-PAGE in the presence (designated "red") and absence ("unred") of β-mercaptoethanol (Figure 7). It is evident that several bands are present for both PtPA and OsPA representing component polypeptide chains.

PtPA- and OsPA-containing preparations prepared in accordance with this method were used in all subsequent experiments, except where otherwise indicated.

Example 1c: Purification and characterisation of notecarin from *Notechis scutatus* venom

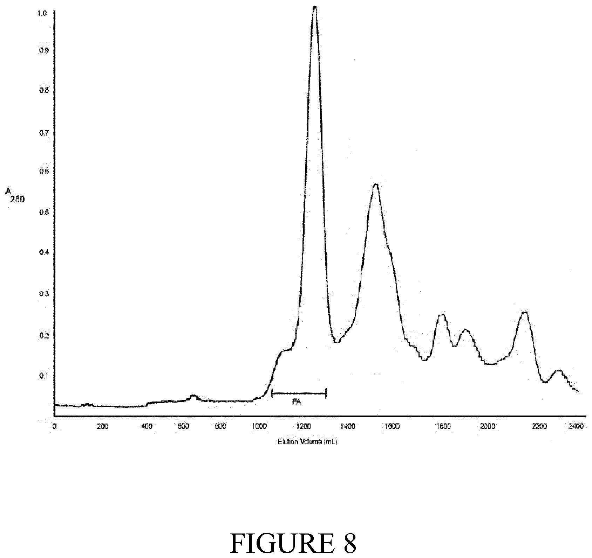

The group D prothrombin activator termed notecarin was purified from the venom of *Notechis scutatus,* using Sephacryl S-300 gel filtration chromatography (5.0 x 9.5 cm) in a similar manner to that described in Example 1b above for the group C prothrombin activators. The elution profile is shown in Figure 8, where "PA" refers to the fraction containing notecarin.

Notecarin-containing preparations prepared in accordance with this method were used in all subsequent experiments, except where otherwise indicated.

Example 1d: Further characterisation of carinactivase-1, carinactivase-2, ecarin, PtPA, OsPA, and notecarin

The prothrombin activator preparations prepared in Examples 1a, 1b, and 1c were further characterised as follows.

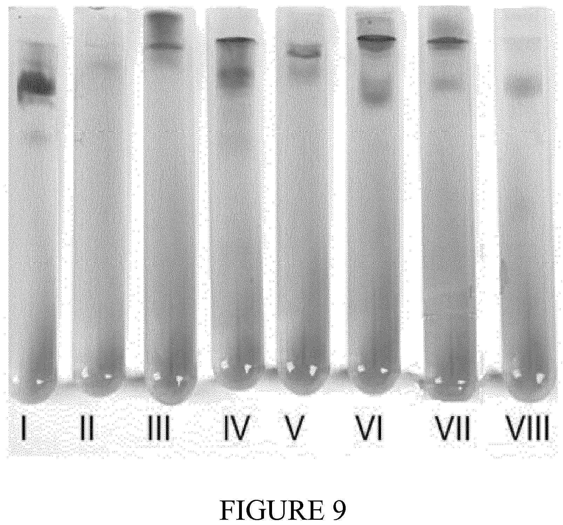

Native PAGE of prothrombin, alpha-thrombin, and the prothrombin activators at pH 8.9 was performed and the results are shown in Figure 9, wherein the labels are as follows: (I) prothrombin; (II) alpha-thrombin; (III) ecarin; (IV) carinactivase-1; (V) carinactivase-2; (VI) PtPA; (VII) OsPA; (VIII) notecarin (20 μg of each prothrombin activator was loaded).

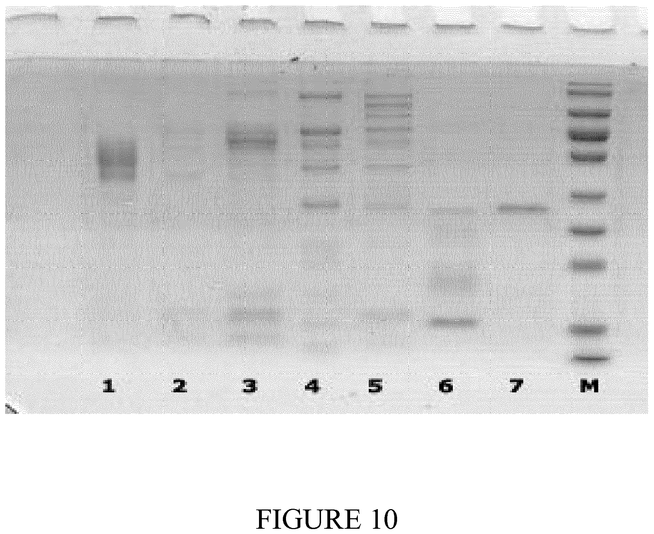

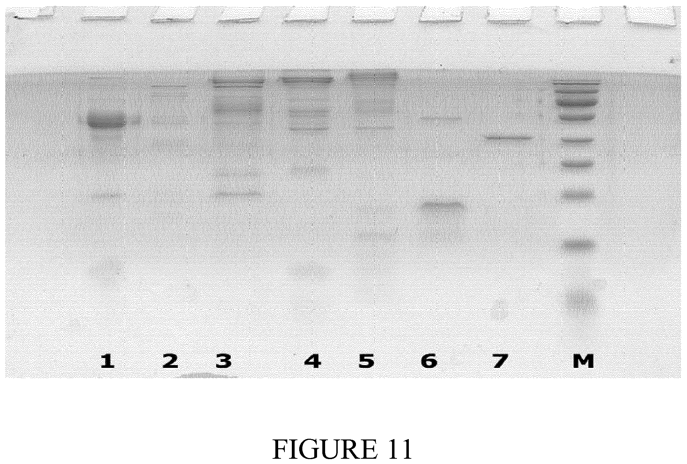

The prothrombin activator preparations were also characterised further by SDS-PAGE in the presence (Figure 10) and absence (Figure 11) of β-mercaptoethanol. In each Figure, the lanes are as follows: (1) carinactivase-1; (2) carinactivase-2; (3) ecarin; (4) PtPA; (5) OsPA; (6) notecarin; (7) thrombin; and (M) molecular weight marker. The PtPA-containing sample in lane (4) and the OsPA-containing sample in lane (5) were the same as those used for the results shown in Figure 7.

The SDS-PAGE shows that in each case the protein bands in the expected regions are present but also that the preparations are not homogeneous. The protein concentrations of the prothrombin activator preparations were calculated using the relationship that 1 mg/mL solution had an $A_{280}$ of 1, and the molar concentrations were calculated by dividing the protein concentrations by the literature values for the molecular weights. The molar concentrations quoted in subsequent experiments described herein that used these preparations are therefore upper estimates because the preparations are not homogenous.

EXAMPLE 2: PROTHROMBIN TO THROMBIN CONVERSION BY PROTHROMBIN ACTIVATORS

Example 2a: Five-minute incubation period

The aim of this experiment was to determine how much prothrombin was converted to thrombin by six different prothrombin activators from snake venom prepared in Example 1, namely preparations containing ecarin, carinactivase-1, carinactivase-2, PtPA, OsPA, and notecarin, after incubation for five minutes at room temperature.

The experiment was performed as follows.

20 mM HEPES Buffer pH 7.4, 150 mM NaCl, 5 mM $CaCl_2$, 0.05% surfactant p20 was prepared.

A solution of human prothrombin in 0.05 M Tris-HCl pH 7.4 was prepared by diluting a stock solution of purified human prothrombin (17.8 mg/mL; #HCP-0200; HTI, USA) 1/50 with distilled water to provide a working solution of 0.36 mg/mL, giving a final concentration in the tube of 72 μg/mL or 0.99 μM. For the gel loading the 0.36 mg/L prothrombin solution was used.

The stock solutions of the prothrombin activator preparations prepared as described earlier were diluted to provide final concentrations of 6 nM.

Stock alpha-thrombin (10 mg/mL, #HCT-0020; HTI, USA) was diluted (1/30) with distilled water containing 0.01% Tween 20 to provide a working thrombin solution of 0.33 mg/L which was used to load on the SDS-PAGE gels.

Samples were then prepared with the volumes shown in Table 3, and incubated for five minutes at room temperature.

Table 3: Volumes in each sample for incubation

| Sample | 1 | 2 | 3 | 4 | 5 | 6 | 7 | 8 |
|---|---|---|---|---|---|---|---|---|
| HEPES buffer (μL) | 80 | 60 | 60 | 60 | 60 | 60 | 60 | 80 |
| Prothrombin (μL) | 20 | 20 | 20 | 20 | 20 | 20 | 20 | 0 |
| Prothrombin activator | 0 | 50 | 50 | 50 | 50 | 50 | 50 | 0 |
| α-thrombin (μL) | 0 | 0 | 0 | 0 | 0 | 0 | 0 | 20 |
| Total volume (μL) | 100 | 100 | 100 | 100 | 100 | 100 | 100 | 100 |

The prothrombin activators in the above samples were as follows: (1) no prothrombin activator (human prothrombin alone in buffer); (2) carinactivase-1; (3) carinactivase-2; (4) ecarin; (5) PtPA; (6) OsPA; (7) notecarin; and (8) highly purified α-thrombin (no prothrombin activator).

SDS PAGE gels used were nUView Precast Mini Gels (#NB10-420, 4-20%) with running buffer Tris-Glycine (#BG-143). Sample buffer (#BG-145) (NuSEP, Australia) was obtained (*i.e.*, non-reducing sample buffer), and a reducing sample buffer of the sample buffer with 5% β-mercaptoethanol (#44143, BDH, UK) was prepared.

After the five minute incubation, a 40 μL aliquot from each sample was transferred into equivalent volume of non-reducing sample buffer or reducing sample buffer. The samples were then incubated for 10 minutes at 100 °C in a heating block. An aliquot of 25 μL of each sample and 12 μL of the pre-stained molecular weight marker (#SM0671 Page Ruler Prestained Protein Ladder, Fermentas, Hanover, MD, USA) were loaded on the gels. The gels were run at 100 V using a Mini-Protein II Cell PAGE apparatus (Bio-Rad) until the dye-front reached the bottom of the gel. The gels were stained with Commassie Brilliant Blue G (#B-0770, Sigma) (0.25% w/v Commassie Brilliant Blue G, 45% methanol, 10% acetic acid), and excess stain was removed by destainer (45% methanol, 45% water and 10% acetic acid).

The results are shown in Figure 12, where Figure 12A shows the SDS-PAGE gel using the sample buffer without β-mercaptoethanol (non-reducing sample buffer) and Figure 12B shows the SDS-PAGE gel using the sample buffer containing 5% β-mercaptoethanol (reducing sample buffer). The sample numbers as used in Table 3 above are the same as the lane numbers in the gels in Figure 12, and "m" represents the molecular weight marker.

This experiment showed complete conversion of prothrombin to thrombin in five minutes by PtPA and OsPA, but very little conversion by the other prothrombin activator preparations under these conditions.

Example 2b: Time course for conversion by PtPA and by notecarin

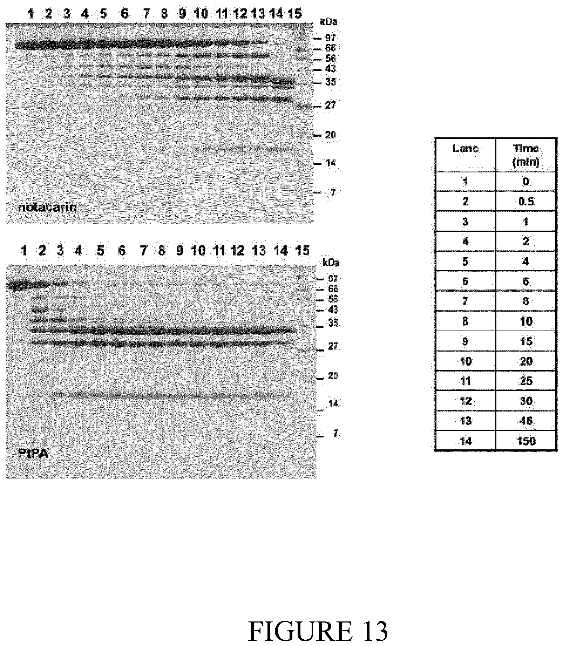

A time course for conversion of prothrombin (14 μM) to thrombin by PtPA (6 nM) and by notecarin (6 nM) was determined, and the SDS-page gel results are shown in Figure 13, where each lane is numbered according to the incubation time as shown in Table 4 below, using methodology similar to Example 2a.

Table 4: Incubation time for each lane shown in Figure 13.

| Lane | Time (min) |
|---|---|
| 1 | 0 |
| 2 | 0.5 |
| 3 | 1 |
| 4 | 2 |
| 5 | 4 |
| 6 | 6 |
| 7 | 8 |
| 8 | 10 |
| 9 | 15 |
| 10 | 20 |
| 11 | 25 |
| 12 | 30 |

(continued)

| Lane | Time (min) |
|------|------------|
| 13 | 45 |
| 14 | 150 |

These results show that PtPA is more efficient than notecarin in converting prothrombin to thrombin, but that notecarin still gave complete cleavage of prothrombin after longer reaction times.

Example 2c: N-terminal sequencing of selected bands from the Example 2b results

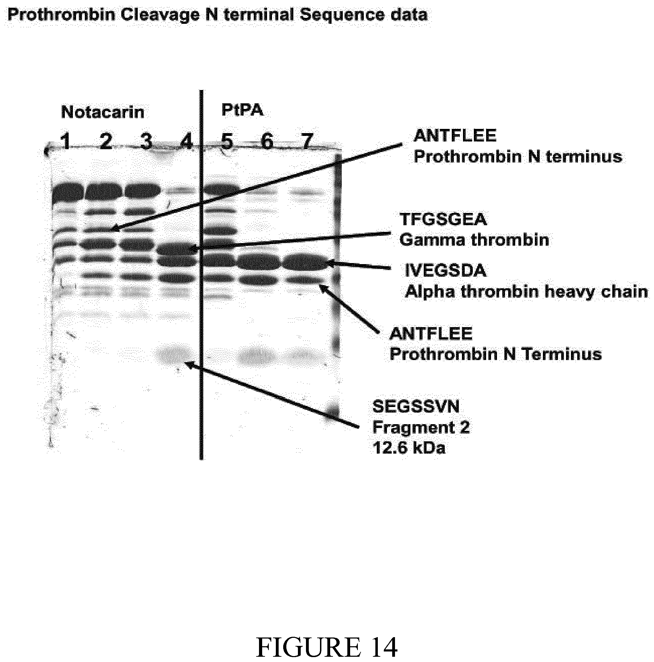

Selected bands from the SDS-PAGE results in Example 2b, produced by the action of PtPA and notecarin on prothrombin (*i.e.*, the fragmentation of prothrombin to thrombin), were eluted and subjected to N-terminal sequencing using mass-spectrometry analysis in order to assign to specific molecular domains. These results are shown in Figure 14. The SDS-PAGE N-terminal sequencing confirmed conversion of prothrombin to alpha-thrombin by PtPA and notecarin, as shown by the major band with N-terminal sequence IVEGSDA which corresponds to the alpha-thrombin heavy chain.

Example 2d: Estimation of thrombin generated by kinetic analysis

This experiment was designed to determine the amount of thrombin generated from prothrombin by each of the prothrombin activators: ecarin; carinactivase-1; carinactivase-2; PtPA; OsPA; and notecarin, at 0.6 nM concentration, by way of kinetic analysis.

The absorbance of the p-nitroaniline (pNA) generated from the chromogenic thrombin substrate S-2238 was continuously monitored at 405 nm, for samples containing different concentrations of thrombin and for samples containing one of the prothrombin activators listed above.

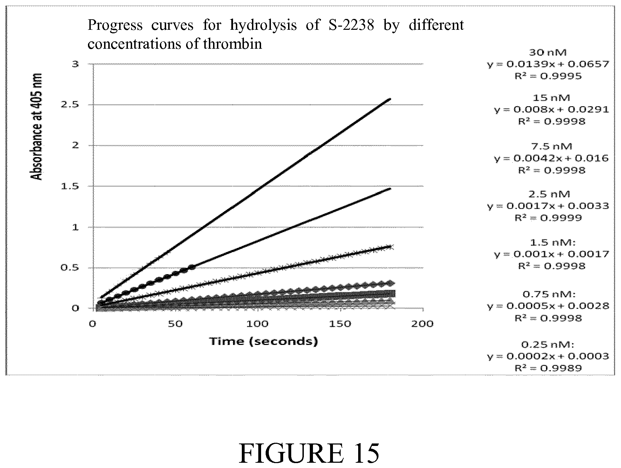

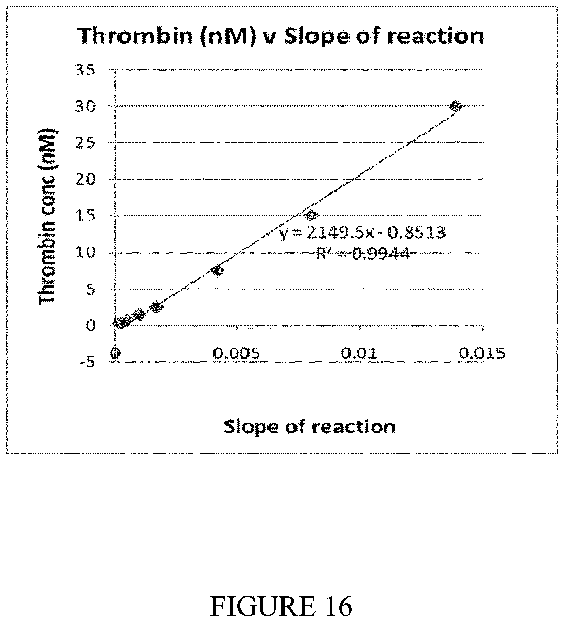

For the thrombin study, each cuvette contained 50 $\mu$L of S-2238 substrate (134 $\mu$M), 50 $\mu$L of thrombin at different concentrations (range of 0.25-30 nM) and 900 $\mu$L of HEPES buffer. Figure 15 shows progress curves for S-2238 hydrolysis by thrombin used to generate the standard curve shown in Figure 16.

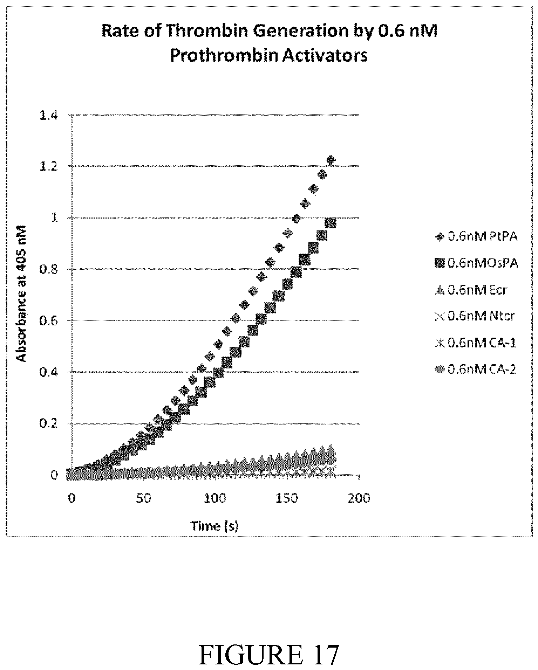

For the study of the prothrombin activators, each cuvette contained 50 $\mu$L of prothrombin (247 nM), 10 $\mu$L (0.6 nM) of the prothrombin activator (ecarin, carinactivase-1, carinactivase-2, PtPA, OsPA, or notecarin), 100 $\mu$L (267 $\mu$M) S-2238 substrate and 840 $\mu$L of HEPES buffer. The reaction slope for each prothrombin activator was determined at 155 seconds and the amount of thrombin generated at 155 seconds read from the standard curve in Figure 16, as shown in Figure 17 and Table 5.

Table 5: Amount of thrombin generated by 0.6 nM of the prothrombin activators over 155 seconds

| Prothrombin activator (0.6 nM) | Calculated slope from standard curves in Figure 16 | Amount of thrombin generated (nM) at 155 seconds |
|------|------|------|
| Ecarin | 0.00085 | 1.74 |
| Carinactivase-1 | 0.00007 | 0.07 |
| Carinactivase-2 | 0.0005 | 0.99 |
| PtPA | 0.0094 | 20.12 |
| OsPA | 0.0077 | 16.47 |
| Notecarin | 0.0001 | 0.13 |

All prothrombin activator preparations produced thrombin as expected, but again, the PtPA and OsPA were the most efficient.

These results indicate the group C prothrombin activators (PtPA and OsPA) were the most efficient enzymes in hydrolysing prothrombin to thrombin. This was confirmed by the amount of thrombin formed that was able to hydrolyse the S-2238 substrate to form the colour product pNA. The next most efficient prothrombin activator was ecarin which showed it hydrolysed ~4% of the S-2238, followed by carinactivase-2 (2.5%) then carinactivase-1 and lastly notecarin (<1%) being the least efficient of the prothrombin activators in this purified system. Group D prothrombin activators (e.g., notecarin) require Factor Va, calcium and phospholipid which were absence in the system but all of which are present in blood.

Example 2e: Kinetic studies on comparative rates of thrombin generation from thrombin by prothrombin activators

This experiment was designed to determine the rate of thrombin formation that is generated from pure prothrombin by each of the prothrombin activator preparations.

Each cuvette contained 50 $\mu$L of prothrombin (247 nM), 10 $\mu$L of the different prothrombin activators, 100 $\mu$L (267 $\mu$M) of S-2238 substrate and 840 $\mu$L of HEPES buffer.

The progress curves for S-2238 hydrolysis (thrombin activity) for each prothrombin activator was determined at 3-5 different concentrations ranging from 0.006-6 nM. The data was analysed using DynaFit 4.04.64 Enzyme Kinetic Data Analysis Software (BioKin Ltd, Watertown, MA, USA) (Kuzmic, P., 1996).

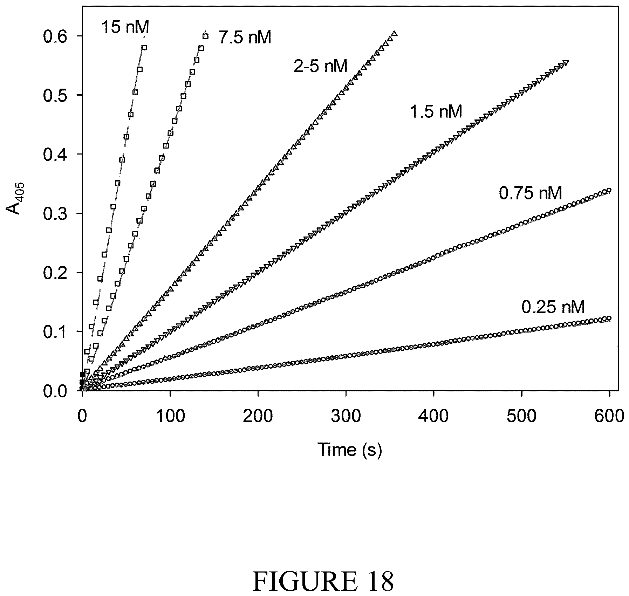

The thrombin catalysed hydrolysis of S-2238 curve fitting analysis is shown in Figure 18.

Kinetic parameters were then calculated as follows:

Model: B $\rightarrow$ B + pNA + P (a pseudo first order rate constant for the thrombin hydrolysis of S-2238), where:

B = thrombin;

pNA = p-nitroaniline;

P = inhibition product; and

S-2238 is in excess and therefore not included in the reaction.

Differential equations as follows:

d [B]/dt = -kb[B]+kb[B];

d [pNA]/dt = +kb[B]; and

d [P]/dt = +kb[B].

Table 6 shows the estimated kinetic parameters for thrombin.

Table 6: Estimated kinetic parameters for thrombin

| Units | Parameter | Initial | Fit | CV % |
|---|---|---|---|---|
| $s^{-1}$ | kb | 61.3 (Sonder, *et al.*, 1986) | 66.2 | 0.4 |

Estimation of rate constant for prothrombin activators:

Model:

A + Z $\rightarrow$ A + B : ka (second order rate constant for activation of prothrombin);

B $\rightarrow$ B + pNA + P : kb; where

A = prothrombin activator.

Differential equations as follows:

d [A]/dt = -ka[A][Z]+ka[A][Z];

d [Z]/dt = -ka[A][Z];

d [B]/dt = +ka[A][Z]-kb[B]+kb[B];

d [pNA]/dt = +kb[B]; and

d [P]/dt = +kb[B].

The kinetic parameters for PtPA were estimated and are shown in Table 7.

Table 7: Estimated kinetic parameters for PtPA

| Units | Parameter | Initial | Fit | CV % |
|---|---|---|---|---|
| $s^{-1}$ | . kb | | 66.2 | |
| $s^{-1}nM^{-1}$ | ka | 0.001 | 6.722 e-4 | 1.8 |
| $s^{-1}M^{-1}$ | ka | | $6.72 \times 10^5$ | 1.8 |

The rate of thrombin generation:

$$= ka\ (s^{-1}\ M^{-1}) \times 60\ (s) \times 10^{-6}$$

$$= 40.3\ \mu M\ thrombin\ /\ min\ /\ M\ prothrombin\ /\ M\ activator$$

$$= 40.3\ nmol\ thrombin\ /\ mL\ /\ min\ /\ \mu mol\ prothrombin\ /\ \mu mol\ activator$$

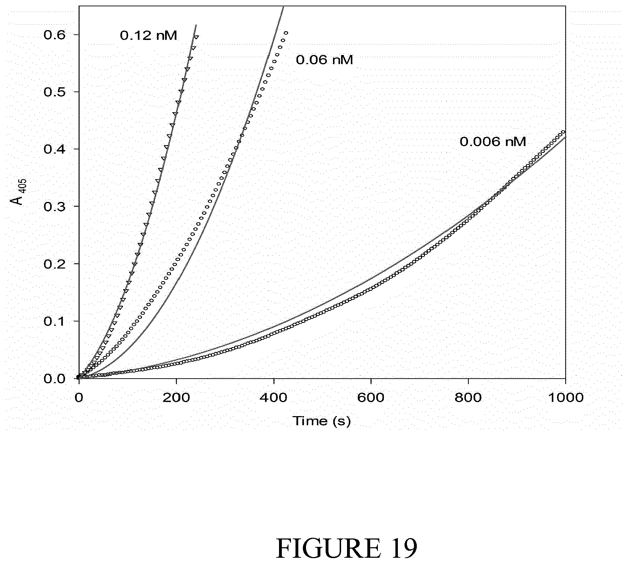

Figure 19 plots the PtPA activation of prothrombin, demonstrating the reaction rates of different PtPA concentrations (0.006 - 0.12 nM) with prothrombin and then the thrombin generated with the chromogenic substrate S-2238, and the best fit curve for PtPA activation of prothrombin. The dotted lines are the experimental results and the continuous lines are those calculated as best fit. These experimental and calculated progress curves show good agreement.
The kinetic parameters for OsPA were then estimated and are shown in Table 8.

Table 8: Estimated kinetic parameters for OsPA

| Units | Parameter | Initial | Fit | CV % |
|---|---|---|---|---|
| $s^{-1}$ | kb | | 66.2 | |
| $s^{-1}nM^{-1}$ | ka | 0.001 | 7.325 e-4 | 3.3 |
| $s^{-1}\ M^{-1}$ | ka | | $7.33 \times 10^5$ | 3.3 |

The rate of thrombin generation:

$$= 44.0\ nmol\ thrombin\ /\ mL\ /\ min\ /\ \mu mol\ prothrombin\ /\ \mu mol\ activator$$

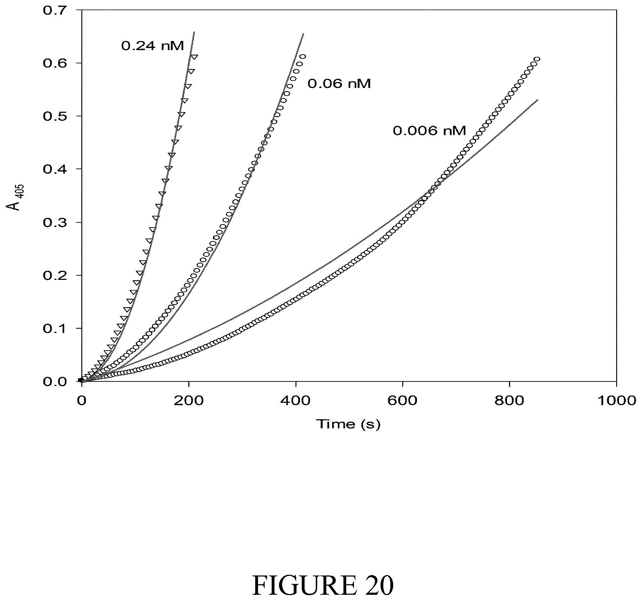

Figure 20 plots the OsPA activation of prothrombin, demonstrating the reaction rates of different OsPA concentrations (0.006 - 0.24 nM) with prothrombin and then the thrombin generated with the chromogenic substrate S-2238, and the best fit curve for OsPA activation of prothrombin. The dotted lines are the experimental results and the continuous lines are those calculated as best fit. These experimental and calculated progress curves show good agreement.
The kinetic parameters for ecarin were then estimated and are shown in Table 9.

Table 9: Estimated kinetic parameters for ecarin

| Units | Parameter | Initial | Fit | CV % |
|---|---|---|---|---|
| | kb | | 66.2 | |
| $s^{-1}\ nM^{-1}$ | ka | 0.001 | 4.607 e-005 | 1.2 |
| $s^{-1}\ M^{-1}$ | ka | | $4.61 \times 10^4$ | 1.2 |

The rate of thrombin generation:

$$= 2.76\ \mu M\ thrombin\ /\ min\ /\ \mu M\ prothrombin\ /\ \mu M\ activator$$

= 2.76 nmol thrombin / mL / min / μmol prothrombin / μmol activator

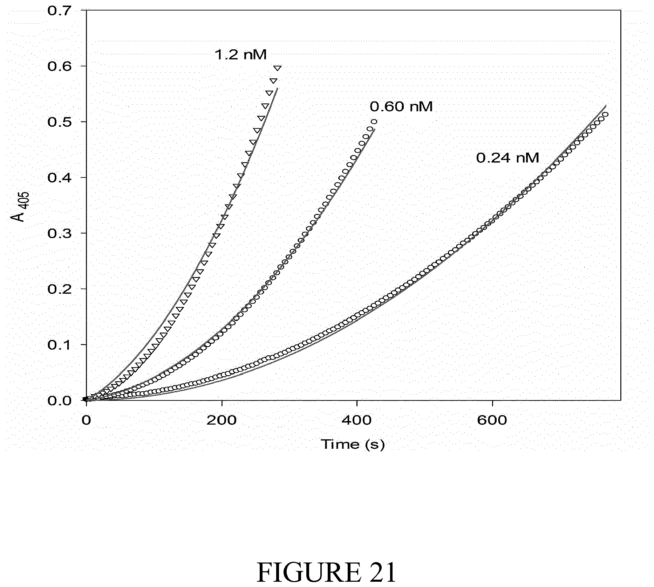

Figure 21 plots the ecarin activation of prothrombin, demonstrating the reaction rates of different ecarin concentrations (0.24 ~ 1.2 nM) with prothrombin and then the thrombin generated with the chromogenic substrate S-2238, and the best fit curve for ecarin activation of prothrombin. The dotted lines are the experimental results and the continuous lines are those calculated as best fit. These experimental and calculated progress curves show good agreement.

The kinetic parameters for carinactivase-1 were then estimated and are shown in Table 10.

Table 10: Estimated kinetic parameters for carinactivase-1

| Units | Parameter | Initial | Fit | CV % |
|---|---|---|---|---|
| $s^{-1}$ | kb | | 66.2 | |
| $s^{-1} nM^{-1}$ | ka | 0.001 | 3.944 e-006 | 0.3 |
| $s^{-1} M^{-1}$ | ka | | $3.94 \times 10^3$ | 0.3 |

The rate of thrombin generation:

= 0.24 μM thrombin / min / μM prothrombin / μM activator

= 0.24 nmol thrombin / mL / min / μmol prothrombin / μmol activator

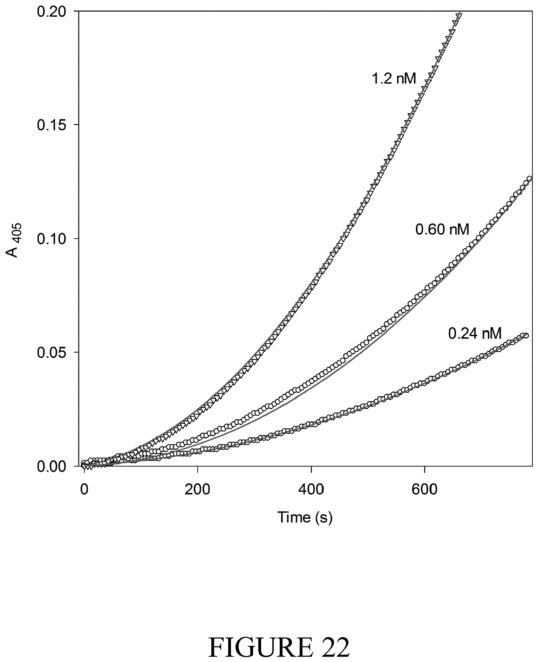

Figure 22 plots the carinactivase-1 activation of prothrombin, demonstrating the reaction rates of different carinactivase-1 concentrations (0.24 - 1.2 nM) with prothrombin and then the thrombin generated with the chromogenic substrate S-2238, and the best fit curve for carinactivase-1 activation of prothrombin. The dotted lines are the experimental results and the continuous lines are those calculated as best fit. These experimental and calculated progress curves show good agreement.

The kinetic parameters for carinactivase-2 were then estimated and are shown in Table 11.

Table 11: Estimated kinetic parameters for carinactivase-2

| Units | Parameter | Initial | Fit | CV % |
|---|---|---|---|---|
| $s^{-1}$ | kb | | 66.2 | |
| $s^{-1} nM^{-1}$ | ka | 0.001 | 3.183 e-005 | 1.8 |
| $s^{-1} M^{-1}$ | ka | | $3.18 \times 10^4$ | 1.8 |

The rate of thrombin generation:

= 1.91 μM thrombin / min / μM prothrombin μM activator

= 1.91 nmol thrombin / mL / min / μmol prothrombin / μmol activator

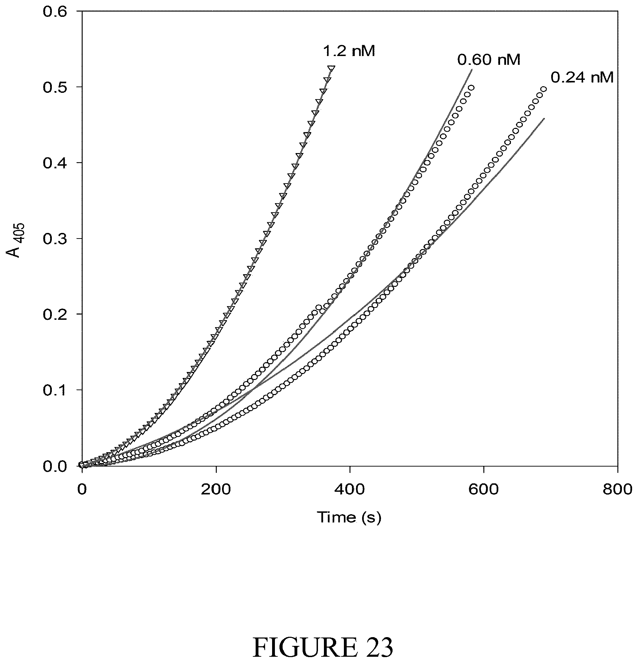

Figure 23 plots the carinactivase-2 activation of prothrombin, demonstrating the reaction rates of different carinactivase-2 concentrations (0.24 -1.2 nM) with prothrombin and then the thrombin generated with the chromogenic substrate S-2238, and the best fit curve for carinactivase-2 activation of prothrombin. The dotted lines are the experimental results and the continuous lines are those calculated as best fit. These experimental and calculated progress curves show good agreement.

The kinetic parameters for notecarin were then estimated and are shown in Table 12.

Table 12: Estimated kinetic parameters for notecarin

| Units | Parameter | Initial | Fit | CV % |
|---|---|---|---|---|
| $s^{-1}$ | **kb** | | 66.2 | |
| $s^{-1} nM^{-1}$ | **ka** | 0.001 | 7.139 e-007 | 0.3 |
| $s^{-1} M^{-1}$ | **ka** | | $7.14 \times 10^2$ | 0.3 |

The rate of thrombin generation:

$$= 0.043\ \mu M\ thrombin\ /\ min\ /\ \mu M\ prothrombin\ /\ \mu M\ activator;$$

$$= 0.043\ nmol\ thrombin\ /\ mL\ /\ min\ /\ \mu mol\ prothrombin\ /\ \mu mol\ activator.$$

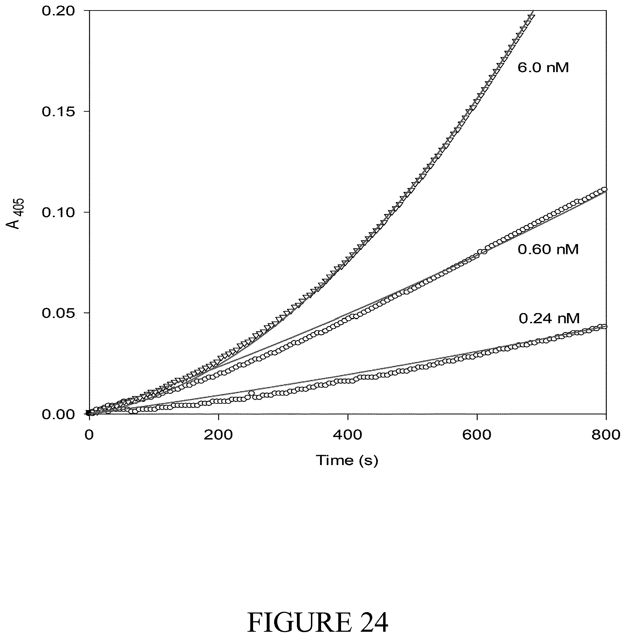

Figure 24 plots the notecarin activation of prothrombin, demonstrating the reaction rates of different notecarin concentrations (0.24 - 6.0 nM) with prothrombin and then the thrombin generated with the chromogenic substrate S-2238, and the best fit curve for notecarin activation of prothrombin. The dotted lines are the experimental results and the continuous lines are those calculated as best fit. These experimental and calculated progress curves show good agreement.

A summary of the estimated activation rate constants and thrombin generation rate for each of the prothrombin activators is provided in Table 13.

Table 13: Summary of the estimated activaton rate constants and the thrombin generation rate for the different prothrombin activators

| Prothrombin activator | Activation rate constant ka $(s^{-1}\ nM^{-1})$ | Thrombin generation rate (nmol thrombin / mL / min / $\mu$mol prothrombin / $\mu$mol activator) |
|---|---|---|
| PtPA | $6.72 \times 10^5$ | 40.3 |
| OsPA | $7.33 \times 10^5$ | 44.0 |
| ecarin | $4.61 \times 10^4$ | 2.76 |
| carinactivase-1 | $3.94 \times 10^3$ | 0.24 |
| carinactivase-2 | $3.18 \times 10^4$ | 1.91 |
| notecarin | $7.14 \times 10^2$ | 0.043 |

There was no reaction between the different prothrombin activators and the thrombin substrate S-2238, nor was there any reaction by prothrombin with S-2238. The activation rate data constants confirmed the group C prothrombin activators (PtPA and OsPA) are most potent in hydrolysing the prothrombin to thrombin. In fact they were more than 20 times more effective than ecarin and carinactivase-2, more than 200 times then carinactivase-1 and approximately 1000 times more effective than notecarin. The estimated thrombin generation rates equally show the same efficacy ratio, the group C activators were able to generate the largest amount of thrombin, followed by ecarin, carinactivase-2, carinactivase-1 and notecarin being the least efficient. As noted in Example 2d, notecarin requires cofactors for activity.

MATERIALS AND METHODS AS USED IN FOLLOWING EXAMPLES

Where indicated in the Examples that follow, the following Materials and Methods were used.

(A) CONTAINERS FOR PREPARING SAMPLES

A typical container (*e.g.* tube) for preparation of a serum sample or a plasma sample contains: (1) a gel barrier to separate cells (and the clot in the case of serum) from plasma or serum during centrifugation and to ensure no subsequent re-mixing; (2) a surfactant coating the inner wall to prevent cell and protein adhesion to the tube wall to minimise subsequent cell lysis; and (3) a procoagulant that enhances the clotting process (*e.g.* silica particles) for preparation of a serum sample or an anticoagulant (*e.g.* lithium heparin, citrate, or EDTA) for preparation of a plasma sample.

In the Examples, a number of commercially available tubes were used to prepare serum samples or plasma samples as described below.

Greiner Vacuette™ plasma tube. This is a plasma separator tube supplied by Greiner under reference number 456083. The inner wall of the tube is coated with spraydried lithium heparin (89 IU) that acts as an anticoagulation agent. The tube also contains a separation gel in the base of the tube. Greiner states that this gel acts to form a stable barrier between the plasma and the blood cells allowing parameters to remain stable for up to 48 hours. The fill volume is 5.0 mL.

Becton Dickinson (BD) Vacutainer™ plasma tube (BD PST II). This is a plasma separator tube supplied by BD under reference number 367375. Each tube contains 77 IU/mL of lithium heparin (0.2 - 1.0 mg) as an anticoagulant. The tube contains an acrylic gel (0.8 - 1.9 g) that forms a barrier between the cells and the plasma during centrifugation.

BD state that this gel provides enhanced plasma purity demonstrated by a reduction in fibrin and measurable white blood cells, red blood cells and platelets, and enhanced analyte stability as most analytes are stable in the tube for 24 hours at 25 °C. The fill volume is 4.0 mL.

Greiner Vacuette™ serum tube. This tube is supplied by Greiner under reference number 456078. The tube contains silica particles as the clot activator. The fill volume is 4.0 mL. The recommended clotting time in this tube is 30 minutes.

Becton Dickinson (BD) Vacutainer™ serum tube (BD SST II). This tube is supplied by BD under reference number 367954. This tube is used to obtain and separate a serum sample. Each tube contains 0.20 - 2.56 mg amorphous (or fused) silica as the coagulation agent, and also a gel (0.90 - 3.50 g) that forms a barrier between the clot and the serum after centrifugation. BD recommend a clotting time of 30 minutes for this tube. The fill volume is 4.0 mL.

Becton Dickinson (BD) Vacutainer™ Rapid Serum tube (BD RST). This tube is supplied by BD under reference number 368771. The tube contains a thrombin-based medical clotting agent and gel. BD claims that these tubes will provide a clotted sample within five minutes, (*i.e.* faster clotting than a standard commercially available serum tube) and will have "minimal fibrin formation". The fill volume is 4.0 mL.

Greiner Vacuette™ No Additive tube. These are plain plastic tubes supplied by Greiner under reference number 45400 to which prothrombin activators at particular concentrations were added as described in the Examples below.

Sarstedt serum tube. This tube is supplied under catalogue number 5092503 with a light brown top and has a fill volume of 4.7 mL. These tubes contain silica particles and a gel barrier.

Terumo plain tube. This tube is supplied under the trade mark VENOSAFE under catalogue number VF-076SP and has a fill volume of 6.0 mL. These tubes contain silica particles and a gel barrier.

Terumo Red Top (RT) tube. This tube is supplied under the trade mark VENOSAFE under catalogue number VF-108SAS and has a fill volume of 8.0 mL. These tubes are internally coated with silica and have a gel barrier.

Greiner Vacuette™ citrate tube. This plastic tube contains 3.2% citrate with a 4.0 mL fill volume. The tube is sold under catalogue number 454327.

Greiner Vacuette™ K2EDTA tube. This plastic tube contains 8 mM EDTA and has a 4.0 mL fill volume. The tube is sold under catalogue number 454023.

## (B) SAMPLE ANALYSES - CLOTEK ANALYSER

The Clotek analyser (Hyland, USA) uses a sample tube containing a magnetic ball which oscillates vertically when the tube is inserted into the machine. The magnetic steel ball is held in position by a magnetic field while the tube oscillates. The sample, buffer, $\pm$ calcium and procoagulant are added and the timer is triggered. Thrombin converts fibrinogen into clottable fibrin, and the time required to form the detectable clot is the clotting time. At clot formation the steel ball is removed from the magnetic suspension field and the light path, causing the light beam to strike the photocell and stop the timer (Austen, D. E., *et al.* 1975 and Starr, H., *et al.*, 1980).

## (C) SAMPLE ANALYSES - THROMBOELASTOGRAPHY

Thromboelastography (TEG) measures clotting of whole blood by determining the rate of blood clot formation and lysis and the elastic properties of the blood clot during its formation and lysis. Where indicated in some Examples below, TEG analysis was performed.

The TEG analysis was performed using a Thrombelastograph® 5000 (TEG, Haemoscope Corporation Pty Ltd, Niles, IL, USA) with TEG Analytical Software version 4.

The clotting parameters used here for the TEG analysis were as follows:

R - reaction time (seconds / minutes): time from start of the analysis until the TEG tracing amplitude reaches 2 mm, indicating detectable clot formation;

$\alpha$ - angle: measure of the rate of fibrin formation and cross-linking (*i.e.* clot strengthening);

MA - maximum amplitude (mm): maximum strength of the clot;

TMA - time (seconds / minutes) from start of the analysis until MA is reached; and

K -time (s): time from the start of the clot formation to the time the curve reaches amplitude of 20 mm.

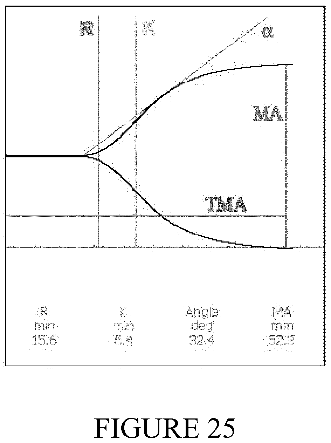

An example of a TEG plot (clot formation part) with the above clotting parameters labelled is provided in Figure 25. Only the clotting parameters are presented as the clot lysis parameters were not included.

## EXAMPLE 3: CLOTTING OF PLASMA SAMPLES BY VENOM PROTHROMBIN ACTIVATORS

Example 3a: Clotting times of normal citrated plasma by PtPA versus bovine thrombin

The clotting times of pooled citrated plasma (combined plasma from 6 normal participants) by PtPA (prepared as outlined in Example 1d) and bovine $\alpha$-thrombin (3276 U/mg; Sigma Chemical Co.), with and without added calcium (10 mM), were measured in duplicate samples.

The results are shown in Table 14, where the values are the means of duplicate experiments.

Table 14: Clotting times of citrated plasma with PtPA and bovine thrombin, measured in a Hyland-Clotek machine (seconds).

| Concentration of enzyme (nM) | Clotting time (seconds) | | | |
|---|---|---|---|---|
| | PtPA + calcium | PtPA calcium | thrombin + calcium | thrombin - calcium |
| 100 | 4.2 | 4.7 | 4.8 | 7.9 |
| 50 | 4.2 | 4.8 | 6.4 | 17.9 |
| 30 | 4.3 | 5.0 | **13.3** | 27.2 |
| 10 | 4.5 | 5.4 | 33.2 | 57.2 |
| 3 | 5.0 | 6.2 | **78.6** | >200# |
| 1 | 6.0 | 6.6 | >200# | >200 |
| 0.1 | 8.7 | 8.8 | >200 | >200 |
| 0.001 (1pM) | **13.5** | 29.0 | >200 | >200 |
| 0.0001 (0.1pM) | **74.1** | 98.6 | >200 | >200 |
| 0 | >200 | >200 | >200 | >200 |
| # Weak clot observed. | | | | |

The PtPA clotted citrated plasma efficiently at concentrations as low as 0.1 pM with or without recalcification. A concentration of 1 nM gave a clotting time of about 6 seconds, close to the minimum clotting time achieved at 100 nM of 4.2 seconds.

These results also show that PtPA is about $3 \times 10^4$ times more efficient in the clotting of citrated plasma (with or without re-calcification) on a molar basis than bovine thrombin. For example, in the presence of 10 mM added calcium, 1 pM PtPA gave the same clotting time as 30 nM thrombin and 0.1 pM PtPA had a very similar clotting time to 3 nM thrombin (values in bold in Table 14).

Example 3b: Clotting times of normal citrated plasma by prothrombin activators

This experiment used the same plasma as in Example 3a.

Each Clotek tube contained 100 $\mu$L of normal "pooled" citrated plasma, 100 $\mu$L of Tris Buffer (150 mM Tris HCl, 150 mM NaCl, pH 7.4), 50 $\mu$L 0.2 M $CaCl_2$ or saline, and.50 $\mu$L of each procoagulant (either prothrombin activator preparation or thrombin).

The clotting results from each prothrombin activator preparation and thrombin with the normal "pooled" citrated plasma (with and without re-calcification) are shown in Tables 15 and 16. In these tables (and elsewhere), CA-1 is carinactivase-1 and CA-2 is carinactivase-2.

Table 15: Clotting times of normal "pooled" citrated plasma with different prothrombin activator preparations and thrombin, with calcium.

| [Procoagulant] (mM) | With $Ca^{2+}$ (seconds) | | | | | | |
|---|---|---|---|---|---|---|---|
| | Ecarin | CA-1 | CA-2 | PtPA | OsPA | Notecarin | Thrombin |
| 100 | 43.0 | 108 | 65.8 | 3.5 | 5.7 | 42.4 | 4.3 |
| 50 | 56.0 | 145 | 85.0 | 4.6 | 6.5 | 52.8 | 6.7 |

(continued)

| [Procoagulant] (mM) | With Ca$^{2+}$ (seconds) | | | | | | |
|---|---|---|---|---|---|---|---|
| | Ecarin | CA-1 | CA-2 | PtPA | OsPA | Notecarin | Thrombin |
| 30 | 69.0 | 181 | 167 | 4.9 | 7.9 | 57.5 | 10.5 |
| 10 | 105 | >300 | >300 | 12.5 | 12.5 | 68.5 | 32.8 |
| 3 | 208 | >300 | >300 | 20.1 | 18.3 | 99.8 | 92.3 |
| 1 | >300 | >300 | >300 | 61 | 30.0 | 144 | >300 |
| 0.1 | >300 | >300 | >300 | 120 | 108 | 284 | ND |
| 0.01 | >300 | >300 | >300 | 212 | >300 | >300 | ND |
| 0.001 | >300 | >300 | >300 | >300 | >300 | >300 | ND |

Table 16: Clotting times of normal "pooled" citrated plasma with different prothrombin activator preparations and thrombin, without calcium

| [Procoagulant] (mM) | Without Ca$^{2+}$ (seconds) | | | | | | |
|---|---|---|---|---|---|---|---|
| | Ecarin | CA-1 | CA-2 | PtPA | OsPA | Notecarin | Thrombin |
| 100 | 94.0 | >300 | 216 | 3.9 | 7.3 | 51.7 | 5.6 |
| 50 | 129 | >300 | >300 | 5.0 | 8.2 | 67.6 | 9.2 |
| 30 | 167 | >300 | >300 | 6.1 | 8.9 | 76.7 | 14.6 |
| 10 | >300 | >300 | >300 | 9.0 | 14.0 | 115 | 43.6 |
| 3 | >300 | >300 | >300 | 15.5 | 21.5 | 250 | 145 |
| 1 | >300 | >300 | >300 | 24.6 | 34.5 | >300 | >300 |
| 0.1 | >300 | >300 | >300 | 93.0 | 152 | >300 | ND |
| 0.01 | >300 | >300 | >300 | >300 | >300 | >300 | ND |
| 0.001 | >300 | >300 | >300 | >300 | >300 | >300 | ND |

The results show that in the presence of added calcium all the prothrombin activators (representing groups A-D) efficiently clotted normal citrated plasma.

Citrated plasma is not completely calcium depleted. The total calcium concentration in normal citrated plasma is ~1.3 mmol/L and the ionised calcium concentration is unmeasurable (<0.25 mmol/L). However, citrate is a chelator of calcium, binding to the calcium in the sample so that free calcium is not readily available to be used for the clot formation. Calcium is part of the prothrombinase complex and plays an important role in the prothrombin to thrombin catalysis. Citrate also binds other metal ions including magnesium and zinc ions. The amount of citrate present in the tube, 0.109 mol/L is more than sufficient to chelate all the metal ions in the tube.

These results showed that ecarin, the group A prothrombin activator, was more than twice as effective in clotting the plasma in the presence of added calcium, and therefore calcium has a significant enhancement on ecarin's ability to accelerate the clot formation. The group B prothrombin activators, carinactivase-1 and carinactivase-2, were strongly affected by the absence of calcium, with a decrease in activity of ~4-fold, confirming the calcium enhances clotting activity with these prothrombin activators. The activity of notecarin, the group D prothrombin activator, was stimulated by the presence of added calcium, and the requirement for calcium was most evident with the decrease in the notecarin concentration with the addition of calcium increasing its effectiveness by ~30%.

Thrombin itself was affected to a smaller degree by the absence of the added calcium. Thrombin itself generates thrombin formation in the sample via the coagulation cascade of prothrombin which requires calcium. Therefore the amount of thrombin formed from the sample prothrombin decreased with the absence of calcium.

The calcium dependence of the procoagulants was less when they were in high concentrations due to their potency. The results showed that the group C prothrombin activators, PtPA and OsPA, were the most efficient in clotting the normal "pooled" citrate plasma both in the presence or absence of added calcium. In fact, the absence of calcium made minimal difference on the clotting ability of these two procoagulants. With and without the presence of added calcium the group C prothrombin activators required ~0.1 nM to achieve clotting in <5 minutes.

Example 3c: Clotting_times of citrated plasma samples and fibrinogen by PtPA

The ability of PtPA to clot the following recalcified citrated plasma samples and a sample of purified fibrinogen was determined in the following duplicate samples:

(i) citrated plasma;

(ii) citrated plasma adsorbed by $Al(OH)_3$ (adsorption by $Al(OH)_3$ removes prothrombin and other γ-carboxyglutamate (GLA)-containing proteins);

(iii) citrated plasma from patients on long term warfarin therapy (with International Normalized Ratio (INR) greater than 4.0);

(iv) citrated plasma from patients on long term warfarin therapy (with INR greater than 4.0) adsorbed by $Al(OH)_3$;

(v) citrated Factor X-deficient plasma obtained from congenitally deficient patients;

(vi) citrated Factor V-deficient plasma obtained from congenitally deficient patients; and

(vii) purified human fibrinogen (2 mg/mL in isotonic saline), adsorbed with $Al(OH)_3$ (to remove traces of prothrombin).

With respect to (iii) and (iv), warfarin inhibits liver γ-carboxylase, an enzyme which inserts γ-carboxyl groups into 10 N-terminal glutamic acid residues of prothrombin. Warfarin plasma therefore contains an uncarboxylated precursor of prothrombin, descarboxyprothrombin, which is hot removed by adsorption with $Al(OH)_3$.

With respect to (v) and (vi), Factor Xa and Factor Va are essential components of the human prothrombinase complex. The Factor X-deficient and Factor V-deficient plasmas were supplied by Ortho-Diagnostics, USA.

The results are shown in Table 17.

Table 17: Clotting times of citrated plasma samples (i) - (vi) and fibrinogen sample (vii).

| PtPA* (nM) | Clotting time (seconds ± 0.5 seconds) for samples | | | | | | |
| --- | --- | --- | --- | --- | --- | --- | --- |
| | (i) | (ii) | (iii) | (iv) | (v) | (vi) | (vii) |
| 54 | 12.8 | > 180 | 15.6 | 18.4 | 16.5 | 12.8 | >180 |
| 26 | 16.4 | > 180 | 18.4 | 26.8 | n.d. | n.d. | n.d. |
| 16 | 22.5 | > 180 | 24.6 | 28.6 | n.d. | n.d. | n.d. |
| 7.3 | 34.6 | > 180 | 38.8 | 42.3 | n.d. | n.d. | n.d. |
| 3.8 | 48.2 | > 180 | 55.0 | 59.8 | n.d. | n.d. | n.d. |
| 0# | 10.0 | 10.6 | 7.7 | 8.6 | 14.6 | 10.6 | 5.4 |

* In this experiment, a different preparation of PtPA was used, accounting for the lower clotting activity than in Table 14 above.
n.d. means that the measurements were not determined.
# Where no PtPA was added, 0.1 Units of bovine thrombin (Parke-Davis, now Pfizer, USA) was added to all samples as a positive control. The rapid clotting observed on addition of thrombin confirmed the presence of fibrinogen in all samples.

The results in Table 17 show that:

(1) prothrombin is necessary for clotting by PtPA since $Al(OH)_3$-adsorbed citrated plasma (sample (ii)) was not clotted by PtPA;

(2) PtPA clots warfarin plasma (samples (iii) and (iv)) indicating that descarboxyprothrombin can be converted to

$\alpha$-thrombin by PtPA;

(3) the result for warfarin plasma adsorbed by Al(OH)$_3$ (sample (iv)) confirms that descarboxyprothrombin is converted to $\alpha$-thrombin, since traces of normal prothrombin in warfarin plasma would be removed by Al(OH)$_3$;

(4) Factor Xa is not required for clotting by PtPA (results for sample (v));

(5) Factor Va is not required for clotting by PtPA (results for sample (vi)); and

(6) PtPA is unable to clot a fibrinogen solution and is therefore not functioning as a thrombin-like enzyme.

In summary, these results shows that clotting induced by PtPA is due to prothrombin activation; that descarboxyprothrombin is converted efficiently to thrombin by PtPA; that Factors Xa and Va are not required for prothrombin activation by PtPA; and PtPA does not convert fibrinogen to fibrin under these conditions.

Example 3d: Clotting of EDTA-treated plasma

A small but significant percentage of samples (serum or lithium heparin plasma samples) received for biochemical analysis is contaminated by EDTA. It is therefore important to determine if the prothrombin activators were effective in clotting EDTA plasma and EDTA whole blood. This example is therefore provided as Example 5b below, so that a comparison of the studies of EDTA plasma and EDTA whole blood could be made.

Example 3e: Clotting of citrated plasma from warfarin medicated participants by prothrombin activators

Warfarin is a commonly used anticoagulant, and therefore the purpose of this experiment was to determine if warfarin therapy affects the ability of the prothrombin activators: ecarin; carinactivase-1; carinactivase-2; PtPA; OsPA; and notecarin, to clot samples from patients medicated with warfarin.
Participant plasma samples with different clotting times (related to warfarin dosage) based on the INR (1.1-7.6) were selected and tested with the different procoagulants (prothrombin activators and thrombin).
The Clotek tubes contained 100 $\mu$L of lithium heparin plasma, 100 $\mu$L of Tris Buffer (150 mM Tris HCl, 150 mM NaCl, pH 7.4), 50 $\mu$L of 0.2 M CaCl$_2$, and 50 $\mu$L of each procoagulant.
The results are shown in Tables 18 and 19. As used in these tables and elsewhere, CA-1 is carinactivase-1 and CA-2 is carinactivase-2.

Table 18: Clotting times (seconds) with re-calcified citrated plasma from warfarin medicated patients, by prothrombin activators.

| [Prothrombin activator] 30 nM | Sample INR | | | | | | |
|---|---|---|---|---|---|---|---|
| | 1.1 | 1.2 | 1.5 | 2.0 | 2.9 | 6.4 | 7.6 |
| PtPA | 5.0 | 5.7 | 6.7 | 6.1 | 7.0 | 9.8 | 10.5 |
| OsPA | 9.0 | 9.1 | 12.6 | 16.8 | 17.2 | 17.2 | 19.3 |
| Notecarin | 69.4 | 67.1 | 68.6 | 66.6 | 71.0 | 91.0 | 121 |
| Ecarin | 72.2 | 63.4 | 63.4 | 69.4 | 85.6 | 114 | 111 |
| CA-1 | 231 | 198 | 232 | 229 | >300 | >300 | >300 |
| CA-2 | 115 | 103 | 115 | 109 | 149.2 | 242 | 240 |

Table 19: Clotting times (seconds) with re-calcified citrated plasma from warfarin medicated patients by PtPA and thrombin.

| [procoagulant] 30 nM | Sample INR | | | | | |
|---|---|---|---|---|---|---|
| | 1.1 | 1.7 | 2.3 | 2.8 | 5.8 | 6.4 |
| PtPA | 7.4 | 8.1 | 8.1 | 8.3 | 11.1 | 13.3 |
| Thrombin | 13.4 | 13.7 | 14.7 | 14.0 | 14.7 | 16.3 |

Warfarin leads to a decrease in the synthesis of normal Factor-X (FX) and prothrombin from their active precursors (descarboxyfactor-X and descarboxyprothrombin). Prothrombin and descarboxyprothrombin are the substrates targeted by the prothrombin activators. The decrease in prothrombin means less thrombin was generated by the prothrombin activators, hence the prolonged clotting times observed. Thrombin itself was not inhibited by warfarin thus the clotting times with thrombin are not affected as has been observed in this experiment. The results in this experiment showed that the carinactivase-1 was the only prothrombin activator unable to clot plasma samples with INR ≥2.9. The results showed PtPA and OsPA were the most effective procoagulants for plasma from patients taking warfarin.

Example 3f: Clotting of FV-deficient and FX-deficient plasma by prothrombin activators

The prothrombin activator preparations were tested for their ability to clot commercially available FV-deficient plasma (#0008466150) and FX-deficient plasma (#0008466350) (Instrumentation Laboratory, Lexington, MA, USA) using the Clotek analyser (Hyland USA).

The Clotek tube contained 100 $\mu$L of FV- or FX- deficient plasma, 100 $\mu$L of Tris Buffer (150 mM Tris HCl, 150 mM NaCl, pH 7.4), 50 $\mu$L 0.2 M CaCl$_2$ and 50 $\mu$L of each procoagulant. All procoagulants were at a concentration of 30 nM, and an additional sample containing carinactivase-1 at a concentration of 100 nM was also tested.

The results are shown in Table 20.

Table 20: Clotting times (seconds) of FV- or FX- deficient plasma by the prothrombin activator preparations and thrombin

| [Procoagulant] 30 nM | Clotting time (seconds) | | |
|---|---|---|---|
| | Normal "pooled" citrated plasma | FV-deficient plasma | FX-deficient plasma |
| Ecarin | 66.8 | 74.8 | 77.5 |
| CA-1 | 184 | >300 | >300 |
| CA-1 (100nM) | ND | 216 | 218 |
| CA-2 | 133 | 159 | 159 |
| OsPA | 13.3 | 12.7 | 12.7 |
| PtPA | 9.0 | 7.4 | 7.6 |
| Notecarin | 47.5 | >300 | 71.9 |
| Thrombin | 13.8 | 44.6 | 50.2 |
| "ND" means this was not determined. | | | |

Notecarin was not able to clot the FV-deficient plasma in 5 minutes, and its clotting efficiency was also diminished in the absence of FX. Furthermore, the clotting activity of the group A and group B prothrombin activators was also diminished in the absence of FV or FX. The group C prothrombin activators, PtPA and OsPA, were clearly the most efficient in clotting the plasma, and have no requirement for FV or FX from plasma.

Thrombin clotting time was significantly increased to be more than 3 times longer in the FV- and FX-deficient plasma compared to the clotting time for the normal "pooled" citrated plasma.

Example 3g: Estimation of amount of thrombin generated by PtPA and OsPA in normal and heparinised citrated plasma

This experiment was designed to provide an estimate of the amount of thrombin that is generated by PtPA and OsPA using different concentrations and in the presence of heparin.

Normal citrated plasma was obtained from a healthy participant into a number of tubes (Greiner citrate) which were immediately centrifuged and the citrated plasma pooled.

The thrombin used was Thrombin-JMI, Topical (Bovine) 5000USP (GenTrac Inc, King Pharmacia, Middleton, WI, USA). The USP units are equivalent to IU, and one IU is equal to 9 nM. The thrombin substrate used was S-2238 (25 mg stock solution MW 626 Da was dissolved in 15 mL to give 2.67 mM (#820324 Chromogeinix IL Lexington MA, USA). The heparin used was 5000 IU/5 mL (1000 IU/mL), sodium heparin, Pfizer, NZ. For heparin, 190.9 IU = 1 mg (Camenzind, *et al.* 1997); a MWt of ~12000 Da was used to calculate molar concentrations. The buffer used was: 20 mM Hepes buffer; 150 mM NaCl; pH 7.4; 0.05% surfactant p20.

For the plasma incubation the tubes contained 0.5 mL citrated plasma, 50 $\mu$L of 0.5 M CaCl$_2$, and 5-40 $\mu$L thrombin or a prothrombin activator plus 20 $\mu$L of heparin to provide concentrations of 860, 4300, and 8600 nM (2, 10 and 20 IU) or saline. The tubes were incubated for 5 minutes at room temperature. Immediately after this 5 minute period, the clot was ringed using two wood applicator sticks, removed and discarded.

For thrombin activity assays, each spectrophotometric cuvette contained 930 $\mu$L of buffer, 50 $\mu$L of S-2238 and 20 $\mu$L of the clear serum that was remaining after the clot was removed from each tube in the incubation above. The absorbance change was monitored at 405 nm for 5 minutes.

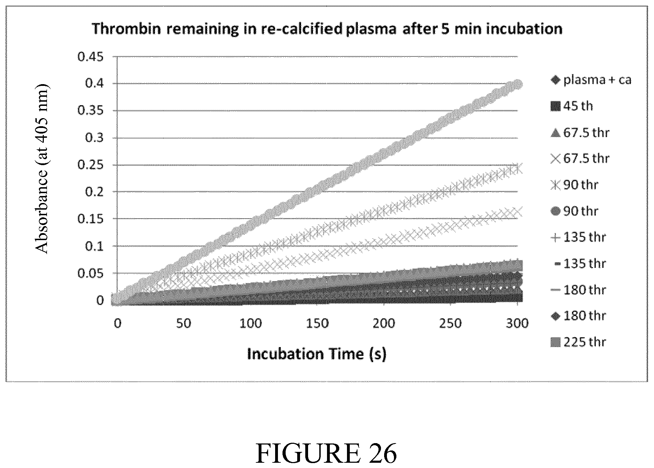

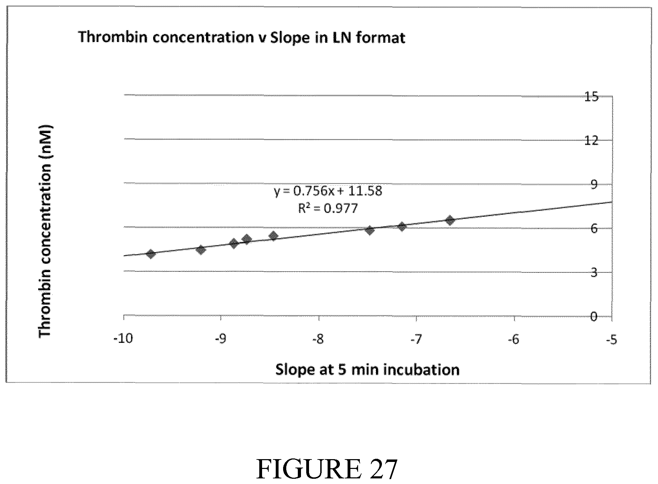

Figure 26 shows the activities of remaining thrombin in those tubes to which different concentrations of thrombin had been added. For each curve, the slope at 275 seconds was determined using the time range of 250-300 seconds, and these slopes along with the thrombin concentrations were then used to develop the standard curve in logarithmic format to provide a linear relationship over higher thrombin concentration, as shown in Figures 26 and 27.

The re-calcified plasma (no procoagulant) took ~37 minutes to clot at room temperature. All the thrombin containing samples exhibited latent clotting. The lowest concentration of thrombin was the slowest to exhibit latent clotting (~3 minutes). The time to latent clot decreased as the concentration of thrombin increased. The samples with concentrations >200 nM thrombin exhibited latent clotting immediately after the clot was removed as the serum was being aliquoted. At times, a second clot removal was necessary to ensure sufficient sample was obtained from the tube for the photometric analysis.

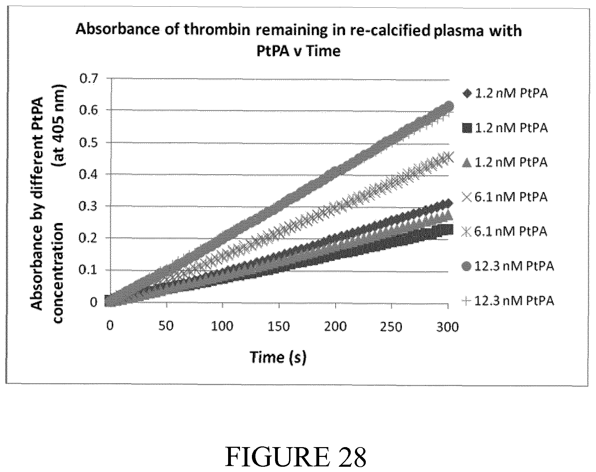

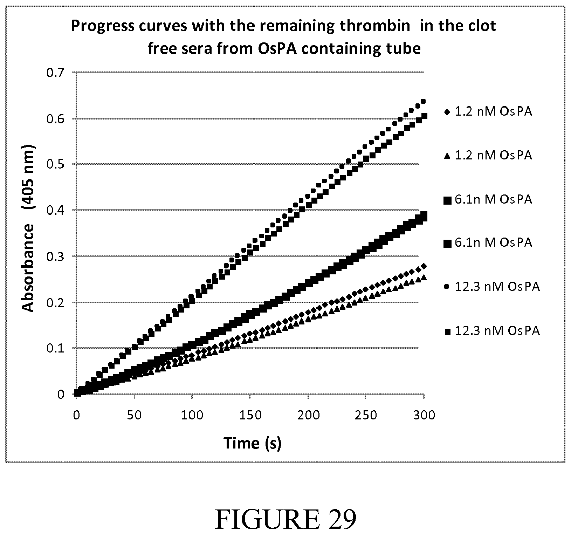

For the samples containing different concentrations of the prothrombin activators PtPA and OsPA, but without heparin, the results were as follows. The spectrophotometric results for the samples containing different concentrations of PtPA are shown in Figure 28, and for the samples containing different concentrations of OsPA are shown in Figure 29.

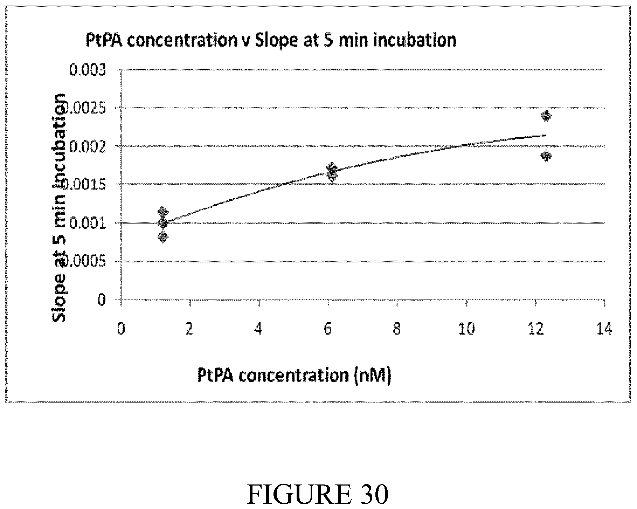

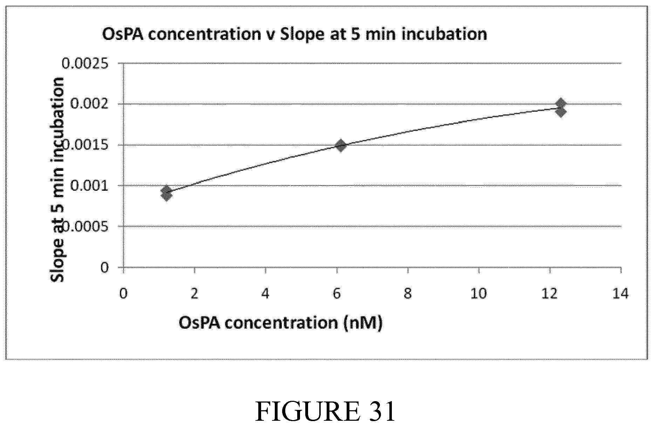

The thrombin concentrations generated by PtPA was then plotted against the slope at 5 minutes, and the results are shown in Figure 30, and the thrombin concentrations generated by OsPA was then plotted against the slope at 5 minutes with the results are shown in Figure 31.

The concentration of thrombin generated by the different PtPA and OsPA concentrations remaining active in the serum was estimated and is shown in Table 21.

Table 21: Estimation of the active thrombin present in the serum generated by different concentration of PtPA and OsPA determined from the standard curve regression equation, Figure 27.

| [Prothrombin activator] (nM) | Slope at 5 minutes per second | Estimated concentration of active thrombin (nM) |
|---|---|---|
| PtPA | | |
| 1.2 | 0.00098 | 574 |
| 6.1 | 0.00171 | 869 |
| 12.3 | 0.00196 | 965 |
| OsPA | | |
| 1.2 | 0.00094 | 554 |
| 6.1 | 0.00149 | 784 |
| 12.3 | 0.00195 | 961 |

The results indicated that very significant amounts of thrombin were generated even by 1.2 nM PtPA and OsPA. Very small amounts of PtPA and OsPA were capable of completely clotting the re-calcified normal citrated plasma in < 5 minutes. No latent clotting was observed, indicating that the amount of thrombin generated was sufficient to completely catalyse the fibrinogen and prevent latent clotting which would equate to very high quality serum.

For the samples containing different concentrations of heparin, the results were as follows.

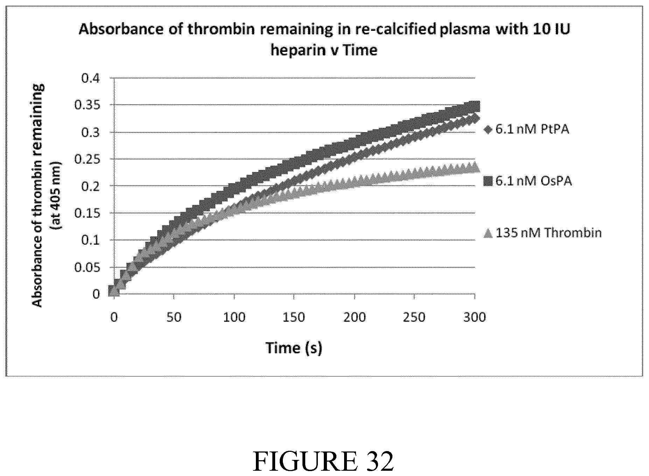

First, the concentration of thrombin remaining in the serum component from the activity of thrombin, PtPA and OsPA in the presence of 4300 nM (10 IU) heparin was determined and is shown in Figure 32 (change in A$_{405}$ for S-2238 hydrolysis / minute on the *y*-axis is proportional to thrombin concentration). The 135 nM thrombin failed to fully clot the plasma sample, with only a minor 2-3 mm "fluff-like" clot observed. The 6.1 nM concentrations of both prothrombin activators clotted the sample containing 4300 nM (10 IU) of heparin in approximately one minute and no latent clotting was observed even after 24 hours storage at room temperature.

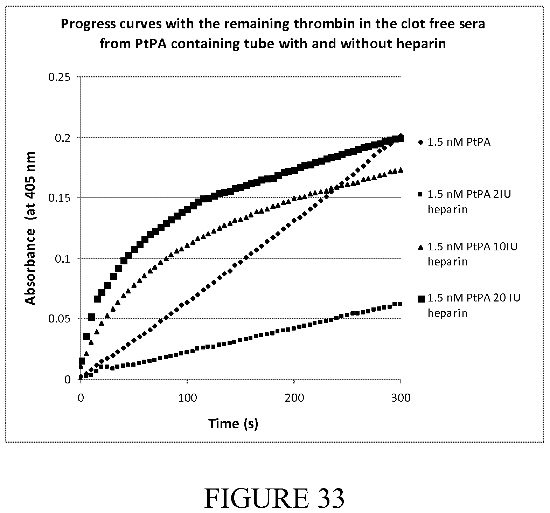

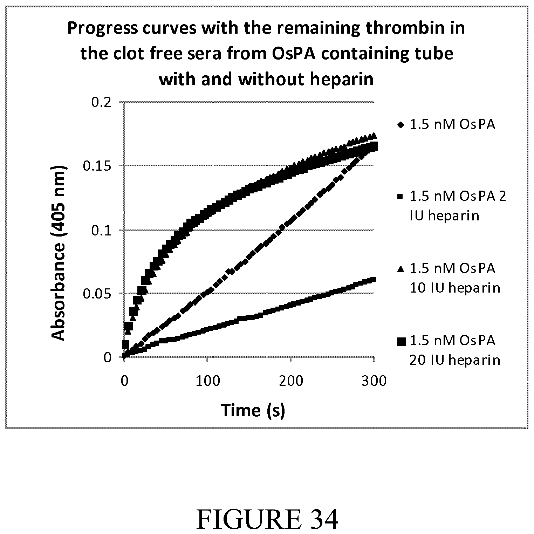

It was then investigated whether the reaction was of similar nature with different heparin concentrations. Normal pooled citrated plasma was was obtained from the Coagulation Laboratory, Pathology Queensland, Princess Alexandra Hospital as there was insufficient plasma from the normal participant. The results are shown in Figure 33 (PtPA) and Figure 34 (OsPA).

The amount of thrombin generated by 1.5 nM PtPA and OsPA concentrations remaining active in the serum was estimated and is shown in Table 22.

Table 22: Estimation of the active thrombin present in the serum generated by 1.5 nM of PtPA and OsPA determined from the standard curve regression equation, Figure 27.

| [Prothrombin activator] (nM) | Heparin nM (IU)/mL | Slope at 5 minutes per second | Estimated concentration of active thrombin (nM) |
|---|---|---|---|
| PtPA | | | |
| 1.5 | 0 | 0.0007 | 443 |
| 1.5 | 860 (2) | 0.00018 | 159 |
| 1.5 | 4300 (10) | 0.00024 | 197 |
| 1.5 | 8600 (20) | 0.00024 | 197 |
| OsPA | | | |
| 1.5 | 0 | 0.0006 | 394 |
| 1.5 | 860 (2) | 0.0002 | 172 |
| 1.5 | 4300 (10) | 0.00022 | 185 |
| 1.5 | 8600 (20) | 0.00020 | 172 |

The samples containing 860 and 4300 nm (2 and 10 IU) heparin clotted within two minutes and showed no latent clotting. The clot was solid but not as solid as those observed in the samples that did not contain heparin. The 860 nM (2 IU) heparin-containing sample gave a thrombin activity that was about 25% of that of the plasma samples not containing heparin. In contrast, the sample containing 8600 nM (20 IU) heparin produced a "jelly-like" clot (more similar to the clots formed in the thrombin-containing samples) within 3 minutes the sample then exhibited latent clotting within 5 minutes. In Figures 33 and 34, the reason for the shape of the progress curves at higher heparin concentrations is unknown.

Example 3h: Estimation of amount of thrombin generated by BD RST tube using normal pooled citrated plasma, and the effect of heparin

The purpose of this experiment was to provide an estimate of the amount of thrombin generated and present in the active form in the BD RST tube filled with different volumes of plasma. Additionally the effect of heparin on the clotting ability of the BD RST tubes was also studied.

Normal pooled citrated plasma (< 24 hours old) was obtained from the Coagulation Laboratory, Pathology Queensland, Princess Alexandra Hospital.

For the standard curve, the BD RST tubes contained 1, 2, 3 and 4 mL of citrated plasma and 50 $\mu$L of 0.5 M CaCl$_2$. The tubes were inverted 8-10 times to mix and incubated for 5 minutes. The clot was ringed using two wood applicator sticks, removed and discarded.

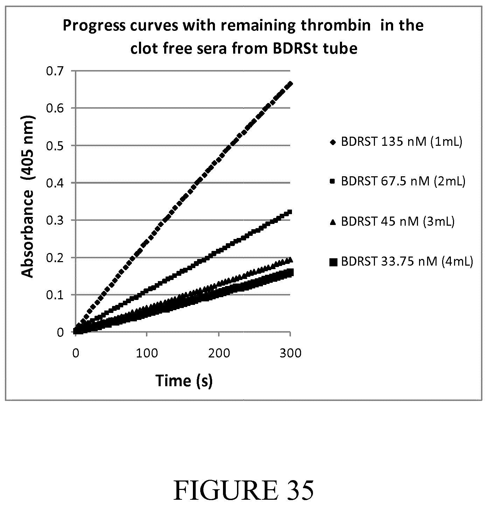

The spectrophotometric cuvette contained 930 $\mu$L of buffer, 50 $\mu$L of S-2238 and 20 $\mu$L of the clear serum that was remaining after the clot was removed from each BD RST tube above. The absorbance change was monitored at 405 nM for 5 minutes, and the results are shown in Figure 35. Thrombin concentrations were estimated from the slopes using the standard curve in Figure 16, and plotted against the slopes from Figure 35. The original BD RST tube thrombin concentrations and remaining thrombin concentrations in the clot free sera from the four samples were are as follows; 135 versus 4.3; 67.5 versus 1.4; 45 versus 0.5; and 33.8 versus 0.3 nM respectively. It is likely that most of the thrombin was removed during the clot removal as it would be bound to the clot fibrin.

Unlike the samples with pure thrombin described in Example 3g, each sample in each of the BD RST tubes clotted within one minute and no latent clotting was observed. However, the clot strength was much weaker that those observed with PtPA or OsPA described in Example 3g.

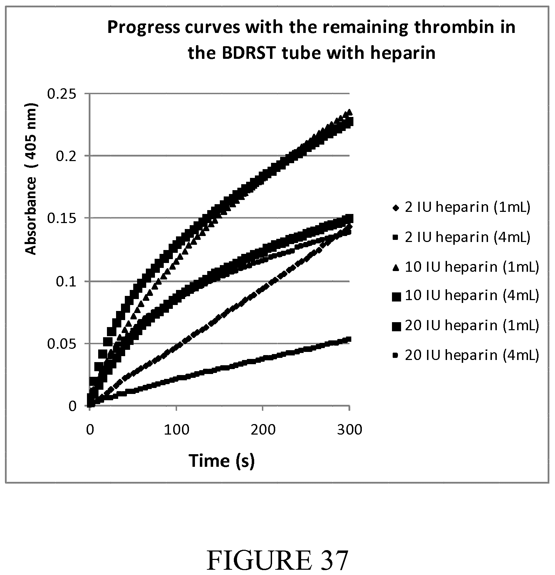

For the samples containing different volumes of citrated plasma and different heparin concentrations, the absorance change of each sample was monitored at 405 nM for 5 minutes, and the results are shown in Figure 37. The reason for the shape of the progress curves at high heparin concentrations is unknown.

There were no samples that produced complete clotting in the presence of any of the three heparin concentrations. The strongest clot was observed with the samples containing 830 nM (2 IU) heparin and one mL of plasma, although the clot was loose by comparison to the solid clots in the absence of heparin. The weakest clots that formed were those in the tube filled with 4 mL of plasma and containing 8600 nM (20 IU) heparin. The clot was "fairy-floss" like and only in part of the sample. All tubes containing heparin showed some latent clotting after 5 minutes. The actual curve shape with the 4300 and 8600 nM (10 and 20 IU) heparin was very similar to the curves observed with PtPA and OsPA.

The slopes and the estimation of active thrombin in the presence of the different heparin concentrations and the different thrombin concentrations are shown in Table 23.

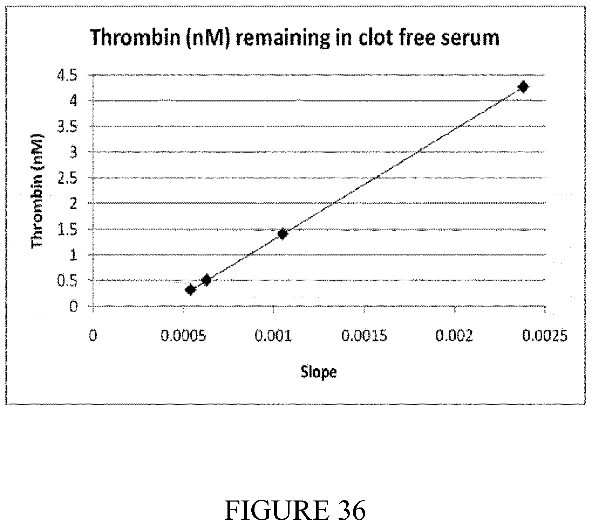

Table 23: Estimation of the active thrombin in the presence of different concentrations of heparin and different concentrations of thrombin from BD RST tubes, using the regression equation from the standard curve in Figure 36.

| [Heparin] (IU) [nM] | [BD RST thrombin] per mL (nM) | Slope | Estimated [active BDRST thrombin] (nM) | Ratio of decrease in presence of heparin (column 2/column 4) |
|---|---|---|---|---|
| 2 [830 nM] | 135 | 0.00050 | 30 | 4.5 |
| 2 [830 nM] | 33.8 | 0.00016 | 6 | 5.6 |
| 10 [2150 nM] | 135 | 0.00050 | 30 | 4.5 |
| 10 [2150 nM] | 33.8 | 0.00024 | 12 | 3.0 |
| 20 [8300 nM] | 135 | 0.00042 | 25 | 5.4 |
| 20 [8300 nM] | 33.8 | 0.00020 | 9 | 3.8 |

The results showed that the same nominal thrombin concentrations (in column 2) produced similar active thrombin concentrations in the presence of different heparin concentrations. Thrombin concentrations measured after clotting were lower by several fold than the nominal concentrations.

EXAMPLE 4: CLOTTING OF WHOLE BLOOD SAMPLES FROM HEALTHY PARTICIPANTS BY PROTHROMBIN ACTIVATORS

Example 4a: Clotting by ecarin

For this experiment, ecarin, purified from *Echis carinatus* venom, was purchased from Sigma (catalogue number EO 504-1 VL; batch number 128K 1536). Citrated blood (pool of three samples with normal coagulation profiles) was obtained from Pathology Queensland, Princess Alexandra Hospital, Queensland, Australia.

One vial of ecarin (50 units) was reconstituted in 100 of $H_2O$ resulting in a stock concentration of 500 units/mL. Dilutions of this stock in distilled water (1:100, *i.e.* 5 units/mL; 1:1000, *i.e.* 0.5 units/mL; 1:10000, *i.e.* 0.05 units/mL) were used in TEG assays. Each assay mixture consisted of 310 μL citrated blood, 20 μL 0.2 M $CaCl_2$, and 10 μL saline (control) or ecarin dilution. Therefore, the concentration range of ecarin in the assay mixture was 0 to 0.15 units/mL. All experiments were done in duplicate. The results are shown in Table 24.

Table 24: TEG results for clotting of re-calcified citrated blood samples by ecarin.

| Ecarin (units/mL) | R(min) | TMA(min) | α(deg) | MA(mm) |
|---|---|---|---|---|
| control | 6.95 | 26.45 | 52.65 | 65.60 |
| 0.0015 | 7.3 | 27.60 | 59.80 | 65.65 |
| 0.015 | 4.5 | 17.90 | 74.15 | 67.25 |
| 0.15 | 2.1 | 15.75 | 69.25 | 73.80 |

Addition of ecarin to recalcified citrated blood resulted in a concentration dependent decrease in both R-time and TMA, and a small increase in MA. At the highest concentration tested, 0.15 units/mL, ecarin reduced the R-time from 6.95 to 2.1 minutes and the TMA from 26.45 to 15.75 minutes. The lowest concentration of ecarin tested, 0.0015 units/mL, had no significant effect on clotting time and clot strength whereas ecarin at a concentration of 0.015 units/mL had an intermediate effect.

These results suggest that ecarin would also be an effective prothrombin activator to rapidly produce serum for analyte measurements in a clinical laboratory.

Example 4b: Clotting of normal "pooled" re-calcified citrated blood by six prothrombin activators

Purified preparations of ecarin, carinactivase-1, carinactivase-2, PtPA, OsPA, and notecarin prepared as described in Example 1 were used. Clotting of normal pooled citrated blood (as in Example 4a) by the six prothrombin activators, by thrombin, and in BD RST tubes was studied by TEG, as described in Example 4a.

The results are provided in Table 25.

Table 25: TEG clotting study with normal "pooled" re-calcified citrate blood with the different procoagulants.

| Procoagulant (nM) | R (min) | MA (mm) | TMA (min) |
|---|---|---|---|
| **Ecarin** | | | |
| 32 | 1.2 | 60.5 | 18.2 |
| 9 | 2.2 | 61.6 | 20.1 |
| 3.2 | 3.4 | 59.0 | 21.6 |
| 0.9 | 6.5 | 59.5 | 29.7 |
| **Carinactivase-1** | | | |
| 50 | 3.3 | 69.1 | 21.7 |
| 32 | 4.6 | 68.5 | 27.1 |
| 9 | 6.2 | 63.9 | 31.4 |
| 3.2 | 7.4 | 64.9 | 30.3 |
| **Carinactivase-2** | | | |
| 50 | 1.8 | 64.9 | 19.9 |
| 32 | 2.1 | 68.2 | 20.0 |
| 9 | 3.3 | 67.8 | 19.7 |
| 3.2 | 4.6 | 68.0 | 23.6 |
| **OsPA** | | | |
| 0.9 | 0.7 | 63.7 | 18.4 |
| 0.09 | 2.9 | 61.8 | 20.5 |
| 0.009 | 4.2 | 63.5 | 22.5 |
| 0.0009 | 6.2 | 59.3 | 24.8 |
| **PtPA** | | | |
| 0.9 | 1.5 | 62.4 | 22.1 |
| 0.09 | 2.6 | 63.1 | 23.6 |
| 0.009 | 3.9 | 63.9 | 24.8 |
| 0.0009 | 5.8 | 53.4 | 28.1 |
| **Notecarin** | | | |
| 3.2 | 1.2 | 62.1 | 20.2 |
| 0.9 | 1.8 | 63.7 | 18.3 |
| 0.09 | 3.7 | 63.2 | 20.3 |
| 0.009 | 7.3 | 59.0 | 26.3 |

(continued)

| Thrombin | | | |
|---|---|---|---|
| 32 | 2.3 | 65.2 | 22.8 |
| 9 | 3.5 | 64.0 | 23.8 |
| 3.2 | 5.5 | 62.4 | 27.6 |
| BD RST | | | |
| 24 | 0.8 | 54.8 | 24.8 |
| 6 | 1.5 | 59.3 | 23.7 |

These results provided further support to the plasma clotting findings (Example 3) that all prothrombin activators clot blood very efficiently; and that the group C prothrombin activators (PtPA and OsPA) were the most effective in clotting the normal "pooled" re-calcified citrated whole blood. For example, 9 pM PtPA and OsPA achieved R times of about 5 minutes, and the maximum clot strength as confirmed by the MA results. The next most effective prothrombin activator was notecarin, the group D prothrombin activator, which was able to produce an R time of 5 minutes and maximum clot strength with a minimum concentration between 0.09 and 0.009 nM. Although notecarin itself does not contain FVa, the FV present in plasma is converted to active form by the small amount of thrombin formed by the notecarin FXa. The next most effective prothrombin activator was ecarin, the group A prothrombin activator, which was able to achieve the desired R time and maximum clot strength with a minimum concentration between 0.9 and 3.2 nM. The least effective prothrombin activators were the group B prothrombin activators, carinactivase-1 and carinactivase-2 which required between $\geq 9$ and $\geq 3$ nM concentrations respectively to achieve R times of < 5 minutes and maximum clot strength. The required minimum thrombin concentration to achieve the desired R time and maximum clot strength was $\geq 3$ nM.

Example 4c: Comparison of commercially available serum tubes and PtPA-containing tubes

Blood from a healthy participant was drawn sequentially into the following tubes using an inline butterfly needle:

(1) a 4.0 mL Greiner VacuetteTM No Additive tube;

(3) Greiner Vacuette™ serum tube;

(4) BD Vacutainer™ serum tube;

(5) Sarstedt serum tube;

(6) Terumo RT tube; and

(7) Terumo plain tube.

The tubes were filled with freshly collected blood to the required fill mark and mixed immediately for 30 seconds by gentle inversion 8-10 times. Then a 340 $\mu$L sample was transferred into a TEG cup for immediate analysis. This method of blood collection allowed for rapid transfer of a blood sample for TEG analysis immediately after mixing in the commercially available tube so that no anticoagulant or re-calcification in TEG analysis was required. An aliquot, 330 $\mu$L of the blood from the plain tube (1) was immediately transferred to a TEG cup and 10 $\mu$L (1.41 $\mu$g) of PtPA was added (2), and analysis was immediately started.

All TEG assays were done in duplicate. The TEG parameters (average of the duplicate samples) are shown in Table 26 below.

Table 26: TEG results for blood from a healthy participant in different tubes

| Sample | R(min) | TMA(min) | $\alpha$(deg) | MA(mm) |
|---|---|---|---|---|
| (1) Plain tube | 14.3 | 46.5 | 26.9 | 49.8 |
| (2) PtPA | 0.8 | 12.2 | 70.8 | 54.9 |

(continued)

| Sample | R(min) | TMA(min) | $\alpha$(deg) | MA(mm) |
|---|---|---|---|---|
| (3) Greiner | 3.0 | 23.5 | 71.2 | 68.2 |
| (4) BD serum | 4.9 | 25.3 | 71.0 | 68.1 |
| (5) Sarstedt | 5.1 | 23.8 | 70.8 | 69.1 |
| (6) Terumo RT | 9.6 | 22.5 | 63.2 | 58.1 |
| (7) Terumo | 13.4 | 31.2 | 38.2 | 54.6 |

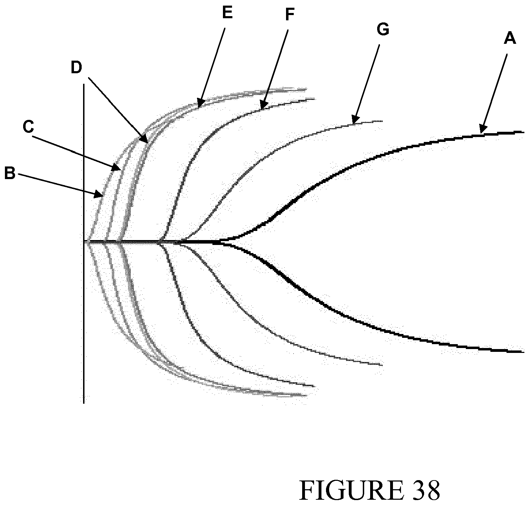

The TEG parameters of Table 26 are shown in the TEG plot in Figure 38 where: trace A represents the plain plastic tube (1); trace B represents the plain tube with added PtPA (2); trace C represents the Greiner serum tube (3); trace D represents the BD serum tube (4); trace E represents the Sarstedt serum tube (5); trace F represents the Terumo RT tube (6); and trace G represents Terumo serum tube (7).

The blood sample in the PtPA tube (2) clotted most rapidly (R time = 0.8 minutes) in the TEG cup. R times for the commercial tubes (3)-(7) varied from 3.0 minutes for the Greiner tube (3) to 13.4 minutes for the Terumo tube (7). These R times can be compared with the R time of 14.3 minutes for the plain tube (1). It is believed that the differences in R times between the commercial tubes reflect the presence of different procoagulants and additives, and/or different amounts of procoagulants or additives in these tubes.

Clot formation in all tubes was also visually observed. The commercially available tubes were allowed to clot for the standard time of 30 minutes. The PtPA specimen was allowed to stand for 5 minutes. The tubes were visually inspected for clot formation prior to loading in the centrifuge. All tubes were centrifuged at 3000 g for 10 minutes at 20°C. The specimens were stored at room temperature, ~21°C for up to 6 hours and inspected on hourly intervals for latent clot formation. All tubes including the PtPA-containing tube formed solid clots, with the PtPA-containing tube forming a solid immobile clot within 2 minutes. No latent clotting was observed in any of the tubes up to 6 hours after collection.

This example shows that inclusion of a small amount of PtPA led to faster clotting than in any of the commercial tubes tested, demonstrating the potential for rapid production of serum to provide a faster turn-around time for analyte measurements in a clinical laboratory.

Example 4d: Clotting of normal citrated blood by PtPA, OsPA, ecarin, and some commercial tubes

The purpose of this experiment was to investigate how three prothrombin activators: PtPA, OsPA, and ecarin compare to three routinely used commercial clotting tubes: Greiner serum, BD SST II and BD RST.

Blood from a healthy participant was collected into a citrate-containing tube.

For the prothrombin activators, 30 $\mu$L of activator (concentration of PtPA and OsPA 0.56 nM; ecarin 5.3 nM in TEG cup), 20 $\mu$L of 0.2 M $CaCl_2$ and 290 $\mu$L of citrated blood was added to the TEG cup.

For the commercial serum tubes, 1 mL of distilled water was added and allowed to mix on a roller for ~2 hours to dissolve the content of the tube. The BD RST contains 135 nM bovine thrombin (33.8 nM/mL of blood), whereas the concentration used in this experiment in the TEG cup was 53 nM/mL. For the commercial serum tubes 45 $\mu$L of the dissolved content, 20 $\mu$L of 0.2 M $CaCl_2$ and 275 $\mu$L of citrated blood was added to the TEG cup.

The results are shown in Table 27.

Table 27: TEG data with PtPA, OsPA, ecarin, and routinely used commercial serum tubes with native blood from a healthy participant.

| Tube | Tube Catalogue # | R time (min) | MA | TMA |
|---|---|---|---|---|
| Greiner Vacuette Serum | 455078 | 2.8 | 62.5 | 20.6 |
| BD SST II | 367958 | 4.3 | 62.8 | 23.5 |
| BD RST (18 nM) | 368771 | 0.5 | 58.8 | 20.2 |
| PtPA (0.53 nM) | | 1.4 | 65.6 | 19.8 |
| OsPA (0.53 nM) | | 1.5 | 64.0 | 19.7 |
| Ecarin (5.3 nM) | | 2.6 | 64.3 | 19.3 |
| Native blood (plain tube) | | 6.4 | 67.6 | 27.8 |

EP 2 619 540 B1

The results demonstrated that the routinely used commercial serum tubes vary in the time at which significant clot formation was detected by the TEG (R time), with the time ranging from 0.5-6.4 minutes for re-calcified citrated blood from a healthy participant. The manufacturers of these tubes recommend a minimum clotting time of 30 minutes prior to centrifugation of the serum tubes. The results also demonstrated that PtPA and OsPA at 0.5 nM gave an R time of <2 minutes with an equivalent clot strength which make them very suitable for evaluation as procoagulant to be used in producing serum rapidly.

EXAMPLE 5: CLOTTING OF WHOLE BLOOD SAMPLES CONTAINING ANTICOAGULANTS BY PROTHROMBIN ACTIVATORS

Many blood samples are taken from patients being treated with anticoagulants such as heparin, warfarin and citrate. In addition, some blood samples are collected into tubes containing anticoagulants such as EDTA and citrate; or are contaminated with anticoagulants during the collection process. The following experiments were performed to study the clotting of blood samples containing anticoagulants by prothrombin activators.

Example 5a: Concentration dependent clotting of citrated or EDTA treated blood from a healthy participant

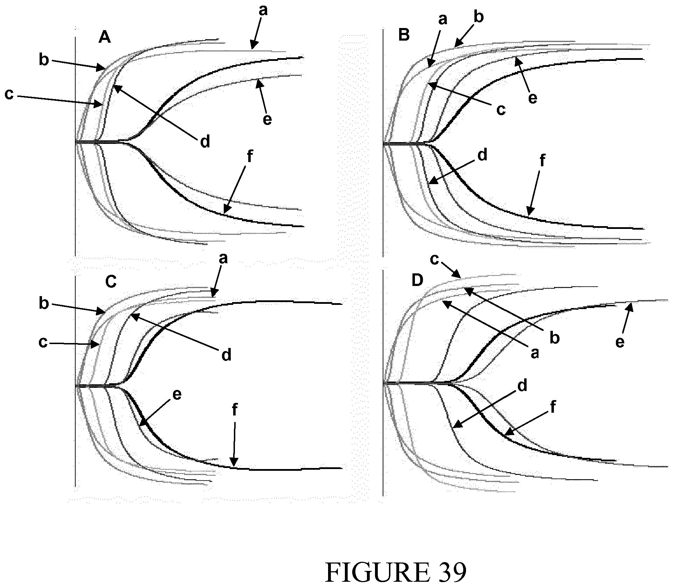

TEG analysis was performed where each TEG assay mixture consisted of 310 $\mu$L of citrated or EDTA treated blood, 20 $\mu$L of CaCl$_2$ (0.2 M) and 10 $\mu$L of saline or prothrombin activator solution at a range of concentrations (stock solutions - PtPA, 4.8 mg/mL or OsPA, 2.0 mg/mL). The results are shown in Figure 39 and in Tables 28 and 29 or PtPA and Tables 30 and 31 for OsPA.

Table 28: TEG results for clotting of normal citrated blood with PtPA

| PtPA ($\mu$g/mL) | R(min) | TMA(min) | $\alpha$(deg) | MA(mm) |
|---|---|---|---|---|
| 0 | 10.9 | 40.4 | 23.8 | 55.2 |
| 0.00141 | 10.3 | 39.6 | 26.2 | 44.0 |
| 0.0141 | 4.6 | 22.8 | 54.0 | 67.2 |
| 0.141 | 3.8 | 23.5 | 67.7 | 67.0 |
| 1.41 | 1.0 | 21.2 | 72.4 | 64.8 |
| 14.1 | 0.3 | 20.2 | 74.7 | 59.7 |

Table 29: TEG results for clotting of re-calcified EDTA-treated blood with PtPA

| PtPA ($\mu$g/mL) | R(min) | TMA(min) | $\alpha$(deg) | MA(mm) |
|---|---|---|---|---|
| 0 | 9.8 | 39.2 | 25.8 | 54.6 |
| 0.00141 | 9.1 | 31.5 | 34.9 | 60.9 |
| 0.0141 | 6.8 | 27.0 | 62.4 | 64.4 |
| 0.141 | 5.5 | 25.3 | 62.0 | 64.4 |
| 1.41 | 1.3 | 19.8 | 72.1 | 66.3 |
| 14.1 | 0.3 | 21.2 | 70.1 | 60.9 |

Table 30: TEG results for clotting of normal citrated blood with OsPA

| OsPA ($\mu$g/mL) | R(min) | TMA(min) | $\alpha$(deg) | MA(mm) |
|---|---|---|---|---|
| 0 | 9.0 | 31.3 | 29.5 | 55.0 |
| 0.000588 | 9.0 | 24.0 | 34.2 | 49.0 |
| 0.00588 | 5.9 | 21.3 | 54.3 | 62.0 |
| 0.0588 | 2.8 | 15.3 | 65.9 | 54.8 |

(continued)

| OsPA (μg/mL) | R(min) | TMA(min) | $\alpha$(deg) | MA(mm) |
|---|---|---|---|---|
| 0.588 | 0.8 | 17.9 | 73.6 | 64.2 |
| 5.88 | 0.3 | 19.8 | 72.3 | 58.2 |

Table 31: TEG results for clotting of re-calcified EDTA-treated blood with OsPA

| OsPA (μg/mL) | R(min) | TMA(min) | $\alpha$(deg) | MA(mm) |
|---|---|---|---|---|
| 0 | 13.9 | 40.8 | 31.2 | 50.7 |
| 0.000588 | 16.3 | 45.4 | 29.8 | 53.7 |
| 0.00588 | 9.8 | 30.0 | 36.2 | 62.4 |
| 0.0588 | 3.3 | 18.3 | 64.3 | 70.7 |
| 0.588 | 0.8 | 17.0 | 73.1 | 64.0 |
| 5.88 | 0.3 | 20.8 | 77.0 | 61.1 |

The R time of re-calcified blood without addition of a prothrombin activator was approximately 10 minutes which decreased to 1 minute or 1.3 minutes in the presence of 1.41 μg/mL PtPA (Tables 28 and 29). The rate of clotting was rapid (angle = 54-72°) (between 0.0141 and 1.41) and full strength clots with MA values of 60-70 mm were obtained. The R time of re-calcified blood without addition of a prothrombin activator of approximately 10 minutes was decreased to 0.8 minutes in the presence of 0.59 μg/mL OsPA (Tables 30 and 31).

It is believed that the very short R-times and large $\alpha$ observed on addition of the PtPA or OsPA may be explained by generation of a massive burst of thrombin, while downstream reactions (conversion of fibrinogen to fibrin, activation of factor XIII, cross-linking of fibrin monomers) are rate limiting and result in a comparatively smaller improvement of TMA.

The TEG results from Tables 28, 28, 30 and 31 are shown in Figure 39, where TEG plot A shows the Table 28 results (citrated blood and PtPA); TEG plot B shows the Table 29 results (EDTA treated blood and PtPA); TEG plot C shows the Table 30 results (citrated blood and OsPA) and TEG plot D shows the Table 31 results (EDTA treated blood and OsPA).

In Figure 39, the traces labelled A in each TEG plot represent 14.1 μg/mL for PtPA and 5.88 μg/mL for OsPA ; the traces labelled B represent 1.41 μg/mL for PtPA and 0.588 μg/mL for OsPA; the traces labelled C represent 0.141 μg/mL for PtPA and 0.0588 μg/mL for OsPA; the traces labelled D represent 0.0141 μg/mL for PtPA and 0.00588 μg/mL for OsPA; the traces labelled E represent 0.00141 μg/mL for PtPA and 0.000588 μg/mL for OsPA; and the traces labelled F represent 0 μg/mL PtPA or 0 μg/mL OsPA.

In summary, PtPA and OsPA both clot re-calcified citrated and EDTA-treated blood in a highly efficient concentration-dependent manner. Thus in practice, blood collected in an EDTA tube could be rapidly clotted to produce serum by adding PtPA or OsPA for biochemical and other laboratory analysis.

Example 5b: Clotting of EDTA plasma and blood obtained from a healthy participant

As mentioned in Example 3d above, a small but significant percentage of samples (serum or lithium heparin plasma samples) received for biochemical analysis is contaminated by EDTA. It is therefore important to determine if prothrombin activators are effective in clotting EDTA plasma and EDTA whole blood. The purpose of this experiment was to do so, and to make a comparison of EDTA plasma and EDTA whole blood.

Blood from a healthy patient was collected into a Greiner EDTA tube containing 1.8 mg/mL (6.2 mmol) EDTA when filled to the fill mark. At this concentration, the EDTA is able to bind all the metal ions (including $Ca^{2+}$, $Mg^{2+}$, $Fe^{2+}$, $Zn^{2+}$, and $Cu^{2+}$) present in the tube. To obtain EDTA plasma, a portion of each sample was centrifuged under standard protocols.

The Clotek tube contained 100 μL of EDTA plasma, 100 μL of Tris Buffer (150 mM Tris HCl, 150 mM NaCl, pH 7.4), 50 μL of 0.2 M $CaCl_2$ or Tris buffer and 50 μL of each procoagulant.

The TEG cup contained 20 μL of 0.2 M $CaCl_2$ or saline, 60 μL of procoagulant and 260 μL of the EDTA blood.

For the BD RST experiment the contents of two tubes were dissolved with 1 and 4 mL of distilled water respectively, mixed for 5 minutes and 60 μL of the content used in the TEG cup as the procoagulant.

The results of the plasma clotting study are shown in Table 32 and in Table 33.

Table 32: Clotting times for EDTA plasma obtained from a healthy participant with different procoagulants (prothrombin activators and thrombin) at different concentrations, with calcium.

| [Procoagulant] (nM) | With Ca$^{2+}$ (seconds) | | | | | | |
|---|---|---|---|---|---|---|---|
| | Ecarin | CA-1 | CA-2 | PtPA | OsPA | Notecarin | Thrombin |
| 50 | 68.9 | 165.4 | 82.5 | 6.5 | 10.0 | 85.5 | 12.7 |
| 10 | 127 | 232 | 101 | 13.5 | 15.8 | 117 | 43.0 |
| 1 | 224 | >300 | 151 | 35.7 | 42.7 | 206 | >300 |
| 0.1 | ND | ND | ND | 98.6 | 123 | ND | >300 |
| "ND" means this was not determined. | | | | | | | |

Table 33: Clotting times for EDTA plasma obtained from a healthy participant with different procoagulants (prothrombin activators and thrombin) at different concentrations, without calcium.

| [Procoagulant] (nM) | Without Ca$^{2+}$ (seconds) | | | | | | |
|---|---|---|---|---|---|---|---|
| | Ecarin | CA-1 | CA-2 | PtPA | OsPA | Notecarin | Thrombin |
| 50 | >300 | >300 | >300 | 31.7 | 56.4 | 132 | 17.9 |
| 10 | >300 | | | 48.7 | 68.2 | 242 | 53.2 |
| 1 | >300 | | | 86.4 | 107 | >300 | >300 |
| 0.1 | | | | 190 | 251 | | >300 |

The results of the BD RST plasma clotting study are shown in Table 34.

Table 34: Clotting times for the BD RST tubes, with and without calcium.

| BDRST Tube (nM) | With Ca$^{2+}$ (seconds) | Without Ca$^{2+}$ (seconds) |
|---|---|---|
| 1 mL (23) | 12.3 | 44.9 |
| 4 mL (6) | 34.3 | 101 |

EDTA is a metal chelator which chelates the calcium and in turn prevents the normal clotting process from occurring.

These results show that the calcium-dependent prothrombin activators carinactivase-1 (CA-1) and carinactivase-2 (CA-2) did not clot EDTA plasma even at 50 nM concentration in the pre-requisite time of < 5 minutes, although they were able to after re-calcification. The effect on ecarin was significant: without re-calcification the clotting time was more than 5 minutes. The addition of calcium in excess caused only a moderate decrease in clotting time with PtPA and OsPA (for example, PtPA at 0.1 nM, 190 seconds to 98.6 seconds). Although other examples described herein have suggested that notecarin is less effective as a procoagulant than PtPA and OsPA, the results showed it was the prothrombin activator that was the least affected by the absence of calcium. The addition of calcium in excess caused 3-fold increase in the clotting of the plasma with thrombin.

The results from the whole blood clotting study are shown in Table 35.

Table 35: TEG results showing the effect of different procoagulants on EDTA blood with and without the presence of calcium.

| Procoagulant (nM) | R (min) | MA (mm) | TMA (min) |
|---|---|---|---|
| Ecarin | | | |
| 53 (re-calcified) | 2.5 | 56.8 | 22.1 |
| 53 (not re-calcified) | 16.9* | ND | ND |

(continued)

| Carinactivase - 1 | | | |
|---|---|---|---|
| 53 (re-calcified) | 4.8 | 57.6 | 29.5 |
| 53 (not re-calcified) | 16.9* | ND | ND |
| **Carinactivase - 2** | | | |
| 53 (re-calcified) | 2.6 | 55.7 | 25.6 |
| 53 (not re-calcified) | 34.8* | ND | ND |
| **OsPA** | | | |
| 53 (re-calcified) | 1.6 | 41.5 | 21.6 |
| 53 (not re-calcified) | 17.7* | ND | ND |
| **PtPA** | | | |
| 53 (re-calcified) | 0.5 | 58.2 | 22.9 |
| 53 (not re-calcified) | 17.8* | ND | ND |
| 1 (re-calcified) | 1.6 | 64.0 | 22.9 |
| **PtPA** | | | |
| 690 nM (Run 1) (not re-calcified) | 50.1* | ND | ND |
| 690 nM (Run 2) (not re-calcified) | 50.3* | ND | ND |
| **Notecarin** | | | |
| 53 (re-calcified) | 1.9 | 54.2 | 25.8 |
| 53 (not re-calcified) | 17.2* | ND | ND |
| **Thrombin** | | | |
| 53 (re-calcified) | 2.6 | 49.9 | 31.5 |
| 53 (not re-calcified) | 45.4* | ND | ND |
| **BD RST** | | | |
| 23 (re-calcified) | 1.6 | 45.4 | 24.0 |
| 23 (re-calcified) | 1.8 | 57.7 | 28.3 |
| 6 (re-calcified) | 30.1* | ND | ND |
| 6 (re-calcified) | 32.5* | ND | ND |
| **Re-calcified EDTA blood alone** | | | |
| Run 1 | 11.2 | 58.5 | 40.8 |
| Run 2 | 10.6 | 56.7 | 38.5 |
| * means measurement was stopped, and ND means this was not determined. | | | |

This experiment showed that EDTA whole blood presents a difficult challenge for the procoagulants. Unlike with EDTA plasma, none of the procoagulants were able to clot EDTA blood in the absence of re-calcification. Clotting was not achieved even with very high PtPA concentration (690 nM). However, all procoagulants were able to clot the EDTA blood when it was re-calcified. Rapid and complete clotting was achieved even with a very low PtPA concentration (1nM). EDTA is the anticoagulant of choice for preserving cellular morphology, and by chelating calcium the EDTA also prevents platelet activation and platelet clumping.

Two additional plasma clotting experiments using 30 nM PtPA were performed. In the first, water was added to the Clotek tube instead of Tris buffer and the sample still clotted in 6.4 seconds. In the second, no buffer was used, only 200 μL EDTA plus 50 μL PtPA and the clotting time was 5.4 seconds. For the TEG study, the following solutions were added to a TEG cup 50 μL Tris buffer, 60 μL PtPA, 230 μL EDTA blood and still no clotting was observed.

Additionally, a BD RST tube was filled to the fill mark with EDTA blood and after 60 minutes it showed very partial clotting. It is obvious from these experiments that whenever EDTA may be present, addition of calcium with the procoagulant should be considered in order to achieve clotting.

Example 5c: Clotting of blood from warfarin-treated participants

The effect of PtPA and OsPA on coagulation of blood samples from two participants ("W1" and "W2") on warfarin was determined.

The coagulation parameters of each participant, determined by standard coagulation protocols, are shown in Table 36, where:

aPTT = activated partial thromboplastin time;

PT = prothrombin time;

INR = international normalised ratio; and

Fib-D = prothrombin time derived fibrinogen.

Table 36: Coagulation parameters for the participants on anticoagulant treatment

| Participant | aPTT (s) | PT (s) | INR | Fib-D (g/L) |
|---|---|---|---|---|
| **W1** | 30.27 | 21.3 | 2.08 | 1.55 |
| **W2** | 36.15 | 27.4 | 2.61 | 5.20 |
| **Normal plasma** | 30-32 | 10-11 | 1.0 | 2.5-4.0 |

The results in Table 36 confirm that the blood from participants W1 and W2 was anticoagulated as expected.

TEG analysis was then performed. Each TEG assay mixture consisted of 310 $\mu$L of citrated blood, 20 $\mu$L of CaCl$_2$ (0.2 M) and 10 $\mu$L of saline or solution of prothrombin activator (PtPA or OsPA) resulting in final assay concentrations of prothrombin activator of between 0 and 14.1 $\mu$g/mL.

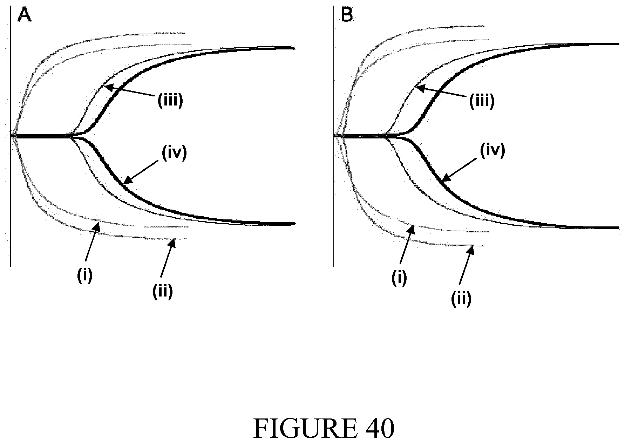

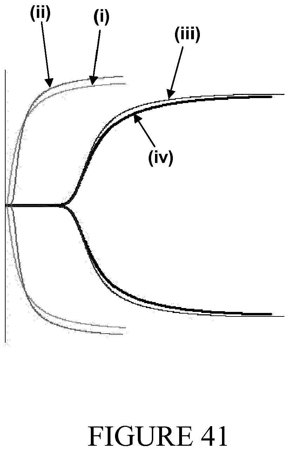

The TEG results are shown in Tables 37, 38 and 39 below. The values therein are derived from the TEG data as shown in Figures 40 and 41.

Table 37: TEG results for clotting of citrated blood of W1 with OsPA

| OsPA ($\mu$g/mL) | R(min) | TMA(min) | $\alpha$(deg) | MA(mm) |
|---|---|---|---|---|
| 0 | 12.2 | 35.5 | 23.6 | 50.4 |
| 0.0588 | 4.4 | 22.9 | 68.1 | 59.5 |
| 0.588 | 1.6 | 16.7 | 73.8 | 60.8 |
| 5.88 | 0.6 | 17.8 | 67.5 | 53.0 |

Table 38: TEG results for clotting of citrated blood of W1 with PtPA

| PtPA ($\mu$g/mL) | R(min) | TMA(min) | $\alpha$(deg) | MA(mm) |
|---|---|---|---|---|
| 0 | 12.2 | 35.5 | 23.6 | 50.4 |
| 0.0141 | 9.4 | 31.5 | 48.4 | 54.3 |
| 1.41 | 0.8 | 18.8 | 69.7 | 59.6 |
| 14.1 | 0.4 | 20.6 | 65.1 | 53.1 |

Table 39: TEG results for clotting of citrated blood of W2 with PtPA

| PtPA (µg/mL) | R(min) | TMA(min) | α(deg) | MA(mm) |
|---|---|---|---|---|
| 0 | 12.3 | 37.6 | 49.1 | 60.8 |
| 0.0141 | 7.7 | 26.1 | 62.9 | 66.4 |
| 1.41 | 1.3 | 17.9 | 77.7 | 73.3 |
| 14.1 | 0.4 | 18.4 | 79.6 | 69.4 |

TEG plots of the results in Tables 37 and 38 are provided in Figure 40 where TEG plot A shows the results from Table 37 (OsPA) and TEG plot B shows the results from Table 38 (PtPA). In Figure 40, for the A plot the trace labelled (i) shows the results for 5.88 µg/mL OsPA, the trace labelled (ii) for 0.588 µg/mL OsPA, the trace labelled (iii) for 0.0588 µg/mL OsPA, and the trace labelled (iv) for 0 µg/mL. In Figure 40, for the B plot the trace labelled (i) shows the results for 14.1 µg/mL PtPA, the trace labelled (ii) for 1.41 µg/mL PtPA, the trace labelled (iii) for 0.0141 µg/mL PtPA, and the trace labelled (iv) for 0 µg/mL.

In Figure 41, the trace labelled (i) shows the results for 14.1 µg/mL PtPA, the trace labelled (ii) for 1.41 µg/mL PtPA, the trace labelled (iii) for 0.0141 µg/mL PtPA, and the trace labelled (iv) for 0 µg/mL.

In summary, both PtPA and OsPA at low concentrations rapidly clotted blood samples from participants on warfarin therapy, with R times, α values and MA values similar to those in comparable experiments with blood from a healthy participant (see Tables 28-31 above). These results confirm the observation on warfarin plasma in Example 3c.

Example 5d: Clotting of blood from a high dose heparinised participant with PtPA and OsPA

Blood was taken from a participant ("H1") who had been given 35,000 IU of heparin 30 minutes prior to the blood sample being taken. The concentration of heparin was calculated to be approximately 7 IU/mL of blood. Blood was collected in a plain syringe and then transferred to a Greiner Vacuette™ citrate tube.

The coagulation parameters of the participant were measured and are shown in Table 40.

Table 40: Coagulation parameters for the heparinised participant.

| Participant | aPTT(s) | PT(s) | INR | TT(s) | PTNH(s) | REPT(s) | Fib-D(g/L) |
|---|---|---|---|---|---|---|---|
| H1 | failed | 41.2 | 3.75 | failed | 17.75 | 15.28 | 2.40 |

All coagulation parameters in Table 40 are known in the art, being: aPTT = activated partial thromboplastin times; PT = prothrombin time; INR = international normalised ratio; TT = thrombin time; PTNH = protamine sulphate time; REPT = repitalise time; Fib-D = prothrombin time derived fibrinogen.

The results in Table 40 are in the range of expected results for a heavily heparinised sample.

TEG analysis was then performed. Each TEG assay mixture consisted of 310 µL of citrated blood, 20 µL of $CaCl_2$ (0.2 M) and 10 µL of saline or solution of prothrombin activator (PA).

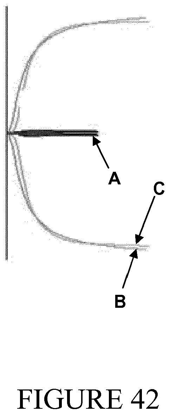

The TEG results are shown in Table 41 below and in Figure 42.

Table 41: TEG results for clotting of blood from a participant on very high doses of heparin.

| PA | PA concentration (µg/mL) | Citrated | | | |
|---|---|---|---|---|---|
| | | R(min) | TMA(min) | α(deg) | MA(mm) |
| | 0 | n.c. | n.c. | n.c. | n.c. |
| PtPA | 14.1 | 0.4 | 20.9 | 72.6 | 64.3 |
| OsPA | 5.9 | 1.3 | 12.8 | 70.6 | 61.3 |

In Table 41 above, "n.c." indicates no clotting.

TEG traces of the results in Tables 41 are provided in Figure 42 where the trace labelled A shows the results for no prothrombin activator; the trace labelled B shows the results for 14.1 µg/mL PtPA; and the trace labelled C shows the results for 5.9 µg/mL OsPA.

At the concentration tested both PtPA and OsPA were able to clot citrated blood from the heparinised participant rapidly. The values for the TEG parameters monitored (R-time, TMA, angle, MA) were comparable with the results obtained from blood of a healthy participant, indicating that the activity of the prothrombin activators was not inhibited by the concentration of heparin present in the blood sample.

Example 5e: Clotting of blood from a fully heparinised participant with a PtP A-containing tube and a BD RST tube.

A 10 mL blood sample was collected from a participant undergoing cardiac surgery who had been given 38,000 IU of heparin 30 minutes before sample collection. The blood was collected in a plain syringe and within 10 minutes delivered to the laboratory for analysis.

The BD RST tube was filled to the fill mark, inverted/mixed for 30 seconds and 340 μL of this blood was transferred to a TEG cup in channel 1.

330 μL of the original heparinised participant blood sample was added to 10 μL of PtPA (final concentration 1.41 μg/mL) in a TEG cup in channel 2.

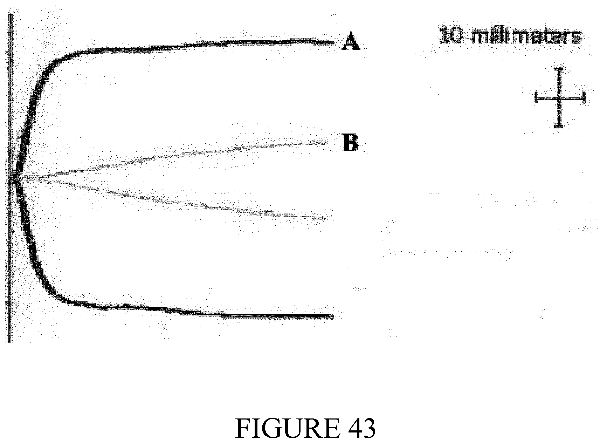

The results of the TEG analysis are shown in Table 42 and in Figure 43.

Table 42: TEG analysis results for heparinised blood in BD RST tube and with PtPA

| Sample | R (min) | K (min) | Angle (deg) | MA (mm) | TMA (min) |
|---|---|---|---|---|---|
| PtPA (outer trace) | 0.7 | 1.3 | 74.4 | 43.8 | 9.8 |
| BD RST tube (inner trace) | 6.2 | N/A | 7.8 | 11.5 | 24.0 |

For the BD RST tube, the R time was 6.2 minutes with a maximum amplitude (MA) value of 11.5 mm, indicating that only partial clotting was achieved. This clot strength did not increase further during the 2 hour period of the analysis.

With PtPA, the R time was 0.7 minutes, the maximum amplitude (MA) was 43.8 mm and the time to maximum amplitude was 9.8 minutes indicating that a strong and stable clot was achieved quickly.

The results from Table 42 are also shown in Figure 43 as a TEG plot where trace "A" is the trace of the TEG cup in channel 2 (PtPA) and trace "B" is the trace of the TEG cup in channel 1 (BD RST tube).

The blood collected in the plain syringe, and the blood in the BD RST tube were also monitored visually for clotting. No clotting was observed in the plain syringe for up to 2 days. Blood in the BD RST tube showed partial clotting at 30 minutes and complete clotting within 24 hours.

In summary, the heavily heparinised sample clotted rapidly and completely in a tube containing PtPA but only very slowly and incompletely in a BD RST tube.

Example 5f: Clotting of blood samples containing heparin by prothrombin activators

The ability of venom prothrombin activators to clot blood from a healthy participant collected in a Greiner lithium heparin tube (18 IU heparin per mL of blood) was determined using TEG analysis and the results are shown in Table 43. The citrate sample was collected for the comparison of the MA values.

Table 43: TEG results with blood collected in Greiner lithium heparin tube from a healthy participant.

| Procoagulant (nM) | R (min) | MA (mm) | TMA (min) |
|---|---|---|---|
| Ecarin | | | |
| 56 (black) | 9.9 | 18.8 | 37.8 |
| 32 (green) | 13.8 | 18.6 | 39.8 |
| Carinactivase - 1 | | | |
| 100 (black) | 35.6* | ND | ND |
| 50 (green) | 34.8* | ND | ND |
| Carinactivase - 2 | | | |
| 56 (black) | 10.4 | 23.0 | 37.8 |
| 34 (green) | 18.0 | 18.5 | 47.5 |
| OsPA (nM) | | | |
| 56 (magenta) | 1.9 | 37.6 | 24.7 |

(continued)

| OsPA (nM) | | | |
|---|---|---|---|
| 34 (pink) | 2.3 | 36.5 | 26.1 |
| 9 (green) | 2.9 | 29.8 | 25.2 |
| 3 (black) | 10.3 | 6.2 | 20.0 |
| PtPA | | | |
| 56 (magenta) | 2.3 | 33.5 | 26.6 |
| 34 (brown) | 2.3 | 39.8 | 27.2 |
| 9 (green) | 2.8 | 31.3 | 25.7 |
| 3 (black) | 6.8 | 14.7 | 25.8 |
| Notecarin | | | |
| 56 (black) | 2.5 | 34.1 | 30.7 |
| 32 (green) | 7.9 | 18.0 | 28.4 |
| BD RST | | | |
| 25 (black) | 35.6* | ND | ND |
| 6 (green) | 35.8* | ND | ND |
| Re-calcified citrate blood | | | |
| Run 1 | 6.3 | 61.2 | 30.1 |
| Run 2 | 6.5 | 61.5 | 27.1 |
| * means measurement was stopped, and ND means this was not determined. | | | |

The results of this experiment with lithium heparin whole blood showed that some of the procoagulants were capable of clotting the heparinised blood at lower procoagulant concentrations, while other procoagulants were unable to clot the blood even at the highest concentration range tested in this experiment. The results show that the group C procoagulants (PtPA and OsPA) were the most effective in clotting the blood collected in Greiner lithium heparin tubes, requiring $\geq$ 9 nM for OsPA and PtPA to achieve R time within 5 minutes. This was significantly lower than the MAs (~61) achieved in the recalcified citrated blood. Although not wishing to be bound by theory, it is believed likely that the ATIII-heparin complex inhibits the thrombin formed, and subsequently prevents proper or stronger clot formation. The minimum concentrations required for the other prothrombin activators are >56 nM for ecarin and carinactivase-2, >100 nM for carinactivase-1, and >32 nM of notecarin. The thrombin concentrations of 6 and 25 nM from the BDRST tubes failed to clot the blood at all.

Again, although not wishing to be bound by theory, it is believed likely that the group C prothrombin activators (OsPA and PtPA) produced a much larger and more sustained burst of thrombin to overwhelm the ATIII-heparin complex that inhibits both thrombin and FXa and subsequently the clotting process.

Example 5g: Clotting of normal "pooled" citrated plasma spiked with different concentrations of heparin

This experiment was performed to determine the required concentrations of the different prothrombin activators to clot plasma spiked with heparin at different concentrations to mimic levels likely to be found in plasma from heparinised patients.

Normal "pooled" citrated plasma had the total calcium adjusted to 2.71 mmol/L and then aliquots of heparin were added to give final heparin concentrations in the Clotek Tube of 0.8, 2.0, 10 and 20 IU/mL which is the range that may be encountered in patient blood samples. Additionally I mL aliquots of the citrated plasma were dispensed in Greiner lithium heparin tubes, and then mixed on a roller for 15 minutes to give lithium heparin concentration of 90 IU/mL. The Clotek tube contained 100 $\mu$L of sodium or lithium heparin plasma, 100 $\mu$L of Tris Buffer (150 mM Tris HCl, 150 mM NaCl, pH 7.4) and 50 $\mu$L of each procoagulant.

The clotting results for each prothrombin activator with the heparin spiked plasma samples are shown in Tables 44-49, and for thrombin is shown in Table 50.

Table 44: Clotting by PtPA of normal "pooled" citrated plasma spiked with heparin or dispenses in Greiner lithium heparin tubes

| Heparin (IU/mL) | PtPA (nM)/Clotting Time (secs) | | | | | |
|---|---|---|---|---|---|---|
| | 120 nM | 60 nM | 36 nM | 30 nM | 20 nM | 12 nM |
| 0 | 3.3 | 4.3 | 5.2 | 5.5 | 6.4 | 10.1 |
| 0.8 | 3.5 | 5.3 | 7.4 | 7.6 | 10.4 | 14.0 |
| 2.0 | 6.2 | 9.8 | 12.7 | 15.7 | 16.6 | 26.5 |
| 10 | 25.3 | 39.1 | 46.1 | 60.2 | 72.3 | 142 |
| 20 | 38.0 | 47.2 | 68.2 | 80.6 | 232 | >300 |
| 90 (Greiner Li Hep tube) | **17.4** | **27.7** | **53.7** | **69.5** | **228** | **>300** |

Table 45: Clotting by OsPA of normal "pooled" citrated plasma spiked with heparin or dispenses in Greiner lithium heparin tubes

| Heparin (IU/mL) | OsPA(nM)//Clotting Time (secs) | | | | | |
|---|---|---|---|---|---|---|
| | 120 nM | 60 nM | 36 nM | 30 nM | 20 nM | 12 nM |
| 0 | 6.9 | 7.2 | 8.3 | 8.6 | 8.9 | 9.4 |
| 0.8 | 9.1 | 10.8 | 12.9 | 13.7 | 16.4 | 21.7 |
| 2.0 | 13.8 | 15.2 | 17.6 | 19.1 | 22.6 | 27.9 |
| 10 | 27.3 | 35.2 | 49.2 | 56.5 | 86.1 | 270 |
| 20 | 93.9 | 120 | >300 | >300 | >300 | >300 |
| 90 (Greiner Li Hep tube) | >300 | >300 | >300 | >300 | >300 | >300 |

Table 46: Clotting by notecarin of normal "pooled" citrated plasma spiked with heparin or dispenses in Greiner lithium heparin tubes

| Heparin (IU/mL) | Notecarin (nM)/Clotting Time (secs) | | | |
|---|---|---|---|---|
| | 120 nM | 60 nM | 36 nM | 12 nM |
| 0 | 44.3 | 55.1 | 61.6 | 87.0 |
| 0.8 | 49.8 | 65.0 | 78.3 | 126 |
| 2.0 | 57.0 | 92.2 | 129 | 262 |
| 10 | >300 | >300 | >300 | >300 |
| 20 | >300 | >300 | >300 | >300 |
| 90 (Greiner Li Hep tube) | >300 | >300 | >300 | >300 |

Table 47: Clotting by ecarin of normal "pooled" citrated plasma spiked with heparin or dispenses in Greiner lithium heparin tubes

| Heparin (IU/mL) | Ecarin (nM)/Clotting Time (secs) | | | |
|---|---|---|---|---|
| | 120 nM | 60 nM | 36 nM | 12 nM |
| 0 | 28.5 | 40.0 | 49.3 | 92.3 |

(continued)

| Heparin (IU/mL) | Ecarin (nM)/Clotting Time (secs) | | | |
|---|---|---|---|---|
| | 120 nM | 60 nM | 36 nM | 12 nM |
| 0.8 | 30.1 | 41.3 | 51.8 | 109 |
| 2.0 | 33.1 | 50.9 | 70.7 | 156.6 |
| 10 | 75.7 | 142 | 269 | >300 |
| 20 | 140 | >300 | >300 | >300 |
| 90 (Greiner Li Hep tube) | >300 | >300 | >300 | >300 |

Table 48: Clotting by carinactivase-1 of normal "pooled" citrated plasma spiked with heparin or dispenses in Greiner lithium heparin tubes

| Heparin (IU/mL) | CA-1(nM)//Clotting Time (secs) | | | |
|---|---|---|---|---|
| | 120 nM | 60 nM | 36 nM | 12 nM |
| 0 | 170 | 258 | >300 | >300 |
| 0.8 | 215 | 297 | >300 | >300 |
| 2.0 | >300 | >300 | >300 | >300 |
| 10 | >300 | >300 | >300 | >300 |
| 20 | >300 | >300 | >300 | >300 |
| 90 (Greiner Li Hep tube) | >300 | >300 | >300 | >300 |

Table 49: Clotting by carinactivase-2 of normal "pooled" citrated plasma spiked with heparin or dispenses in Greiner lithium heparin tubes

| Heparin (IU/mL) | CA-2 (nM)/Clotting Time (secs) | | | |
|---|---|---|---|---|
| | 120 nM | 60 nM | 36 nM | 12 nM |
| 0 | 80.6 | 120 | 168 | 267 |
| 0.8 | 87.8 | 126 | 181 | >300 |
| 2.0 | 130 | 230 | >300 | >300 |
| 10 | >300 | >300 | >300 | >300 |
| 20 | >300 | >300 | >300 | >300 |
| 90 (Greiner Li Hep tube) | >300 | >300 | >300 | >300 |

Table 50: Clotting by thrombin of normal "pooled" citrated plasma spiked with heparin or dispenses in Greiner lithium heparin tubes

| Heparin (IU/mL) | Thrombin (nM)/Clotting Time (secs) | | | |
|---|---|---|---|---|
| | 100 | 60 | 30 | 10 |
| 0 | 5.1 | 7.0 | 13.5 | 24.7 |
| 0.8 | 23.2 | >300 | >300 | >300 |
| 2.0 | >300 | >300 | >300 | >300 |
| 10 | >300 | >300 | >300 | >300 |
| 20 | >300 | >300 | >300 | >300 |

(continued)

| Heparin (IU/mL) | Thrombin (nM)/Clotting Time (secs) | | | |
|---|---|---|---|---|
| **90** (Greiner Li Hep tube) | >300 | >300 | >300 | >300 |

As was shown in the previous experiment (Example 5f) the presence of heparin (and thrombin inhibition due to the ATIII-heparin complex) presents a major challenge for the procoagulants in preventing partial or complete clotting of blood. The results of this experiment showed the relative effectiveness of the procoagulants in clotting of heparinised plasma.

The results show that PtPA and OsPA were the most effective prothrombin activators in overcoming the anti-coagulant effect from the ATIII-heparin complex. PtPA required the lowest concentration (12-20 nM) to clot plasma containing highest heparin concentrations that may be encountered in patient samples. One interesting finding was PtPA was the only prothrombin activator able to effectively clot the heparinised plasma from the Greiner lithium heparin tube in <5 minutes with all but the lowest PtPA concentration of 12 nM even though the heparin concentration was 90 IU/mL compared with the spiked sample containing 20 IU/mL heparin. It was quite possible the PtPA only was aided by the surfactant contained in the Greiner lithium heparin tube. OsPA required >36 nM and ecarin >60 nM to be able to clot the plasma containing 20 IU of heparin, and both were unable to clot the heparinised plasma from the Greiner lithium heparin tube in < 5 minutes respectively. Carinactivase-2 and notecarin were unable to clot the plasma containing ≥10 IU of heparin while the carinactivase-1 was unable to clot the plasma containing >0.8 IU of heparin in the pre-requisite time of < 5 minutes even when the concentration was 120 nM. The thrombin study showed thrombin concentration of 100 nM was only able to clot the sample spiked with the lowest heparin concentration, 0.8 IU/mL. Thus, when considering the BD RST tube which contains 135 nM of thrombin it is unlikely to clot samples containing heparin >2 IU/mL in the prerequisite time of 5 minutes.

Example 5h: Clotting of lithium heparin plasma from a healthy participant

Samples collected in Greiner lithium heparin tubes are likely to be the most highly heparinised samples a laboratory will receive, particularly if the tubes are not filled to the recommended fill volume. The purpose of this experiment was to provide a guide on the minimum concentration required of the procoagulants to clot all other samples that may be received from heparin anticoagulated patients and produce high quality serum.

Greiner lithium heparin tubes were filled to the recommended fill mark, giving a heparin concentration of 18 IU/mL. The Clotek tubes contained 100 μL of lithium heparin plasma, 100 μL of Tris Buffer (150 mM Tris HCl, 150 mM NaCl, pH 7.4) and 50 μL of each procoagulant. The TEG cups contained 60 μL procoagulant and 260 μL of the participant blood collected in Greiner lithium heparin tube.

For the BD RST tube, deionised water was added to two tubes (BDRST1- 1 mL; BDRST2 -4 mL), allowed to mix on a roller for 30 minutes to dissolve the thrombin and the content was used as with the other procoagulants.

The clotting results for each procoagulant with the lithium heparin plasma are shown in Table 51.

Table 51: Clotting study with lithium heparin plasma collected in Greiner Lithium Heparin Tube.

| [Prothrombin activator] (nM) | Clotting Time (s) | | | | | | |
|---|---|---|---|---|---|---|---|
| | Ecarin | CA-1 | CA-2 | PtPA | OsPA | Notecarin | Thrombin |
| 120 | 69.0 | >300 | 100.1 | 7.5 | 15.6 | 47.8 | >300 |
| 60 | 122.6 | >300 | 180.3 | 10.7 | 18.1 | 62.7 | >300 |
| 36 | 175.2 | | 270.6 | 13.1 | 21.0 | 82.4 | >300 |
| 12 | >300 | | >300 | 32.7 | 37.1 | 269.3 | |
| 4 | >300 | | >300 | 78.5 | 102.1 | >300 | |
| 1 | >300 | | >300 | 207.8 | >300 | >300 | |
| 0.1 | >300 | | >300 | >300 | >300 | | |
| 0.01 | >300 | | >300 | >300 | >300 | | |

The experiment design was to show the effectiveness of the procoagulants in clotting heparinised plasma from blood collection tubes. It was found that the minimum required concentration range to achieve clotting of <5 minutes for ecarin and carinactivase-2 was 12 to 36 nM, >120 nM for carinactivase-1, and 4 to 12 nM for notecarin. For the thrombin, concentration of >120 nM was necessary. The BD RST tube was tested as well with 7.7 and 27 nM of thrombin and both concentrations producing clotting time >5 minutes which suggested that the BDRST may be ineffective in clotting samples from patients on high doses of heparin. The results show the group C prothrombin activators (PtPA and OsPA) were easily the most effective in clotting the lithium heparin plasma obtained from blood of a healthy participant collected in Greiner lithium heparin tubes, requiring between 0.1 to 1 nM of PtPA and between 1 to 4 nM of OsPA respectively to achieve clotting in <5 minutes.

Example 5i: Clotting of blood and plasma obtained from a "fully heparinised" cardiac surgery participant

The highest concentration of heparin given intravenously is in complex surgical procedures such as cardiac surgery. If samples are collected for biochemistry during the maximum heparinisation period the results usually need to be provided in the shortest possible time. If serum is used, this will require the clotting to be completed by the procoagulant by the time these serum samples arrive in the laboratory, say in < 10 minutes.

The heparin that is given to patients is sodium heparin while in blood collection tubes or syringes it is lithium heparin. The maximum amount of heparin infused in such patients is ~45000 IU. These patients are also haemodiluted (*i.e.*, there is an increase in blood plasma volume to ~ 4 L), giving a heparin concentration of ~10 IU per mL of plasma.

This experiment was designed to determine whether the selected procoagulant concentrations were able to clot "fully" heparinised cardiac surgery patient blood and produce high quality serum. The participant had received 37000 IU of heparin and the sample was collected ~30 minutes post heparin infusion, equating to ~9.3 IU of heparin per mL of plasma. A portion of the blood sample was centrifuged to obtain plasma.

For plasma clotting, the Clotek tube contained 100 μL of cardiac participant plasma, 100 μL of Tris Buffer (150 mM Tris HCl, 150 mM NaCl, pH 7.4) and 50 μL of each procoagulant.

For the whole blood clotting, the TEG cup contained 60 μL of procoagulant and 260 μL of the participant blood. Being fully heparinised the blood did not clot on its own.

For the BD RST-1 experiment, the contents of two BDRST tubes were dissolved with 1 and 4 mL of distilled water respectively, and 60 μL of the solution used in the TEG cup as the procoagulant.

For the BDRST-2 experiment, the tubes were filled with 1 and 4 mL of blood respectively, the tubes were inverted 10 times (~30 secs) and 340 μL of the blood was transferred to the TEG cup.

The clotting results for each procoagulant with the patient's plasma are shown in Table 52, and the whole blood results are shown in Table 53.

Table 52: Clotting times in seconds of plasma from a "fully heparinised" cardiac surgery participant with the different prothrombin activators, thrombin and BD RST tube contents at different concentrations.

| Procoagulant conc (nM) | PtPA | OsPA | Ecarin | CA-1 | CA-2 | Notecarin | Thrombin | BDRST |
|---|---|---|---|---|---|---|---|---|
| 120 | 10.0 | ND | 64.8 | 175 | 66.5 | ND | >300 | >300 (27 nM) |
| 60 | 14.8 | 18.7 | 89.7 | >300 | 95.1 | 68.6 | >300 | >300 (6 nM) |
| 36 | 15.9 | 27.3 | >300 | >300 | 124 | 84 | >300 | ND |
| 12 | 36.3 | 35.5 | >300 | >300 | >300 | 163 | ND | ND |
| 6 | **65.9** | ND | ND | ND | ND | ND | ND | ND |
| 4 | 301.6 | 100 | >300 | >300 | >300 | 179 | ND | ND |
| 3 | ND | **123** | ND | ND | ND | ND | ND | ND |
| 1 | ND | >300 | ND | ND | ND | >300 | ND | ND |
| ND means this was not determined. | | | | | | | | |

The results from this experiment showed the concentrations of each procoagulant required to clot the plasma in the prerequisite time of < 5 minutes. The maximum activator concentration giving clotting time of about 5 minutes were: PtPA 4 nM, OsPA 1-3 nM, notecarin 3 nM, carinactivase-2 >12 nM, ecarin >36 nM, carinactivase-1 >60 nM, and thrombin >120 nM. It was surprising to note that the notecarin efficacy was comparable to the group C activators.

Table 53: Results of the TEG clotting study with blood from a "fully heparinised" cardiac surgery participant with the different procoagulants.

| Procoagulant (nM) | R (min) | MA (mm) | TMA (min) |
|---|---|---|---|
| **Ecarin** | | | |
| 56 | 2.3 | 47.6 | 19.4 |
| 34 | 3.7 | 49.0 | 25.1 |
| 11 | 16.6 | 35.2* | 53.3* |
| 3 | 28.8* | ND | ND |
| **Carinactivase - 1** | | | |
| 112 | 5.3 | 36.9 | 23.7 |
| 56 | 9.8 | 33.1* | 37.7* |
| **Carinactivase - 2** | | | |
| 56 | 2.8 | 38.1 | 25.8 |
| 34 | 3.3 | 36.9 | 24.3 |
| 11 | 8.9 | 30.7* | 38.5* |
| **OsPA (nM)** | | | |
| 56 | 0.2 | 64.2 | 18.6 |
| 3 | 2.9 | 61.4 | 25.8 |
| 2.3 | 4.2 | 54.8 | 28.4 |
| 2 | 11.0 | 29.4* | 37.9* |
| **PtPA** | | | |
| 56 | 0.4 | 62.9 | 20.1 |
| 6 | 4.4 | 60.9 | 27.9 |
| 3 | 9.7 | 42.8* | 48.6* |
| 2 | 28.7* | ND | ND |
| **Notecarin** | | | |
| 56 | 0.8 | 51.9 | 23.3 |
| 34 | 1.1 | 51.0 | 19.6 |
| 11 | 2.2 | 46.6 | 22.2 |
| 3 | 7.2 | 39.5 | 33.6 |
| **BD RST - 1** | | | |
| 25 | 18.9* | ND | .ND |
| 6 | 19.3* | ND | ND |
| **BD RST - 2** | | | |
| 135 (1 mL) | 0.4 | 9.9 | 13.3 |
| 34 (4 mL) | 0.3 | 12.7 | 8.1 |
| * means measurement was stopped, and ND means this was not determined. | | | |

The concentrations determined to achieve an R time of <5 minutes and maximum clot strength from these results for the different prothrombin activators were: PtPA 6 nM, OsPA 2.3 nM, notecarin 3-11 nM, ecarin 11-34 nM, carinactivase-2 11-34 nM, and carinactivase-1 57-112 nM.

Surprisingly, the notecarin produced clots with higher MAs than the group A and B prothrombin activators, and without wishing to be bound by theory, it is postulated that additives within the blood of such patients may have aided clotting efficacy.

The strongest clots formed as indicated by the MA were with the PtPA and OsPA, and the weakest with the carinactivase-1 and carinactivase-2 prothrombin activators.

The thrombin in the BD RST tubes (BDRST-2 experiment with 135 nM of thrombin) produced incomplete clotting and weak clots as indicated by the MA. Such samples are very likely to form latent clotting in the serum post centrifugation.

Example 5j: Clotting of normal "pooled" citrated plasma spiked with rivaroxaban

This experiment was designed to examine the effect of one of the new Factor Xa inhibitor anticoagulants, rivaroxaban, on the clotting ability of the procoagulants.

One rivaroxaban table (10 mg, Mol Wt 435, Xarelto, Bayer Schering Pharm, Germany) tablet was crushed in 5 mL deionised water (2 mg/mL), allowed to mix for 30 minutes, and then centrifuged to remove undissolved particles. The typical therapeutic dose given is 10-40 mg once daily. For a 70 kg person ($\sim$3000 mL plasma) the total amount is 0.14-0.57 mg/kg, 0.0033-0.013 mg/mL of plasma. The concentrations prepared and tested in the Clotek tube were: 0.0033, 0.0083, 0.017, 0.033, and 0.17 mg/mL.

The Clotek tubes contained 50 $\mu$L of Tris Buffer (150 mM Tris HCl, 150 mM NaCl, pH 7.4), 50 $\mu$L 0.2 mM CaCl$_2$, 100 $\mu$L normal "pooled" citrated plasma, 50 $\mu$L of Tris buffer or anticoagulant of different concentration, and 50 $\mu$L of prothrombin activator (30 nM).

The clotting times are shown in Table 54.

Table 54: Clotting times (seconds) with normal "pooled" citrated plasma and Rivaroxaban.

| Procoagulant (30 nM) | Anticoagulant concentration | Rivaroxaban concentration (mg/mL) | | | | |
|---|---|---|---|---|---|---|
| | 0 | 0.003 | 0.008 | 0.017 | 0.033 | 0.17 |
| **PtPA** | 4.8 | 5.8 | 6.2 | 7.7 | 8.8 | 12.0 |
| **OsPA** | 8.6 | 8.6 | 8.8 | 8.8 | 9.3 | 16.9 |
| **Ecarin** | 42.6 | 50.7 | 51.6 | 53.9 | 58.9 | 83.9 |
| **Notecarin** | 41.7 | 42.7 | 44.9 | 46.2 | 47.6 | 67.3 |
| **CA-1** | 128 | 133 | 152 | 176 | 194 | 258 |
| **CA-2** | 60.9 | 61.1 | 62.8 | 66.1 | 70.6 | 98.0 |
| **Thrombin** | 7.1 | 7.0 | 7.3 | 7.4 | 8.0 | 9.8 |
| **BDRST 23 nM** | 5.1 | 5.3 | 5.2 | 5.8 | 5.7 | 7.7 |

The results show that rivaroxaban has minimal effect on the procoagulants in the therapeutic concentration range 0.0033-0.013 mg/mL of plasma that may be encountered in patients. However, at higher concentrations, rivaroxaban had a significant inhibitory effect on clotting in the presence of each of the procoagulants. This effect may be due to inhibition of Factor Xa produced from human FX in the plasma by the thrombin generated by the procoagulants.

Example 5k: Clotting of blood from a patient undergoing citrate anticoagulation

In previous experiments, it has been shown that the prothrombin activators are capable of clotting citrated blood collected in Greiner citrate tubes. The purpose of this experiment was to confirm that *in vivo* citration presented no clotting problems with the prothrombin activators.

In this example, the patient was on 3.0 mmol of citrate per litre of blood flow. The blood was collected in a 50 mL plain syringe (BD Plastipak #300866) for routine pathology testing from which <3 mL was left to perform the very limited study with PtPA only.

The coagulation parameters were: PT 13 s (RR 9-13), INR 1.3, APTT 45 s (RR 24-39), fibrinogen (derived) 7.2 g/L (RR 1.7-4.5), confirming anticoagulation.

The TEG cup contained 20 $\mu$L of 0.2 M CaCl$_2$ or saline, 5 $\mu$L of PtPA and 320 $\mu$L of the citrated blood.

The results are shown in Table 55.

Table 55: TEG results for citrated anticoagulated participant.

| Citrated anticoagulated participant | R(min) | MA(mm) | TMA(min) |
|---|---|---|---|
| Re-calcified citrate blood alone | 4.8 | 77.1 | 18.0 |
| 2.8 nM PtPA re-calcified | 0.6 | 74.7 | 15.8 |
| 2.8 nM PtPA non re-calcified | 0.8 | 76.2 | 18.9 |

The results indicated that the citrate concentration used for anticoagulation of patients did not present clotting problems when PtPA was used as the procoagulant in re-calcified and non re-calcified blood. The citrate concentration in blood collected in Greiner Citrate tubes is 3.2% or 109 mM, which is about 36 times the concentration the patient was receiving. Additionally, it was not expected there would be any clotting problems as patients being citrated are continuously monitored for ionised calcium and ionised calcium is replenished as required. The R time in normal "pooled" citrate blood was 6.4 minutes (as shown in Table 43) compared to the 4.8 min in this patient. The re-calcification adds calcium in exceeds to requirement thus theoretically it should not contribute to the difference. It is postulated that the difference is due to higher concentrations of clotting factors including fibrinogen which was 7.2 g/L.

EXAMPLE 6: CLOTTING OF WHOLE BLOOD SAMPLES FROM PARTICIPANTS WITH PROLONGED COAGULATION PROFILE OR LOW PLATELET COUNTS

Some patients present with prolonged coagulation parameters and/or reduced platelet count as a result of drug treatment or genetic factors (*e.g.* haemophilia). The following experiments were conducted to determine whether PtPA or **OsPA** would be able to rapidly clot blood samples from such patients.

Example 6a: Participants with prolonged clotting time

Blood from 4 participants ("A", "B", "C" and "D") with prolonged aPTT indicating a defect in the intrinsic pathway were obtained from Pathology Queensland, Princess Alexandra Hospital, Queensland, Australia. The coagulation parameters of each participant were determined and are shown in Table 56.

Table 56: Coagulation parameters* of each participant

| Participant | aPTT(s) | PT(s) | INR | TT(s) | Fib-D(g/L) | plat(x10$^9$/L) |
|---|---|---|---|---|---|---|
| A | 48 | 16 | 1.6 | 19 | 7.1 | 453 |
| B | 44 | 34 | 3.0 | 16 | 4.1 | 97 |
| C | >200 | n.d. | n.d. | n.d. | n.d. | n.d. |
| D | 45 | 16 | 1.5 | 15 | 1.5 | 96 |
| Reference Interval | 25-38 | 8-14 | 0.9-1.3 | 10-15 | 1.5-4.0 | 140-400 |

* aPTT = activated partial thromboplastin times; PT = prothrombin time; INR = international normalised ratio; TT = thrombin time; Fib-D = prothrombin time derived fibrinogen; Plat = platelets, n.d. = not able to be determined. The Reference Intervals were obtained from Pathology Queensland.

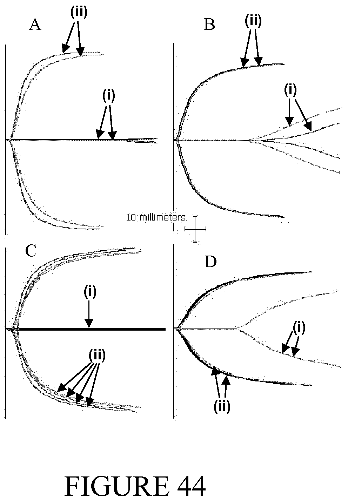

TEG analysis was performed. Each TEG assay mixture consisted of 310 μL citrated blood from A-D participant samples, 20 μL of CaCl$_2$ (0.2 M) and 10 μL of saline or PtPA (final concentration 1.41 μg/mL). CaCl$_2$ was added at zero time to initiate clotting.
TEG traces for the four samples are shown in Figure 44 and TEG parameters from these traces are shown in Table 57.

Table 57: TEG results for clotting of blood samples from participants with abnormal coagulation parameters

| | R(min) | TMA(min) | α(deg) | MA(mm) |
|---|---|---|---|---|
| A | >35 | n.d. | n.d. | n.d. |
| A + PtPA | 1.3 | 16.6 | 70.9 | 67.4 |
| B | 23.0 | >39 | 17.0 | n.d. |

(continued)

| | R(min) | TMA(min) | $\alpha$(deg) | MA(mm) |
|---|---|---|---|---|
| **B + PtPA** | 0.9 | 21.3 | 70.0 | 59.0 |
| **C** | >52 | n.d. | n.d. | n.d. |
| **C + PtPA** | 2.1 | 24.5 | 67.6 | 60.4 |
| **D** | 16.1 | 43.3 | 26.4 | 31.7 |
| **D + PtPA** | 1.4 | 19.2 | 52.0 | 40.8 |
| "n.d." indicates that this was not measured | | | | |

TEG plots for the four samples are shown in Figure 44 where TEG plot A represents participant A and in that plot the two traces labelled (i) represent the saline control with added calcium; the two traces labelled (ii) are the samples with PtPA. TEG plot B represents participant B and in that plot the two traces labelled (i) are saline control with added calcium; the two traces labelled (ii) are the samples with PtPA. TEG plot C represents participant C and in that plot the trace labelled (i) is the saline control; and the four traces labelled (ii) are samples with PtPA added. TEG plot D represents participant D and in that plot the two traces labelled (i) are the saline controls; and the two traces labelled (ii) are the samples with PtPA. Triplicate determinations were carried out for patient C due to grossly abnormal clotting parameters. The data in Table 57 represents averages from the TEG data.

In summary, blood samples from participants A-D show an aPTT time ranging from 44 to >200 seconds indicating a defect in the intrinsic coagulation pathway. Without PtPA the samples did not clot or produced very weak clots. In all cases PtPA (1.41 $\mu$g/ml) caused rapid clotting, reducing the clotting time to 1-2 minutes. Thus PtPA is effective in rapidly clotting blood from patients with a deficiency in this coagulation pathway.

Example 6b: Participants with low platelet counts

In participants with low platelet counts, clotting times are significantly increased compared to blood from the healthy population. This is because platelets provide a phospholipid surface for the formation of the prothrombinase complex and accelerate blood clotting.

Blood samples from four participants with low platelet counts ("E", "F", "G" and "H") were obtained from Princess Alexandra Hospital, Queensland, Australia. The coagulation parameters of each participant were determined and are shown in Table 58, confirming the low platelet count.

Table 58: Coagulation parameters of each participant

| Participant | aPTT(s) | PT(s) | INR | TT(s) | fib(g/L) | plat(x10$^9$/L) |
|---|---|---|---|---|---|---|
| **E** | 31 | 10 | 1.1 | n.d. | 4.5 | 10 |
| **F** | 29 | 14 | 1.4 | n.d. | 1.2 | 29 |
| **G** | 34 | 12 | 1.2 | n.d. | 2.7 | 18 |
| **H** | 54 | 11 | 1.2 | 21 | 6.4 | 46 |
| **Reference Interval** | 25-38 | 8-14 | 0.9-1.3 | 10-15 | 1.5-4.0 | 140-400 |
| n.d. indicates that the measurements were not determined. | | | | | | |

TEG analysis was performed. Each TEG assay mixture consisted of 310 $\mu$L citrated blood from E-H participant samples, 20 $\mu$L of CaCl$_2$ (0.2 M) and 10 $\mu$L of saline or solution of prothrombin activator (either PtPA at a final concentration of 1.41 $\mu$g/mL or 14.1 $\mu$g/mL, or OsPA at a final concentration of 0.59 $\mu$g/mL or 5.9 $\mu$g/mL).

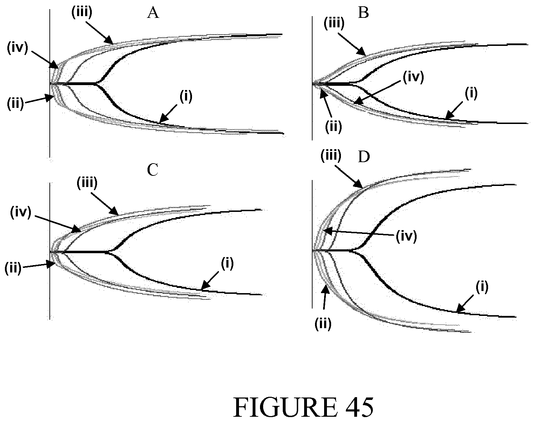

The TEG traces are shown in Figure 45 and TEG parameters are listed in Tables 59 and 60.

Table 59: TEG results for clotting of blood samples from participants with low platelet counts with PtPA

| Participant | $\mu$g/mL | R(min) | TMA(min) | $\alpha$(deg) | MA(mm) |
|---|---|---|---|---|---|
| **E** | 0 | 10 | 30.3 | 42.3 | 34.6 |

(continued)

| Participant | µg/mL | R(min) | TMA(min) | $\alpha$(deg) | MA(mm) |
|---|---|---|---|---|---|
| | 1.41 | 1.5 | 22.5 | 61.6 | 35.3 |
| | 14.1 | 0.4 | 20.3 | 73.2 | 31.1 |
| F | 0 | 9.3 | 29.8 | 15.6 | 28.2 |
| | 1.41 | 1.5 | 24.2 | 29.0 | 31.2 |
| | 14.1 | 0.6 | 24.3 | 38.4 | 28.0 |
| G | 0 | 12.8 | 32.8 | 29.5 | 30.2 |
| | 1.41 | 1.4 | 23.4 | 57.1 | 33.7 |
| | 14.1 | 0.4 | 25.0 | 66.9 | 31.3 |
| H | 0 | 9.1 | 32.0 | 39.3 | 48.2 |
| | 1.41 | 1.7 | 24.2 | 66.3 | 58.8 |
| | 14.1 | 0.3 | 22.2 | 73.7 | 54.7 |

Table 60: TEG results for clotting of blood samples from participants with low platelet counts with **OsPA**

| Participant | µg/mL | R(min) | TMA(min) | $\alpha$(deg) | MA(mm) |
|---|---|---|---|---|---|
| E | 0 | 10 | 30.3 | 42.3 | 34.6 |
| | 10 | 3.8 | 23.8 | 41.0 | 32.7 |
| | 100 | 0.9 | 21.8 | 63.7 | 32.9 |
| F | 0 | 9.3 | 29.8 | 15.6 | 28.2 |
| | 10 | 3.2 | 25.3 | 27.4 | 28.7 |
| | 100 | 1.6 | 25.6 | 27.5 | 29.3 |
| G | 0 | 12.8 | 32.8 | 29.5 | 30.2 |
| | 10 | 3.3 | 24.6 | 38.8 | 31.7 |
| | 100 | 0.9 | 24.5 | 56.5 | 29.9 |
| H | 0 | 9.1 | 32.0 | 39.3 | 48.2 |
| | 10 | 3.4 | 24.1 | 59.8 | 59.7 |
| | 100 | 0.8 | 22.6 | 66.7 | 54.9 |

The TEG plots are shown in Figure 45 where plot A shows the results for participant E; plot B shows the results for participant F; plot C shows the results for participant G; and plot D shows the results for participant H. In each plot, the trace labelled (i) represents no PtPA or OsPA; the trace labelled (ii) represents 14.1 µg/mL PtPA; the trace labelled (iii) represents 1.41 µg/mL PtPA; the trace labelled (iv) represents 5.88 µg/mL OsPA; and the blue trace represents 0.588 µg/mL OsPA.

In summary, both PtPA and OsPA clot blood from participants with low platelet counts rapidly to give high strength clots.

EXAMPLE 7: COMPARISON OF CLOTTING ACTIVITY OF PROTRHOMBIN ACTIVATOR-CONTAINING VENOMS

Five freshly reconstituted venoms from the snakes *Pseudonaja textilis* (Pt), *Oxyuranus scutellatus* (Os), *Oxyuranus microlepidotus* (Om), *Notchis scutatus* (Ns), and *Echis carinatus* (Ec) were reconstituted in distilled water with a stock concentration of 50 mg/mL. A working stock dilution of 6 mg/mL was freshly prepared and their clotting activity was measured on the clotting of re-calcified citrated plasma. The venoms were serially diluted in duplicates of each concentration in the assay from 2 mg/mL to 500 pg/mL. The results shown are the average of the duplicate measurements.

The assay consisted of: 100 μL of 0.02 M Hepes buffer pH 7.4 with added 10 mM calcium, plus 100 μL of citrated plasma and clotting time (in seconds) was measured from the time of the addition of venom dilution.

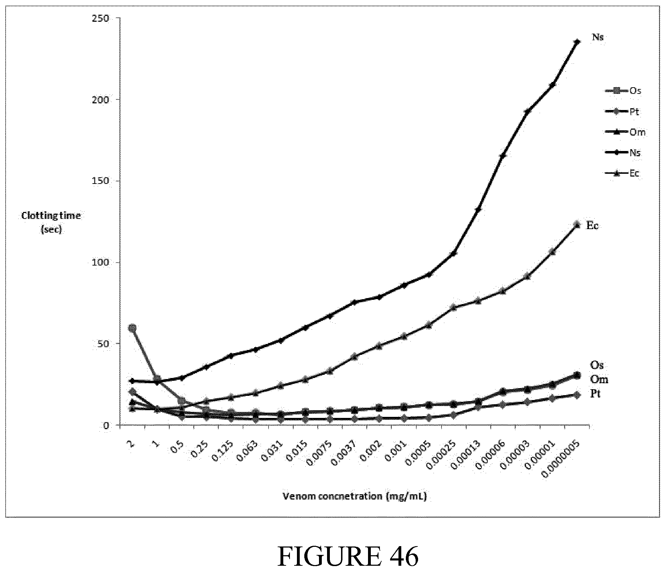

The results are shown in Table 61 and Figure 46.

Table 61: Clotting times of snake venoms containing prothrombin activators at different concentrations

| Venom (mg/mL). | Clotting time (seconds) | | | | |
|---|---|---|---|---|---|
| | Os | Pt | Om | Ns | Ec |
| 2 | 59.5 | 20.6 | 14.6 | 27.4 | 10.3 |
| 1 | 28.4 | 9.8 | 9.8 | 26.5 | 9.7 |
| 0.5 | 15 | 5.2 | 7.8 | 29.2 | 10.9 |
| 0.25 | 9.3 | 5 | 6.6 | 36 | 14.7 |
| 0.125 | 7.6 | 4.2 | 6.1 | 42.7 | 17.1 |
| 0.063 | 7.4 | 3.6 | 6.8 | 46.4 | 19.8 |
| 0.031 | 6.5 | 3.5 | 6.9 | 52.00 | 24.3 |
| 0.015 | 8.2 | 3.6 | 8.0 | 59.9 | 28.1 |
| 0.0075 | 8.6 | 3.7 | 8.6 | 67.2 | 33.4 |
| 0.0037 | 9.2 | 3.8 | 9.3 | 75.7 | 42.1 |
| 0.002 | 10.6 | 4.0 | 10.7 | 78.8 | 48.6 |
| 0.001 | 11.2 | 4.2 | 11.1 | 86.00 | 54.6 |
| 0.0005 | 12.4 | 4.5 | 12.6 | 92.5 | 61.4 |
| 0.00025 | 12.8 | 6.2 | 13.0 | 105.6 | 72.3 |
| 0.00013 | 14.5 | 11.2 | 14.8 | 132.4 | 76.4 |
| 0.00006. | 20.4 | 12.6 | 21.1 | 165.5 | 82.5 |
| 0.00003 | 21.8 | 14.3 | 22.4 | 192.6 | 91.3 |
| 0.00001 | 24.6 | 16.5 | 25.6 | 209 | 106.4 |
| 0.0000005 | 30.7 | 18.6 | 31.2 | 235.4 | 123.2 |

As can be seen in both Table 61 and Figure 46, all five venoms clotted recalcified citrated plasma very efficiently down to concentration of 500 pg/mL. The Group C prothrombin activator-containing venoms (Pt, Os and Om) were the most active achieving clotting times of less 50 seconds at a concentration of 500 pg/mL.

EXAMPLE 8: PRECIPITATION AND LATENT CLOTTING IN PLASMA AND SERUM SAMPLES PREPARED USING COMMERCIALLY AVAILABLE TUBES OR USING PTPA AND EFFECTS ON TROPONIN I ESTIMATION

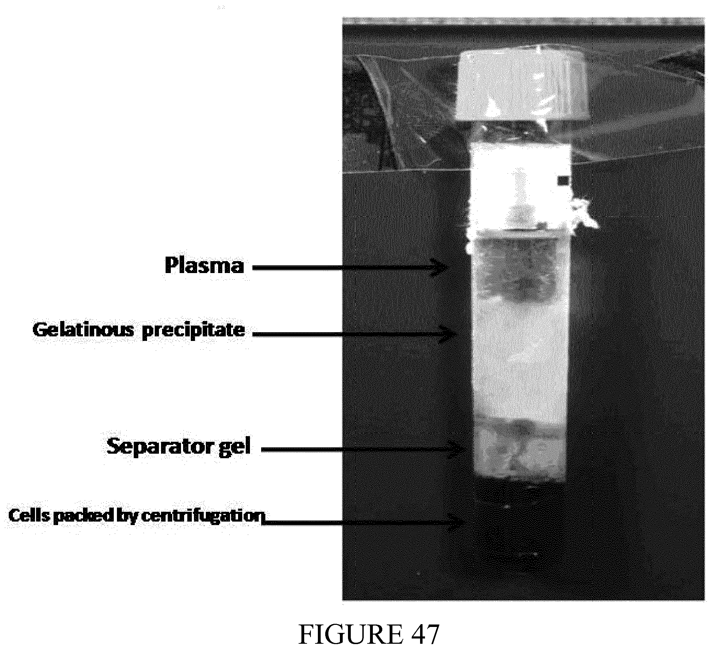

As discussed in the earlier parts of this specification, a number of problems have been associated with the use of commercial serum and plasma tubes such as latent clotting or no clotting (serum tubes), microclots and fibrinogen strings (serum or plasma tubes), and precipitation of proteins and leakage of cellular material (serum or plasma tubes). To investigate whether a tube containing a prothrombin activator would exhibit some of these problems, blood samples in commercially available tubes were visually inspected and compared against blood samples in PtPA-containing tubes. The results are shown in Figures 47-49.

Example 8a: Precipitation and latent clotting

Figure 47 shows the results of plasma preparation from a blood sample collected in a commercially available Greiner plasma (lithium heparin containing) tube. Four sections of the tube are marked (from top to bottom of the Figure): plasma; gelatinous precipitate; separator gel; cells packed by centrifugation. The gelatinous precipitate is composed of fibrinogen and other plasma proteins and forms when the tube is stored at 2-8°C. This precipitate has the potential to interfere with instrument function and analyte determination. Such precipitates occur in ~5% of lithium heparin plasma samples.

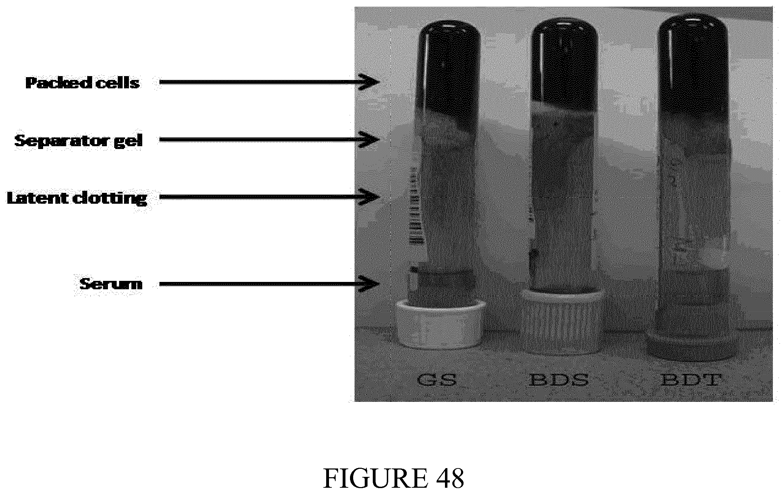

Figure 48 shows the results of serum preparation in three commercially available serum tubes: left tube (GS) is a Greiner serum tube; middle tube (BDS) is a BD serum tube and right tube (BDT) is a BD RST tube. Four sections of the tubes are marked (from top to bottom of the Figure): packed cells; separator gel; latent clotting; serum. Post-centrifugation (latent) clotting is evident in the supernatants or serum component of all three tubes. The tubes are inverted to illustrate the presence of the clots. These latent clots have the potential to interfere with instrumentation and functional assays.

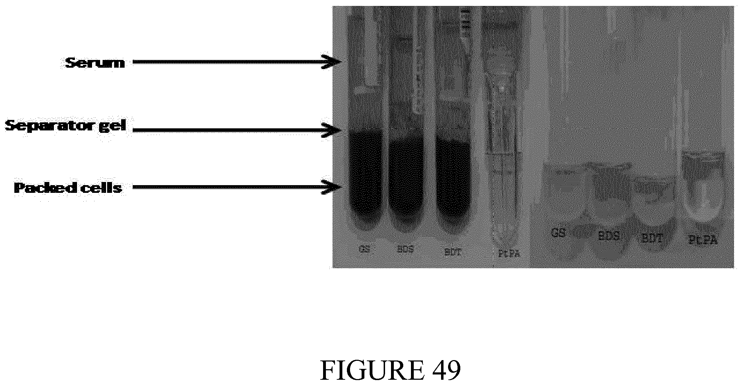

Figure 49 is in two halves. The left half shows the results of serum preparation from a single blood sample from the same fully heparinised patient in three commercially available tubes (left to right): a Greiner Vacuette™ serum tube (GS), a BD Vacutainer™ serum tube/SST II (BDS) and a BD RST tube (BDT), and also in a Greiner Vacuette™ No Additive tube with PtPA added. Three sections of the tubes are marked (from top to bottom of the Figure): serum; separator gel; packed cells. All four tubes from Figure 49 were centrifuged then left standing overnight, after which the serum in each tube was transferred to a clear glass tube and photographed as shown in the right half of Figure 49 (left to right: GS, BDS, BDT and PtPA tubes). The sera from all three commercially available tubes showed evidence of clotting, by clot formation on the side of the tube and observation of fibrin strands, but there was no evidence of precipitation in the serum prepared in the PtPA-containing tube which had a clarity that considerably exceeded that observed with the other samples.

Example 8b: Troponin I levels

It has been observed that at least some false positive results showing elevated troponin I (one of the most specific markers of cardiac events) are due to precipitation/latent clotting in serum and plasma samples.

Troponin I levels in blood samples from 64 participants were measured using serum and plasma samples prepared in commercially available tubes and PtPA-containing tubes.

Serum and plasma samples were prepared for each participant in five commercially available tubes: Greiner Vacuette™ plasma; BD PST II; Greiner Vacuette™ serum; BD SST II; and BD RST, and in Greiner Vacuette™ No Additive tubes containing PtPA (1.2 $\mu$g/4 mL) and analysed for troponin I (TnI) using the Beckman Coulter AccuTnI assay.

Four discrepant results were obtained as indicated in Table 62 for participants 18, 20, 38 and 63. Participants 18 and 38 were healthy, and participants 20 and 63 were both patients undergoing cardiac surgery and on heparin. Three false positive results (participants 18, 20 and 38) were obtained. Re-centrifugation of an aliquot from the primary tube followed by re-assay gave negative results, as expected when compared to results from the other tubes from the same blood sample. A further result was exceptionally high (participant 63). Re-centrifugation of an aliquot and re-assay gave a comparable result with the other tubes.

The fact that centrifugation of samples resolved the discrepancy strongly suggests that the discrepant results were due to latent clotting/precipitate formation causing inaccurate sampling. No discrepant results were obtained with the PtPA serum.

Table 62: Troponin I assay results showing discrepancies.

| Participant | Original TnI result (Repeat TnI result) $\mu$g/L | | | | | |
| --- | --- | --- | --- | --- | --- | --- |
| | PtPA | Greiner plasma | BD PST II | (Greiner serum | BD SST II | BD RST |
| 18 | 0.003 | 0.000 | 0.000 | 0.000 | **0.053** (0.004) | 0.000 |
| 20 | 0.007 | 0.015 | **0.093** (0.015) | 0.013 | 0.013 | 0.004 |
| 38 | 0.009 | 0.000 | 0.006 | 0.006 | **1.334** (0.002) | 0.004 |
| 63 | 0.675 (0.690) | 0.665 (0.704) | 0.643 (0.665) | 0.652 (0.650) | 0.542 (0.579) | **1.251** (0.734) |

In summary, precipitation and latent clotting observed during the preparation of plasma and serum samples in commercially available tubes represent significant problems for analyte determination. No precipitation or evidence of microclot formation was observed when PtPA was used to prepare a serum sample.

EXAMPLE 9: FIBRINOGEN, DEGRADED FIBRINOGEN AND FIBRIN LEVELS IN PLASMA AND SERUM SAMPLES

Soluble fibrinogen, soluble partially degraded fibrinogen (fdp) and unpolymerised fibrin (FDP) are undesirable components in serum and plasma samples for analyte determination as discussed in the earlier parts of this specification. Briefly, fibrinogen/fdp/FDP should be minimal in a high quality serum sample to avoid further conversion into insoluble fibrin (microclots) after standard serum preparation conditions especially on standing. Microclots or fibrin strands can interfere with instrumentation and affect analyte determination. For serum samples prepared by standard methods, the concentration of fibrinogen/fdp/FDP in the sample is believed to depend on the extent to which the patient/individual is anticoagulated, the health status of the patient (*e.g.* the presence of liver disease), on the type of sample collection container and in rare cases on inadequate mixing of the sample in the tube. In this example, the concentration of fibrinogen/fdp/FDP in serum and plasma samples prepared using different tubes was investigated to assist in establishing conditions for preparation of a "high quality" serum sample.

Serum and plasma fibrinogen/fdp/FDP concentrations were measured by a sandwich enzyme immunoassay (ELISA) as described below. This assay uses a polyclonal antiserum against fibrinogen which recognises soluble soluble fibrinogen, fdp and FDP.

The ELISA method requires anti-fibrinogen antiserum (as a purified IgG fraction: AHFAS) and anti-fibrinogen antiserum-horseradish peroxidase conjugate (AHFAS-HRP). Preparations of each were purchased from MP Biochemical, USA. International fibrinogen standard was purchased from NIBSC, London, and diluted from a working stock concentration of 1 mg/mL in 50% glycerol/saline.

The wash buffer consisted of 0.05 M phosphate buffer, pH 7.4, 0.5 M NaCl, 0.05 % Tween 20 and 1% BSA, while the binding buffer used was 0.1 M bicarbonate buffer, pH 9.6.

Working dilutions of the antibodies were prepared by diluting AHFAS and AHFAS-HRP stocks 1:500 in binding buffer. The HRP substrate solution consisted of 20 mM tetramethylbenzidine, 0.4 mL of 30% $H_2O_2$ and 50 $\mu$L of 0.05 M citrate buffer pH 4.0 in a 50 mL reaction solution.

Nunc ELISA IMMUNOSORB 96 well plates (Thermo Fisher Scientific, Rochester, NY) were coated with 100 $\mu$L of AHFAS working dilution per well by incubating the plates with AHFAS at 4°C overnight. The plates were then blocked with 100 $\mu$L of 2% bovine serum albumin (Sigma Chemical, Co) in wash buffer at 4°C overnight and subsequently washed three times with wash buffer.

Blood samples from healthy volunteers (healthy) and patients (cardiac and renal disease) were collected into the following tubes:

Greiner serum tubes (GS or GRS);

BD serum tubes (BDS);

BD RST tubes (BDT or BD RST);

Greiner No Additive tubes with added 1.2 $\mu$g PtPA from a PtPA stock solution 4.8 mg/mL (PtPA);

Greiner No Additive tubes with added 0.5 $\mu$g OsPA from a OsPA stock solution 2.0 mg/mL (OsPA);

Greiner No Additive tubes with added 0.6 U ecarin;

Greiner No Additive tubes with added 1.2 U ecarin;

Greiner No Additive tubes with added 0.63 U/4mL ecarin (Example 9b);

Greiner No Additive tubes with added 1.25 U/4mL ecarin (Example 9c);

Greiner No Additive tubes with added 2.5 U/4mL ecarin (Example 9c);

Greiner Vacuette™ plasma tubes (GRLH);

BD Vacutainer™ plasma (PST II) tubes (BDLH);

Greiner Vacuette™ citrate tube (3.2% citrate) for plasma (CIT); and

Greiner Vacuette™ K2EDTA tube (1.5-2.2 mg/mL of EDTA) for plasma (EDTA).

Plasma and serum samples prepared in each tube above were prepared under standard Pathology Queensland (Australia) analyte sample preparation procedure where blood was collected into each tube, and the tube was left to stand prior to centrifugation at 3,000 g. The BD RST, PtPA, OsPA and ecarin-containing tubes were left to stand for 5 minutes, while all remaining tubes were left to stand for 30 minutes for normal blood and 60 minutes for anticoagulated blood.

From each tube, 100 µL aliquots of the plasma or serum samples (diluted 1/1,000 dilution and 1/10 dilution, respectively) were plated in triplicate. Serial dilutions of the fibrinogen standard (11 dilutions covering a concentration range of 1,000 ng/mL to 10 ng/mL) were plated out in duplicate. The plates were incubated overnight at 4°C.

The plates were washed six times with wash buffer to remove unbound components and incubated overnight at 4°C with 100 µL of AHFAS-HRP working solution per well. The plates were again washed 6 times with wash buffer before adding 100 µL HRP substrate solution per well.

Development of a blue colour was monitored in a dark environment until $A_{450}$ nm reached an absorbance of 1.0 at a fibrinogen concentration of 1 µg/mL. The reaction was then stopped by adding 100 µL per well of 1.0 M sulphuric acid solution, yielding a yellow colour. Plates were then read at $A_{450}$ nm in a Thermo Scientific Multiskan Ascent plate reader with Ascent software.

Example 9a: Fibrinogen/fdp/FDP levels in 48 serum samples collected in Greiner serum tubes (GS) from patients

Fibrinogen/fdp/FDP concentration was measured using the ELISA method outlined above in serum samples from Greiner serum (GS) tubes from 48 randomly selected patients requiring analyte determination for their clinical conditions.

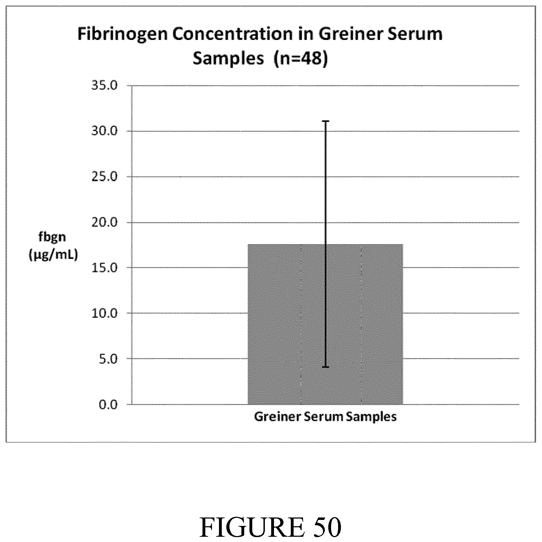

Figure 50 shows the results. The concentration of fibrinogen/fdp/FDP in these samples ranged from 4.4-32 µg/mL, compared with fibrinogen concentration in these plasma samples of 2.0-5.0 mg/mL. These data show that greater than or equal to 99% of fibrinogen is removed by the clotting process in standard commercially available serum tubes. These data also provide a reference interval range of fibrinogen in serum preparation in Greiner serum tubes from blood samples from a hospital population.

Example 9b: Fibrinogen/fdp/FDP levels in normal serum and plasma samples

36 normal serum samples (as determined by prothrombin time, aPTT and fibrinogen assays) were selected to investigate the effect of PtPA addition (300 ng/mL or 1.2 µg/4 mL tube) on the fibrinogen/fdp/FDP levels. Sera were prepared in Greiner serum tubes (primary tubes) under standard Pathology Queensland procedure prior to aliquoting two equal serum samples of 1 mL from each Greiner serum tube into plain plastic tubes (secondary tubes). PtPA (300 ng/mL or 1.2 µg/4 mL tube) was added to one tube and an equal volume (50 µL) saline to the second to provide matched pairs of secondary tubes.

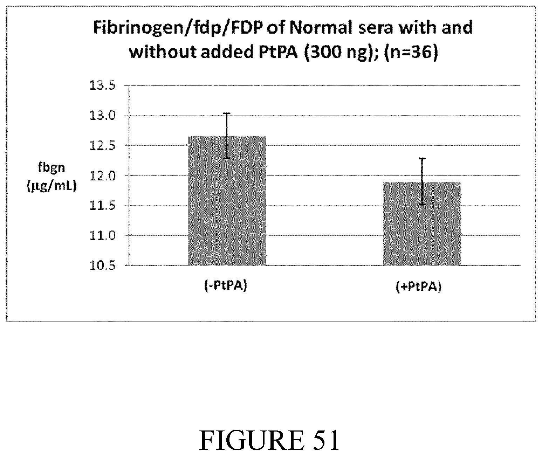

The fibrinogen/fdp/FDP concentration in each secondary tube was measured using the ELISA method outlined above and the results are shown in Figure 51. In both secondary tubes residual fibrinogen was reduced to less than 1% of that present in blood. However, addition of PtPA further reduced the fibrinogen/fdp/FDP levels from a mean of 12.8 µg/mL to 11.8 µg/mL. This reduction was significant ($p < 0.04$) in a paired t-test analysis. Thus the PtPA was capable of further reducing serum fibrinogen/fdp/FDP even in normal individual sera in which residual fibrinogen/fdp/FDP levels were very low. The very small amounts remaining (<1.0%) are likely to be molecules which react with antibody but are not able to polymerise to form insoluble clot.

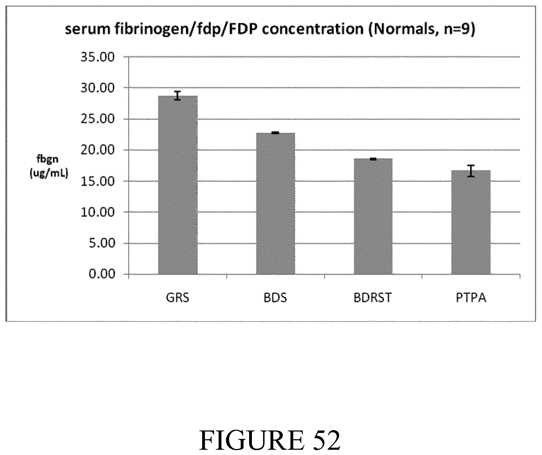

9 normal blood samples were then collected into four different serum tubes: Greiner serum, BD serum, BD RST and PtPA-containing Greiner Vacuette™ No Additive tubes. The fibrinogen/fdp/FDP concentration in each secondary tube was measured using the ELISA method outlined above and the results are shown in Figure 52. The fibrinogen concentration was considerably lower in the sera prepared in PtPA-containing Greiner Vacuette™ No Additive tube.

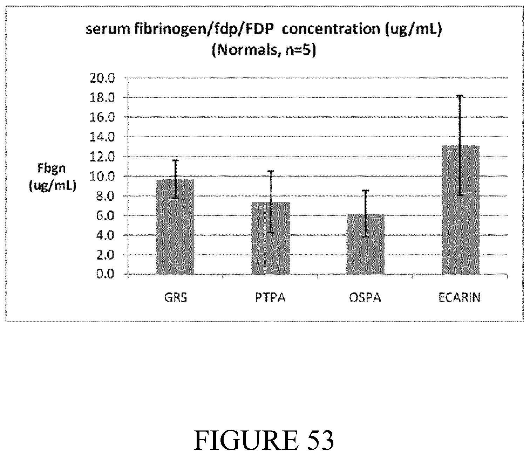

The concentration of fibrinogen/fdp/FDP in serum produced using OsPA and ecarin was then investigated. Serum samples were prepared from blood of 5 normal participants in Greiner serum tubes (GRS) and in Greiner No Additive tubes containing OsPA (0.50 µg/4 mL tube), ecarin (0.63 units/4 mL tube) and PtPA (1.2 µg/4 mL tube). Fibrinogen/fdp/FDP concentration in the sera of each tube was measured using the ELISA method outlined above. The results are shown in Figure 53. In all cases residual fibrinogen/fdp/FDP levels were less than 1% that in normal blood or plasma.

Experiment 9c: Fibrinogen/fdp/FDP levels in serum samples prepared from heparinised patients

Patients undergoing renal dialysis require moderate levels of heparinisation (1,000 - 10,000 U of heparin during the treatment period) to avoid prothrombotic events during dialysis. 3 patients from this category were chosen to test the ability of PtPA tubes (1.2 µg/4 mL tube) and BD RST tubes to efficiently clot blood from dialysis patients in a 5 minute incubation time, compared to the clotting of blood taken in Greiner serum (GRS) and BD serum (BD SST II) blood collection tubes in a 30 minute clotting time period. The effectiveness of clotting was determined by measuring the fibrinogen/fdp/FDP concentration in the respective sera using the ELISA method outlined above.

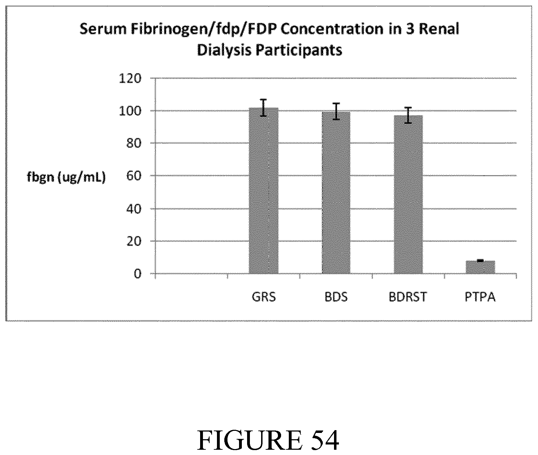

The results are shown in Figure 54 and reveal that the residual levels of fibrinogen/fdp/FDP in the PtPA-produced sera from the heparinised blood were comparable to those values in the PtPA-produced sera from normal blood (Figures 51 and 53). In contrast, residual fibrinogen/fdp/FDP levels in sera produced in the GRS, BDS and BD RST tubes were much higher. At these levels, latent clot formation and microclots are especially likely in the presence of heparin. The values in Figure 54 for GRS, BDS and BDRST are minimal estimates based on a single dilution of sample.

Blood samples from 2 cardiac surgery patients (treated with 25,000-41,000 Units of heparin) were collected into the following tubes: Greiner plasma tubes, Greiner serum tubes, BD serum tubes, BD RST tubes, Greiner Vacuette™ No Additive tubes with PtPA added (1.2 $\mu$g/4mL tube), Greiner Vacuette™ No Additive tubes with OsPA added (0.50 $\mu$g/4mL tube), Greiner Vacuette™ No Additive tubes with 1.25 units/L of ecarin per 4mL tube added (EC1), or Greiner Vacuette™ No Additive tubes with 2.5 units/L of ecarin per 4mL tube added (EC2).

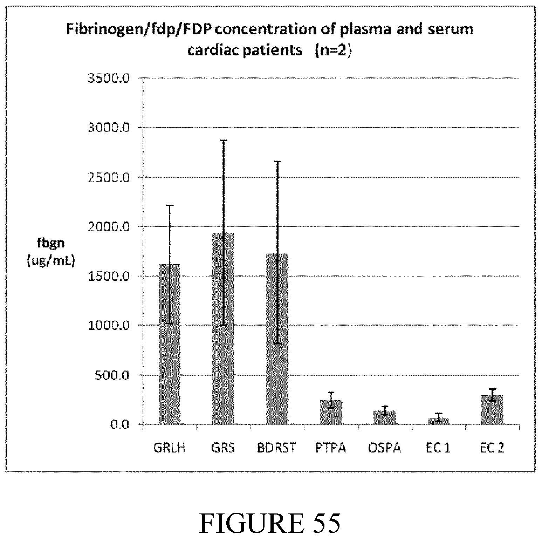

The fibrinogen/fdp/FDP concentration in the sera and plasma of each tube was determined using the ELISA method outlined above and the results are shown in Figure 55. The fibrinogen/fdp/FDP concentration was greatly reduced in serum obtained using the prothrombin activator-containing tubes compared to the plasma and other serum tubes. Furthermore, the time allowed for clotting was only five minutes with the prothrombin activator-containing tubes compared to 30 minutes in the other tubes or 60 minutes in the tubes for the anticoagulated blood.

In summary, the tubes containing prothrombin activators were able to produce serum samples with a lower concentration of residual fibrinogen/fdp/FDP than in serum samples produced using commercially available serum or plasma tubes. For normal samples, the effect was relatively small. However, with heparinised samples, the prothrombin activators were able to produce serum with low residual fibrinogen compared to serum prepared in commercial serum tubes. The results suggest that a sufficiently low concentration of residual fibrinogen/fdp/FDP should be achievable by using a prothrombin activator to avoid latent clotting or precipitation in all serum samples. This ability to produce "high quality" serum from patients on high anticoagulant doses (e.g. cardiac care, dialysis) and in "fully heparinised" samples from patients undergoing cardiac surgery is useful.

EXAMPLE 10: HAEMOGLOBIN CONCENTRATION IN PLASMA AND SERUM SAMPLES

The haemolytic index is used routinely in chemical pathology as a measure of the haemoglobin present in serum and plasma samples and hence the extent of cell lysis. Lysis of all types of blood cells, including erythrocytes, white cells and platelets, can occur *in vivo,* during blood collection and serum/plasma preparation and on standing. *In vitro* cell lysis commonly occurs during collection of samples or transfer of samples using small gauge needles or transfer devices which are normally under pressure. *In vivo* red cell lysis alone may occur in haemolytic anaemias. During the lysis of cells *in vitro,* cellular content is released into the serum or plasma, falsely altering the results of some analytes; release of cellular content may even cause dilution of other analytes if the haemolysis is extensive. Haemoglobin in plasma or serum may cause spectral problems during analysis and other cellular analytes may cross react. Serum normally contains slightly higher haemoglobin than lithium heparin plasma and this is considered to be due to the clotting process lysing a small number of cells as the clot expands and contracts. A low haemoglobin concentration is therefore an important criterion for a "high quality" serum sample.

The following experiment was performed to compare the haemoglobin content of serum produced in the presence and absence of PtPA and in plasma produced using lithium heparin. Blood samples were collected from 2 patients undergoing citrate anticoagulation therapy and 9 healthy participants. Samples were collected in Greiner plasma tubes, Greiner serum tubes, and Greiner Vacuette™ No Additive tubes containing PtPA (1.2 $\mu$g/4 mL of blood). Tubes were centrifuged 5 minutes after collection for the Greiner plasma tubes and Greiner Vacuette™ No Additive tubes with PtPA tubes and 30 minutes for the Greiner Vacuette™ No Additive tubes (without PtPA). The samples were analysed for haemoglobin within 30 minutes after centrifugation. Results are shown in Table 63.

Table 63: Haemoglobin concentrations in plasma and serum samples.

| Sample | Haemoglobin concentration (mg/L) | | |
|---|---|---|---|
| | Greiner plasma | Greiner serum | Greiner Vacuette™ No Additive tubes with PtPA |
| **Number of samples (2 citrated and 9 healthy participants)** | 11 | 11 | 11 |
| **Mean** | 55 | 67 | 46 |
| **Standard deviation** | 26 | 28 | 25 |

The mean haemoglobin concentration in the plasma samples was 55 mg/L, somewhat lower than the 67 mg/L found for the Greiner serum, as expected from the literature. The mean value for the PtPA serum was 46 mg/L, considerably lower than that found for Greiner serum and even lower than that found for plasma. The standard deviations of the mean are large because of the individual variation from person to person. They do not give an estimate of the significance of differences between the three types of tubes for any one person. To check for the significance of these differences, paired two tailed t-tests were performed using the data from the 11 blood samples. The results were as follows:

Greiner plasma v Greiner serum: p = 0.1243

Greiner serum v Greiner Vacuette™ No Additive tubes with PtPA: p = 0.0188 (statistically significant P<0.05)

Greiner plasma v Greiner Vacuette™ No Additive tubes with PtPA: p = 0.1038

The conclusions that can be drawn from this Example are that:

(1) The haemoglobin concentration in the plasma samples prepared in the Greiner plasma tubes was lower than that in serum prepared in the Greiner serum tubes, as expected from the literature;

(2) The haemoglobin concentration in the sera prepared in the Greiner Vacuette™ No Additive tubes with PtPA was significantly lower than that in the Greiner serum. The lower level of haemoglobin in these serum samples may reflect the much faster rate of clotting, limiting the amount of cell lysis which occurs during the clotting process; and

(3) The mean haemoglobin concentration in the PtPA serum samples was lower than that in the plasma samples and the difference was of borderline significance (p = 0.1038).

Thus, the use of the PtPA tube gave sera of higher quality in terms of haemoglobin content than use of the commercial Greiner serum tube.

EXAMPLE 11: PRESENCE OF CELLS AND CELLULAR CONTENT IN PLASMA AND SERUM SAMPLES

Lithium heparin plasma prepared in commercially available tubes contains residual cells in suspension or in the buffy coat layer on top of the gel barrier in contact with the plasma after centrifugation. Serum prepared from healthy participant samples in commercially available tubes contains fewer cells in contact with the serum (compared with plasma). Storage of plasma and serum for at least 7 days at 2-8°C is a regulatory requirement in Australia under the National Pathology Laboratory Accreditation Advisory Council (NPACC) in case of re-analysis or requests for additional analyses. The presence of cells can have two effects during storage and analysis of serum or plasma. Firstly, cells may lyse, releasing cellular contents (*e.g.* $K^+$, lactate dehydrogenase) into the serum or plasma. This can lead to significant differences between measurements made immediately after centrifugation and measurements after a period of storage. Secondly, cells continue to be metabolically active and may use up significant amounts of nutrients (*e.g.* glucose) and release metabolic products (*e.g.* lactate) on storage. Changes can even be observed in blood samples of many tubes when the samples are stored for the usual recommended 30 minute clotting time when the samples are from healthy participants.
The degree of cellular contamination is therefore an important quality criterion for serum samples and an important advantage of using serum over plasma.
The following experiments were performed to compare serum prepared using PtPA with serum prepared using Greiner serum tubes and lithium heparin plasma prepared using Greiner plasma tubes. The extent of contamination of serum and plasma samples by cells and cell contents can be assessed using several markers of contamination. Three markers of cellular contamination that were used here are: increase in lactate dehydrogenase (LD) activity on storage; decrease in glucose concentration on storage; and direct observation of cells.

Example 11a: Comparison of PtPA serum with lithium heparin plasma from healthy participants prepared in commercially available tubes

Blood was collected from 10 healthy participants in Greiner plasma tubes, and Greiner serum tubes containing PtPA, prepared as follows. The insides of the Greiner serum tubes were cleaned to remove the Greiner procoagulants/additives by filling the tubes with sterile de-ionized water, inverted ~20 times, allowed to stand 10 minutes, and then the inside wall and inner part of tube cap were scrubbed with sterile cotton swab without disturbing the gel barrier. The content was discarded and the tubes were further filled/inverted/rinsed 3 times with de-ionised water. The cleaned tubes were finally placed in a drying oven at 40°C to completely dry any water droplets before the PtPA was dispensed (1.2 μg/4 mL of blood). Blood was then collected into the tubes. The two tubes (Greiner plasma and PtPA) from each participant were centrifuged and immediately analysed for lactate dehydrogenase (LD) and glucose (zero time) levels. The samples were allowed to stand at room temperature (21°C) and re-analysed ~8 hours later on the same analyser. Results are shown in Tables 64 and 65.

Table 64: LD activity (U/L) of samples from 10 participants measured at 0 and 8 hours post centrifugation stored at 21°C.

| A | Greiner plasma | | | | PtPA | | | |
|---|---|---|---|---|---|---|---|---|
| | 0 hr | 8 hr | 8-0 hr* | % 8-0 hr* | 0 hr | 8 hr | 8-0 hr* | % 8-0 hr* |
| Mean | 177 | 199 | 22 | +12.5 | 175 | 173 | 1.3 | -0.6 |
| SD | 14 | 23 | 16 | 9.2 | 19 | 16 | 3.4 | 2.0 |
| * = Difference between 8 hours and 0 hours and percent difference between 8 hours and 0 hours. | | | | | | | | |

Table 65: Glucose concentration (mmol/L) of samples from 10 participants measured at 0 and 8 hours post-centrifugation stored at 21°C.

| B | Greiner plasma | | | | PtPA | | | |
|---|---|---|---|---|---|---|---|---|
| | 0 hr | 8 hr | 8-0 hr* | % 8-0 hr* | 0 hr | 8 hr | 8-0 hr* | % 8-0 hr* |
| Mean | 5.1 | 4.7 | 0.5 | -9.1 | 5.2 | 5.1 | 0.08 | -1.5 |
| SD | 0.8 | 0.9 | 0.2 | 5.1 | 0.8 | 0.8 | 0.04 | 0.9 |
| * = Difference between 8 hours and 0 hours and percent difference between 8 hours and 0 hours. | | | | | | | | |

In serum samples from the PtPA tubes, the LD and glucose results showed 0.6% and 1.5% change over the 8 hour period compared with the 12.5% and 9.1% change for the Greiner plasma tube samples respectively. In the plasma samples, the presence of cells resulted in glucose consumption, and the LD increased due to leakage from the cells lysed.

The results confirm that clotting of the blood with PtPA removes cells effectively and this prevents any significant changes in the most affected analytes, glucose and LD, for up to 8 hours. Thus, inclusion of PtPA in a serum tube provided high quality stable serum from the healthy participants' blood samples.

Example 11b: Cellular contamination of serum and plasma prepared from anticoagulated patients

Blood samples were collected from two participants (PI and P2) undergoing cardiac surgery who had received in total: P1 - 30000, and P2 - 35000 units of heparin just prior to blood collection (within 15 minutes post heparin infusion). The samples were collected in 50 mL plain syringes (BD Plastipak REF #300866) filled with ~30 mL blood. The syringes were delivered to the laboratory within 15 minutes of collection. The following tubes were filled with blood: Greiner plasma tube, Greiner serum tube, and Greiner serum tube (cleaned as per the procedure above) containing PtPA (1.2 μg/4 mL of blood). The plasma and PtPA containing tubes were centrifuged immediately on arrival at the laboratory. The Greiner serum tube was allowed to stand for 60 minutes before centrifugation. The samples were analysed, then allowed to stand at room temperature (21°C) and re-analysed 24 hours later on the same analyser. Results are shown in Tables 66 and 67.

Table 66: LD activity (U/L) of samples from 2 cardiac surgery participants measured at 0 and 24 hours post-centrifugation stored at 21°C.

| A | Greiner Plasma | | | | Greiner Serum | | | | PtPA Serum | | | |
|---|---|---|---|---|---|---|---|---|---|---|---|---|
| | 0 hr | 24 hrs | Dfce (24-0 hrs) | % Dfce (24-0 hrs) | 0 hr | 24 hrs | Dfce (24-0 hrs) | % Dfce (24-0 hrs) | 0 hr | 24 hrs | Dfce (24-0 hrs) | % Dfce (24-0 hrs) |
| Mean | 87 | 94 | 8 | +9 | 87 | 98 | 11 | +13 | 84 | 81 | -3 | -3 |
| SD | 11 | 1 | 9 | 12 | 8 | 9 | 1 | 1 | 9 | 10 | 1 | 1 |

Table 67: Glucose concentration (mmol/L) of samples from 2 cardiac surgery participants measured at 0 and 24 hours post-centrifugation stored at 21°C.

| B | Greiner Plasma | | | | Greiner Serum | | | | PTPA Serum | | | |
|---|---|---|---|---|---|---|---|---|---|---|---|---|
| | 0 hr | 24 hrs | Dfce (24-0 hrs) | % Dfce (24-0 hrs) | 0 hr | 24 hrs | Dfce (24-0 hrs) | % Dfce (24-0 hrs) | 0 hr | 24 hrs | Dfce (24-0 hrs) | % Dfce (24-0 hrs) |
| Mean | 5.4 | 4.6 | -0.8 | -14.8 | 5.4 | 4.7 | -0.8 | -13.9 | 5.3 | 5.3 | -0.1 | -0.9 |
| SD | 0.1 | 0.1 | 0.0 | 0.4 | 0.1 | 0.1 | 0.1 | 1.0 | 0.1 | 0.1 | 0.1 | 1.3 |

In the PtPA-produced serum samples the LD and glucose results showed a change of -3 and -0.9% at 24 hours compared with the 9%, and -14.8% change in the lithium heparin plasma, and 13% and -13.9% for serum samples respectively. Similar to the healthy participant group of Examples 11a, the presence of cells in plasma resulted in glucose consumption and with cell lysis, the activity of LD was increased. The Greiner serum tubes showed plasma like results, as expected, since the samples never clotted.

In the Greiner serum tube no blood clotting was observed nor was any latent clotting detected visually or by analysers over the 24 hours post-centrifugation. The samples in the PtPA-containing tubes clotted within 3-5 minutes. The 3 tubes of participant P1 were photographed to indicate the presence of cells on top of the gel barrier. In the PtPA tube, very few cells were present above or within the gel barrier, in contrast to the other two tubes where cells were visible throughout and above the gel barrier.

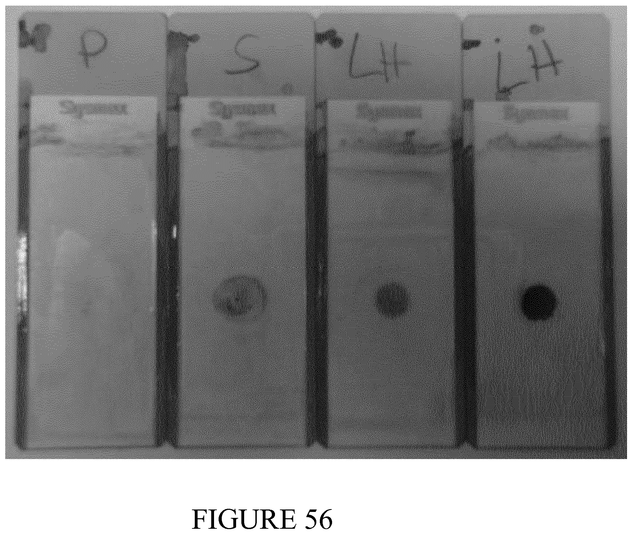

From each of the 3 tubes the majority of the plasma or serum content was gently removed without disturbing the layer on top of the gel barrier, leaving about 0.5 mL of the plasma or serum in the tube. The residual plasma or serum in each tube was re-mixed with the buffy coat and transferred into a slide centrifuge, Cytospin (Shandon-Elliott Cytospin, Shandon-Elliott Instruments Limited) to concentrate the cells and produce a Giemsa-stained slide of the cells, cell stroma, *etc* for microscopic examination. The slides (shown in Figure 56) clearly indicate presence of cells in abundance in the plasma, slightly fewer in the Greiner serum and minimal cells in the PtPA-produced serum.

These results confirm clotting of the blood with PtPA was achieved even in so called "fully heparinised" participants, in a very short time, <5 minutes. After centrifugation, the number of residual cells was minimal and insufficient to have an effect on LD activity or glucose concentrations.

Example 11c: Effect of prolonged storage of serum and plasma samples from one cardiac surgery participant on analytes

The 3 samples from cardiac surgery participant P2 after 24 hours at room temperature (21°C) were stored in the primary tubes at 4°C for 13 additional days, (total of 14 days or 336 hours post collection) and then were re-analysed for $K^+$, glucose, LD and phosphate (Pi). The results are shown in Table 68.

Table 68: Concentration of analytes in serum and plasma samples from a cardiac surgery participant measured at 0, 24 and 336 hours post-centrifugation

| Analyte | 0 hr | 24 hrs | 336 hrs | Dfce (24-0) | %Dfce (24-0) | Dfce (336-0) | %Dfce (336-0) |
|---|---|---|---|---|---|---|---|
| **Greiner Plasma** | | | | | | | |
| $K^+$(mmol/L) | 4.7 | 4.6 | 4.9 | -01 | -2.1 | 0.2 | **4.3** |
| Glucose (mmol/L) | 5.5 | 4.1 | 3.1 | -1.4 | -25.5 | -2.4 | **-43.6** |
| LD(U/L) | 79 | 93 | 208 | 14 | 17.7 | 129 | **163.0** |
| Pi (mmol/L) | 0.59 | 0.6 | 0.69 | 0.01 | 1.7 | 0.1 | **16.9** |
| **Greiner Serum** | | | | | | | |
| $K^+$ (mmol/L) | 4.7 | 4.7 | 5.2 | 0 | 0 | 0.5 | **10.6** |
| Glucose (mmol/L) | 5.5 | 4.7 | 3.8 | -0.8 | -14.5 | -1.7 | **-30.9** |
| LD (U/L) | 81 | 91 | 180 | 10 | 12.3 | 99 | **122.2** |

(continued)

| Greiner Serum | | | | | | | |
|---|---|---|---|---|---|---|---|
| Pi (mmol/L) | 0.58 | 0.63 | 10.74 | 0.05 | 7.8 | 10.16 | **27.6** |
| PtPA Serum | | | | | | | |
| $K^+$ (mmol/L) | 4.6 | 4.6 | 4.6 | 0 | 0 | 0 | **0** |
| Glucose (mmol/L) | 5.4 | 5.3 | 5.3 | 0.1 | 1.9 | -0.1 | **-1.9** |
| LD (U/L) | 77 | 74 | 64 | 3 | 3.9 | -13 | **-20.3** |
| Pi (mmol/L) | 0.57 | 0.58 | 0.65 | -0.01 | -1.8 | 0.08 | **12.3** |

Unlike the lithium heparin plasma and "serum" from the Greiner tubes, the PtPA serum sample showed insignificant changes in the $K^+$ and glucose results, while the LD and Pi results showed minimal changes.

In this extreme case scenario, serum produced using PtPA provided easily the most stable sample type that will allow laboratories to provide additional analyte testing at later dates and have confidence the results will be accurate. The 20% fall in LD activity in the PtPA sample was probably due to slow denaturation of existing LD on storage.

Overall Conclusion

PtPA produced serum offers outstanding stability in analyte concentrations that are most likely to be affected by the incomplete removal of cells from the plasma or serum component above the gel barrier during the clotting and/or centrifugation process in all patients. Our results also show by direct observation the relative absence of cells from the PtPA produced sera compared with Greiner lithium heparin plasma prepared from healthy participants or patients on high doses of heparin.

EXAMPLE 12: MEASUREMENT OF BIOCHEMICAL ANALYTES IN PLASMA AND SERUM SAMPLES PREPARED IN COMMERCIAL TUBES AND SERUM SAMPLES PREPARED USING VENOM PROTHROMBIN ACTIVATORS ECARIN,PTPA, AND OSPA)

As discussed in earlier parts of this specification, current commercially available blood collection tubes are unable to produce completely clotted serum from all blood samples in a timely manner to meet the quality and turn-around time expectations from biochemistry laboratories for optimal patient care. The results in the above examples demonstrate that prothrombin activators can be employed to produce a quality serum sample rapidly with low levels of fibrinogen/fdp/FDP and without cellular contaminations (as determined by visual inspection, clarity of the serum and analysis) in blood from a wide variety of patients/individuals.

It was important therefore to determine whether the prothrombin activators might interfere with analysis of analytes commonly used for clinical management of patients. Prothrombin activators are proteolytic enzymes which could in principle cleave serum proteins or proteins involved in analytical methodology, thereby affecting analytical results. Proteins are involved in analytical methods either as the analyte of interest or as reactants (e.g. enzymes, antibodies) used to measure the analytes of interest.

The purpose of this example was to investigate whether serum prepared using prothrombin activators gives the same analytical results as plasma and serum prepared in current commercially available tubes using current commercial methods.

It is not uncommon for more than 30 biochemical analytes to be requested and performed from a single serum or plasma tube. As the range of analytes increases and the analytical volumes per analyte decreases with technological improvements, the number of analyses from a single tube will further increase. Hence it is essential tube additives, specifically procoagulants, be inert and not impose any analytical effect on analytes yet provide highest quality sample for the most accurate estimation of analytes.

Standard analytical test procedures were used in the following experiments for each of the 33 assays which are listed below:

Test 1: Sodium ($Na^+$)

Test 2: Potassium ($K^+$)

Test 3: Chloride (Cl⁻)

Test 4: Bicarbonate ($HCO_3^-$)

Test 5: Glucose (Glue)

Test 6: Urea (Urea)

Test 7: Creatinine (Creat)

Test 8: Urate (Urate)

Test 9: Total Protein (TP or T Prot)

Test 10: Albumin (Alb)

Test 11: Total Bilirubin (T Bili)

Test 12: Alkaline Phosphatase (ALP)

Test 13: Gamma-Glutamyl Transferase (GGT)

Test 14: Alanine Aminotransferase (ALT)

Test 15: Aspartate Aminotransferase (AST)

Test 16: Lactate Dehydrogenase (LD)

Test 17: Creatine Kinase (CK)

Test 18: Total Calcium (TCa)

Test 19: Phosphate (Pi or Phos)

Test 20: Magnesium ($Mg^{2+}$)

Test 21: Lipase (Lipase)

Test 22: Cholesterol (Chol)

Test 23: Triglycerides*

Test 24: High-Density Lipoprotein Cholesterol (HDL-C or HDL)

Test 25: Iron ($Fe^{2+}$)

Test 26: Transferrin (Trf)

Test 27: C Reactive Protein (CRP)

Test 28: Cortisol Test (Cortisol)

Test 29: Free Thyroxine (FT4)

Test 30: Thyroid Stimulating Hormone (TSH)

Test 31: Ferritin (Ferritin)

Test 32: Troponin (TnI)

Test 33: Haemolytic Index (Haem index)**

Test 34: Icteric Index*

Test 35: Lipemia Index*

*Triglycerides were not measured in these experiments because of the presence of glycerol in the PtPA preparation. Icteric and Lipaemic indices were also not determined in this study.

** See also Example 10.

Test Analysis

Analysis was performed on Beckman DxC800 general chemistry analysers and a DxI800 immunoassay analyser (Beckman Coulter, Fullerton, CA, USA). Samples were loaded on the same instruments at the same time and within 1-2 hours post-centrifugation, except where recurrent latent clotting was encountered.

The analytes tested plus semi-quantitative haemolysis levels are listed in the result tables. The upper limit of imprecision of the between-run coefficient of variations (CVs) from the two and three internal quality control concentrations for the 35 analytes tested on the Beckman DxC800 analysers the DxI800 analyser respectively are shown in Table 69. We also measured the activated partial thromboplastin time (aPTT) on an AC[TOPS]. (Instrumentation Laboratory, Lexington MA, USA) since it was difficult to ascertain the exact anticoagulant concentration in the participants' blood samples at the time of the specimen collection in the cardiac care unit and dialysis participants. In all cases, results were consistent with listed degree of anticoagulation.

Data Analysis

The results of each test were obtained and then data analysis was performed as follows. The mean and standard deviation (SD) were calculated for each test for each type of tube. The difference (actual and %) between each result pair for each test between the different tubes (*e.g.* PtPA tube result versus Greiner serum result type) was also calculated. The % difference was then compared with the between-run precision values obtained on the analysers (Table 69) to determine if there were analytically significant differences between the different serum tubes and the different serum tubes versus plasma tubes. The participant data was also separated into healthy participants and anticoagulated participants, and the same analysis performed. If a measurement was not obtained for an analyte in any of the three tubes (due to recurrent latent clotting leading to insufficient specimen, insufficient specimen collected, not requested on analyser, or insufficient reagent), the result was not included in the calculation accounting for the variability in the number of specimens analysed per assay.

Table 69: Quality Control (QC) imprecision on the Beckman DxC800 and DxI800 analysers (*DxI800)

| Analyte | QC Level 1 | | | QC Level 2 | | | QC Level 3 | | |
|---|---|---|---|---|---|---|---|---|---|
| | Mean | SD | CV % | Mean | SD | CV % | Mean | SD | CV % |
| $Na^+$ | 132 | 1.27 | 1.0 | 150 | 1.33 | 0.9 | | | |
| $K^+$ | 3.9 | 0.06 | 1.4 | 6.0 | 0.07 | 1.2 | | | |
| Cl- | 85 | 1.17 | 1.4 | 100 | 1.19 | 1.2 | | | |
| $HCO_3^-$ | 17 | 0.66 | 3.8 | 29 | 0.85 | 2.9 | | | |
| Gluc | 4.8 | 0.12 | 2.6 | 16.7 | 0.28 | 1.8 | | | |
| Urea | 5.2 | 0.17 | 3.3 | 15.9 | 0.41 | 2.6 | | | |
| Creat | 68 | 3.34 | 4.9 | 491 | 8.93 | 1.8 | | | |
| Urate | 0.23 | 0.001 | 1.9 | 0.49 | 0.01 | 1.5 | | | |
| T Prot | 41 | 0.73 | 1.8 | 67 | 1.07 | 1.6 | | | |
| Alb | 26 | 0.37 | 1.4 | 40 | 0.54 | 1.3 | | | |

(continued)

| Analyte | QC Level 1 | | | QC Level 2 | | | QC Level 3 | | |
|---|---|---|---|---|---|---|---|---|---|
| | Mean | SD | CV % | Mean | SD | CV % | Mean | SD | CV % |
| T Bili | 21 | 1.37 | 6.4 | 93 | 1.96 | 2.1 | | | |
| ALP | 106 | 2.79 | 2.6 | 471 | 7.93 | 1.7 | | | |
| GGT | 38.7 | 2.03 | 5.3 | 155 | 2.88 | 1.9 | | | |
| ALT | 25 | 1.52 | 6.2 | 94 | 2.00 | 2.1 | | | |
| AST | 34 | 1.35 | 3.9 | 201 | 2.39 | 1.2 | | | |
| LD | 149 | 3.21 | 2.2 | 408 | 5.65 | 1.4 | | | |
| CK | 139 | 2.53 | 1.8 | 459 | 5.8 | 1.3 | | | |
| TCa | 2.07 | 0.03 | 1,6 | 2.86 | 0.04 | 1.3 | | | |
| Phos | 1.00 | 0.02 | 1.9 | 2.86 | 0.04 | 1.5 | | | |
| $Mg^{2+}$ | 0.84 | 0.02 | 2.5 | 1.63 | 0.04 | 2.3 | | | |
| Lipase | 29 | 2.39 | 8.2 | 57 | 4.05 | 7.1 | | | |
| Chol | 3.0 | 0.05 | 1.7 | 6.5 | 0.15 | 2.3 | | | |
| Trig | 1.0 | 0.04 | 3.6 | 2.0 | 0.05 | 2.7 | | | |
| HDL | 1.1 | 0.04 | 3.5 | 1.9 | 0.05 | 2.6 | | | |
| $Fe^{2+}$ | 10 | 0.29 | 2.8 | 34 | 1.93 | 5.7 | | | |
| Trf | 1.0 | 0.04 | 3.5 | 2.3 | 0.08 | 3.5 | | | |
| CRP | 4.6 | 0.36 | 7.9 | 10.0 | 0.55 | 5.4 | | | |
| TnI* | 0.052 | 0.01 | 16.1 | 0.55 | 0.04 | 7.9 | 10.2 | 0.69 | 6.7 |
| Cortisol* | 139 | 8.85 | 6.4 | 552 | 28.48 | 5.2 | 906 | 41.7 | 4.6 |
| fT4* | 7.51 | 0.62 | 8.2 | 23.93 | 0.94 | 4.0 | 50.9 | 1.92 | 3.8 |
| TSH* | 0.71 | 0.05 | 7.1 | 4.17 | 0.26 | 6.3 | 23.1 | 1.53 | 6.6 |
| Ferritin* | 19 | 1.68 | 8.8 | 159 | 9.67 | 6.1 | 358 | 22.42 | 6.3 |

Example 12a: Comparison of PtPA serum with serum and lithium heparin plasma prepared in commercial tubes

A total of 61 participants were recruited. All 61 participants were adults, > 18 years of age, with a mix of males and females. The participants consisted of two groups: 26 healthy volunteers and 35 anticoagulated patients.

Of the 35 anticoagulated patients, 1 was an outpatient on low dose warfarin therapy and 34 were inpatients.

Of the 34 inpatients, 11 were undergoing cardiac surgery, 8 were cardiac care unit patients, and 15 were on dialysis.

The 11 inpatients undergoing cardiac surgery had received in total 25,000-41,000 units of heparin at the time of the blood collection which was within 30 minutes post heparin infusion. The specimens were collected while participants were on bypass (where blood is pumped by machine not by the heart).

The 8 cardiac care unit participants were recruited the night before specimen collection and were receiving heparin by IV infusion, 950-1450 units of heparin per hour. Seven remained on IV heparin infusion (blood collection was ≥ 12 hours after IV heparin infusion started) at the time of specimens being collected, and the other was due for surgery on the day so had had the infusion stopped approximately three hours prior to the specimen collection (blood collection was > 9 hours after IV infusion had started and ~3 hours after IV heparin infusion was stopped). From the information in the patient records the heparin concentration in the infusate and the infusion rate were unchanged for the participants over the period between when IV heparin infusion started and blood was collected.

Of the 15 inpatients on dialysis, 12 were on heparin IV infusion, and 1 was on warfarin/heparin (~1750-7000 units of heparin, initial bolus plus hourly top up dose), and 2 were on clexane. Blood from these patients was collected at least one hour after dialysis commenced.

Blood was drawn using a standardised draw order. The blood was drawn by venipuncture from healthy volunteers, via bypass port from cardiac patients, and via bloodline from dialysis patients. The following tubes were used:

Greiner Vacuette™ citrate tube (for coagulation studies);

Greiner Vacuette™ No Additive tube with PtPA added (1.2 $\mu$g/4 mL) (PtPA);

Greiner Vacuette™ plasma tube (GLH);

BD Vacutainer™ plasma/PST II tube (BDLH);

Greiner Vacuette™ serum tube (GRS);

BD serum tube/SST II (BDS); and

BD RST tube (BD RST).

The blood for the PtPA-containing tube was collected in a plain syringe and then transferred from the syringe into the Greiner tube containing the PtPA without a needle to minimise cell lysis.

The Greiner serum and BD SST tubes were allowed to clot for the standard time of 30 minutes for healthy participants and 60 minutes for anticoagulated participants. These tubes were visually inspected for clot formation prior to loading in the centrifuge.

The BD RST and PtPA tubes were visually inspected for clot formation at 5 minutes for all participants at the point of collection (phlebotomy or clinical unit). The BD RST specimens for the healthy participants and for the anticoagulated participants that formed a solid clot at 5 minutes were centrifuged as soon as delivered to the laboratory (<20 minutes). If clotting was incomplete the specimens were re-checked every 10-15 minutes for clotting and left to clot for 60 minutes maximum. The lithium heparin and PtPA tubes were centrifuged immediately on delivery in the laboratory (<20 minutes from collection).

All tubes were centrifuged at 3000g for 10 minutes at 20°C in swing bucket centrifuges, and then stored at ~21°C. The tubes were visually inspected for latent clotting immediately after centrifugation and again just prior to loading onto analysers. These tube (primary tubes) were used for analysis except in cases where latent clotting was observed. In tubes where latent clotting was observed, the serum in the primary tubes was transferred to an aliquot tube, re-centrifuged to remove the clots, and the clean serum transferred to another aliquot tube (secondary tube) which was used for analysis. The Greiner No Additive tube does not contain a serum gel barrier, thus the PtPA generated serum was also transferred to a secondary tube for analysis to prevent re-mixing and prolonged contact with the cells.

Results

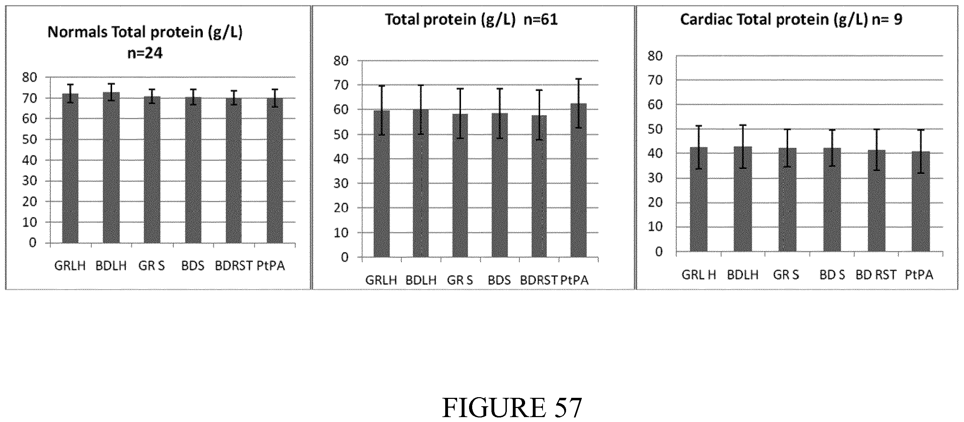

The results are shown in Table 70. Also, an example of results is provided in Figure 57 for the measurement of total protein (TP). The left graph shows the mean with standard deviation value of all normal patient results (n=26) for each tube type. The centre graph shows the mean value of all patient results (n=61) for each tube type. The right graph shows the mean value of results for cardiac patients (n=11) for each tube type. The units on the *y*-axis are g/L protein. The error bars are standard deviation.

Comparison of PtPA sera with commercial tube sera from healthy and anticoagulated participants.

No significant differences were observed with any of the analytes. This conclusion is based on comparison of the mean test values in Table 70 and a paired-wise statistical analysis described above.

Comparison of PtPA sera with commercial tube lithium heparin plasma from healthy and anticoagulated participants.

Significant differences were observed in some analytes ($K^+$, TP, AST and Pi) as expected because of the well established differences between plasma and serum. Sera prepared in the commercially available serum tubes showed similar analytical differences from plasma to those shown with PtPA sera.

Table 70: Analytical data for 32 analytes on serum and plasma samples.

| Number of samples | Analyte | Units | Mean ± SD | | | | | |
|---|---|---|---|---|---|---|---|---|
| | | | PtPA | GLH | BDLH | GS | BDS | BD RST |
| 60 | Na⁺ | mmol/L | 136.9 ±2.24 | 137.4 ±2.44 | 137.2 ±2.38 | 137.5 ±2.29 | 137.5 ±2.34 | 137.4 ±2.38 |
| 61 | K⁺ | mmol/L | 4.09 ±0.59 | 3.91 ±0.63 | 3.93 ±0.65 | 4.13 ±0.62 | 4.15 ±0.60 | 4.07 ±0.60 |
| 61 | Cl⁻ | mmol/L | 104.1 ±3.8 | 104.6 ±4.2 | 104.8 ±4.1 | 104.6 ±4.0 | 104.8 ±3.8 | 104.7 ±3.9 |
| 61 | HCO₃⁻ | mmol/L | 24.8 ±2.8 | 25.8 ±3.0 | 25.3 ±2.7 | 25.7 ±3.1 | 25.1 ±2.9 | 25.0 ±3.0 |
| 61 | Gluc | mmol/L | 6.2 ±2.09 | 6.3 ±2.15 | 6.3 ±2.11 | 6.2 ±2.07 | 6.2 ±2.11 | 6.3 ±2.08 |
| 61 | Urea | mmol/L | 5.3 ±2.0 | 5.3 ±2.0 | 5.4 ±2.0 | 5.3 ±2.1 | 5.4 ±2.1 | 5.3 ±2.1 |
| 61 | Creat | µmol/L | 134.1 ±112.8 | 135.1 ±115.0 | 136.3 ±114.7 | 137.1 ±116.0 | 136.7 ±116.0 | 138.1 ±116.6 |
| 60 | Urate | mmol/L | 0.27 ±0.11 | 0.27 ±0.12 | 0.27 ±0.12 | 0.28 ±0.11 | 0.28 ±0.11 | 0.27 ±0.12 |
| 61 | TP | g/L | 62.6 ±13.0 | 65.0 ±13.2 | 65.5 ±13.3 | 63.0 ±12.6 | 63.0 ±12.5 | 62.8 ±12.6 |
| 61 | Alb | g/L | 36.1 ±8.3 | 35.9 ±8.1 | 35.9 ±8.1 | 36.1 ±8.3 | 36.1 ±8.3 | 36.2 ±8.3 |
| 58 | T Bili | µmol/L | 13.5 ±3.8 | 13.6 ±3.7 | 13.4 ±3.4 | 13.3 ±3.6 | 13.4 ±4.0 | 13.2 ±3.8 |
| 61 | ALP | U/L | 74,1 ±26.9 | 68.9 ±26.5 | 69.4 ±26.6 | 71.3 ±26.9 | 71.7 ±27.5 | 71.4 ±26.9 |
| 61 | GGT | U/L | 27.9 ±17.6 | 28.3 ±17.0 | 28.1 ±16.9 | 27.3 ±17.1 | 28.1 ±17.1 | 227.8 ±17.5 |
| 61 | §ALT | U/L | 30.4 ±19.9 | 29.6 ±18.8 | 29.6 ±19.1 | 30.4 ±19.1 | 29.8 ±19.2 | 30.7 ±20.1 |
| 59 | §AST | U/L | 34.2 ±37.7 | 32.5 ±37.5 | 32.6 ±36.9 | 32.0 ±34.9 | 32.6 ±38.7 | 32.4 ±39.1 |
| 58 | §LD | U/L | 212.9 ±110.0 | 215.4 ±113.1 | 215.3 ±110.7 | 213.8± 106.0 | 222.8 ±120.0 | 212.5 ±119.2 |
| 59 | §CK | U/L | 147.1 ±250.3 | 146.6 ±250.0 | 145.3 ±242.8 | 146.0 ±241.3 | 149.4 ±259.6 | 147.7 ±253.3 |
| 61 | TCa | mmol/L | 2.19 ±0.26 | 2.17 ±0.24 | 2.17 ±0.24 | 2.20 ±0.26 | 2.20 ±0.26 | 2.19 ±0.26 |
| 61 | Pi | mmol/L | 1.07 ±0.28 | 1.02 ±0.28 | 1.02 ±0.28 | 1.08 ±0.30 | 1.08 ±0.30 | 1.08 ±0.30 |
| 57 | Mg²⁺ | mmol/L | 0.94 ±0.19 | 0.95 ±0.20 | 0.94 0.19 | 0.94 ±0.18 | 0.94 ±0.20 | 0.93 ±0.20 |
| 59 | Lipase | U/L | 28.2 ±9.0 | 28.3 ±9.0 | 28.3 ±8.9 | 28.2 ±9.0 | 28.6 ±9.2 | 28.2 ±9.0 |
| 57 | Chol | mmol/L | 4.2 ±1.6 | 4.2 ±1.6 | 4.2 ±1.6 | 4.3 ±1.6 | 4.3 ±1.6 | 4.3 ±1,6 |
| 58 | HDL-C | mmol/L | 1.17 ±0.6 | 1.25 ±0.58 | 1.26 ±0.60 | 1.20 ±0.58 | 1.21 ±0.58 | 1.21 ±0.59 |
| 59 | Fe²⁺ | µmol/L | 14.6 ±6.6 | 14.5 ±6.5 | 14.6 ±6.7 | 14.7 ±6.7 | 14.9 ±6.7 | 14.6 ±6.6 |

(continued)

| Number of samples | Analyte | Units | Mean ± SD | | | | | |
| --- | --- | --- | --- | --- | --- | --- | --- | --- |
| | | | PtPA | GLH | BDLH | GS | BDS | BD RST |
| 59 | Trf | g/L | 2.17 ±0.74 | 2.13 ±0.71 | 2.14 ±0.72 | 2.17 ±0.73 | 2.19 ±0.73 | 2.18 ±0.77 |
| 57 | CRP | µg/L | 14.4 ±49.3 | 14.5 ±49.5 | 14.6 ±50.5 | 14.2 ±46.8 | 15.0 ±52.3 | 15.1 ±52.4 |
| 59 | Cortisol* | nmol/L | 356 ±247 | 354 ±241 | 356 ±254 | 356 ±249 | 352 ±248 | 358 ±250 |
| 60 | FT4* | Pmol/L | 13.6 ±4.1 | 12.7 ±3.7 | 12.7 ±3.6 | 12.7 ±3.9 | 12.7 ±3.9 | 12.8 ±3.7 |
| 61 | TSH* | µIU/mL | 1.81 ±2.12 | 1.87 ±2.05 | 1.89 ±2.12 | 1.83 ±2.10 | 1.82 ±2.02 | 1.86 ±2.08 |
| 43 | Ferritin* | µg/L | 204 ±191 | 199 ±191 | 202 ±192 | 208 ±203 | 207 ±198 | 204 ±187 |
| 60 | §TnI* | µg/L | 0.873 ±5.33 | 0.994 ±6.06 | 0.945 ±5.75 | 0.782 ±4.77 | 0.869 ±5.25 | 0.951 ±5.75 |
| 61 | Haem Index | | 0.2 ±0.7 | 0.2 ±0.7 | 0.3 ±0.7 | 0.3 ±0.7 | 0.2 ±0.7 | 0.2 ±0.7 |
| * Analysis performed on a DxI800 immunoassay analyser. § signifies non-parametric distribution used. The *p* value was determined by Wilcoxen Matched-Paris Ranks test. | | | | | | | | |

Example 12b: Comparison of PtPA, OsPA and ecarin serum with serum and plasma prepared in commercial tubes

7 participants were recruited consisting of 5 healthy volunteers with a mix of 4 males and 1 female, and 2 patients undergoing cardiac surgery (all adults >18 years of age). The two cardiac patients had received 34,000 and 43,000 units of heparin within 30 minutes post heparin infusion.

Blood from the healthy participants was drawn by venipuncture using standardized draw order: Greiner citrate plasma tube; Greiner serum tube; Greiner plasma tube; a plain syringe (Thermo 10 mL #CE0197) for the PtPA, OsPA or ecarin containing Greiner No Additive tubes (the concentration of the prothrombin activators in these 4 mL tubes was PtPA 1.2 µg, OsPA 0.5 µg and ecarin 0.625 units for healthy and 1.25 units for cardiac surgery participants). The blood from the syringe was transferred into the Greiner No Additive tubes containing the prothrombin activators without a needle to minimise cell lysis.

For the cardiac participants blood was collected in a plain syringe (30 mL) while on bypass. The blood was delivered to the laboratory within 15 minutes and dispensed into the various tubes listed above.

The Greiner serum tubes were allowed to clot for the standard time of 30 minutes for healthy participants and 60 minutes for the cardiac surgery participants, and then visually inspected prior to loading in the centrifuge for clot formation. The PtPA, OsPA and ecarin specimens were visually inspected for clot formation at 3 and 5 minute mark for all participants at the point of collection (phlebotomy for healthy participants and in the clinical unit for the cardiac surgery participants). The lithium heparin, PtPA, OsPA and ecarin tubes were centrifuged immediately on delivery in the laboratory (<30 minutes from collection).

All tubes were centrifuged at 3000g for 10 minutes at 20°C in a swing bucket centrifuge, and then stored at ~21°C. The tubes were visually inspected for latent clotting immediately after centrifugation and again just prior to loading onto analysers. The Greiner primary tubes were used for analysis. The Greiner No Additive tube does not contain a serum gel barrier, thus the serum generated using prothrombin activators was transferred to aliquot (secondary) tubes to prevent re-mixing and prolonged contact with the cells.

Results

Results are shown in Table 71 and support the following conclusions:

1. Comparison of sera produced by the prothrombin activators versus Greiner sera - no significant differences were observed with any of the analytes.

2. Comparison of sera produced by prothrombin activators versus Greiner lithium heparin plasma - significant differences were observed in some analytes ($K^+$, TP, AST and Pi) as expected because of the well established differences between plasma and serum. Sera prepared in commercial serum tubes showed similar analytical differences from plasma to those shown with prothrombin activator sera.

Table 71: Data for each analyte from the three different snake procoagulants and the Greiner tubes.

| No of Samples | Analyte | Mean ± SD | | | | | |
|---|---|---|---|---|---|---|---|
| | | Units | GLH | GS | PtPA | OsPA | Ecarin |
| 7 | Na$^+$ | mmol/L | 138.1±1.22 | 138.1±1.46 | 137.3±1.25 | 136.7±2.36 | 137.1±1.57 |
| 7 | K$^+$ | mmol/L | 4.40±0.82 | 4.61±0.71 | 4.53±00.77 | 4.50±0.76 | 4.49±0.79 |
| 7 | Cl$^-$ | mmol/L | 104.0±2.8 | 104.0±2.6 | 103.0±2.6 | 102.9±2.34 | 103.4±2.4 |
| 7 | HCO$_3^-$ | mmol/L | 25.1±3.2 | 25.3±3.5 | 23.9±2.4 | 24.1 ±2.7 | 23.3±2.2 |
| 7 | Gluc | mmol/L | 5.76±0.88 | 5.69±0.94 | 5.63±0.0.81 | 5.60±0.80 | 5.64±0.0.90 |
| 7 | Urea | mmol/L | 4.63±0.82 | 4.66±0.80 | 4.64±0.85 | 4.54±0.83 | 4.54±0.88 |
| 7 | Creat | μmol/L | 75.1±10.9 | 76.7±12.35 | 75.9±12.10 | 76.29±12.54 | 75.0±12.7 |
| 7 | Urate | mmol/L | 0.33±0.08 | 0.33±0.08 | 0.33±0.08 | 0.32±0.07 | 0.32±0.08 |
| 7 | TP | g/L | 68.5±11.0 | 62.7±11.6 | 63.9±11.8 | 62.4±14.1 | 63.4±13.4 |
| 7 | Alb | g/L | 38.3±5.5 | 38.3±5.5 | 38.6±5.8 | 38.4±6.0 | 38.6±5.8 |
| 7 | T Bili | μmol/L | 15.6±4.6 | 16.6±4.7 | 16.4±4.5 | 16.1±4.6 | 15.7±5.2 |
| 7 | ALP | U/L | 65.7±18.6 | 66.0±18.4 | 66.1±20.7 | 69.3±16.7 | 67.9±18.7 |
| 7 | GGT | U/L | 27.4±15.2 | 25.1±15.0 | 25.4±14.7 | 25.7±15.8 | 24.9±15.8 |
| 7 | §ALT | U/L | 30.4±11.4 | 31.0±11.8 | 32.0±11.4 | 31.7±12.2 | 32.1±11.8 |
| 7 | §ASTI | U/L | 22.6±7.8 | 25.0±8.3 | 24.1±7.2 | 24.9±6.5 | 24.9±6.7 |
| 7 | §LD | U/L | 194.4±23.1 | 193.6±41.6 | 195.6±22.4 | 189.0±18.9 | 192.7±21.2 |
| 7 | §CK | U/L | 98.9±34.9 | 97.9±37.5 | 98.6±36.8 | 97.1±34.0 | 98.0±36.1 |
| 7 | TCa | mmol/L | 2.20±0.236 | 2.23±0.245 | 2.22±0.255 | 2.20±0260 | 2.21±0.25 |
| 7 | Pi | mmol/L | 1.13±0.13 | 1.18±0.14 | 1.19±0.13 | 1.16±0.14 | 1.17±0.13 |
| 7 | Mg$^{2+}$ | mmol/L | 1.04±0.23 | 1.06±0.22 | 1.03±0.20 | 1.03±0.19 | 1.04±0.21 |
| 7 | Chol | mmol/L | 4.3±1.7 | 4.3±1.7 | 4.3±1.7 | 4.3±1.7 | 4.3±1.7 |
| 7 | HDL-C | mmol/L | 1.07±0.27 | 1.04±0.23 | 1.01±0.27 | 1.03±0.28 | 1.01±0.06 |
| 7 | Fe$^{2+}$ | μmol/L | 16.1±5.8 | 16.3±5.9 | 16.4±5.8 | 16.4±5.7 | 16.1±5.8 |
| 7 | Trf | g/L | 2.18±0.51 | 2.18±0.51 | 2.21±0.54 | 2.20±0.53 | 2.21±0.55 |

(continued)

| No of Samples | Analyte | Mean ± SD | | | | | |
|---|---|---|---|---|---|---|---|
| | | Units | GLH | GS | PtPA | OsPA | Ecarin |
| 7 | CRAP | μg/L | 1.7±1.5 | 1.9±1.6 | 1.7±1.5 | 1.9±1.6 | 1,7±1.5 |
| 7 | Cortisol* | nmol/L | 382.1±251.4 | 379.7±247.2 | 373.6±256.9 | 366.3±241.2 | 359.3±237.7 |
| 7 | FT4* | Pmol/L | 13.53±2.63 | 13.56±3.26 | 13.46±1.2.3 | 13.76±3.17 | 14.23±3.13 |
| 7 | FT3* | Pmol/L | 4.83±0.35 | 4.66±0.43 | 4.91±0.51 | 4.74±0.35 | 4.97±0.48 |
| 7 | TSH* | μIU/mL | 1.86±0.48 | 1.88±0.56 | 1.72±0.30 | 1.75±0.30 | 1.73±0.32 |
| 7 | Ferritin* | μg/L | 236.6±297.2 | 234.9±304.8 | 226.4±280.8 | 207.3±236.5 | 225.3±280.1 |
| 7 | §TnI* | μg/L | 0.079±0.136 | 0.071±0.137 | 0.091±0.165 | 0.092±0.170 | 0.093±0.174 |
| 7 | Haem Index | | 0.4±0.5 | 0.6±0.8 | 0.6±0.5 | 0.4±0.5 | 0.4±0.5 |

§ signifies non-parametric distribution used. The *p* value was determined by Wilcoxen Matched-Paris Ranks test.
* Analysis performed on a DxI800 immunoassay analyser.

Summary of Example 12

The blood of all the healthy and anticoagulated participants ranging from the lowest dose to the highest "fully" heparinised participant clotted within 5 minutes in the PtPA, OsPA and ecarin-containing tubes to give firm immobile clots. No latent clotting was visually observed or detected by the analysers in any of the sera produced by the prothrombin activators. Despite the prothrombin activators being proteolytic enzymes, they did not produce any analytically or clinically significant effect on any of the analytes measured irrespective of whether the analytes were proteins or whether proteins were used as reactive compounds in the analytical methods. There were no significant differences observed in any of the analytes between the sera prepared in the commercial tube and the sera prepared using prothrombin activators. The analytical and clinical differences observed in analytes between lithium heparin plasma prepared in a commercial tube and sera produced using prothrombin activators were in line with published data.

EXAMPLE 13: FURTHER MEASUREMENT OF BIOCHEMICAL ANALYTES IN PLASMA AND SERUM SAMPLES PREPARED IN COMMERCIAL TUBES AND SERUM SAMPLES PREPARED USING VENOM PROTHROMBIN ACTIVATORS (NOTECARIN AND CARINACTIVASE-2)

This Example follows from Example 12 and uses the same methodology described therein.

Example 13a: Analyte Measurement

Blood from 5 healthy participants was collected into Greiner No Additive tubes (Cat No 454001) to which 25 μL of notecarin or carinactivase-2 had been added to give concentrations of 12 nmol/mL and 45 nmol/mL respectively. Greiner Vacuette serum tubes (Cat No 456078; GS) were used as controls. The GS tubes were allowed to clot for 30 minutes as per manufacturer's recommendation prior to centrifugation. The tubes containing the prothrombin activators were observed for clotting immediately upon addition of the blood.

Notecarin and carinactivase-2 tubes clotted within 2 minutes and the tubes were brought to the laboratory and centrifuged within 7 to 15 minutes of collection. The clots formed in the tubes containing the activators were solid, and no latent clot formation was observed or detected.

The samples were then analysed for 31 analytes, and the results are shown in Table 72. For 27 analytes, the values obtained were equal within experimental error, with differences between paired results less than the least significant change (LSC%). The results where the differences were greater than the stated LSC (highlighted) can be explained as follows:

(1) For LD, the slightly lower activities in the two prothrombin activator samples may reflect decreased cellular contamination of these compared to Greiner serum;

(2) The high triglyceride (Trig) level in the Notecarin sample is due to interference by glycerol in which the Notecarin was stored;

(3) Notecarin serum produced higher AST levels, however the differences were clinically insignificant;

(4) Troponin results for the five patients were too low for accurate differentiation.

Table 72: Analytical results for serum samples generated by notecarin and carinactivase-2.

| No of Samples | Analyte | Units | Mean ± SD | | | Notec -GS Mean dffce between pairs (%) | CA2-GS Mean dffce between pairs (%) | LSC (%) | CAL (%) |
|---|---|---|---|---|---|---|---|---|---|
| | | | GS | Notecarin | CA-2 | | | | |
| 5 | Na$^+$ | mmol/L | 138.6±1.5 | 138±1.6 | 138±1.5 | -0.4 | -0.4 | 2.8 | 3 |
| 5 | K$^+$ | mmol/L | 3.94±0.17 | 3.94±0.18 | 3.83±0.18 | 0 | -2.9 | 3.3 | 5 |
| 5 | Cl$^-$ | mmol/L | 104.2±2.4 | 104.2±2.7 | 106.5±2.6 | 0 | 2.2 | 3.3 | 5 |
| 5 | HCO$_3^-$ | mmol/L | 26.8±2.6 | 25.6±2.5 | 25.6±2.2 | -4.5 | -4.5 | 4.8 | 10 |
| 5 | Gluc | mmol/L | 4.86±0.86 | 4.76±0.82 | 4.74±0.65 | -2.1 | -2.4 | 4.4 | 10 |
| 5 | Urea | mmol/L | 5.58±2.49 | 5.56±2.44 | 5.55±2.42 | -0.4 | -0.5 | 5.0 | 10 |
| 5 | Creat | μmol/L | 69.2±25.0 | 69.2±22.9 | 67.2±22.9 | -2.9 | -2.9 | 5.5 | 10 |
| 5 | Urate | mmol/L | 0.242±0.026 | 0.240±0.026 | 0.240±0.023 | -0.8 | -1.2 | 3.8 | 10 |
| 5 | TP | g/L | 66.0±2.0 | 65.0±2.2 | 63.6±2.4 | -1.5 | -3.3 | 3.5 | 5 |
| 5 | Alb | g/L | 40.8±1.9 | 40.2±1.9 | 39.5±1.4 | -1.5 | -3.1 | 3.2 | 5 |
| 5 | T Bili | μmol/L | 11.4±8.8 | 11.6±9.0 | 10.6±6.9 | 1.8 | -7.0 | 7.0 | 10 |
| 5 | ALP | U/L | 60.0±4.1 | 60.4±4.2 | 58.4±4.3 | 0.7 | -2.6 | 4.8 | 10 |
| 5 | GGT | U/L | 14.6±4.9 | 14.4±6.5 | 14.8±5.0 | -1.4 | 1.2 | 7.3 | 10 |
| 5 | ALT | U/L | 22.6±8.0 | 23.2±7.6 | 23.9±7.6 | 2.7 | 5.8 | 6.8 | 10 |
| 5 | AST | U/L | 17.2±3.8 | 19.8±4.4 | 17.3±3.5 | **15.1§** | 0.4 | **6.2** | **10** |
| 5 | LD | U/L | 192.2±17.0 | 184.8±18.3 | 179.9±17.6 | **-3.9§** | **-6.3§** | **3.7** | 15 |
| 5 | CK | U/L | 81.4±15.9 | 81.0±15.4 | 78.8±14.1 | -0.5 | -3.2 | 3.7 | 15 |
| 5 | TCa | mmol/L | 2.330±0.07 | 2.290±0.07 | 2.267±0.07 | -1.7 | -2.7 | 3.5 | 5 |
| 5 | Pi | mmol/L | 1.314±0,118 | 1.282±0.117 | 1.271±0.099 | -2.4 | -3.3 | 3.8 | 10 |
| 5 | Mg$^{2+}$ | mmol/L | 0.804±0.046 | 0.802±0.051 | 0.792±0.034 | -0.3 | -1.4 | 4.4 | 10 |
| 5 | Chol | mmol/L | 4.94±0.35 | 4.78±0.39 | 4.70±0.34 | -1.2 | -2.9 | 3.6 | 10 |
| 5 | Trig | mmol/L | 1.52±0.55 | 5.61±0.96 | 1.56±0.65 | **269#§** | 2.6 | **5.3** | **10** |
| 5 | HDL-C | mmol/L | 1.420±0.399 | 1.424±0.293 | 1.388±0.277 | 0.3 | -2.6 | 5.2 | 10 |
| 5 | Fe$^{2+}$ | μmol/L | 17.2±3.8 | 16.8±4.1 | 16.6±3.6 | -2.3 | -3.3 | 3.7 | 10 |

(continued)

| No of Samples | Analyte | | Mean ± SD | | | Notec -GS | CA2-GS | LSC (%) | CAL (%) |
|---|---|---|---|---|---|---|---|---|---|
| | | Units | GS | Notecarin | CA-2 | Mean dffce between pairs (%) | Mean dffce between pairs (%) | | |
| 5 | Trf | g/L | 2.596±0.187 | 2.566±0.188 | 2.508±0.188 | -1.16 | -3.4 | 4.8 | 10 |
| 5 | Cortisol* | nmol/L | 236.0±27.2 | 244.4±21.5 | 231.9±297.7 | 3.6 | -1.7 | 6.4 | 15 |
| 5 | FT4* | Pmol/L | 10.54±1.45 | 11.38±2.05 | 11.08±1.05 | -2.7 | 5.1 | 6.5 | 30 |
| 5 | TSH* | μIU/mL | 1.142±0.783 | 1.142±0.783 | 1.080±0.783 | 1.1 | -4.5 | 7.3 | 15 |
| 5 | Ferritin* | μg/L | 43.6±20 | 43.6±17.5 | 14.2±17.7 | 0.0 | 1.4 | 7.2 | 15 |
| 5 | TnI* | μg/L | 0.009±0.003 | 0.010±0.005 | 0.011±0.002 | **11.6** | **25.6** | **14** | 30 |
| 5 | Haem Index | | 0±0 | 0±0 | 0±0 | 0 | 0 | | |
| Analysis performed on Beckman DxC800 and *DxI800 immunoassay analysers. §= Those results which differ by more than the LSC and/or CAL. | | | | | | | | | |

Example 13b: Analyte stability

Greiner Serum tubes were washed (x5 with distilled water) to remove the surfactant and procoagulant but retain the gel barrier then oven dried. Notecarin and carinactivase-2 (25 $\mu$L aliquots) were added to the tubes to give concentrations of 12 nmol/mL and 45 nmol/mL respectively. Blood (5 mL) was collected from a healthy volunteer into one tube containing notecarin and one containing carinactivase-2. The tubes were centrifuged after 5 minutes. K$^+$, glucose, LD and phosphate levels were determined at 3 time intervals after serum preparation. The samples were stored at 23 °C for 5 hours then from 5 to 26.5 hours at 4°C. The level of haemoglobin was also measured.

The results are shown in Table 73.

Table 73: Stability of analytes in serum samples generated by Notecarin and Carinactivase-2

| Analyte | Zero time | 5 hrs | 26.5 hrs | Serum Hb mg/L |
|---|---|---|---|---|
| **Notecarin** | | | | **32** |
| K$^+$ | 4.0 | 4.1 | 4.0 | |
| Glucose | 5.4 | 5.3 | 5.1 | |
| LD | 176 | 175 | 173 | |
| Phosphate | 1.05 | 1.13 | 1.05 | |
| **Carinactivase 2** | | | | **46** |
| K$^+$ | 3.9 | 3.9 | 3.9 | |
| Glucose | 5.3 | 5.0 | 4.9 | |
| LD | 176 | 177 | 170 | |
| Phosphate | 1.03 | 1.04 | 1.06 | |

A haemoglobin level of <50 mg/L is considered clinically insignificant. These results therefore indicate that the serum samples produced by the prothrombin activators were effectively free of haemoglobin (therefore there is very little haemolysis during the clotting process), and free of cellular contamination. If cells or cell debris had been present, the concentration of K$^+$, LD and phosphate would be expected to increase and the concentration of glucose to decrease on storage. Samples of the sera were taken and analysed for cells using the Cytospin method. Very few cells were detected.

In summary, serum samples prepared by using a group B prothrombin activator (carinactivase-2) and a group D prothrombin activator (notecarin) were of high quality based on completeness of clotting, analytical results and absence of cellular contaminants.

EXAMPLE 14: MEASUREMENT OF BIOCHEMICAL ANALYTES IN PLASMA AND SERUM SAMPLES PREPARED IN COMMERCIAL TUBES AND SERUM SAMPLES PREPARED USING PROTHROMBIN ACTIVATOR-CONTAINING VENOMS

This Example uses the same analyte analysis methodology described in Examples 12 and 13.

The discoveries made in the preceding examples surprisingly demonstrated that snake venom prothrombin activators would be suitable for use as procoagulants in clinical environments. This then raised the question whether the crude venoms which contain these prothrombin activators could be used in blood clotting devices without prior purification of the prothrombin activators.

The following experiments were therefore designed and conducted to show whether the venoms could be used to rapidly produce high quality serum from human blood samples. The amount of venom used was approximately four times the amount of purified prothrombin activators used in the preceding experiments, reflecting the fact that the venoms contain other proteins than the prothrombin activators.

Example 14a: Use of crude *P. textilis* and *O. scutellatus* venoms as procoagulants in clotting tubes

Blood was collected from 2 healthy volunteers into 10 Greiner No Additive tubes (#454001, Griener Bio-One, Kremsmuster, Austraia) (4 mL capacity) containing 2 and 4 $\mu$g (in duplicate for the 4 $\mu$g *O. scutellatus* venom) of either *P. textilis* or *O. scutellatus* venom. Blood samples were also collected into Greiner standard serum tubes (#456071, Greiner Bio-One, Kremsmuster, Austraia) for comparison.

Clotting in the venom-containing tubes appeared complete by visual observation in 2 minutes. Samples in the venom-containing tubes were centrifuged after 15 minutes and those in the Greiner serum tubes after 30 minutes. Samples were centrifuged at 3000g, 10 min and 20°C (Hereaus 1 S-R centrifuge, Germany). The sera from the Greiner No Additive tubes were immediately transferred into Beckman plain plastic tubes (#448778, Beckman coulter, Brea, CA, USA) for observation and analysis. Latent clotting was observed in about half (6 out of the 10) of the venom-containing tubes but not in either of the Greiner serum tubes. This indicates that in some tubes, clotting was incomplete at the concentrations of venom used. Samples in the plain plastic tubes were re-centrifuged to remove cells, cell stroma and latent clots and the clear sera obtained analysed within 2 hours for 31 analytes. Sample analysis was performed within two hour post centrifugation for 31 analytes on the Beckman DxC800 general chemistry analyser and DxI800 immuno-analyser (Beckman Coulter, Brea, CA, USA). There were no clinically significant differences at the concentration levels tested between results obtained for the 12 sera (4 *P. textilis* sera and 6 *O. scutellatus* sera and the 2 Greiner sera).

The results are shown in Table 74.

Table 74: Analytical results for serum samples generated by *P. textilis* sera and *O. scutellatus* venom.

| No of Samples | Analyte | Units | Mean ± SD | | | | | | Mean difference between pairs (%) | | | | | LSC (%) | CAL (%) |
|---|---|---|---|---|---|---|---|---|---|---|---|---|---|---|---|
| | | | GS | Brown 2.0 μg | Brown 4.0 μg | Taipan 2.0 μg | Taipan 4.0 μg | Taipan 4.0 μg | Br2 - GS | BR4-GS | T2-GS | T4-GS | T4R-GS | | |
| 2 | Na$^+$ | mmol/L | 136.5±0.71 | 137.0±1.41 | 136.5±0.71 | 137.0±1.41 | 136±0.00 | 136.0±0.00 | -0.4 | 0.0 | 0.4 | -0.4 | -0.4 | 2.8 | 3 |
| 2 | K$^+$ | mmol/L | 4.05±0.21 | 4.05±0.07 | 4.0±0.00 | 4.20±0.28 | 4.00±0.00 | 4.25±0.35 | 0.0 | -1.2 | 3.7 | -1.2 | 4.9 | 3.3 | 5 |
| 2 | Cl$^-$ | mmol/L | 103.5±0.71 | 103.5±0.71 | 103.5±0.71 | 103.5±0.71 | 104.0±0.00 | 103.5±0.71 | 0.0 | 0.0 | 0.0 | 0.5 | 0.0 | 3.3 | 5 |
| 2 | HCO$_3^-$ | mmol/L | 26.0±0.0 | 24.5±0.7 | 24.0±0.0 | 24.5±0.7 | 23.5±0.7 | 24.0±0.0 | -5.8 | -7.7 | -5.8 | -9.6 | -7.7 | 4.8 | 10 |
| 2 | Gluc | mmol/L | 5.45±0.35 | 5.20±0.42 | 5.30±0.14 | 5.35±0.50 | 5.2±0.28 | 5.4±0.42 | -4.6 | -2.8 | -1.8 | **-4.6** | -0.9 | 4.4 | 10 |
| 2 | Urea | mmol/L | 7.45±2.76 | 7.30±2.83 | 7.25+2.90 | 7.30±2.97 | 7.3±2.97 | 7.35±2.90 | -2.0 | -2.7 | -2.0 | -1.3 | -2.0 | 5.0 | 10 |
| 2 | Creat | μmol/L | 106.0±2.8 | 101.5±12.0 | 104.0±7.1 | 103.5±6.4 | 107.0±0.00 | 105.0±8.5 | -4.2 | -1.9 | -2.4 | 0.9 | -0.9 | 5.5 | 10 |
| 2 | Urate | mmol/L | 0.31±0.13 | 0.30±0.14 | 0.30±0.14 | 0.30±0.14 | 0.30±0.14 | 0.30±0.14 | -3.2 | -3.2 | -3.2 | -3.2 | -3.2 | 3.8 | 10 |
| 2 | TP | g/L | 65.0±0.0 | 64.5±0.71 | 65.5±0.71 | 66.0±0.0 | 63.5±0.71 | 65.5±0.71 | -0.8 | 0.8 | 1.5 | -2.3 | 0.8 | 3.5 | 5 |
| 2 | Alb | g/L | 39.5±0.7 | 40.5±0.7 | 40.5±0.7 | 40.5±0.71 | 40.0±0.0 | 40.0±0.0 | 2.5 | 2.5 | 2.5 | 1.3 | 1.3 | 3.2 | 5 |
| 2 | T Bili | μmol/L | 13.5±5.0 | 11.5±5.0 | 11.0±4.2 | 11.0±5.7 | 12.5±5.0 | 12.5±5.0 | -14.8 | -18.5 | -18.5 | -7.4 | -7.4 | 7.0 | 20 |
| 2 | ALP | U/L | 66.5±17.7 | 65.5±20.5 | 69.0±21.2 | 67.5±21.9 | 66.0±21.2 | 68.0±19.8 | -1.5 | 3.88 | 1.5 | -0.8 | 2.3 | 4.8 | 10 |
| 2 | GGT | U/L | 14.5±6.4 | 15.0±2.8 | 15.0±7.1 | 16.5±5.0 | 15.5±9.2 | 14.0±7.1 | 3.4 | 3.4 | **13.8$^§$** | 6.9 | -3.4 | 7.3 | 10 |
| 2 | ALT | U/L | 3200±2.8 | 30.5±6.4 | 30.5±5.0 | 31.5±3.5 | 31.5±5.0 | 30.5±2.1 | -4.7 | -4.7 | -1.6 | -1.6 | -4.7 | 6.8 | 10 |
| 2 | AST | U/L | 23-5±0.7 | 26.5±0.7 | 30.0±0.0 | 295±2.1 | 29.5±2.1 | 29.0±0.0 | **12.8** | **27.7$^§$** | **25.5$^§$** | **25.5$^§$** | **23.4$^§$** | **6.2** | **15** |
| 2 | LD | U/L | 189.5±10.6 | 189.5±20.5 | 193.5±6.4 | 197.5±20.5 | 201.5±14.8 | 201.5±13.4 | 0.0 | 2.1 | **4.2** | **6.3** | **6.3** | **3.7** | 15 |
| 2 | CK | U/L | 152.0±83.4 | 154.5±84.0 | 145.5±90.0 | 145.5±89.8 | 147.5±92.6 | 145.5±94.0 | 1.6 | -4.3 | -4.3 | -3.0 | -4.3 | 3.7 | 15 |
| 2 | TCa | mmol/L | 2.17±0.06 | 2.18±0.09 | 2.17±0.09 | 2.18±0.06 | 2.15±0.08 | 2.17±0.09 | 0.5 | 0.0 | 0.2 | -1.2 | -0.2 | 3.5 | 5 |
| 2 | Pi | mmol/L | 1.22±0.04 | 1.22±0.04 | 1.21±0.07 | 1.21±0.06 | 1.21±0.04 | 1.20±0.04 | 0.0 | -0.8 | -1.2 | -1.2 | -2.0 | 3.8 | 10 |
| 2 | Mg$^{2+}$ | mmol/L | 0.915±0.049 | 0.935±0.05 | 0.935±0.049 | 0.930±0.057 | 0.950±0.057 | 0.945±0.049 | 2.2 | 2.2 | 1.6 | 3.8 | 3.3 | 4.4 | 10 |
| [0907] | Chol | mmol/L | 4.60±0.00 | 4.70±0.00 | 4.70±0.14 | 4.70±0.00 | 4.65±0.07 | 4.70±0.00 | 2.2 | 2.9 | 2.2 | 1.1 | 2.2 | 3.6 | 10 |
| 2 | Trig | mmol/L | 2.40±0.28 | 2.50±0.57 | 2.55±0.50 | 2.50±0.57 | 2.50±0.42 | 2.50±0.42 | 4.2 | **6.2** | 4.2 | 4.2 | 4.2 | **5.3** | 10 |
| 2 | HDL-C | mmol/L | 1.50±0.42 | 1.35±0.07 | 1.50±0.14 | 1.45±0.07 | 1.50±0.28 | 1.50±0.14 | -10.0 | 0.0 | -3.3 | 0.0 | 0.0 | 5.2 | 10 |
| 2 | Fe$^{2+}$ | μmol/L | 13.0±5.7 | 13.0±5.7 | 13.5±5.0 | 13.0±5.7 | 13.0±5.7 | 13.5±5.0 | 0.0 | 3.8 | 0.0 | 0.0 | 3.8 | 3.7 | 10 |

| No of Samples | Analyte | Units | Mean ± SD | | | | | | Mean difference between pairs (%) | | | | | LSC (%) | CAL (%) |
|---|---|---|---|---|---|---|---|---|---|---|---|---|---|---|---|
| | | | GS | Brown 2.0 μg | Brown 4.0 μg | Taipan 2.0 μg | Taipan 4.0 μg | Taipan 4.0 μg | Br2 -GS | BR4-GS | T2-GS | T4-GS | T4R-GS | | |
| 2 | Trf | g/L | 2.59±0.46 | 2.61±0.44 | 2.58±0.35 | 2.62±0.43 | 2.56±0.44 | 2.55±0.36 | 1.0 | -0.2 | 1.2 | -1.0 | -1.5 | 4.8 | 10 |
| 2 | Cortisol * | nmol/L | 236.0±27. 2 | ± | ± | ± | ± | ± | 3.6 | -1.7 | | | | 6.4 | 15 |
| 2 | FT4* | Pmol/L | 11.55±1.34 | 12.80±0.28 | 12.90±2.55 | 12.70±2.12 | 13.25±4.03 | 12.75±1.34 | 10.8 | 11.7 | 10.0 | 14.7 | 10.4 | 6.5 | 30 |
| 2 | TSH* | μIU/mL | 2.185±1.011 | 2.135±0.841 | 2.430±1.202 | 2.160±0.834 | 2.070±0.891 | 2.085±0.884 | -2.3 | **11.2** | -1.1 | -5.3 | -4.6 | 7.3 | 15 |
| 2 | Ferritin* | μg/L | 50.0±0.0 | 56.0±0.0 | 56.5±2.1 | 51.5±9.2 | 55±9.9 | 51.0±7.1 | **12.0** | **13.0** | 3.0 | **10.0** | 2.0 | 7.2 | 15 |
| 2 | TnI* | μg/L | 0.010=0.005 | 0.012±0.008 | 0.010±0.002 | 0.012±0.007 | 0.010±0.003 | 0.007±0002 | **26.3** | 0.0 | **26.3** | 5.3 | **-31.6** | **14** | 30 |
| 2 | Haem Index | | 0±0 | 0±0 | 0±0 | 0±0 | 0.5±0.7 | 0.5±0.7 | | | | | | | |
| § = results differed by more than the CAL. | | | | | | | | | | | | | | | |

Analysis was performed on Beckman DxC800 and *DxI800 analysers Br2 - Brown snake venom 2.0 $\mu$g; Br4 - Brown snake venom 4.0 $\mu$g; T2 - Taipan snake venom 2.0 $\mu$g; T4 and T4R - Taipan snake venom 4.0 $\mu$g.

In summary, we have shown that crude *P. textilis* and *O. scutellatus* venoms are potential procoagulants for producing serum in blood clotting tubes. The optimal concentrations need to be determined to avoid latent clotting and maintain analytical integrity.

Example 14b: Use of crude *E. carinatus* venom as procoagulant in clotting tubes

The following experiments were conducted to show whether *E. carinatus* venom could be used to rapidly produce high quality serum from human blood samples.

Blood was collected from 2 healthy volunteers into 10 Greiner No Additive tubes (#454001, Griener Bio-One, Kremsmuster, Austraia) (4 mL capacity) containing 4 $\mu$g of *E. carinatus* venom. At the same time blood was collected into Greiner standard serum tubes (# 456071, Greiner Bio-One, Kremsmuster, Austraia) for comparison.

Clotting in the venom-containing tubes appeared complete by visual observation in 3 minutes. Samples in the venom-containing tubes were centrifuged after 10 minutes and those in the Greiner serum tubes after 30 minutes. Samples were centrifuged at 3000g, 10 min and 20°C (Hereaus 1 S-R centrifuge, Germany). The sera from the Greiner No Additive tubes were immediately transferred into Beckman plain plastic tubes (#448778, Beckman coulter, Brea, CA, USA) for observation and analysis. No latent clotting was observed in the venom-containing tubes or in the Greiner serum tubes. Samples in the plain plastic tubes were re-centrifuged to remove any cells during the transfer of serum and the clear sera obtained analysed within 2 hours for 31 analytes. Sample analysis was performed within two hour post centrifugation for 31 analytes on the Beckman DxC800 general chemistry analyser and DxI800 immuno-analyser (Beckman Coulter, Brea, CA, USA). The results are shown in Table 75. There were clinically significant differences with AST and HDL and no other analyte at the concentration level tested between results obtained for the *E. carinatus* and the Greiner sera.

Table 75: Analytical results for serum samples generated by *Echis carinatus* snake venom.

| No of Samples | Analyte | Mean ± SD | | | Mean difference between pairs (%) | LSC (%) | CAL (%) |
|---|---|---|---|---|---|---|---|
| | | Units | GS | E.carinatus 4.0 $\mu$g | EC4-GS | | |
| 2 | Na$^+$ | mmol/L | 138.5±0.71 | 137.5±0.71 | -0.7 | 2.8 | 3 |
| 2 | K$^+$ | mmol/L | 4.0±0.14 | 4.0±0.14 | 0.0 | 3.3 | 5 |
| 2 | Cl$^-$ | mmol/L | 103±0.0 | 103.5±0.7 | -0.5 | 3.3 | 5 |
| 2 | HCO$_3^-$ | mmol/L | 27.4±0.92 | 26.1±0.14 | -4.6 | 4.8 | 10 |
| 2 | Gluc | mmol/L | 5.45+0.07 | 5.35±0.07 | -1.8 | 4.4 | 10 |
| 2 | Urea | mmol/L | 7.25±0.64 | 7.30±0.71 | 0.7 | 5.0 | 10 |
| 2 | Creat | $\mu$mol/L | 88.5±12.1 | 86.0±17.0 | -2.8 | 5.5 | 10 |
| 2 | Urate | mmol/L | 0.28±0.09 | 0.28±0.09 | 0 | 3.8 | 10 |
| 2 | TP | g/L | 63.5±0.7 | 63.5+0.7 | 0 | 3.5 | 5 |
| 2 | Alb | g/L | 39.5±0.7 | 40.5±0.7 | 2.5 | 3.2 | 5 |
| 2 | T Bili | $\mu$mol/L | 13.5±6.4 | 12.0±5.7 | -11.1 | 7.0 | 20 |
| 2 | ALP | U/L | 71.5±23.3 | 70.0±19.8 | -2.1 | 4.8 | 10 |
| 2 | GGT | U/L | 14.0±4.2 | 14.5±7.8 | 3.6 | 7.3 | 10 |
| 2 | ALT | U/L | 34.0±0.0 | 35.5±2.1 | 4.4 | 6.8 | 10 |
| 2 | AST | U/L | 20.5±2.1 | 27.0±00 | **31.7[§]** | **6.2** | **15** |
| 2 | LD | U/L | 185±5.7 | 193±11.3 | 4.3 | 3.7 | 15 |
| 2 | CK | U/L | 135±50.9 | 133.5±48.8 | -1.1 | 3.7 | 15 |
| 2 | TCa | mmol/L | 2.20±0.014 | 2.17±0.021 | -1.6 | 3.5 | 5 |

(continued)

| No of Samples | Analyte | Mean ± SD | | | Mean difference between pairs (%) | LSC (%) | CAL (%) |
|---|---|---|---|---|---|---|---|
| | | Units | GS | E.carinatus 4.0 $\mu$g | EC4-GS | | |
| 2 | Pi | mmol/L | 1.215±0.247 | 1.17±0.226 | -3.7 | 3.8 | 10 |
| 2 | $Mg^{2+}$ | mmol/L | 0.91±0.0 | 0.93±0.04 | 1.6 | 4.4 | 10 |
| 2 | Chol | mmol/L | 4.75±0.64 | 4.75±0.64 | 0.0 | 3.6 | 10 |
| 2 | Trig | mmol/L | 1.45±0.50 | 1.50±0.14 | 3.4 | **5.3** | 10 |
| 2 | HDL-C | mmol/L | 1.25±0.21 | 1.80±0.57 | **44.0[§]** | **5.2** | **10** |
| 2 | $Fe^{2+}$ | $\mu$mol/L | 13.5±5.0 | 13.5±5.0 | 0 | 3.7 | 10 |
| 2 | Trf | g/L | 2.62±0.49 | 2.60±0.44 | 1.3 | 4.8 | 10 |
| 2 | Cortisol* | nmol/L | 232+10.6 | 238±7.8 | 2.6 | 6.4 | 15 |
| 2 | FT4* | Pmol/L | 12.2±0.6 | 12.6±0.7 | 3.7 | 6.5 | 30 |
| 2 | TSH* | $\mu$IU/mL | 2.16±0.76 | 2.10±0.67 | -3.0 | 7.3 | 15 |
| 2 | Ferritin* | $\mu$g/L | 50±2.8 | 47±2.8 | -6.0 | 7.2 | 15 |
| 2 | TnI* | $\mu$g/L | 0.024±0.000 | 0.020±0.006 | **-18.8** | **14** | 30 |
| 2 | Haem Index | | 0±0 | 0±0 | | | |
| § = results were different by more than the CAL. | | | | | | | |

Analysis was performed on Beckman DxC800 and *DxI800 analysers EC4 - *Echis carinatus* venom 4.0 $\mu$g.

As these are preliminary results, further investigation is needed to establish why there were clinically significant differences for the AST and HDL results.

In summary, we have shown that use of *crude E carinatus* venom as procoagulant in blood clotting tubes may be feasible. The optimal concentration and further studies on analyte interference need to be accurately determined to avoid latent clotting and maintain analytical integrity.

EXAMPLE 15: STABILITY STUDIES

For a venom prothrombin activator to be useful as a component of blood collection devices, it must meet three types of stability requirement:

(1) Stability during bulk storage: It needs to be stable for the period after purification before use in manufacture of the tubes. For certainty of supply, this storage period may be for several months. Storage could be as a concentrated aqueous solution or as lyophilized solid. Storage could be under refrigeration if required.

(2) Stability during manufacture: It needs to be stable during the manufacture of the blood collection device. This process is likely to involve addition of an aliquot of a stock solution of the prothrombin activator to tubes which already contain other components such as the separating gel, the surfactant and possible a particulate procoagulant. This would be followed by drying to give a surface layer of prothrombin activator, sealing the tubes under vacuum and sterilization by radiation.

(3) Stability during storage of the device prior to its use: It would be necessary for the activator to retain close to full activity for a period of at least 12 months at room temperature, say 23 C, preferably for a longer period at a higher temperature.

Experiments performed to date suggest that conditions will be found to meet these requirements for at least some of the venom prothrombin activators.

Example 15a: Stability during-bulk storage

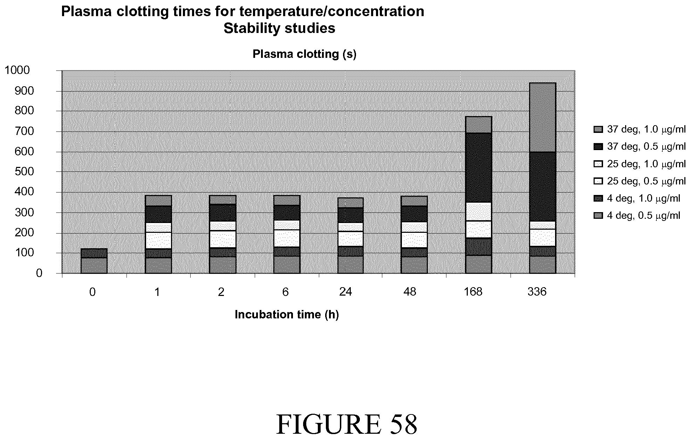

The stability of PtPA when maintained at a range of temperatures as a dilute solution in phosphate buffered saline at pH 7 in plain plastic Eppendorf tubes was determined. Results of plasma clotting assays are shown in Figure 58. The results show that there was no significant loss of clotting activity after two weeks (336 hours) at 4 or 25 °C. Similar results were obtained when the activity of PtPA was measured against the Factor Xa chromogenic substrate S-2222. At 37 °C PtPA lost a significant amount of its activity after 168 hours.

In related experiments, it was demonstrated that pH 7.4 was the optimal temperature for storage and that the addition of calcium ions to the storage buffer stabilized the plasma clotting activity.

These experiments indicate that it should be possible to find conditions for the bulk storage of PtPA and the closely related OsPA so that they retain all or almost all of their activity on storage for extended periods.

Example 15b: Stability during manufacture

An experiment was performed to determine the effect of gamma radiation on OsPA added to blood collection tubes as a dilute solution.

Stock OsPA (1.089 mg/mL; 4.36 mM) was diluted 1 in 25 with 0.02 M Hepes buffer pH 7.4. 50 $\mu$L was added to each of 5 plain blood collection tubes with no other additives were gamma irradiated (16 megaBeq) and to 5 similar tubes which were not irradiated. Similarly, 50 $\mu$L of diluted OsPA was added to each of 10 Greiner silica-containing blood collection tubes (Greiner serum tubes) and 5 of these tubes were irradiated.

After gamma irradiation, all tubes were made up to 2.0 mL with 0.02 M Hepes buffer pH 7.4. Aliquots were then taken and tested for hydrolytic activity against S-2765 and clotting activity using normal citrated plasma with added 10 mM calcium.

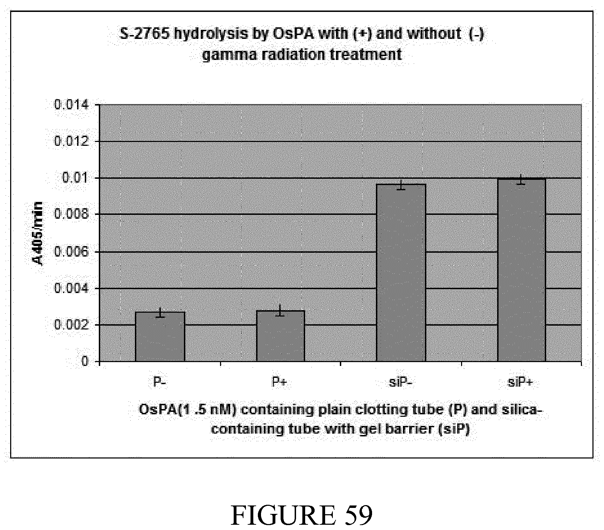

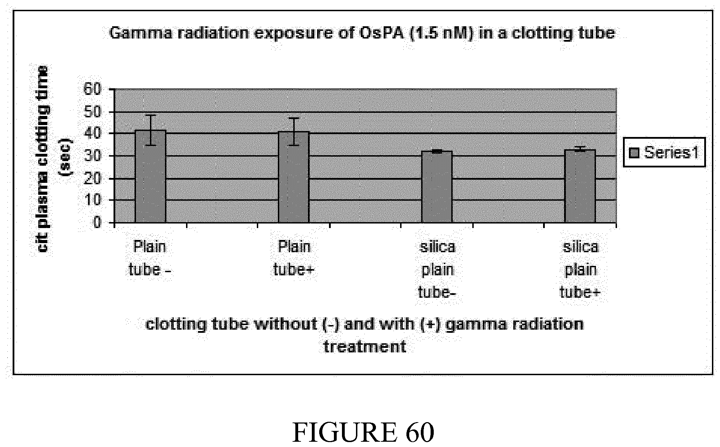

The results are shown in Figures 59 and 60, and show that irradiation had no effect on either clotting activity against recalcified citrated plasma or hydrolytic activity against the chromogenic substrate S-2765.

The addition of OsPA (1.5 nM) to plain blood collection tube showed a significant loss of activity as compared to the freshly diluted OsPA. This can be explained by hydrophobic binding of the OsPA to the plain plastic tube. Adding OsPA to silica-containing blood collection tubes showed recovery of greater than 95% the hydrolytic and clotting activities. Again, no loss activity was observed with gamma irradiation treatment.

In conclusion, gamma irradiation of OsPA in a blood collection tube did not affect either hydrolytic or clotting activity. Adding OsPA to a clotting tube containing silica, gel barrier, and surfactant is the most effective combination determined to date to clot normal blood in less than 1 minute.

Example 15c: Stability during storage of the device.

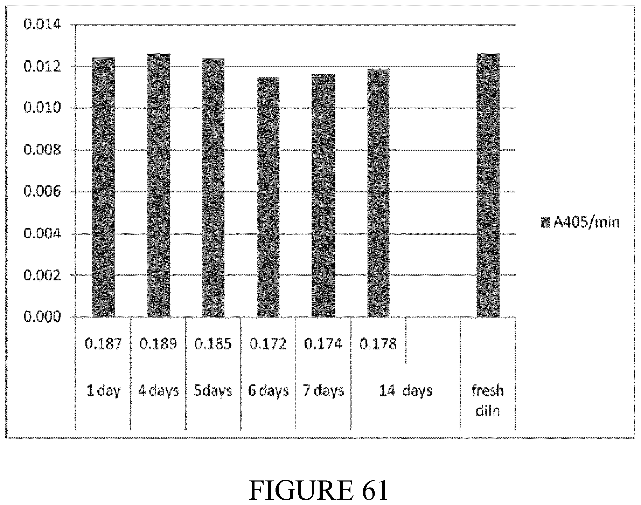

Aliquots (50 $\mu$L) of a diluted solution of OsPA in 0.02 M Hepes buffer, pH 7.4 (1.5 nM) were placed in Greiner standard serum tubes. Each tube was rotated to coat the surface with the OsPA and the tubes were placed in a vacuum dessicator and purged by a stream of dry nitrogen before applying a low vacuum for 3 days at 23 °C to dry. The dessicator was opened, flushed with nitrogen and the tubes re-sealed. The tubes were then maintained at room temperature (23 °C). After specified times, three tubes were opened, the contents redissolved in 2 mL water and assayed against the Factor Xa specific substrate S-2222. The results are shown in Figure 61, and show that the activity (expressed as increase in A405/min) of OsPA towards S-2222 was effectively unchanged by storage at 23 °C as a dry surface layer in the Greiner serum tubes for 14 days. All assays were done in triplicate. In Figure 61, the right hand bar is the activity of a freshly diluted sample of OsPA stock solution (4.05 uM) and its activity was the same as that found in the tubes, showing that the process used to prepare the tubes did not cause any significant loss of OsPA activity.

EXAMPLE 16: PREPARATION OF BLOOD COLLECTION TUBE

All commercial serum and plasma tubes are now plastic and contain a number of components which have been developed by the suppliers to enhance the quality of the samples prepared in them. As discussed above, some container (*e.g.* tube) for preparation of a serum sample or a plasma sample contains:

(1) a gel barrier which aims to separate cells (and clot in the case of serum) from the supernatant (serum or plasma) after centrifugation and to limit re-mixing. The gel is added as a warm liquid which solidifies on cooling to room temperature;

(2) a surfactant sprayed on the inner surface of the plastic tube above the gel barrier to decrease attachment of cells, debris and clot material and cell lysis; and

(3) a procoagulant/anticoagulant, usually particulate, sprayed as a suspension onto the inner surface of the tube on top of the surfactant.

The present invention envisages the a serum tube (*e.g.*, a plastic tube) comprising components (1), (2), and (3) as described above, where the tube is prepared by adding components (1) and (2) to the tube first, and subsequently adding component (3) to the tube. Component (3) comprises a procoagulant comprising a clotting composition comprising, consisting essentially of, or consisting of a prothrombin activator as herein defined, for example, component (3) may comprise existing particulate procoagulants used in some commercial serum tubes. The amount of prothrombin activator within each tube can be determined using the experimental results described in detail above, and routine techniques known in the art. The tubes are then evacuated and sterile capped. The tubes are then stored at room temperature.

Also envisaged a serum tube (*e.g.*, a plastic tube) comprising component (3) and optionally comprising components (1) and/or (2). The amount of prothrombin activator within each tube can be determined using the experimental results described in detail above, and routine techniques known in the art. The tubes are evacuated and sterile capped and can be stored at room temperature.

EXAMPLE 17: POINT OF CARE EMBODIMENT

As discussed above, "point-of-care" testing means that the testing is performed at or near the site of patient care. Point of care testing is becoming increasingly popular in hospital and other environments where there is a need for fast results. It is accomplished using a variety of devices some of which are relatively inexpensive, small, and portable.

In current practice, many point-of-care devices work as follows: a droplet of blood is placed on a membrane which retains blood cells but allows plasma to diffuse through the membrane into a microfluidic device with multiple channels in which a number of analyses are performed. As discussed elsewhere, serum is preferred to plasma as the sample for biochemical or other pathology assays. In this embodiment, the present invention contemplates the use of clotting compositions comprising, consisting essentially of, or consisting of a prothrombin activator to produce serum in devices designed for point of care testing.

In one such device, suitable for hospital and doctors' surgery use, a blood sample obtained by venipuncture is collected into a syringe (or similar) containing a clotting composition comprising, consisting essentially of, or consisting of a prothrombin activator, where composition is achieved in ~30 seconds. The device is designed to allow either filtration or centrifugation to rapidly separate the serum from the clot. The serum so obtained is used in existing microfluidic devices for biochemical or other pathology assays, with the range of analyses increased as the sample being analysed is serum rather than plasma that is used in existing point-of-care devices. Furthermore, a sample of the serum may be retained to allow further analysis in the pathology laboratory.

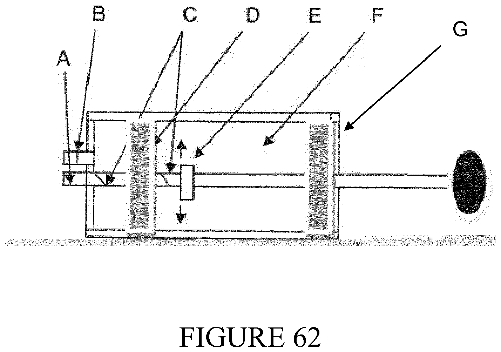

The filtration device in the syringe described above may comprise a filtration mechanism that allows the serum to be pushed through after 5 minutes for analysis. Suitably, the syringe may be divided in compartments as illustrated in Figure 62, with the syringe labelled as follows:

A - entry port that will have blood collection needle adapter that would unscrew to minimising prodruding component;

B - port for dispensing serum or entry point for point of care sample needles;

C - valves to allow for one way entry of blood and prevent blood being pushed into serum compartment;

D - plunger filter that would allow for serum to be pushed out into the serum compartment (front part of the syringe);

E - opening for blood to enter blood compartment.

F - compartment ion which clotting occurs.

In Figure 62, G is coated with the clotting composition on the inside to set the clotting process in place. It is then pushed to force the serum across the filter (D).

In another such device, the point-of-care device involves a capillary system, where part of the device is coated with a clotting composition comprising, consisting essentially of, or consisting of a prothrombin activator and an absorbant that can draw the serum into the reaction chamber for analysis.

In another such device, a clotting composition comprising, consisting essentially of, or consisting of a prothrombin activator could be coupled to the membrane of a microfluidic device so as to achieve rapid clotting of a blood droplet placed on the membrane. The serum so formed diffuses into the device and is analysed in place of the plasma currently analysed in such devices.

## SUMMARY OF EXAMPLES

Obtaining a suitable sample for analysis in a timely manner is pivotal in clinical chemistry service provision. Serum is recognised as being the cleaner sample but because of time required for completion of clotting (30 minutes to ensure complete clotting in most currently available commercial tubes plus analysis time in the standard protocols) and an ever-increasing number of patients on anti-coagulant therapy, plasma is commonly used, especially in hospital environments. However, latent clotting (clotting after centrifugation) can occur in plasma samples leading to analytical errors and delays in delivery of critical results. Some of these problems are illustrated in the above examples.

The results in the above examples also show that use of prothrombin activators can produce high quality serum very quickly from blood samples from a wide variety of patients, including patients being treated with anticoagulants. They provide suitable alternatives to lithium heparin plasma and to conventional serum tubes by:

- having the clotting process completed in ~< 5 minutes (time taken to deliver samples to the laboratory) allowing rapid analysis after blood sample collection and hence rapid turn around times comparable to the times for plasma tubes and better than those for most current serum tubes;

- at the same time providing high quality serum samples for blood from both healthy and all anticoagulated individuals that may be encountered in a clinical environment with minimal analyte interference from cellular debris and/or latent microclotting; and

- producing a single serum sample that is suitable for a very wide range of assays typically used in clinical environment, the serum sample being superior to plasma for analyte analysis, and the single sample reducing the burden on scarce resources (blood in critically ill patients, staff time and consumables by needing to collect fewer blood tubes).

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## BIBLIOGRAPHY

Altschul, S. F., Madden, T. L., Schäffer, A. A., Zhang, J., Zhang, Z., Miller, W., Lipman, D. J., "Gapped BLAST and PSI-BLAST: a new generation of protein database search programs." Nucleic Acids Res. 1997 Sep 1; 25(17): 3389-402.

Arkin, A. P., Youvan, D. C., "An algorithm for protein engineering: simulations of recursive ensemble mutagenesis" 1992 Proc. Natl. Acad. Sci. USA 89: 7811-7815.

Atherton, E., Shephard, R. C., Solid Phase Peptide Synthesis - A Practical Approach 1989, IRL Press, Oxford England.

Austen, D. E., Rhymes, I. L., "Laboratory manual of blood coagulation" (1975) Blackwell Scientific Publications, London, p37.

Bos, M. H. A., Boltz, M., St Pierre, L., Masci, P. P., de Jersey, J., Lavin, M. F., Camire, R. M., "Venom factor V from the common brown snake escapes hemostatic regulation through procoagulant adaptations" Blood 16 July 2009; 114(3): 686-692.

Camenzind, E., Bakker, W. H., Reijs, A., van Geijlswijk, I. M., Boersma, E., Kutryk, M. J., Krenning, E. P., Roelandt, J. R., Serruys, P. W., "Site-specific intracoronary heparin delivery in humans after balloon angioplasty. A radioisotopic assessment of regional pharmacokinetics." Circulation 1997; 96: 154-65.

Ciuti, R., Rinaldi, G., "Serum and plasma compared for use in 19 common chemical tests performed in the Hitachi 737 analyzer." Clin Chem. 1989; 35: 1562-3.

Cowley, D. M., Nagle, B. A., Chalmers, A. H., Sinton, T. J., "Effects of platelets on collection of specimens for assay of ammonia in plasma." Clin Chem 1985; 31: 332.

Davidson, A. S., Dam, S. M., Sodi, R., "Can lithium heparin plasma be used for protein electrophoresis and paraprotein identification?" Ann Clin Biochem 2006; 43: 31-34.

Dayhoff, M. O., Schwartz, R. M., Orcutt, B. C., et al., "A model of evolutionary change in proteins. Matrices for determining distance relationships" 1978 In Atlas of protein sequence and structure (Dayhoff, M. O. ed.), vol5, pp345-358, National Biomedical Research Foundation, Washington DC.

Delagrave, S., Goldman, E. R., Youvan, D. C., "Recursive ensemble mutagenesis" Apr 1993 Protein Eng. 6(3): 327-31.

Devereux, J., Haeberli, P., Smithies, O., "A comprehensive set of sequence analysis programs for the VAX." Nucleic Acids Res. 1984 Jan 11; 12(1 Pt 1): 387-95.

Dimeski, G., Masci, P.P., Trabi, M., Lavin, M.F., de Jersey, J., "Evaluation of the Becton-Dickinson rapid serum tube: does it provide a suitable alternative to lithium heparin plasma tubes?" Clin Chem Lab Med 2010; 48(5): 2111-2120.

Dimeski, G., Hadrick, T., Flatman, R., Ormiston, B., "Roche IFCC Methods for Lactate Dehydrogenase Tested for Duplicate Errors with Greiner and Becton-Dickinson Lithium-Heparin and Greiner Serum Samples" Clin Chem 2004; 50(12): 2391-2392.

Dimeski, G., Carter, A., "Rare IgM Interference with Roche/Hitachi Modular Glucose and γ-Glutamyltransferase Methods in Heparin Samples" Clin Chem 2005; 51(11): 2202-2204.

Dimeski, G., Clague, A. E., Hickman, P. E., "Correcting and reporting of potassium results in haemolysed samples" Ann. Clin. Biochem. 2005; 42: 119-123.

Dimeski, G., McWhinney, B., Jones, B., Mason, R., Carter, A., "Extent of bilirubin interference in Beckman-Coulter creatinine methods." Ann Clin Biochem 2008; 45:91-92.

Filippovich, I., Sorokina, N., St Pierre, L., Filght, S., de Jersey, J., Perry, N., Masci, P. P., Lavin, M. F. "Cloning and functional expression of venom prothrombin activator protease from Pseudonaja textilis with whole blood procoagulant activity" British Journal of Haematology 2005; 131: 237-246.

Gonnet, G. H., Cohen, M. A., Benner, S. A., "Exhaustive matching of the entire protein sequence database" Jun 5 1992 Science 256(5062): 1443-5.

Hartland, A. J., Neary, R. H., "Serum potassium is unreliable as an estimate of in vivo plasma potassium" Clin Chem 1999; 45:1091-1092.

Kini, R. M., Morita, T., Rosing, J., "Classification and Nomenclature of Prothrombin Activators Isolated from Snake Venoms" Thromb. Haemost. 2001 85:710-711.

Kini, R. M., "The intriguing world of prothrombin activators from snake venom" Toxicon (2005) 45: 1133-1145.

Kunkel, T. A., "Rapid and Efficient site-specific mutagenesis without phenotypic selection" 1985 Proc. Natl. Acad. Sci. USA, 82: 488-492.

Kunkel, T. A., Roberts, J. D., Zakour, R. A., "Rapid and Efficient site-specific mutagenesis without phenotypic selection" 1987 Methods in Enzymol. 154: 367-382.

Kuzmic, P., "Program DYNAFIT for the Analysis of Enzyme Kinetic Data: Application to HIV Proteinase" 1996 Anal. Biochem. 237: 260-273.

Lavin, M. F., Masci, P. P., "Prothrombinase complexes with different physiological roles" Thromb Haemost 2009; 102: 421-423.

Masci, P. P., "The Effects of Australian Snake Venoms on Coagulation and Fibrinolysis" Thesis for Masters of Science in the subject of Biochemistry, July 1986, University of Queensland, St Lucia, Brisbane, Australia.

Masci, P. P., Whitaker, A. N., de Jersey, J., "Purification and characterization of a prothrombin activator from the venom of the Australian brown snake, Pseudonaja textilis textilis" Biochem. Int. 1988; 17(5):825-835.

Miles, R. R., Roberts, R. F., Putnam, A. R., Roberts, W. L., "Comparison of serum and heparinized plasma samples for measurement of chemistry analytes." Clin Chem 2004; 50: 1704-5.

National Pathology Laboratory Accreditation Advisory Council - Requirements for the Retention of Laboratory Records and Diagnostic Material (Fifth Edition 2009), available online at: http://www.health.gov.au/internet/main/publishing.nsf.Content/0392138A9970DFD1CA2573 71000D9200/$File/NPAAC%20Pub%20-%20Ret%20Records%20040110.pdf

Nishida, S., Fujita, T., Kohno, N., Atoda, H., Morita, T., Takeya, H., Kido, I., Paine, M. J. I., Kawabata, S-i., Iwanaga, S. "cDNA Cloning and Deduced Amino Acid Sequence of Prothrombin Activator (Ecarin) from Kenyan Echis carinatus venom." Biochemistry 1995; 34: 1771-1778.

Morita, T., Iwanaga, S., "Prothrombin activator from Echis carinatus venom" Meth Enzymol 1981; 80-pt. C: 303-311.

Nicholson, J., Mirtschin, P., Madaras, F., Venning, M., Kokkinn, M., "Digestive properties of the venom of the Australian Coastal Taipan, Oxyuranus scultellatus (Peters, 1867)" Toxicon 2006: 48: 422-428.

O'Keane M. P., Cunningham, S. K., "Evaluation of three different specimen types (serum, plasma lithium heparin and serum gel separator) for analysis of certain analytes: clinical significance of differences in results and efficiency in use." Clin Chem Lab Med. 2006; 44: 662-8.

Parker, H. W., Grandison, A. G. C., "Snakes: A natural history" University of Queensland Press, St. Lucia, Queensland 1977: pp5, 59 and 87.

Roberge, J. Y., Beebe, X., Danishefsky, S. J., "A strategy for a convergent synthesis of N-linked glycopeptides on a solid support" Science 1995 269(5221); 202-204.

Rosing, J., Tans, G., "Inventory of Exogenous Prothrombin Activators" Thrombosis and Haemostasis (Stuttgart) 1991 65(2): 627-630.

Rosing, J., Tans, G., "structural and Functional Properties of Snake Venom Prothrombin Activators" Toxicon 30(12): 1515-1527, 1992.

Sambrook, J., Russell, D. W., Molecular cloning. A laboratory manual (third edition) 2001; Cold Spring Harbor Laboratory Press.

Schieck, A., Komalik, F., Habermann, E. "The prothrombin-activating principle from Echis carinatus venom. I. Preparation and biochemical properties" Naunyn-Schmiedeberg's Arch Pharmacol. 1972; 272: 402-416.

Sheppard, C.A:, Allen, R.C., Austin, G.E., Young, A.N., Ribeiro, M.A., Fantz, C.R., "Paraprotein interference in automated chemistry analyzers." Clin Chem 2005 Jun;51(6): 1077-8.

Smith, G. F., Craft, T. J., "Heparin reacts stoichiometrically with thrombin during thrombin inhibition in human plasma." Biochem. Biophys. Res. Commun. 1976; 71: 738-45.

Sonder, S. A., Fenton, J. W., "Thrombin specificity with tripeptide chromogenic substrates: comparison of human and bovine thrombins with and without fibrinogen clotting activities" Clin. Chem. 1986;32:934-7.

Speijer, H., Goversriemslag, J. W. P., Zwaal, R. F. A., Rosing, J., "Prothrombin activation by an activator from the venom of Oxyuranus scutellatus (Taipan snake)" J. Biol. Chem. 1986; 261(28): 3258-3267.

Starr, H., Rhodes, P., Lam-Po-Tang, P. R., Archer, G. T., "Prothrombin times: an evaluation of four thromboplastins and four machines." Pathology 1980; 12: 567-574.

St Pierre, L., Masci, P., Filippovich, I., Sorokina, N., Marsh, N., Miller, D. J., Lavin, M. F., "Comparative Analysis of Prothrombin Activators from the Venom of Australian Elapids" Mol. Biol. Evol. 2005; 22(9): 1853-1864.

Tans, G., Govers-Riemslag, J. W., van Rijn, J. L., Rosing, J. J., "Purification and properties of a prothrombin activator from the venom of Notechis scutatus scutatus" Biol. Chem. 1985 Aug 5; 260(16): 9366-72.

Thorelli, E., Kaufman, R. J., Dahlbäck, B., "Cleavage requirements of factor V in tissue-factor induced thrombin generation." Thromb. Haemost. 1998 Jul; 80(1) 92-98.

Wannaslip, N., Sribhen, K., Pussara, N., Hwanpuch, T., Wangchaijaroekit, S., Opartkiattikul, N., "Heparin is unsuitable anticoagulant for the detection of plasma ammonia." Clin Chimica Acta 2006; 371: 196-7.

Watson, J. D., Hopkins, N. H., Roberts, J. W., Steitz, J. A., Weiner, A. M. Molecular Biology of the Gene. fourth edition, 1987 The Benjamin/Cummings Publishing Co., Inc., Menlo Park, California.

Yamada, D., Sekiya, F., Morita, T., "Isolation and Characterization of Carinactivase, a Novel Prothrombin Activator in Echis carinatus Venom with a Unique Catalytic Mechanism" J. Biol. Chem. 1996; 271(9): 5200-5207.

Yamada, D., Morita, T., "Purification and Characterization of a Ca2+-Dependent Prothrombin Activator, Multactivase, from the Venom of Echis multisquamatus" J. Biochem. 1997; 122: 991-997.

Yamanouye, N., Kerchove, C. M., Moura-da-Silva, A. M., Carneiro, S. M., Markus, R. P., "Long-term primary culture of secretory cells of Bothrops jararaca gland for venom production in vitro" Nature Protocols 2007; 1: 2763-2766.

Yonemura, H., Imamura, T., Soejima, K., Nakahara, Y., Morikawa, W., Ushio, Y., Kamachi, Y., Nakatake, H., Sugawara, K., Nakagaki, T., Nozaki, C., "Preparation of Recombinant $\alpha$-Thrombin: High-Level Expression of Recombinant Human Prethrombin-2 and Its Activation by Recombinant Ecarin" J. Biochem. 2004; 135: 577-582.

Zubay, G., Biochemistry, third edition Wm.C. Brown Publishers, Oxford (1993).

## Claims

1. Use of a clotting composition in the preparation of a serum sample for analyte detection, wherein the clotting composition consists essentially of purified prothrombin activator or comprises prothrombin activator purified or partially purified from snake venom.

2. A container for preparing a serum sample that is suitable for detecting an analyte of interest that is present in the serum sample, wherein the container contains a clotting composition consisting essentially of purified prothrombin activator or comprising prothrombin activator purified or partially purified from snake venom, and wherein the container is evacuated.

3. Use of a clotting composition consisting essentially of a purified prothrombin activator or comprising prothrombin activator purified or partially purified from snake venom in the manufacture of a container for preparing a serum sample for analyte detection.

4. A method of analysing a serum sample for the presence, absence or amount of an analyte of interest, the method comprising: contacting a blood sample with a clotting composition consisting essentially of a purified prothrombin activator or comprising prothrombin activator purified or partially purified from snake venom; and analysing the serum sample for the presence, absence or amount of the analyte of interest.

5.   A method of diagnosing the presence, absence or severity of a disease or condition in a subject, wherein the presence, absence or severity of the disease or condition is associated with the presence, absence or an aberrant amount of an analyte of interest in the subject, the method comprising analysing a serum sample in accordance with the method of claim 4 to thereby determine the presence, absence or severity of the disease or condition in the subject.

6.   A use according to claim 1 or claim 3, container according to claim 2, or method according to claim 4 or claim 5, wherein the prothrombin activator is a group A prothrombin activator.

7.   A use, container, or method according to claim 6, wherein the prothrombin activator is selected from ecarin and basparin.

8.   A use according to claim 1 or claim 3, container according to claim 2, or method according to claim 4 or claim 5, wherein the prothrombin activator is a group B prothrombin activator.

9.   A use, container, or method according to claim 8, wherein the prothrombin activator is selected from carinactivase-1, carinactivase-2 and multactivase.

10.   A use according to claim 1 or claim 3, container according to claim 2, or method according to claim 4 or claim 5, wherein the prothrombin activator is a group C prothrombin activator.

11.   A use, container, or method according to claim 10, wherein the prothrombin activator is selected from pseutarin C, oscutarin C and Omicarin C.

12.   A use according to claim 1 or claim 3, container according to claim 2, or method according to claim 4 or claim 5, wherein the prothrombin activator is a group D prothrombin activator.

13.   A use, container, or method according to claim 12, wherein the prothrombin activator is selected from porpharin D notecarin D, trocarin D, hopsarin D and notenarin D.


**Patentansprüche**

1.   Verwendung einer Gerinnungszusammensetzung in der Herstellung einer Serumprobe für einen Analytennachweis, wobei die Gerinnungszusammensetzung im Wesentlichen aus gereinigtem Prothrombin-Aktivator besteht oder Prothrombin-Aktivator umfasst, der aus Schlangengift aufgereinigt oder teilweise aufgereinigt ist.

2.   Behälter zur Herstellung einer Serumprobe, die zum Nachweisen eines Analyten von Interesse geeignet ist, wobei der Behälter eine Gerinnungszusammensetzung bestehend aus gereinigtem Prothrombin-Aktivator oder umfassend Prothrombin-Aktivator umfasst, der aus Schlangengift aufgereinigt oder teilweise aufgereinigt ist, und wobei der Behälter evakuiert ist.

3.   Verwendung einer Gerinnungszusammensetzung bestehend im wesentlichen aus einem gereinigten Prothrombin-Aktivator oder umfassend Prothrombin-Aktivator, der aus Schlangengift aufgereinigt oder teilweise aufgereinigt ist, in der Herstellung eines Behälters zum Herstellen einer Serumprobe für einen Analytennachweis.

4.   Verfahren zum Analysieren einer Serumprobe hinsichtlich der Anwesenheit, Abwesenheit oder Menge eines Analyten von Interesse, wobei das Verfahren umfasst: Kontaktieren einer Blutprobe mit einer Gerinnungszusammensetzung bestehend im Wesentlichen aus einem gereinigten Prothrombin-Aktivator oder umfassend Prothrombin-Aktivator, der aus Schlangengift aufgereinigt oder teilweise aufgereinigt ist; und Analysieren der Serumprobe hinsichtlich der Anwesenheit, Abwesenheit oder Menge des Analyten von Interesse.

5.   Verfahren zum Diagnostizieren der Anwesenheit, Abwesenheit oder Schwere einer Erkrankung oder eines Zustands in einem Subjekt, wobei die Anwesenheit, Abwesenheit oder Schwere der Erkrankung oder des Zustands mit der Anwesenheit, Abwesenheit oder einer abweichenden Menge eines Analyten von Interesse in dem Subjekt assoziiert ist, wobei das Verfahren ein Analysieren einer Serumprobe gemäß dem Verfahren nach Anspruch 4 umfasst, um somit die Anwesenheit, Abwesenheit oder Schwere der Erkrankung des Zustands in dem Subjekt zu bestimmen.

6. Verwendung nach Anspruch 1 oder Anspruch 3, Behälter nach Anspruch 2 oder Verfahren nach Anspruch 4 oder 5, wobei der Prothrombin-Aktivator einer Gruppe A Prothrombin-Aktivator ist.

7. Verwendung, Behälter oder Verfahren nach Anspruch 6, wobei der Prothrombin-Aktivator aus Ecarin und Basparin ausgewählt ist.

8. Verfahren nach Anspruch 1 oder Anspruch 3, Behälter nach Anspruch 2 oder Verfahren nach Anspruch 4 oder 5, wobei der Prothrombin-Aktivator ein Gruppe B Prothrombin-Aktivator ist.

9. Verwendung, Behälter oder Verwendung nach Anspruch 8, wobei der Prothrombin-Aktivator aus Carinaktivase-1, Carinactivase-2 und Multiaktivase ausgewählt ist.

10. Verwendung nach Anspruch 1 oder Anspruch 3, Behälter nach Anspruch 2 oder Verfahren nach Anspruch 4 oder 5, wobei der Prothrombin-Aktivator ein Gruppe C Prothrombin-Aktivator ist.

11. Verwendung, Behälter oder Verfahren nach Anspruch 10, wobei der Prothrombin-Aktivator aus Pseutarin C, Oscutarin C und Omicarin C ausgewählt ist.

12. Verwendung nach Anspruch 1 oder Anspruch 3, Behälter nach Anspruch 2 oder Verfahren nach Anspruch 4 oder 5, wobei der Prothrombin-Aktivator ein Gruppe D Prothrombin-Aktivator ist.

13. Verwendung, Behälter oder Verfahren nach Anspruch 12, wobei der Prothrombin-Aktivator aus Porpharin D, Notecarin D, Trocarin D, Hopsarin D und Notenarium D ausgewählt ist.

## Revendications

1. Utilisation d'une composition de coagulation dans la préparation d'un échantillon de sérum pour la détection d'analytes, dans laquelle la composition de coagulation est constituée sensiblement d'un activateur de prothrombine purifié ou comprend un activateur de prothrombine purifié ou partiellement purifié à partir de venin de serpent.

2. Récipient pour préparer un échantillon de sérum qui convient à la détection d'un analyte d'intérêt qui est présent dans l'échantillon de sérum, dans lequel le récipient contient une composition de coagulation constituée sensiblement d'un activateur de prothrombine purifié ou comprenant un activateur de prothrombine purifié ou partiellement purifié à partir de venin de serpent, et dans lequel le récipient est placé sous vide.

3. Utilisation d'une composition de coagulation constituée sensiblement d'un activateur de prothrombine purifié ou comprenant un activateur de prothrombine purifié ou partiellement purifié à partir de venin de serpent dans la fabrication d'un récipient de préparation d'un échantillon de sérum pour la détection d'analytes.

4. Procédé d'analyse d'un échantillon de sérum pour la présence, l'absence ou une quantité d'un analyte d'intérêt, le procédé comprenant la mise en contact d'un échantillon de sang avec une composition de coagulation constituée sensiblement d'un activateur de prothrombine purifié ou comprenant un activateur de prothrombine purifié ou partiellement purifié à partir de venin de serpent, et l'analyse de l'échantillon de sérum pour la présence, l'absence ou une quantité de l'analyte d'intérêt.

5. Procédé de diagnostic de la présence, de l'absence ou de la gravité d'une maladie ou d'un état chez un sujet, dans lequel la présence, l'absence ou la gravité de la maladie ou de l'état est associée à la présence, l'absence ou une quantité aberrante d'un analyte d'intérêt chez le sujet, le procédé comprenant l'analyse d'un échantillon de sérum selon le procédé de la revendication 4 pour déterminer ainsi la présence, l'absence ou la gravité de la maladie ou de l'état chez le sujet.

6. Utilisation selon la revendication 1 ou la revendication 3, récipient selon la revendication 2 ou procédé selon la revendication 4 ou la revendication 5, dans laquelle l'activateur de prothrombine est un activateur de prothrombine du groupe A.

7. Utilisation, récipient ou procédé selon la revendication 6, dans lesquels l'activateur de prothrombine est choisi parmi l'écarine ou la basparine.

8. Utilisation selon la revendication 1 ou la revendication 3, récipient selon la revendication 2 ou procédé selon la revendication 4 ou la revendication 5, dans lesquels l'activateur de prothrombine est un activateur de prothrombine du groupe B.

9. Utilisation, récipient ou procédé selon la revendication 8, dans lesquels l'activateur de prothrombine est choisi parmi la carinactivase-1, la carinactivase-2 et la multactivase.

10. Utilisation selon la revendication 1 ou la revendication 3, récipient selon la revendication 2 ou procédé selon la revendication 4 ou la revendication 5, dans lesquels l'activateur de prothrombine est un activateur de prothrombine du groupe C.

11. Utilisation, récipient ou procédé selon la revendication dans lesquels l'activateur de prothrombine est choisi parmi la pseutarine C, l'oscutarine C et l'omicarine C.

12. Utilisation selon la revendication 1 ou la revendication 3, récipient selon la revendication 2 ou procédé selon la revendication 4 ou la revendication 5, dans lesquels l'activateur de prothrombine est un activateur de prothrombine du groupe D.

13. Utilisation, récipient ou procédé selon la revendication 12, dans lesquels l'activateur de prothrombine est choisi par la porpharine D, la notécarine D, la trocarine D, l'hopsarine D et la noténarine D.

FIGURE 1

FIGURE 2

FIGURE 3

FIGURE 4

EP 2 619 540 B1

FIGURE 5

FIGURE 6

FIGURE 7

FIGURE 8

FIGURE 9

FIGURE 10

FIGURE 11

FIGURE 12

FIGURE 13

Prothrombin Cleavage N terminal Sequence data

FIGURE 14

FIGURE 15

**FIGURE 16**

FIGURE 17

FIGURE 18

FIGURE 19

# FIGURE 20

FIGURE 21

FIGURE 22

FIGURE 23

FIGURE 24

EP 2 619 540 B1

FIGURE 25

127

FIGURE 26

FIGURE 27

FIGURE 28

**Progress curves with the remaining thrombin in the clot free sera from OsPA containing tube**

Legend:
- ◆ 1.2 nM OsPA
- ▲ 1.2 nM OsPA
- ■ 6.1n M OsPA
- ■ 6.1n M OsPA
- ● 12.3 nM OsPA
- ■ 12.3 nM OsPA

X-axis: Time (s)
Y-axis: Absorbance (405 nm)

FIGURE 29

FIGURE 30

FIGURE 31

EP 2 619 540 B1

FIGURE 32

EP 2 619 540 B1

FIGURE 33

EP 2 619 540 B1

FIGURE 34

FIGURE 35

EP 2 619 540 B1

FIGURE 36

EP 2 619 540 B1

FIGURE 37

EP 2 619 540 B1

FIGURE 38

FIGURE 39

FIGURE 40

FIGURE 41

FIGURE 42

FIGURE 43

FIGURE 44

FIGURE 45

FIGURE 46

FIGURE 47

FIGURE 48

FIGURE 49

**Fibrinogen Concentration in Greiner Serum Samples (n=48)**

FIGURE 50

Fibrinogen/fdp/FDP of Normal sera with and without added PtPA (300 ng); (n=36)

FIGURE 51

FIGURE 52

**serum fibrinogen/fdp/FDP concentration (ug/mL)
(Normals, n=5)**

FIGURE 53

FIGURE 54

FIGURE 55

FIGURE 56

FIGURE 57

FIGURE 58

FIGURE 59

FIGURE 60

EP 2 619 540 B1

FIGURE 61

FIGURE 62

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- AU 2010904233
- US 4873192 A, Watson
- US 6413737 B
- WO 03082914 A
- US 4227620 A
- US 4256120 A

- US 6416717 B
- US 6592613 B
- US 6686204 B
- US 7488287 B
- US 7699828 B
- EP 0628816 A

### Non-patent literature cited in the description

- **AUSUBEL et al.** Current Protocols in Molecular Biology. John Wiley & Sons Inc, 1994
- **COLIGAN et al.** Current Protocols in Protein Science. John Wiley & Sons, Inc, 1995
- **ALTSCHUL, S. F. ; MADDEN, T. L. ; SCHÄFFER, A. A. ; ZHANG, J. ; ZHANG, Z. ; MILLER, W. ; LIPMAN, D. J.** Gapped BLAST and PSI-BLAST: a new generation of protein database search programs. *Nucleic Acids Res.,* 01 September 1997, vol. 25 (17), 3389-402
- **ARKIN, A. P. ; YOUVAN, D. C.** An algorithm for protein engineering: simulations of recursive ensemble mutagenesis. *Proc. Natl. Acad. Sci. USA,* 1992, vol. 89, 7811-7815
- **ATHERTON, E. ; SHEPHARD, R. C.** Solid Phase Peptide Synthesis - A Practical Approach. IRL Press, 1989
- **AUSTEN, D. E. ; RHYMES, I. L.** Laboratory manual of blood coagulation. Blackwell Scientific Publications, 1975, 37
- **BOS, M. H. A. ; BOLTZ, M. ; ST PIERRE, L. ; MASCI, P. P. ; DE JERSEY, J. ; LAVIN, M. F. ; CAMIRE, R. M.** Venom factor V from the common brown snake escapes hemostatic regulation through procoagulant adaptations. *Blood,* 16 July 2009, vol. 114 (3), 686-692
- **CAMENZIND, E. ; BAKKER, W. H. ; REIJS, A. ; VAN GEIJLSWIJK, I. M. ; BOERSMA, E. ; KUTRYK, M. J. ; KRENNING, E. P. ; ROELANDT, J. R. ; SERRUYS, P. W.** Site-specific intracoronary heparin delivery in humans after balloon angioplasty. A radioisotopic assessment of regional pharmacokinetics. *Circulation,* 1997, vol. 96, 154-65
- **CIUTI, R. ; RINALDI, G.** Serum and plasma compared for use in 19 common chemical tests performed in the Hitachi 737 analyzer. *Clin Chem.,* 1989, vol. 35, 1562-3

- **COWLEY, D. M. ; NAGLE, B. A. ; CHALMERS, A. H. ; SINTON, T. J.** Effects of platelets on collection of specimens for assay of ammonia in plasma. *Clin Chem,* 1985, vol. 31, 332
- **DAVIDSON, A. S. ; DAM, S. M. ; SODI, R.** Can lithium heparin plasma be used for protein electrophoresis and paraprotein identification?. *Ann Clin Biochem,* 2006, vol. 43, 31-34
- A model of evolutionary change in proteins. Matrices for determining distance relationships. **DAYHOFF, M. O. ; SCHWARTZ, R. M. ; ORCUTT, B. C. et al.** Atlas of protein sequence and structure. National Biomedical Research Foundation, 1978, vol. 5, 345-358
- **DELAGRAVE, S. ; GOLDMAN, E. R. ; YOUVAN, D. C.** Recursive ensemble mutagenesis. *Protein Eng.,* April 1993, vol. 6 (3), 327-31
- **DEVEREUX, J. ; HAEBERLI, P. ; SMITHIES, O.** A comprehensive set of sequence analysis programs for the VAX. *Nucleic Acids Res.,* 11 January 1984, vol. 12 (1), 387-95
- **DIMESKI, G. ; MASCI, P.P. ; TRABI, M. ; LAVIN, M.F. ; DE JERSEY, J.** Evaluation of the Becton-Dickinson rapid serum tube: does it provide a suitable alternative to lithium heparin plasma tubes?. *Clin Chem Lab Med,* 2010, vol. 48 (5), 2111-2120
- **DIMESKI, G. ; HADRICK, T. ; FLATMAN, R. ; ORMISTON, B.** Roche IFCC Methods for Lactate Dehydrogenase Tested for Duplicate Errors with Greiner and Becton-Dickinson Lithium-Heparin and Greiner Serum Samples. *Clin Chem,* 2004, vol. 50 (12), 2391-2392
- **DIMESKI, G. ; CARTER, A.** Rare IgM Interference with Roche/Hitachi Modular Glucose and $\gamma$-Glutamyltransferase Methods in Heparin Samples. *Clin Chem,* 2005, vol. 51 (11), 2202-2204

- **DIMESKI, G. ; CLAGUE, A. E. ; HICKMAN, P. E.** Correcting and reporting of potassium results in haemolysed samples. *Ann. Clin. Biochem.,* 2005, vol. 42, 119-123
- **DIMESKI, G. ; MCWHINNEY, B. ; JONES, B. ; MASON, R. ; CARTER, A.** Extent of bilirubin interference in Beckman-Coulter creatinine methods. *Ann Clin Biochem,* 2008, vol. 45, 91-92
- **FILIPPOVICH, I. ; SOROKINA, N. ; ST PIERRE, L. ; FILGHT, S. ; DE JERSEY, J. ; PERRY, N. ; MASCI, P. P. ; LAVIN, M. F.** Cloning and functional expression of venom prothrombin activator protease from Pseudonaja textilis with whole blood procoagulant activity. *British Journal of Haematology,* 2005, vol. 131, 237-246
- **GONNET, G. H. ; COHEN, M. A. ; BENNER, S. A.** Exhaustive matching of the entire protein sequence database. *Science,* 05 June 1992, vol. 256 (5062), 1443-5
- **HARTLAND, A. J. ; NEARY, R. H.** Serum potassium is unreliable as an estimate of in vivo plasma potassium. *Clin Chem,* 1999, vol. 45, 1091-1092
- **KINI, R. M. ; MORITA, T. ; ROSING, J.** Classification and Nomenclature of Prothrombin Activators Isolated from Snake Venoms. *Thromb. Haemost.,* 2001, vol. 85, 710-711
- **KINI, R. M.** The intriguing world of prothrombin activators from snake venom. *Toxicon,* 2005, vol. 45, 1133-1145
- **KUNKEL, T. A.** Rapid and Efficient site-specific mutagenesis without phenotypic selection. *Proc. Natl. Acad. Sci. USA,* 1985, vol. 82, 488-492
- **KUNKEL, T. A. ; ROBERTS, J. D. ; ZAKOUR, R. A.** Rapid and Efficient site-specific mutagenesis without phenotypic selection. *Methods in Enzymol.,* 1987, vol. 154, 367-382
- **KUZMIC, P.** Program DYNAFIT for the Analysis of Enzyme Kinetic Data: Application to HIV Proteinase. *Anal. Biochem.,* 1996, vol. 237, 260-273
- **LAVIN, M. F. ; MASCI, P. P.** Prothrombinase complexes with different physiological roles. *Thromb Haemost,* 2009, vol. 102, 421-423
- The Effects of Australian Snake Venoms on Coagulation and Fibrinolysis. **MASCI, P. P.** Thesis for Masters of Science in the subject of Biochemistry. University of Queensland, July 1986
- **MASCI, P. P. ; WHITAKER, A. N. ; DE JERSEY, J.** Purification and characterization of a prothrombin activator from the venom of the Australian brown snake, Pseudonaja textilis textilis. *Biochem. Int.,* 1988, vol. 17 (5), 825-835
- **MILES, R. R. ; ROBERTS, R. F. ; PUTNAM, A. R. ; ROBERTS, W. L.** Comparison of serum and heparinized plasma samples for measurement of chemistry analytes. *Clin Chem,* 2004, vol. 50, 1704-5
- National Pathology Laboratory Accreditation Advisory Council - Requirements for the Retention of Laboratory Records and Diagnostic Material. 2009
- **NISHIDA, S. ; FUJITA, T. ; KOHNO, N. ; ATODA, H. ; MORITA, T. ; TAKEYA, H. ; KIDO, I. ; PAINE, M. J. I. ; KAWABATA, S-I. ; IWANAGA, S.** cDNA Cloning and Deduced Amino Acid Sequence of Prothrombin Activator (Ecarin) from Kenyan Echis carinatus venom. *Biochemistry,* 1995, vol. 34, 1771-1778
- **MORITA, T. ; IWANAGA, S.** Prothrombin activator from Echis carinatus venom. *Meth Enzymol,* 1981, vol. 80, 303-311
- **NICHOLSON, J. ; MIRTSCHIN, P. ; MADARAS, F. ; VENNING, M. ; KOKKINN, M.** Digestive properties of the venom of the Australian Coastal Taipan, Oxyuranus scutellatus (Peters, 1867). *Toxicon,* 2006, vol. 48, 422-428
- **O'KEANE M. P. ; CUNNINGHAM, S. K.** Evaluation of three different specimen types (serum, plasma lithium heparin and serum gel separator) for analysis of certain analytes: clinical significance of differences in results and efficiency in use. *Clin Chem Lab Med.,* 2006, vol. 44, 662-8
- **PARKER, H. W. ; GRANDISON, A. G. C.** Snakes: A natural history. University of Queensland Press, 1977, 5, , 59, , 87
- **ROBERGE, J. Y. ; BEEBE, X. ; DANISHEFSKY, S. J.** A strategy for a convergent synthesis of N-linked glycopeptides on a solid support. *Science,* 1995, vol. 269 (5221), 202-204
- **ROSING, J. ; TANS, G.** Inventory of Exogenous Prothrombin Activators. *Thrombosis and Haemostasis,* 1991, vol. 65 (2), 627-630
- **ROSING, J. ; TANS, G.** structural and Functional Properties of Snake Venom Prothrombin Activators. *Toxicon,* 1992, vol. 30 (12), 1515-1527
- **SAMBROOK, J. ; RUSSELL, D. W.** Molecular cloning. A laboratory manual. Cold Spring Harbor Laboratory Press, 2001
- **SCHIECK, A. ; KOMALIK, F. ; HABERMANN, E.** The prothrombin-activating principle from Echis carinatus venom. I. Preparation and biochemical properties. *Naunyn-Schmiedeberg's Arch Pharmacol.,* 1972, vol. 272, 402-416
- **SHEPPARD, C.A ; ALLEN, R.C. ; AUSTIN, G.E. ; YOUNG, A.N. ; RIBEIRO, M.A. ; FANTZ, C.R.** Paraprotein interference in automated chemistry analyzers. *Clin Chem,* June 2005, vol. 51 (6), 1077-8
- **SMITH, G. F. ; CRAFT, T. J.** Heparin reacts stoichiometrically with thrombin during thrombin inhibition in human plasma. *Biochem. Biophys. Res. Commun.,* 1976, vol. 71, 738-45

- **SONDER, S. A. ; FENTON, J. W.** Thrombin specificity with tripeptide chromogenic substrates: comparison of human and bovine thrombins with and without fibrinogen clotting activities. *Clin. Chem.,* 1986, vol. 32, 934-7
- **SPEIJER, H. ; GOVERSRIEMSLAG, J. W. P. ; ZWAAL, R. F. A. ; ROSING, J.** Prothrombin activation by an activator from the venom of Oxyuranus scutellatus (Taipan snake). *J. Biol. Chem.,* 1986, vol. 261 (28), 3258-3267
- **STARR, H. ; RHODES, P. ; LAM-PO-TANG, P. R. ; ARCHER, G. T.** Prothrombin times: an evaluation of four thromboplastins and four machines. *Pathology,* 1980, vol. 12, 567-574
- **ST PIERRE, L. ; MASCI, P. ; FILIPPOVICH, I. ; SOROKINA, N. ; MARSH, N. ; MILLER, D. J. ; LAVIN, M. F.** Comparative Analysis of Prothrombin Activators from the Venom of Australian Elapids. *Mol. Biol. Evol.,* 2005, vol. 22 (9), 1853-1864
- **TANS, G. ; GOVERS-RIEMSLAG, J. W. ; VAN RIJN, J. L. ; ROSING, J. J.** Purification and properties of a prothrombin activator from the venom of Notechis scutatus scutatus. *Biol. Chem.,* 05 August 1985, vol. 260 (16), 9366-72
- **THORELLI, E. ; KAUFMAN, R. J. ; DAHLBÄCK, B.** Cleavage requirements of factor V in tissue-factor induced thrombin generation. *Thromb. Haemost.,* July 1998, vol. 80 (1), 92-98
- **WANNASLIP, N. ; SRIBHEN, K. ; PUSSARA, N. ; HWANPUCH, T. ; WANGCHAIJAROEKIT, S. ; OPARTKIATTIKUL, N.** Heparin is unsuitable anticoagulant for the detection of plasma ammonia. *Clin Chimica Acta,* 2006, vol. 371, 196-7
- **WATSON, J. D. ; HOPKINS, N. H. ; ROBERTS, J. W. ; STEITZ, J. A. ; WEINER, A. M.** Molecular Biology of the Gene. The Benjamin/Cummings Publishing Co., Inc, 1987
- **YAMADA, D. ; SEKIYA, F. ; MORITA, T.** Isolation and Characterization of Carinactivase, a Novel Prothrombin Activator in Echis carinatus Venom with a Unique Catalytic Mechanism. *J. Biol. Chem.,* 1996, vol. 271 (9), 5200-5207
- **YAMADA, D. ; MORITA, T.** Purification and Characterization of a Ca2+-Dependent Prothrombin Activator, Multactivase, from the Venom of Echis multisquamatus. *J. Biochem.,* 1997, vol. 122, 991-997
- **YAMANOUYE, N. ; KERCHOVE, C. M. ; MOURA-DA-SILVA, A. M. ; CARNEIRO, S. M. ; MARKUS, R. P.** Long-term primary culture of secretory cells of Bothrops jararaca gland for venom production in vitro. *Nature Protocols,* 2007, vol. 1, 2763-2766
- **YONEMURA, H. ; IMAMURA, T. ; SOEJIMA, K. ; NAKAHARA, Y. ; MORIKAWA, W. ; USHIO, Y. ; KAMACHI, Y. ; NAKATAKE, H. ; SUGAWARA, K. ; NAKAGAKI, T.** Preparation of Recombinant α-Thrombin: High-Level Expression of Recombinant Human Prethrombin-2 and Its Activation by Recombinant Ecarin. *J. Biochem.,* 2004, vol. 135, 577-582
- **ZUBAY, G.** Biochemistry. Wm.C. Brown Publishers, 1993